# EUROPEAN PATENT APPLICATION

(11) **EP 3 978 927 A2**
(43) Date of publication of application: **06.04.2022**
(21) Application number: 22152107.3
(22) Date of filing: 19.02.2021
(51) Int. Cl.: G01N 33/569, C07K 14/005, C07K 14/165

(54) **METHODS AND REAGENTS FOR DIAGNOSIS OF SARS-COV-2 INFECTION**

(30) Priority: 19.02.2020 EP 20158348; 20.02.2020 EP 20158626; 21.02.2020 EP 20158821; 20.08.2020 DE 202020003564 U; 28.08.2020 DE 202020104982 U
(62) Divisional of application: 21158065.9
(71) Applicant: EUROIMMUN Medizinische Labordiagnostika AG, 23560 Lübeck (DE); Charité - Universitätsmedizin Berlin, 10117 Berlin (DE)
(72) Inventor: Steinhagen, Katja, 23560 Lübeck (DE); Messing, Claudia, 23560 Lübeck (DE); Lattwein, Erik, 23560 Lübeck (DE); Stiba, Konstanze, 23560 Lübeck (DE); Lindhorst, Fabian, 23560 Lübeck (DE); Neugebauer, Eva, 23560 Lübeck (DE); Müller, Marcel, 10117 Berlin (DE); Corman, Victor, 10117 Berlin (DE)

(57) **Abstract**

The present invention relates to a method for diagnosing a SARS-CoV-2 infection comprising the step of detecting the presence or absence of an antibody to SEQ ID NO: 1, preferably IgA class antibody, in a sample from a subject, a method for the differential diagnosis of a coronavirus infection, a use of an antibody to SEQ ID NO: 1, preferably IgA class antibody for diagnosing a SARS-CoV-2 infection or for the differential diagnosis of a coronavirus infection, preferably for distinguishing between a SARS-CoV-2, MERS and NL63, 229E, OC43 and HKU1 infection, and a kit comprising a polypeptide comprising SEQ ID NO: 1 or a variant thereof, preferably coated to a diagnostically useful carrier and one or more, preferably all reagents from the group comprising an antibody to SEQ ID NO: 1, a washing buffer, a means for detecting the presence of an antibody, preferably IgA class antibody, preferably a secondary antibody binding specifically to IgA class antibodies, preferably comprising a detectable label, and a dilution buffer.

SEQUENCE LISTING

<110>  EUROIMMUN Medizinische Labordiagnostika AG

<120>  Methods and reagents for diagnosis of SARS-CoV-2 infection

<130>  20PP007EP7

<160>  56

<170>  PatentIn version 3.5

<210>  1
<211>  670
<212>  PRT
<213>  Artificial Sequence

<220>
<223>  SARS-CoV-2 S1 domain from S Protein

<400>  1

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu

                165                 170                 175

Phe Val Phe Lys Asn Ile Asp Gly Tyr Phe Lys Ile Tyr Ser Lys His
            180                 185                 190

Thr Pro Ile Asn Leu Val Arg Asp Leu Pro Gln Gly Phe Ser Ala Leu
        195                 200                 205

Glu Pro Leu Val Asp Leu Pro Ile Gly Ile Asn Ile Thr Arg Phe Gln
    210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Lys Ile Ala Asp Tyr Asn Tyr Lys Leu Pro Asp Asp Phe Thr Gly
                405                 410                 415

Cys Val Ile Ala Trp Asn Ser Asn Asn Leu Asp Ser Lys Val Gly Gly
        420                 425                 430

Asn Tyr Asn Tyr Leu Tyr Arg Leu Phe Arg Lys Ser Asn Leu Lys Pro
    435                 440                 445

Phe Glu Arg Asp Ile Ser Thr Glu Ile Tyr Gln Ala Gly Ser Thr Pro
    450                 455                 460

Cys Asn Gly Val Glu Gly Phe Asn Cys Tyr Phe Pro Leu Gln Ser Tyr
465                 470                 475                 480

Gly Phe Gln Pro Thr Asn Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Asp Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro Ile Gly Ala Gly Ile Cys
                645                 650                 655

Ala Ser Tyr Gln Thr Gln Thr Asn Ser Pro Arg Arg Ala Arg
            660                 665                 670

<210>  2
<211>  695
<212>  PRT
<213>  Artificial Sequence

<220>
<223>  SARS-CoV-2 S1 domain from S Protein, C-terminally his-tagged

<400>  2

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Leu Glu His
        675                 680                 685

His His His His His His His
    690                 695

<210>  3
<211>  680

<212>  PRT
<213>  Artificial Sequence

<220>
<223>  SARS-CoV-2 S1 domain from S Protein, C-terminally his-tagged, as
       after cleavage of signal peptide

<400>  3

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu
                165                 170                 175

Phe Val Phe Lys Asn Ile Asp Gly Tyr Phe Lys Ile Tyr Ser Lys His
            180                 185                 190

Thr Pro Ile Asn Leu Val Arg Asp Leu Pro Gln Gly Phe Ser Ala Leu
        195                 200                 205

Glu Pro Leu Val Asp Leu Pro Ile Gly Ile Asn Ile Thr Arg Phe Gln
    210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Lys Ile Ala Asp Tyr Asn Tyr Lys Leu Pro Asp Asp Phe Thr Gly
                405                 410                 415

Cys Val Ile Ala Trp Asn Ser Asn Asn Leu Asp Ser Lys Val Gly Gly
            420                 425                 430

Asn Tyr Asn Tyr Leu Tyr Arg Leu Phe Arg Lys Ser Asn Leu Lys Pro
        435                 440                 445

Phe Glu Arg Asp Ile Ser Thr Glu Ile Tyr Gln Ala Gly Ser Thr Pro
    450                 455                 460

Cys Asn Gly Val Glu Gly Phe Asn Cys Tyr Phe Pro Leu Gln Ser Tyr

465                 470                 475                 480

Gly Phe Gln Pro Thr Asn Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Asp Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro Ile Gly Ala Gly Ile Cys
                645                 650                 655

Ala Ser Tyr Gln Thr Gln Thr Asn Ser Pro Arg Arg Ala Arg Leu Glu
            660                 665                 670

His His His His His His His His
        675                 680

<210>  4
<211>  2088
<212>  DNA
<213>  Artificial Sequence

<220>
<223>  SARS-CoV-2 S1 domain from S Protein, nucleotide sequence encoding
       SEQ ID NO: 2

<400> 4
atgttcgtat tccttgttct gctgcctttg gttagcagtc agtgtgtcaa cctgacaact 60
cgcacgcaac tgccgccagc ttacaccaac tctttcacaa gaggcgtcta ctacccggac 120
aaagtgtttc gctcatcagt gctgcactct acacaagatt tgtttctgcc attcttctct 180
aacgtaacct ggtttcacgc gattcatgtg tctgggacaa atgggaccaa gcgcttcgac 240
aaccccgtgc tgccattcaa tgacggggtg tattttgcct ccaccgagaa atccaatatc 300
atccgaggat ggattttcgg tactacgctg gactctaaaa cgcagtctct cttgatcgtt 360
aataacgcca caaatgttgt cattaaggtg tgcgagtttc agttctgtaa tgatcccttt 420
ctgggtgtgt attaccacaa gaataacaag tcatggatgg aaagcgagtt tcgcgtgtac 480
tcaagtgcca ataactgcac attcgagtat gtgtcccagc ctttcctgat ggatctcgaa 540
ggcaaacagg ggaacttcaa gaatctgcgc gagttcgtgt ttaagaacat cgacggttat 600
ttcaagatct acagcaaaca tacacccatt aacctggtca gggatctccc tcagggattc 660
tccgccctgg aacccttggt ggacttgccc attgggatta acatcactag attccagacc 720
ctgctggccc ttcaccgttc ctatcttact cctggcgaca gtagcagtgg atggaccgca 780
ggagcagccg cttactatgt aggctatctg cagccacgga ccttcctcct caagtacaat 840
gaaaatggta ccataactga tgctgtggac tgcgctctgg atccactctc cgaaactaaa 900
tgcaccctta aaagcttcac ggtcgaaaag ggaatctacc agacaagtaa ctttcgggta 960
caacccactg agtccatcgt gcggtttcct aacatcacaa atctctgccc ctttggtgaa 1020
gtgtttaacg ccactaggtt cgcttctgtt tatgcgtgga atcggaagag gatttccaat 1080
tgcgtggcag actactctgt cctgtataat agcgctagct tcagcacctt caaatgttac 1140
ggggtaagcc caactaaact gaacgacctc tgttttacca acgtgtatgc cgatagcttt 1200
gtcatacgag gagatgaggt tcgtcagatt gctcctggcc aaacggggaa aatcgcagac 1260
tacaactaca agcttcccga cgacttcaca ggatgcgtga tcgcgtggaa ctcaaataat 1320
ctggatagca aggttggtgg caattataac tacctgtatc gactgttcag gaaaagcaac 1380
ctcaaaccct ttgagcgcga catcagcacc gagatatacc aagccggttc aacaccttgc 1440
aatggggtgg aagggtttaa ctgctatttc ccacttcaga gctatgggtt tcagccaacc 1500
aatggagtcg gctaccagcc ctatcgggtg gtagtcctgt cctttgagct gttgcatgcg 1560
cctgccacag tctgtggccc taagaagagt acgaatctgg tgaagaacaa gtgcgtcaac 1620
ttcaatttta acggcttgac tggaacagga gttctgaccg agtccaacaa gaaattcctt 1680
ccttttcagc agtttggaag ggatatagcc gacactaccg atgccgttcg ggatccacag 1740
acactggaga ttctggacat tactccgtgc tcatttggcg gtgtatctgt catcacacct 1800
gggaccaata cctcaaatca ggtggctgtg ctctaccagg atgtgaattg taccgaagtt 1860

ccagtggcaa ttcatgccga tcaactgact cccacctgga gagtgtacag tactggcagt 1920

aacgtgtttc agacaagagc tggctgtctc ataggcgcag aacacgtcaa caacagctat 1980

gagtgtgaca ttccgatcgg cgcaggcatc tgtgcatcct accagacgca aaccaactct 2040

cccagaagag ccaggctcga gcaccaccat caccatcacc atcactaa 2088

<210> 5
<211> 12
<212> PRT
<213> Artificial Sequence

<220>
<223> possible SARS-CoV-2 S1 epitope

<400> 5

Asn Leu Lys Pro Phe Glu Arg Asp Ile Ser Thr Glu
1               5                   10

<210> 6
<211> 734
<212> PRT
<213> Artificial Sequence

<220>
<223> S1 [MERS_CoV]

<400> 6

Tyr Val Asp Val Gly Pro Asp Ser Val Lys Ser Ala Cys Ile Glu Val
1               5                   10                  15

Asp Ile Gln Gln Thr Phe Phe Asp Lys Thr Trp Pro Arg Pro Ile Asp
            20                  25                  30

Val Ser Lys Ala Asp Gly Ile Ile Tyr Pro Gln Gly Arg Thr Tyr Ser
        35                  40                  45

Asn Ile Thr Ile Thr Tyr Gln Gly Leu Phe Pro Tyr Gln Gly Asp His
    50                  55                  60

Gly Asp Met Tyr Val Tyr Ser Ala Gly His Ala Thr Gly Thr Thr Pro
65                  70                  75                  80

Gln Lys Leu Phe Val Ala Asn Tyr Ser Gln Asp Val Lys Gln Phe Ala
                85                  90                  95

Asn Gly Phe Val Val Arg Ile Gly Ala Ala Ala Asn Ser Thr Gly Thr
            100                 105                 110

Val Ile Ile Ser Pro Ser Thr Ser Ala Thr Ile Arg Lys Ile Tyr Pro

115                 120                 125

Ala Phe Met Leu Gly Ser Ser Val Gly Asn Phe Ser Asp Gly Lys Met
    130                 135                 140

Gly Arg Phe Phe Asn His Thr Leu Val Leu Leu Pro Asp Gly Cys Gly
145                 150                 155                 160

Thr Leu Leu Arg Ala Phe Tyr Cys Ile Leu Glu Pro Arg Ser Gly Asn
                165                 170                 175

His Cys Pro Ala Gly Asn Ser Tyr Thr Ser Phe Ala Thr Tyr His Thr
            180                 185                 190

Pro Ala Thr Asp Cys Ser Asp Gly Asn Tyr Asn Arg Asn Ala Ser Leu
        195                 200                 205

Asn Ser Phe Lys Glu Tyr Phe Asn Leu Arg Asn Cys Thr Phe Met Tyr
    210                 215                 220

Thr Tyr Asn Ile Thr Glu Asp Glu Ile Leu Glu Trp Phe Gly Ile Thr
225                 230                 235                 240

Gln Thr Ala Gln Gly Val His Leu Phe Ser Ser Arg Tyr Val Asp Leu
                245                 250                 255

Tyr Gly Gly Asn Met Phe Gln Phe Ala Thr Leu Pro Val Tyr Asp Thr
            260                 265                 270

Ile Lys Tyr Tyr Ser Ile Ile Pro His Ser Ile Arg Ser Ile Gln Ser
        275                 280                 285

Asp Arg Lys Ala Trp Ala Ala Phe Tyr Val Tyr Lys Leu Gln Pro Leu
    290                 295                 300

Thr Phe Leu Leu Asp Phe Ser Val Asp Gly Tyr Ile Arg Arg Ala Ile
305                 310                 315                 320

Asp Cys Gly Phe Asn Asp Leu Ser Gln Leu His Cys Ser Tyr Glu Ser
                325                 330                 335

Phe Asp Val Glu Ser Gly Val Tyr Ser Val Ser Ser Phe Glu Ala Lys
            340                 345                 350

Pro Ser Gly Ser Val Val Glu Gln Ala Glu Gly Val Glu Cys Asp Phe
        355                 360                 365

Ser Pro Leu Leu Ser Gly Thr Pro Pro Gln Val Tyr Asn Phe Lys Arg
    370                 375                 380

Leu Val Phe Thr Asn Cys Asn Tyr Asn Leu Thr Lys Leu Leu Ser Leu
385                 390                 395                 400

Phe Ser Val Asn Asp Phe Thr Cys Ser Gln Ile Ser Pro Ala Ala Ile
                405                 410                 415

Ala Ser Asn Cys Tyr Ser Ser Leu Ile Leu Asp Tyr Phe Ser Tyr Pro
            420                 425                 430

Leu Ser Met Lys Ser Asp Leu Ser Val Ser Ser Ala Gly Pro Ile Ser
        435                 440                 445

Gln Phe Asn Tyr Lys Gln Ser Phe Ser Asn Pro Thr Cys Leu Ile Leu
    450                 455                 460

Ala Thr Val Pro His Asn Leu Thr Thr Ile Thr Lys Pro Leu Lys Tyr
465                 470                 475                 480

Ser Tyr Ile Asn Lys Cys Ser Arg Leu Leu Ser Asp Asp Arg Thr Glu
                485                 490                 495

Val Pro Gln Leu Val Asn Ala Asn Gln Tyr Ser Pro Cys Val Ser Ile
            500                 505                 510

Val Pro Ser Thr Val Trp Glu Asp Gly Asp Tyr Tyr Arg Lys Gln Leu
        515                 520                 525

Ser Pro Leu Glu Gly Gly Gly Trp Leu Val Ala Ser Gly Ser Thr Val
    530                 535                 540

Ala Met Thr Glu Gln Leu Gln Met Gly Phe Gly Ile Thr Val Gln Tyr
545                 550                 555                 560

Gly Thr Asp Thr Asn Ser Val Cys Pro Lys Leu Glu Phe Ala Asn Asp
                565                 570                 575

Thr Lys Ile Ala Ser Gln Leu Gly Asn Cys Val Glu Tyr Ser Leu Tyr
            580                 585                 590

Gly Val Ser Gly Arg Gly Val Phe Gln Asn Cys Thr Ala Val Gly Val
        595                 600                 605

Arg Gln Gln Arg Phe Val Tyr Asp Ala Tyr Gln Asn Leu Val Gly Tyr
    610                 615                 620

Tyr Ser Asp Asp Gly Asn Tyr Tyr Cys Leu Arg Ala Cys Val Ser Val
625                 630                 635                 640

Pro Val Ser Val Ile Tyr Asp Lys Glu Thr Lys Thr His Ala Thr Leu
                645                 650                 655

Phe Gly Ser Val Ala Cys Glu His Ile Ser Ser Thr Met Ser Gln Tyr
            660                 665                 670

Ser Arg Ser Thr Arg Ser Met Leu Lys Arg Arg Asp Ser Thr Tyr Gly
        675                 680                 685

Pro Leu Gln Thr Pro Val Gly Cys Val Leu Gly Leu Val Asn Ser Ser
    690                 695                 700

Leu Phe Val Glu Asp Cys Lys Leu Pro Leu Gly Gln Ser Leu Cys Ala
705                 710                 715                 720

Leu Pro Asp Thr Pro Ser Thr Leu Thr Pro Arg Ser Val Arg
                725                 730

<210>  7
<211>  521
<212>  PRT
<213>  Artificial Sequence

<220>
<223>  S1 [HCoV-229E]

<400>  7

Cys Gln Thr Thr Asn Gly Leu Asn Thr Ser Tyr Ser Val Cys Asn Gly
1               5                   10                  15

Cys Val Gly Tyr Ser Glu Asn Val Phe Ala Val Glu Ser Gly Gly Tyr
            20                  25                  30

Ile Pro Ser Asp Phe Ala Phe Asn Asn Trp Phe Leu Leu Thr Asn Thr
        35                  40                  45

Ser Ser Val Val Asp Gly Val Val Arg Ser Phe Gln Pro Leu Leu Leu
    50                  55                  60

Asn Cys Leu Trp Ser Val Ser Gly Leu Arg Phe Thr Thr Gly Phe Val
65                  70                  75                  80

Tyr Phe Asn Gly Thr Gly Arg Gly Asp Cys Lys Gly Phe Ser Ser Asp
                85                  90                  95

Val Leu Ser Asp Val Ile Arg Tyr Asn Leu Asn Phe Glu Glu Asn Leu
            100                 105                 110

Arg Arg Gly Thr Ile Leu Phe Lys Thr Ser Tyr Gly Val Val Val Phe
        115                 120                 125

Tyr Cys Thr Asn Asn Thr Leu Val Ser Gly Asp Ala His Ile Pro Phe
    130                 135                 140

Gly Thr Val Leu Gly Asn Phe Tyr Cys Phe Val Asn Thr Thr Ile Gly
145                 150                 155                 160

Asn Glu Thr Thr Ser Ala Phe Val Gly Ala Leu Pro Lys Thr Val Arg
                165                 170                 175

Glu Phe Val Ile Ser Arg Thr Gly His Phe Tyr Ile Asn Gly Tyr Arg
            180                 185                 190

Tyr Phe Thr Leu Gly Asn Val Glu Ala Val Asn Phe Asn Val Thr Thr
        195                 200                 205

Ala Glu Thr Thr Asp Phe Cys Thr Val Ala Leu Ala Ser Tyr Ala Asp
    210                 215                 220

Val Leu Val Asn Val Ser Gln Thr Ser Ile Ala Asn Ile Ile Tyr Cys
225                 230                 235                 240

Asn Ser Val Ile Asn Arg Leu Arg Cys Asp Gln Leu Ser Phe Asp Val
                245                 250                 255

Pro Asp Gly Phe Tyr Ser Thr Ser Pro Ile Gln Ser Val Glu Leu Pro
            260                 265                 270

Val Ser Ile Val Ser Leu Pro Val Tyr His Lys His Thr Phe Ile Val
        275                 280                 285

Leu Tyr Val Asp Phe Lys Pro Gln Ser Gly Gly Gly Lys Cys Phe Asn
    290                 295                 300

Cys Tyr Pro Ala Gly Val Asn Ile Thr Leu Ala Asn Phe Asn Glu Thr
305                 310                 315                 320

Lys Gly Pro Leu Cys Val Asp Thr Ser His Phe Thr Thr Lys Tyr Val
                325                 330                 335

Ala Val Tyr Ala Asn Val Gly Arg Trp Ser Ala Ser Ile Asn Thr Gly
            340                 345                 350

Asn Cys Pro Phe Ser Phe Gly Lys Val Asn Asn Phe Val Lys Phe Gly
        355                 360                 365

Ser Val Cys Phe Ser Leu Lys Asp Ile Pro Gly Gly Cys Ala Met Pro
    370                 375                 380

Ile Val Ala Asn Trp Ala Tyr Ser Lys Tyr Tyr Thr Ile Gly Ser Leu
385                 390                 395                 400

Tyr Val Ser Trp Ser Asp Gly Asp Gly Ile Thr Gly Val Pro Gln Pro
                405                 410                 415

Val Glu Gly Val Ser Ser Phe Met Asn Val Thr Leu Asp Lys Cys Thr
            420                 425                 430

Lys Tyr Asn Ile Tyr Asp Val Ser Gly Val Gly Val Ile Arg Val Ser
        435                 440                 445

Asn Asp Thr Phe Leu Asn Gly Ile Thr Tyr Thr Ser Thr Ser Gly Asn
    450                 455                 460

Leu Leu Gly Phe Lys Asp Val Thr Lys Gly Thr Ile Tyr Ser Ile Thr
465                 470                 475                 480

Pro Cys Asn Pro Pro Asp Gln Leu Val Val Tyr Gln Gln Ala Val Val
                485                 490                 495

Gly Ala Met Leu Ser Glu Asn Phe Thr Ser Tyr Gly Phe Ser Asn Val
            500                 505                 510

Val Glu Leu Pro Lys Phe Phe Tyr Ala
        515                 520

<210>  8
<211>  745
<212>  PRT
<213>  Artificial Sequence

<220>
<223>  S1 [HCoV-OC43]

<400>  8

Ala Val Ile Gly Asp Leu Lys Cys Thr Ser Asp Asn Ile Asn Asp Lys
1               5                   10                  15

Asp Thr Gly Pro Pro Pro Ile Ser Thr Asp Thr Val Asp Val Thr Asn
            20                  25                  30

Gly Leu Gly Thr Tyr Tyr Val Leu Asp Arg Val Tyr Leu Asn Thr Thr
        35                  40                  45

Leu Phe Leu Asn Gly Tyr Tyr Pro Thr Ser Gly Ser Thr Tyr Arg Asn
    50                  55                  60

Met Ala Leu Lys Gly Ser Val Leu Leu Ser Arg Leu Trp Phe Lys Pro
65                  70                  75                  80

Pro Phe Leu Ser Asp Phe Ile Asn Gly Ile Phe Ala Lys Val Lys Asn
                85                  90                  95

Thr Lys Val Ile Lys Asp Arg Val Met Tyr Ser Glu Phe Pro Ala Ile
            100                 105                 110

Thr Ile Gly Ser Thr Phe Val Asn Thr Ser Tyr Ser Val Val Val Gln
        115                 120                 125

Pro Arg Thr Ile Asn Ser Thr Gln Asp Gly Asp Asn Lys Leu Gln Gly
    130                 135                 140

Leu Leu Glu Val Ser Val Cys Gln Tyr Asn Met Cys Glu Tyr Pro Gln
145                 150                 155                 160

Thr Ile Cys His Pro Asn Leu Gly Asn His Arg Lys Glu Leu Trp His
                165                 170                 175

Leu Asp Thr Gly Val Val Ser Cys Leu Tyr Lys Arg Asn Phe Thr Tyr
            180                 185                 190

Asp Val Asn Ala Asp Tyr Leu Tyr Phe His Phe Tyr Gln Glu Gly Gly
        195                 200                 205

Thr Phe Tyr Ala Tyr Phe Thr Asp Thr Gly Val Val Thr Lys Phe Leu
    210                 215                 220

Phe Asn Val Tyr Leu Gly Met Ala Leu Ser His Tyr Tyr Val Met Pro
225                 230                 235                 240

Leu Thr Cys Asn Ser Lys Leu Thr Leu Glu Tyr Trp Val Thr Pro Leu
                245                 250                 255

Thr Ser Arg Gln Tyr Leu Leu Ala Phe Asn Gln Asp Gly Ile Ile Phe
            260                 265                 270

Asn Ala Glu Asp Cys Met Ser Asp Phe Met Ser Glu Ile Lys Cys Lys
        275                 280                 285

Thr Gln Ser Ile Ala Pro Pro Thr Gly Val Tyr Glu Leu Asn Gly Tyr
    290                 295                 300

Thr Val Gln Pro Ile Ala Asp Val Tyr Arg Arg Lys Pro Asn Leu Pro
305                 310                 315                 320

Asn Cys Asn Ile Glu Ala Trp Leu Asn Asp Lys Ser Val Pro Ser Pro
                325                 330                 335

Leu Asn Trp Glu Arg Lys Thr Phe Ser Asn Cys Asn Phe Asn Met Ser
            340                 345                 350

Ser Leu Met Ser Phe Ile Gln Ala Asp Ser Phe Thr Cys Asn Asn Ile
        355                 360                 365

Asp Ala Ala Lys Ile Tyr Gly Met Cys Phe Ser Ser Ile Thr Ile Asp
    370                 375                 380

Lys Phe Ala Ile Pro Asn Gly Arg Lys Val Asp Leu Gln Leu Gly Asn
385                 390                 395                 400

Leu Gly Tyr Leu Gln Ser Phe Asn Tyr Arg Ile Asp Thr Thr Ala Thr
                405                 410                 415

Ser Cys Gln Leu Tyr Tyr Asn Leu Pro Ala Ala Asn Val Ser Val Ser
            420                 425                 430

Arg Phe Asn Pro Ser Thr Trp Asn Lys Arg Phe Gly Phe Ile Glu Asp
        435                 440                 445

Ser Val Phe Lys Pro Arg Pro Ala Gly Val Leu Thr Asn His Asp Val
    450                 455                 460

Val Tyr Ala Gln His Cys Phe Lys Ala Pro Lys Asn Phe Cys Pro Cys
465                 470                 475                 480

Lys Leu Asn Gly Ser Cys Val Gly Ser Gly Pro Gly Lys Asn Asn Gly
                485                 490                 495

Ile Gly Thr Cys Pro Ala Gly Thr Asn Tyr Leu Thr Cys Asp Asn Leu
            500                 505                 510

Cys Thr Pro Asp Pro Ile Thr Phe Thr Gly Thr Tyr Lys Cys Pro Gln
        515                 520                 525

Thr Lys Ser Leu Val Gly Ile Gly Glu His Cys Ser Gly Leu Ala Val
    530                 535                 540

Lys Ser Asp Tyr Cys Gly Gly Asn Ser Cys Thr Cys Arg Pro Gln Ala
545                 550                 555                 560

Phe Leu Gly Trp Ser Ala Asp Ser Cys Leu Gln Gly Asp Lys Cys Asn
                565                 570                 575

Ile Phe Ala Asn Phe Ile Leu His Asp Val Asn Ser Gly Leu Thr Cys
            580                 585                 590

Ser Thr Asp Leu Gln Lys Ala Asn Thr Asp Ile Ile Leu Gly Val Cys
        595                 600                 605

Val Asn Tyr Asp Leu Tyr Gly Ile Leu Gly Gln Gly Ile Phe Val Glu
    610                 615                 620

Val Asn Ala Thr Tyr Tyr Asn Ser Trp Gln Asn Leu Leu Tyr Asp Ser
625                 630                 635                 640

Asn Gly Asn Leu Tyr Gly Phe Arg Asp Tyr Ile Ile Asn Arg Thr Phe
                645                 650                 655

Met Ile Arg Ser Cys Tyr Ser Gly Arg Val Ser Ala Ala Phe His Ala
            660                 665                 670

Asn Ser Ser Glu Pro Ala Leu Leu Phe Arg Asn Ile Lys Cys Asn Tyr
        675                 680                 685

Val Phe Asn Asn Ser Leu Thr Arg Gln Leu Gln Pro Ile Asn Tyr Phe
    690                 695                 700

Asp Ser Tyr Leu Gly Cys Val Val Asn Ala Tyr Asn Ser Thr Ala Ile
705                 710                 715                 720

Ser Val Gln Thr Cys Asp Leu Thr Val Gly Ser Gly Tyr Cys Val Asp
                725                 730                 735

Tyr Ser Lys Asn Arg Arg Ser Arg Gly
            740                 745

<210>  9
<211>  744
<212>  PRT
<213>  Artificial Sequence

<220>
<223>  S1 [HCoV-HKU1]

<400>  9

Ala Val Ile Gly Asp Phe Asn Cys Thr Asn Ser Phe Ile Asn Asp Tyr
1               5                   10                  15

Asn Lys Thr Ile Pro Arg Ile Ser Glu Asp Val Val Asp Val Ser Leu
            20                  25                  30

Gly Leu Gly Thr Tyr Tyr Val Leu Asn Arg Val Tyr Leu Asn Thr Thr
        35                  40                  45

Leu Leu Phe Thr Gly Tyr Phe Pro Lys Ser Gly Ala Asn Phe Arg Asp
    50                  55                  60

Leu Ala Leu Lys Gly Ser Ile Tyr Leu Ser Thr Leu Trp Tyr Lys Pro
65                  70                  75                  80

Pro Phe Leu Ser Asp Phe Asn Asn Gly Ile Phe Ser Lys Val Lys Asn
                85                  90                  95

Thr Lys Leu Tyr Val Asn Asn Thr Leu Tyr Ser Glu Phe Ser Thr Ile
            100                 105                 110

Val Ile Gly Ser Val Phe Val Asn Thr Ser Tyr Thr Ile Val Val Gln
        115                 120                 125

Pro His Asn Gly Ile Leu Glu Ile Thr Ala Cys Gln Tyr Thr Met Cys
    130                 135                 140

Glu Tyr Pro His Thr Val Cys Lys Ser Lys Gly Ser Ile Arg Asn Glu
145                 150                 155                 160

Ser Trp His Ile Asp Ser Ser Glu Pro Leu Cys Leu Phe Lys Lys Asn
                165                 170                 175

Phe Thr Tyr Asn Val Ser Ala Asp Trp Leu Tyr Phe His Phe Tyr Gln
            180                 185                 190

Glu Arg Gly Val Phe Tyr Ala Tyr Tyr Ala Asp Val Gly Met Pro Thr
        195                 200                 205

Thr Phe Leu Phe Ser Leu Tyr Leu Gly Thr Ile Leu Ser His Tyr Tyr
    210                 215                 220

Val Met Pro Leu Thr Cys Asn Ala Ile Ser Ser Asn Thr Asp Asn Glu
225                 230                 235                 240

Thr Leu Glu Tyr Trp Val Thr Pro Leu Ser Arg Arg Gln Tyr Leu Leu
                245                 250                 255

Asn Phe Asp Glu His Gly Val Ile Thr Asn Ala Val Asp Cys Ser Ser
            260                 265                 270

Ser Phe Leu Ser Glu Ile Gln Cys Lys Thr Gln Ser Phe Ala Pro Asn
        275                 280                 285

Thr Gly Val Tyr Asp Leu Ser Gly Phe Thr Val Lys Pro Val Ala Thr
    290                 295                 300

Val Tyr Arg Arg Ile Pro Asn Leu Pro Asp Cys Asp Ile Asp Asn Trp
305                 310                 315                 320

Leu Asn Asn Val Ser Val Pro Ser Pro Leu Asn Trp Glu Arg Arg Ile
                325                 330                 335

Phe Ser Asn Cys Asn Phe Asn Leu Ser Thr Leu Leu Arg Leu Val His
            340                 345                 350

Val Asp Ser Phe Ser Cys Asn Asn Leu Asp Lys Ser Lys Ile Phe Gly
        355                 360                 365

Ser Cys Phe Asn Ser Ile Thr Val Asp Lys Phe Ala Ile Pro Asn Arg
    370                 375                 380

Arg Arg Asp Asp Leu Gln Leu Gly Ser Ser Gly Phe Leu Gln Ser Ser
385                 390                 395                 400

Asn Tyr Lys Ile Asp Ile Ser Ser Ser Ser Cys Gln Leu Tyr Tyr Ser
                405                 410                 415

Leu Pro Leu Val Asn Val Thr Ile Asn Asn Phe Asn Pro Ser Ser Trp
            420                 425                 430

Asn Arg Arg Tyr Gly Phe Gly Ser Phe Asn Leu Ser Ser Tyr Asp Val
        435                 440                 445

Val Tyr Ser Asp His Cys Phe Ser Val Asn Ser Asp Phe Cys Pro Cys
    450                 455                 460

Ala Asp Pro Ser Val Val Asn Ser Cys Ala Lys Ser Lys Pro Pro Ser
465                 470                 475                 480

Ala Ile Cys Pro Ala Gly Thr Lys Tyr Arg His Cys Asp Leu Asp Thr
                485                 490                 495

Thr Leu Tyr Val Lys Asn Trp Cys Arg Cys Ser Cys Leu Pro Asp Pro

500                 505                 510

Ile Ser Thr Tyr Ser Pro Asn Thr Cys Pro Gln Lys Lys Val Val Val
        515                 520                 525

Gly Ile Gly Glu His Cys Pro Gly Leu Gly Ile Asn Glu Glu Lys Cys
    530                 535                 540

Gly Thr Gln Leu Asn His Ser Ser Cys Phe Cys Ser Pro Asp Ala Phe
545                 550                 555                 560

Leu Gly Trp Ser Phe Asp Ser Cys Ile Ser Asn Asn Arg Cys Asn Ile
                565                 570                 575

Phe Ser Asn Phe Ile Phe Asn Gly Ile Asn Ser Gly Thr Thr Cys Ser
            580                 585                 590

Asn Asp Leu Leu Tyr Ser Asn Thr Glu Ile Ser Thr Gly Val Cys Val
        595                 600                 605

Asn Tyr Asp Leu Tyr Gly Ile Thr Gly Gln Gly Ile Phe Lys Glu Val
    610                 615                 620

Ser Ala Ala Tyr Tyr Asn Asn Trp Gln Asn Leu Leu Tyr Asp Ser Asn
625                 630                 635                 640

Gly Asn Ile Ile Gly Phe Lys Asp Phe Leu Thr Asn Lys Thr Tyr Thr
                645                 650                 655

Ile Leu Pro Cys Tyr Ser Gly Arg Val Ser Ala Ala Phe Tyr Gln Asn
            660                 665                 670

Ser Ser Ser Pro Ala Leu Leu Tyr Arg Asn Leu Lys Cys Ser Tyr Val
        675                 680                 685

Leu Asn Asn Ile Ser Phe Ile Ser Gln Pro Phe Tyr Phe Asp Ser Tyr
    690                 695                 700

Leu Gly Cys Val Leu Asn Ala Val Asn Leu Thr Ser Tyr Ser Val Ser
705                 710                 715                 720

Ser Cys Asp Leu Arg Met Gly Ser Gly Phe Cys Ile Asp Tyr Ala Leu
                725                 730                 735

Pro Ser Ser Arg Arg Lys Arg Arg
            740

<210>  10
<211>  702
<212>  PRT
<213>  Artificial Sequence

<220>
<223>  S1 [HCoV-NL63]

<400>  10

Phe Phe Thr Cys Asn Ser Asn Ala Asn Leu Ser Met Leu Gln Leu Gly
1               5                   10                  15

Val Pro Asp Asn Ser Ser Thr Ile Val Thr Gly Leu Leu Pro Thr His
            20                  25                  30

Trp Phe Cys Ala Asn Gln Ser Thr Ser Val Tyr Ser Ala Asn Gly Phe
        35                  40                  45

Phe Tyr Ile Asp Val Gly Asn His Arg Ser Ala Phe Ala Leu His Thr
    50                  55                  60

Gly Tyr Tyr Asp Ala Asn Gln Tyr Tyr Ile Tyr Val Thr Asn Glu Ile
65                  70                  75                  80

Gly Leu Asn Ala Ser Val Thr Leu Lys Ile Cys Lys Phe Ser Arg Asn
                85                  90                  95

Thr Thr Phe Asp Phe Leu Ser Asn Ala Ser Ser Ser Phe Asp Cys Ile
            100                 105                 110

Val Asn Leu Leu Phe Thr Glu Gln Leu Gly Ala Pro Leu Gly Ile Thr
        115                 120                 125

Ile Ser Gly Glu Thr Val Arg Leu His Leu Tyr Asn Val Thr Arg Thr
    130                 135                 140

Phe Tyr Val Pro Ala Ala Tyr Lys Leu Thr Lys Leu Ser Val Lys Cys
145                 150                 155                 160

Tyr Phe Asn Tyr Ser Cys Val Phe Ser Val Val Asn Ala Thr Val Thr
                165                 170                 175

Val Asn Val Thr Thr His Asn Gly Arg Val Val Asn Tyr Thr Val Cys
            180                 185                 190

Asp Asp Cys Asn Gly Tyr Thr Asp Asn Ile Phe Ser Val Gln Gln Asp
        195                 200                 205

Gly Arg Ile Pro Asn Gly Phe Pro Phe Asn Asn Trp Phe Leu Leu Thr

210                 215                 220

Asn Gly Ser Thr Leu Val Asp Gly Val Ser Arg Leu Tyr Gln Pro Leu
225                 230                 235                 240

Arg Leu Thr Cys Leu Trp Pro Val Pro Gly Leu Lys Ser Ser Thr Gly
                245                 250                 255

Phe Val Tyr Phe Asn Ala Thr Gly Ser Asp Val Asn Cys Asn Gly Tyr
            260                 265                 270

Gln His Asn Ser Val Val Asp Val Met Arg Tyr Asn Leu Asn Phe Ser
        275                 280                 285

Ala Asn Ser Leu Asp Asn Leu Lys Ser Gly Val Ile Val Phe Lys Thr
    290                 295                 300

Leu Gln Tyr Asp Val Leu Phe Tyr Cys Ser Asn Ser Ser Ser Gly Val
305                 310                 315                 320

Leu Asp Thr Thr Ile Pro Phe Gly Pro Ser Ser Gln Pro Tyr Tyr Cys
                325                 330                 335

Phe Ile Asn Ser Thr Ile Asn Thr Thr His Val Ser Thr Phe Val Gly
            340                 345                 350

Ile Leu Pro Pro Thr Val Arg Glu Ile Val Val Ala Arg Thr Gly Gln
        355                 360                 365

Phe Tyr Ile Asn Gly Phe Lys Tyr Phe Asp Leu Gly Phe Ile Glu Ala
    370                 375                 380

Val Asn Phe Asn Val Thr Thr Ala Ser Ala Thr Asp Phe Trp Thr Val
385                 390                 395                 400

Ala Phe Ala Thr Phe Val Asp Val Leu Val Asn Val Ser Ala Thr Asn
                405                 410                 415

Ile Gln Asn Leu Leu Tyr Cys Asp Ser Pro Phe Glu Lys Leu Gln Cys
            420                 425                 430

Glu His Leu Gln Phe Gly Leu Gln Asp Gly Phe Tyr Ser Ala Asn Phe
        435                 440                 445

Leu Asp Asp Asn Val Leu Pro Glu Thr Tyr Val Ala Leu Pro Ile Tyr
    450                 455                 460

Tyr Gln His Thr Asp Ile Asn Phe Thr Ala Thr Ala Ser Phe Gly Gly
465                 470                 475                 480

Ser Cys Tyr Val Cys Lys Pro His Gln Val Asn Ile Ser Leu Asn Gly
                485                 490                 495

Asn Thr Ser Val Cys Val Arg Thr Ser His Phe Ser Ile Arg Tyr Ile
            500                 505                 510

Tyr Asn Arg Val Lys Ser Gly Ser Pro Gly Asp Ser Ser Trp His Ile
        515                 520                 525

Tyr Leu Lys Ser Gly Thr Cys Pro Phe Ser Phe Ser Lys Leu Asn Asn
    530                 535                 540

Phe Gln Lys Phe Lys Thr Ile Cys Phe Ser Thr Val Glu Val Pro Gly
545                 550                 555                 560

Ser Cys Asn Phe Pro Leu Glu Ala Thr Trp His Tyr Thr Ser Tyr Thr
                565                 570                 575

Ile Val Gly Ala Leu Tyr Val Thr Trp Ser Glu Gly Asn Ser Ile Thr
            580                 585                 590

Gly Val Pro Tyr Pro Val Ser Gly Ile Arg Glu Phe Ser Asn Leu Val
        595                 600                 605

Leu Asn Asn Cys Thr Lys Tyr Asn Ile Tyr Asp Tyr Val Gly Thr Gly
    610                 615                 620

Ile Ile Arg Ser Ser Asn Gln Ser Leu Ala Gly Gly Ile Thr Tyr Val
625                 630                 635                 640

Ser Asn Ser Gly Asn Leu Leu Gly Phe Lys Asn Val Ser Thr Gly Asn
                645                 650                 655

Ile Phe Ile Val Thr Pro Cys Asn Gln Pro Asp Gln Val Ala Val Tyr
            660                 665                 670

Gln Gln Ser Ile Ile Gly Ala Met Thr Ala Val Asn Glu Ser Arg Tyr
        675                 680                 685

Gly Leu Gln Asn Leu Leu Gln Leu Pro Asn Phe Tyr Tyr Val
    690                 695                 700

<210>  11
<211>  657
<212>  PRT

<213>  Artificial Sequence

<220>
<223>  S1 [SARS_CoV]

<400>  11

Ser Gly Ser Asp Leu Asp Arg Cys Thr Thr Phe Asp Asp Val Gln Ala
1               5                   10                  15

Pro Asn Tyr Thr Gln His Thr Ser Ser Met Arg Gly Val Tyr Tyr Pro
            20                  25                  30

Asp Glu Ile Phe Arg Ser Asp Thr Leu Tyr Leu Thr Gln Asp Leu Phe
        35                  40                  45

Leu Pro Phe Tyr Ser Asn Val Thr Gly Phe His Thr Ile Asn His Thr
    50                  55                  60

Phe Gly Asn Pro Val Ile Pro Phe Lys Asp Gly Ile Tyr Phe Ala Ala
65                  70                  75                  80

Thr Glu Lys Ser Asn Val Val Arg Gly Trp Val Phe Gly Ser Thr Met
                85                  90                  95

Asn Asn Lys Ser Gln Ser Val Ile Ile Ile Asn Asn Ser Thr Asn Val
            100                 105                 110

Val Ile Arg Ala Cys Asn Phe Glu Leu Cys Asp Asn Pro Phe Phe Ala
        115                 120                 125

Val Ser Lys Pro Met Gly Thr Gln Thr His Thr Met Ile Phe Asp Asn
    130                 135                 140

Ala Phe Asn Cys Thr Phe Glu Tyr Ile Ser Asp Ala Phe Ser Leu Asp
145                 150                 155                 160

Val Ser Glu Lys Ser Gly Asn Phe Lys His Leu Arg Glu Phe Val Phe
                165                 170                 175

Lys Asn Lys Asp Gly Phe Leu Tyr Val Tyr Lys Gly Tyr Gln Pro Ile
            180                 185                 190

Asp Val Val Arg Asp Leu Pro Ser Gly Phe Asn Thr Leu Lys Pro Ile
        195                 200                 205

Phe Lys Leu Pro Leu Gly Ile Asn Ile Thr Asn Phe Arg Ala Ile Leu
    210                 215                 220

Thr Ala Phe Ser Pro Ala Gln Asp Ile Trp Gly Thr Ser Ala Ala Ala
225                 230                 235                 240

Tyr Phe Val Gly Tyr Leu Lys Pro Thr Thr Phe Met Leu Lys Tyr Asp
                245                 250                 255

Glu Asn Gly Thr Ile Thr Asp Ala Val Asp Cys Ser Gln Asn Pro Leu
            260                 265                 270

Ala Glu Leu Lys Cys Ser Val Lys Ser Phe Glu Ile Asp Lys Gly Ile
        275                 280                 285

Tyr Gln Thr Ser Asn Phe Arg Val Val Pro Ser Gly Asp Val Val Arg
    290                 295                 300

Phe Pro Asn Ile Thr Asn Leu Cys Pro Phe Gly Glu Val Phe Asn Ala
305                 310                 315                 320

Thr Lys Phe Pro Ser Val Tyr Ala Trp Glu Arg Lys Lys Ile Ser Asn
                325                 330                 335

Cys Val Ala Asp Tyr Ser Val Leu Tyr Asn Ser Thr Phe Phe Ser Thr
            340                 345                 350

Phe Lys Cys Tyr Gly Val Ser Ala Thr Lys Leu Asn Asp Leu Cys Phe
        355                 360                 365

Ser Asn Val Tyr Ala Asp Ser Phe Val Val Lys Gly Asp Asp Val Arg
    370                 375                 380

Gln Ile Ala Pro Gly Gln Thr Gly Val Ile Ala Asp Tyr Asn Tyr Lys
385                 390                 395                 400

Leu Pro Asp Asp Phe Met Gly Cys Val Leu Ala Trp Asn Thr Arg Asn
                405                 410                 415

Ile Asp Ala Thr Ser Thr Gly Asn Tyr Asn Tyr Lys Tyr Arg Tyr Leu
            420                 425                 430

Arg His Gly Lys Leu Arg Pro Phe Glu Arg Asp Ile Ser Asn Val Pro
        435                 440                 445

Phe Ser Pro Asp Gly Lys Pro Cys Thr Pro Pro Ala Leu Asn Cys Tyr
    450                 455                 460

Trp Pro Leu Asn Asp Tyr Gly Phe Tyr Thr Thr Thr Gly Ile Gly Tyr
465                 470                 475                 480

Gln Pro Tyr Arg Val Val Val Leu Ser Phe Glu Leu Leu Asn Ala Pro
                485                 490                 495

Ala Thr Val Cys Gly Pro Lys Leu Ser Thr Asp Leu Ile Lys Asn Gln
            500                 505                 510

Cys Val Asn Phe Asn Phe Asn Gly Leu Thr Gly Thr Gly Val Leu Thr
        515                 520                 525

Pro Ser Ser Lys Arg Phe Gln Pro Phe Gln Gln Phe Gly Arg Asp Val
    530                 535                 540

Ser Asp Phe Thr Asp Ser Val Arg Asp Pro Lys Thr Ser Glu Ile Leu
545                 550                 555                 560

Asp Ile Ser Pro Cys Ser Phe Gly Gly Val Ser Val Ile Thr Pro Gly
                565                 570                 575

Thr Asn Ala Ser Ser Glu Val Ala Val Leu Tyr Gln Asp Val Asn Cys
            580                 585                 590

Thr Asp Val Ser Thr Ala Ile His Ala Asp Gln Leu Thr Pro Ala Trp
        595                 600                 605

Arg Ile Tyr Ser Thr Gly Asn Asn Val Phe Gln Thr Gln Ala Gly Cys
    610                 615                 620

Leu Ile Gly Ala Glu His Val Asp Thr Ser Tyr Glu Cys Asp Ile Pro
625                 630                 635                 640

Ile Gly Ala Gly Ile Cys Ala Ser Tyr His Thr Val Ser Leu Leu Arg
                645                 650                 655

Leu

<210>  12
<211>  649
<212>  PRT
<213>  Artificial Sequence

<220>
<223>  fragment of SEQ ID NO1

<400>  12

Asn Ser Phe Thr Arg Gly Val Tyr Tyr Pro Asp Lys Val Phe Arg Ser
1               5                   10                  15

Ser Val Leu His Ser Thr Gln Asp Leu Phe Leu Pro Phe Phe Ser Asn
            20                  25                  30

Val Thr Trp Phe His Ala Ile His Val Ser Gly Thr Asn Gly Thr Lys
        35                  40                  45

Arg Phe Asp Asn Pro Val Leu Pro Phe Asn Asp Gly Val Tyr Phe Ala
    50                  55                  60

Ser Thr Glu Lys Ser Asn Ile Ile Arg Gly Trp Ile Phe Gly Thr Thr
65                  70                  75                  80

Leu Asp Ser Lys Thr Gln Ser Leu Leu Ile Val Asn Asn Ala Thr Asn
                85                  90                  95

Val Val Ile Lys Val Cys Glu Phe Gln Phe Cys Asn Asp Pro Phe Leu
            100                 105                 110

Gly Val Tyr Tyr His Lys Asn Asn Lys Ser Trp Met Glu Ser Glu Phe
        115                 120                 125

Arg Val Tyr Ser Ser Ala Asn Asn Cys Thr Phe Glu Tyr Val Ser Gln
    130                 135                 140

Pro Phe Leu Met Asp Leu Glu Gly Lys Gln Gly Asn Phe Lys Asn Leu
145                 150                 155                 160

Arg Glu Phe Val Phe Lys Asn Ile Asp Gly Tyr Phe Lys Ile Tyr Ser
                165                 170                 175

Lys His Thr Pro Ile Asn Leu Val Arg Asp Leu Pro Gln Gly Phe Ser
            180                 185                 190

Ala Leu Glu Pro Leu Val Asp Leu Pro Ile Gly Ile Asn Ile Thr Arg
        195                 200                 205

Phe Gln Thr Leu Leu Ala Leu His Arg Ser Tyr Leu Thr Pro Gly Asp
    210                 215                 220

Ser Ser Ser Gly Trp Thr Ala Gly Ala Ala Ala Tyr Tyr Val Gly Tyr
225                 230                 235                 240

Leu Gln Pro Arg Thr Phe Leu Leu Lys Tyr Asn Glu Asn Gly Thr Ile
                245                 250                 255

Thr Asp Ala Val Asp Cys Ala Leu Asp Pro Leu Ser Glu Thr Lys Cys
            260                 265                 270

Thr Leu Lys Ser Phe Thr Val Glu Lys Gly Ile Tyr Gln Thr Ser Asn
        275                 280                 285

Phe Arg Val Gln Pro Thr Glu Ser Ile Val Arg Phe Pro Asn Ile Thr
    290                 295                 300

Asn Leu Cys Pro Phe Gly Glu Val Phe Asn Ala Thr Arg Phe Ala Ser
305                 310                 315                 320

Val Tyr Ala Trp Asn Arg Lys Arg Ile Ser Asn Cys Val Ala Asp Tyr
                325                 330                 335

Ser Val Leu Tyr Asn Ser Ala Ser Phe Ser Thr Phe Lys Cys Tyr Gly
            340                 345                 350

Val Ser Pro Thr Lys Leu Asn Asp Leu Cys Phe Thr Asn Val Tyr Ala
        355                 360                 365

Asp Ser Phe Val Ile Arg Gly Asp Glu Val Arg Gln Ile Ala Pro Gly
    370                 375                 380

Gln Thr Gly Lys Ile Ala Asp Tyr Asn Tyr Lys Leu Pro Asp Asp Phe
385                 390                 395                 400

Thr Gly Cys Val Ile Ala Trp Asn Ser Asn Asn Leu Asp Ser Lys Val
                405                 410                 415

Gly Gly Asn Tyr Asn Tyr Leu Tyr Arg Leu Phe Arg Lys Ser Asn Leu
            420                 425                 430

Lys Pro Phe Glu Arg Asp Ile Ser Thr Glu Ile Tyr Gln Ala Gly Ser
        435                 440                 445

Thr Pro Cys Asn Gly Val Glu Gly Phe Asn Cys Tyr Phe Pro Leu Gln
    450                 455                 460

Ser Tyr Gly Phe Gln Pro Thr Asn Gly Val Gly Tyr Gln Pro Tyr Arg
465                 470                 475                 480

Val Val Val Leu Ser Phe Glu Leu Leu His Ala Pro Ala Thr Val Cys
                485                 490                 495

Gly Pro Lys Lys Ser Thr Asn Leu Val Lys Asn Lys Cys Val Asn Phe
            500                 505                 510

Asn Phe Asn Gly Leu Thr Gly Thr Gly Val Leu Thr Glu Ser Asn Lys
        515                 520                 525

Lys Phe Leu Pro Phe Gln Gln Phe Gly Arg Asp Ile Ala Asp Thr Thr
    530                 535                 540

Asp Ala Val Arg Asp Pro Gln Thr Leu Glu Ile Leu Asp Ile Thr Pro
545                 550                 555                 560

Cys Ser Phe Gly Gly Val Ser Val Ile Thr Pro Gly Thr Asn Thr Ser
                565                 570                 575

Asn Gln Val Ala Val Leu Tyr Gln Asp Val Asn Cys Thr Glu Val Pro
            580                 585                 590

Val Ala Ile His Ala Asp Gln Leu Thr Pro Thr Trp Arg Val Tyr Ser
        595                 600                 605

Thr Gly Ser Asn Val Phe Gln Thr Arg Ala Gly Cys Leu Ile Gly Ala
    610                 615                 620

Glu His Val Asn Asn Ser Tyr Glu Cys Asp Ile Pro Ile Gly Ala Gly
625                 630                 635                 640

Ile Cys Ala Ser Tyr Gln Thr Gln Thr
                645

<210>  13
<211>  29903
<212>  DNA
<213>  SARS-CoV-2

<400>  13
attaaaggtt tataccttcc caggtaacaa accaaccaac tttcgatctc ttgtagatct      60

gttctctaaa cgaactttaa aatctgtgtg gctgtcactc ggctgcatgc ttagtgcact     120

cacgcagtat aattaataac taattactgt cgttgacagg acacgagtaa ctcgtctatc     180

ttctgcaggc tgcttacggt ttcgtccgtg ttgcagccga tcatcagcac atctaggttt     240

cgtccgggtg tgaccgaaag gtaagatgga gagccttgtc cctggtttca acgagaaaac     300

acacgtccaa ctcagtttgc ctgttttaca ggttcgcgac gtgctcgtac gtggctttgg     360

agactccgtg gaggaggtct tatcagaggc acgtcaacat cttaaagatg gcacttgtgg     420

cttagtagaa gttgaaaaag gcgttttgcc tcaacttgaa cagccctatg tgttcatcaa     480

acgttcggat gctcgaactg cacctcatgg tcatgttatg gttgagctgg tagcagaact     540

cgaaggcatt cagtacggtc gtagtggtga gacacttggt gtccttgtcc ctcatgtggg     600

cgaaatacca gtggcttacc gcaaggttct tcttcgtaag aacggtaata aaggagctgg     660

tggccatagt tacggcgccg atctaaagtc atttgactta ggcgacgagc ttggcactga     720

tccttatgaa gattttcaag aaaactggaa cactaaacat agcagtggtg ttacccgtga 780

actcatgcgt gagcttaacg gaggggcata cactcgctat gtcgataaca acttctgtgg 840

ccctgatggc taccctcttg agtgcattaa agaccttcta gcacgtgctg gtaaagcttc 900

atgcactttg tccgaacaac tggactttat tgacactaag aggggtgtat actgctgccg 960

tgaacatgag catgaaattg cttggtacac ggaacgttct gaaaagagct atgaattgca 1020

gacacctttt gaaattaaat tggcaaagaa atttgacacc ttcaatgggg aatgtccaaa 1080

ttttgtattt cccttaaatt ccataatcaa gactattcaa ccaagggttg aaaagaaaaa 1140

gcttgatggc tttatgggta gaattcgatc tgtctatcca gttgcgtcac caaatgaatg 1200

caaccaaatg tgcctttcaa ctctcatgaa gtgtgatcat tgtggtgaaa cttcatggca 1260

gacgggcgat tttgttaaag ccacttgcga attttgtggc actgagaatt tgactaaaga 1320

aggtgccact acttgtggtt acttacccca aaatgctgtt gttaaaattt attgtccagc 1380

atgtcacaat tcagaagtag gacctgagca tagtcttgcc gaataccata atgaatctgg 1440

cttgaaaacc attcttcgta agggtggtcg cactattgcc tttggaggct gtgtgttctc 1500

ttatgttggt tgccataaca agtgtgccta ttgggttcca cgtgctagcg ctaacatagg 1560

ttgtaaccat acaggtgttg ttggagaagg ttccgaaggt cttaatgaca accttcttga 1620

aatactccaa aaagagaaag tcaacatcaa tattgttggt gactttaaac ttaatgaaga 1680

gatcgccatt attttggcat ctttttctgc ttccacaagt gcttttgtgg aaactgtgaa 1740

aggtttggat tataaagcat tcaaacaaat tgttgaatcc tgtggtaatt ttaaagttac 1800

aaaaggaaaa gctaaaaaag gtgcctggaa tattggtgaa cagaaatcaa tactgagtcc 1860

tctttatgca tttgcatcag aggctgctcg tgttgtacga tcaattttct cccgcactct 1920

tgaaactgct caaaattctg tgcgtgtttt acagaaggcc gctataacaa tactagatgg 1980

aatttcacag tattcactga gactcattga tgctatgatg ttcacatctg atttggctac 2040

taacaatcta gttgtaatgg cctacattac aggtggtgtt gttcagttga cttcgcagtg 2100

gctaactaac atctttggca ctgtttatga aaaactcaaa cccgtccttg attggcttga 2160

agagaagttt aaggaaggtg tagagtttct tagagacggt tgggaaattg ttaaatttat 2220

ctcaacctgt gcttgtgaaa ttgtcggtgg acaaattgtc acctgtgcaa aggaaattaa 2280

ggagagtgtt cagacattct ttaagcttgt aaataaattt ttggctttgt gtgctgactc 2340

tatcattatt ggtggagcta aacttaaagc cttgaattta ggtgaaacat ttgtcacgca 2400

ctcaaaggga ttgtacagaa agtgtgttaa atccagagaa gaaactggcc tactcatgcc 2460

tctaaaagcc ccaaaagaaa ttatcttctt agagggagaa acacttccca cagaagtgtt 2520

aacagaggaa gttgtcttga aaactggtga tttacaacca ttagaacaac ctactagtga 2580

agctgttgaa gctccattgg ttggtacacc agtttgtatt aacgggctta tgttgctcga 2640

aatcaaagac acagaaaagt actgtgccct tgcacctaat atgatggtaa caaacaatac 2700

cttcacactc aaaggcggtg caccaacaaa ggttactttt ggtgatgaca ctgtgataga 2760

agtgcaaggt tacaagagtg tgaatatcac ttttgaactt gatgaaagga ttgataaagt 2820

acttaatgag aagtgctctg cctatacagt tgaactcggt acagaagtaa atgagttcgc 2880

ctgtgttgtg gcagatgctg tcataaaaac tttgcaacca gtatctgaat tacttacacc 3000

actgggcatt gatttagatg agtggagtat ggctacatac tacttatttg atgagtctgg 3000

tgagtttaaa ttggcttcac atatgtattg ttctttctac cctccagatg aggatgaaga 3060

agaaggtgat tgtgaagaag aagagtttga gccatcaact caatatgagt atggtactga 3120

agatgattac caaggtaaac ctttggaatt tggtgccact tctgctgctc ttcaacctga 3180

agaagagcaa gaagaagatt ggttagatga tgatagtcaa caaactgttg gtcaacaaga 3240

cggcagtgag gacaatcaga caactactat tcaaacaatt gttgaggttc aacctcaatt 3300

agagatggaa cttacaccag ttgttcagac tattgaagtg aatagtttta gtggttattt 3360

aaaacttact gacaatgtat acattaaaaa tgcagacatt gtggaagaag ctaaaaaggt 3420

aaaaccaaca gtggttgtta atgcagccaa tgtttacctt aaacatggag gaggtgttgc 3480

aggagcctta aataaggcta ctaacaatgc catgcaagtt gaatctgatg attacatagc 3540

tactaatgga ccacttaaag tgggtggtag ttgtgtttta agcggacaca atcttgctaa 3600

acactgtctt catgttgtcg gcccaaatgt taacaaaggt gaagacattc aacttcttaa 3660

gagtgcttat gaaaatttta atcagcacga agttctactt gcaccattat tatcagctgg 3720

tatttttggt gctgacccta tacattcttt aagagtttgt gtagatactg ttcgcacaaa 3780

tgtctactta gctgtctttg ataaaaatct ctatgacaaa cttgtttcaa gctttttgga 3840

aatgaagagt gaaaagcaag ttgaacaaaa gatcgctgag attcctaaag aggaagttaa 3900

gccatttata actgaaagta aaccttcagt tgaacagaga aaacaagatg ataagaaaat 3960

caaagcttgt gttgaagaag ttacaacaac tctggaagaa actaagttcc tcacagaaaa 4020

cttgttactt tatattgaca ttaatggcaa tcttcatcca gattctgcca ctcttgttag 4080

tgacattgac atcactttct taaagaaaga tgctccatat atagtgggtg atgttgttca 4140

agagggtgtt ttaactgctg tggttatacc tactaaaaag gctggtggca ctactgaaat 4200

gctagcgaaa gctttgagaa aagtgccaac agacaattat ataaccactt acccgggtca 4260

gggtttaaat ggttacactg tagaggaggc aaagacagtg cttaaaaagt gtaaaagtgc 4320

cttttacatt ctaccatcta ttatctctaa tgagaagcaa gaaattcttg gaactgtttc 4380

ttggaatttg cgagaaatgc ttgcacatgc agaagaaaca cgcaaattaa tgcctgtctg 4440

tgtggaaact aaagccatag tttcaactat acagcgtaaa tataagggta ttaaaataca 4500

agagggtgtg gttgattatg gtgctagatt ttacttttac accagtaaaa caactgtagc 4560

gtcacttatc aacacactta acgatctaaa tgaaactctt gttacaatgc cacttggcta 4620

tgtaacacat ggcttaaatt tggaagaagc tgctcggtat atgagatctc tcaaagtgcc 4680

agctacagtt tctgtttctt cacctgatgc tgttacagcg tataatggtt atcttacttc 4740

ttcttctaaa acacctgaag aacattttat tgaaaccatc tcacttgctg gttcctataa 4800

agattggtcc tattctggac aatctacaca actaggtata gaatttctta agagaggtga 4860

taaaagtgta tattacacta gtaatcctac cacattccac ctagatggtg aagttatcac 4920

ctttgacaat cttaagacac ttctttcttt gagagaagtg aggactatta aggtgtttac 4980

aacagtagac aacattaacc tccacacgca agttgtggac atgtcaatga catatggaca 5040

acagtttggt ccaacttatt tggatggagc tgatgttact aaaataaaac ctcataattc 5100

acatgaaggt aaaacatttt atgttttacc taatgatgac actctacgtg ttgaggcttt 5160

tgagtactac cacacaactg atcctagttt tctgggtagg tacatgtcag cattaaatca 5220

cactaaaaag tggaaatacc cacaagttaa tggtttaact tctattaaat gggcagataa 5280

caactgttat cttgccactg cattgttaac actccaacaa atagagttga agtttaatcc 5340

acctgctcta caagatgctt attacagagc aagggctggt gaagctgcta acttttgtgc 5400

acttatctta gcctactgta ataagacagt aggtgagtta ggtgatgtta gagaaacaat 5460

gagttacttg tttcaacatg ccaatttaga ttcttgcaaa agagtcttga acgtggtgtg 5520

taaaacttgt ggacaacagc agacaaccct taagggtgta gaagctgtta tgtacatggg 5580

cacactttct tatgaacaat ttaagaaagg tgttcagata ccttgtacgt gtggtaaaca 5640

agctacaaaa tatctagtac aacaggagtc accttttgtt atgatgtcag caccacctgc 5700

tcagtatgaa cttaagcatg gtacatttac ttgtgctagt gagtacactg gtaattacca 5760

gtgtggtcac tataaacata taacttctaa agaaactttg tattgcatag acggtgcttt 5820

acttacaaag tcctcagaat acaaaggtcc tattacggat gttttctaca aagaaaacag 5880

ttacacaaca accataaaac cagttactta taaattggat ggtgttgttt gtacagaaat 5940

tgaccctaag ttggacaatt attataagaa agacaattct tatttcacag agcaaccaat 6000

tgatcttgta ccaaaccaac catatccaaa cgcaagcttc gataatttta agtttgtatg 6060

tgataatatc aaatttgctg atgatttaaa ccagttaact ggttataaga aacctgcttc 6120

aagagagctt aaagttacat ttttccctga cttaaatggt gatgtggtgg ctattgatta 6180

taaacactac acaccctctt ttaagaaagg agctaaattg ttacataaac ctattgtttg 6240

gcatgttaac aatgcaacta ataaagccac gtataaacca aatacctggt gtatacgttg 6300

tctttggagc acaaaaccag ttgaaacatc aaattcgttt gatgtactga agtcagagga 6360

cgcgcaggga atggataatc ttgcctgcga agatctaaaa ccagtctctg aagaagtagt 6420

ggaaaatcct accatacaga aagacgttct tgagtgtaat gtgaaaacta ccgaagttgt 6480

aggagacatt atacttaaac cagcaaataa tagtttaaaa attacagaag aggttggcca 6540

cacagatcta atggctgctt atgtagacaa ttctagtctt actattaaga aacctaatga 6600

attatctaga gtattaggtt tgaaaaccct tgctactcat ggtttagctg ctgttaatag 6660

tgtcccttgg gatactatag ctaattatgc taagcctttt cttaacaaag ttgttagtac 6720

aactactaac atagttacac ggtgtttaaa ccgtgtttgt actaattata tgccttattt 6780

ctttacttta ttgctacaat tgtgtacttt tactagaagt acaaattcta gaattaaagc 6840

atctatgccg actactatag caaagaatac tgttaagagt gtcggtaaat tttgtctaga 6900

ggcttcattt aattatttga agtcacctaa tttttctaaa ctgataaata ttataatttg 6960

gtttttacta ttaagtgttt gcctaggttc tttaatctac tcaaccgctg ctttaggtgt 7020

tttaatgtct aatttaggca tgccttctta ctgtactggt tacagagaag gctatttgaa 7080

ctctactaat gtcactattg caacctactg tactggttct ataccttgta gtgtttgtct 7140

tagtggttta gattctttag acacctatcc ttctttagaa actatacaaa ttaccatttc 7200

atcttttaaa tgggatttaa ctgcttttgg cttagttgca gagtggtttt tggcatatat 7260

tcttttcact aggtttttct atgtacttgg attggctgca atcatgcaat tgtttttcag 7320

ctattttgca gtacatttta ttagtaattc ttggcttatg tggttaataa ttaatcttgt 7380

acaaatggcc ccgatttcag ctatggttag aatgtacatc ttctttgcat cattttatta 7440

tgtatggaaa agttatgtgc atgttgtaga cggttgtaat tcatcaactt gtatgatgtg 7500

ttacaaacgt aatagagcaa caagagtcga atgtacaact attgttaatg gtgttagaag 7560

gtccttttat gtctatgcta atggaggtaa aggcttttgc aaactacaca attggaattg 7620

tgttaattgt gatacattct gtgctggtag tacatttatt agtgatgaag ttgcgagaga 7680

cttgtcacta cagtttaaaa gaccaataaa tcctactgac cagtcttctt acatcgttga 7740

tagtgttaca gtgaagaatg gttccatcca tctttacttt gataaagctg gtcaaaagac 7800

ttatgaaaga cattctctct ctcattttgt taacttagac aacctgagag ctaataacac 7860

taaaggttca ttgcctatta atgttatagt ttttgatggt aaatcaaaat gtgaagaatc 7920

atctgcaaaa tcagcgtctg tttactacag tcagcttatg tgtcaaccta tactgttact 7980

agatcaggca ttagtgtctg atgttggtga tagtgcggaa gttgcagtta aaatgtttga 8040

tgcttacgtt aatacgtttt catcaacttt taacgtacca atggaaaaac tcaaaacact 8100

agttgcaact gcagaagctg aacttgcaaa gaatgtgtcc ttagacaatg tcttatctac 8160

ttttatttca gcagctcggc aagggtttgt tgattcagat gtagaaacta aagatgttgt 8220

tgaatgtctt aaattgtcac atcaatctga catagaagtt actggcgata gttgtaataa 8280

ctatatgctc acctataaca aagttgaaaa catgacaccc cgtgaccttg gtgcttgtat 8340

tgactgtagt gcgcgtcata ttaatgcgca ggtagcaaaa agtcacaaca ttgctttgat 8400

atggaacgtt aaagatttca tgtcattgtc tgaacaacta cgaaaacaaa tacgtagtgc 8460

tgctaaaaag aataacttac cttttaagtt gacatgtgca actactagac aagttgttaa 8520

tgttgtaaca acaaagatag cacttaaggg tggtaaaatt gttaataatt ggttgaagca 8580

gttaattaaa gttacacttg tgttcctttt tgttgctgct attttctatt taataacacc 8640

tgttcatgtc atgtctaaac atactgactt ttcaagtgaa atcataggat acaaggctat 8700

tgatggtggt gtcactcgtg acatagcatc tacagatact tgttttgcta acaaacatgc 8760

tgattttgac acatggttta gccagcgtgg tggtagttat actaatgaca aagcttgccc 8820

attgattgct gcagtcataa caagagaagt gggttttgtc gtgcctggtt tgcctggcac 8880

gatattacgc acaactaatg gtgacttttt gcatttctta cctagagttt ttagtgcagt 8940

tggtaacatc tgttacacac catcaaaact tatagagtac actgactttg caacatcagc 9000

ttgtgttttg gctgctgaat gtacaatttt taaagatgct tctggtaagc cagtaccata 9060

ttgttatgat accaatgtac tagaaggttc tgttgcttat gaaagtttac gccctgacac 9120

acgttatgtg ctcatggatg gctctattat tcaatttcct aacacctacc ttgaaggttc 9180

tgttagagtg gtaacaactt ttgattctga gtactgtagg cacggcactt gtgaaagatc 9240

agaagctggt gtttgtgtat ctactagtgg tagatgggta cttaacaatg attattacag 9300

atctttacca ggagttttct gtggtgtaga tgctgtaaat ttacttacta atatgtttac 9360

accactaatt caacctattg gtgctttgga catatcagca tctatagtag ctggtggtat 9420

tgtagctatc gtagtaacat gccttgccta ctattttatg aggtttagaa gagcttttgg 9480

tgaatacagt catgtagttg cctttaatac tttactattc cttatgtcat tcactgtact 9540

ctgtttaaca ccagtttact cattcttacc tggtgtttat tctgttattt acttgtactt 9600

gacattttat cttactaatg atgtttcttt tttagcacat attcagtgga tggttatgtt 9660

cacaccttta gtacctttct ggataacaat tgcttatatc atttgtattt ccacaaagca 9720

tttctattgg ttctttagta attacctaaa gagacgtgta gtctttaatg gtgtttcctt 9780

tagtactttt gaagaagctg cgctgtgcac ctttttgtta aataaagaaa tgtatctaaa 9840

gttgcgtagt gatgtgctat tacctcttac gcaatataat agatacttag ctctttataa 9900

taagtacaag tattttagtg gagcaatgga tacaactagc tacagagaag ctgcttgttg 9960

tcatctcgca aaggctctca atgacttcag taactcaggt tctgatgttc tttaccaacc 10020

accacaaacc tctatcacct cagctgtttt gcagagtggt tttagaaaaa tggcattccc 10080

atctggtaaa gttgagggtt gtatggtaca agtaacttgt ggtacaacta cacttaacgg 10140

tctttggctt gatgacgtag tttactgtcc aagacatgtg atctgcacct ctgaagacat 10200

gcttaaccct aattatgaag atttactcat tcgtaagtct aatcataatt tcttggtaca 10260

ggctggtaat gttcaactca gggttattgg acattctatg caaaattgtg tacttaagct 10320

taaggttgat acagccaatc ctaagacacc taagtataag tttgttcgca ttcaaccagg 10380

acagactttt tcagtgttag cttgttacaa tggttcacca tctggtgttt accaatgtgc 10440

tatgaggccc aatttcacta ttaagggttc attccttaat ggttcatgtg gtagtgttgg 10500

ttttaacata gattatgact gtgtctcttt ttgttacatg caccatatgg aattaccaac 10560

tggagttcat gctggcacag acttagaagg taacttttat ggaccttttg ttgacaggca 10620

aacagcacaa gcagctggta cggacacaac tattacagtt aatgttttag cttggttgta 10680

cgctgctgtt ataaatggag acaggtggtt tctcaatcga tttaccacaa ctcttaatga 10740

ctttaacctt gtggctatga agtacaatta tgaacctcta acacaagacc atgttgacat 10800

actaggacct ctttctgctc aaactggaat tgccgtttta gatatgtgtg cttcattaaa 10860

agaattactg caaaatggta tgaatggacg taccatattg ggtagtgctt tattagaaga 10920

tgaatttaca ccttttgatg ttgttagaca atgctcaggt gttactttcc aaagtgcagt 10980

gaaaagaaca atcaagggta cacaccactg gttgttactc acaattttga cttcactttt 11040

agttttagtc cagagtactc aatggtcttt gttctttttt ttgtatgaaa atgccttttt 11100

accttttgct atgggtatta ttgctatgtc tgcttttgca atgatgtttg tcaaacataa 11160

gcatgcattt ctctgtttgt ttttgttacc ttctcttgcc actgtagctt attttaatat 11220

ggtctatatg cctgctagtt gggtgatgcg tattatgaca tggttggata tggttgatac 11280

tagtttgtct ggttttaagc taaaagactg tgttatgtat gcatcagctg tagtgttact 11340

aatccttatg acagcaagaa ctgtgtatga tgatggtgct aggagagtgt ggacacttat 11400

gaatgtcttg acactcgttt ataaagttta ttatggtaat gctttagatc aagccatttc 11460

catgtgggct cttataatct ctgttacttc taactactca ggtgtagtta caactgtcat 11520

gtttttggcc agaggtattg tttttatgtg tgttgagtat tgccctattt tcttcataac 11580

tggtaataca cttcagtgta taatgctagt ttattgtttc ttaggctatt tttgtacttg 11640

ttactttggc ctcttttgtt tactcaaccg ctactttaga ctgactcttg gtgtttatga 11700

ttacttagtt tctacacagg agtttagata tatgaattca cagggactac tcccacccaa 11760

gaatagcata gatgccttca aactcaacat taaattgttg ggtgttggtg gcaaaccttg 11820

tatcaaagta gccactgtac agtctaaaat gtcagatgta aagtgcacat cagtagtctt 11880

actctcagtt ttgcaacaac tcagagtaga atcatcatct aaattgtggg ctcaatgtgt 11940

ccagttacac aatgacattc tcttagctaa agatactact gaagcctttg aaaaaatggt 12000

ttcactactt tctgttttgc tttccatgca gggtgctgta gacataaaca agctttgtga 12060

agaaatgctg gacaacaggg caaccttaca agctatagcc tcagagttta gttcccttcc 12120

atcatatgca gcttttgcta ctgctcaaga agcttatgag caggctgttg ctaatggtga 12180

ttctgaagtt gttcttaaaa agttgaagaa gtctttgaat gtggctaaat ctgaatttga 12240

ccgtgatgca gccatgcaac gtaagttgga aaagatggct gatcaagcta tgacccaaat 12300

gtataaacag gctagatctg aggacaagag ggcaaaagtt actagtgcta tgcagacaat 12360

gcttttcact atgcttagaa agttggataa tgatgcactc aacaacatta tcaacaatgc 12420

aagagatggt tgtgttccct tgaacataat acctcttaca acagcagcca aactaatggt 12480

tgtcatacca gactataaca catataaaaa tacgtgtgat ggtacaacat ttacttatgc 12540

atcagcattg tgggaaatcc aacaggttgt agatgcagat agtaaaattg ttcaacttag 12600

tgaaattagt atggacaatt cacctaattt agcatggcct cttattgtaa cagctttaag 12660

ggccaattct gctgtcaaat tacagaataa tgagcttagt cctgttgcac tacgacagat 12720

gtcttgtgct gccggtacta cacaaactgc ttgcactgat gacaatgcgt tagcttacta 12780

caacacaaca aagggaggta ggtttgtact tgcactgtta tccgatttac aggatttgaa 12840

atgggctaga ttccctaaga gtgatggaac tggtactatc tatacagaac tggaaccacc 12900

ttgtaggttt gttacagaca cacctaaagg tcctaaagtg aagtatttat actttattaa 12960

aggattaaac aacctaaata gaggtatggt acttggtagt ttagctgcca cagtacgtct 13020

acaagctggt aatgcaacag aagtgcctgc caattcaact gtattatctt tctgtgcttt 13080

tgctgtagat gctgctaaag cttacaaaga ttatctagct agtgggggac aaccaatcac 13140

taattgtgtt aagatgttgt gtacacacac tggtactggt caggcaataa cagttacacc 13200

ggaagccaat atggatcaag aatcctttgg tggtgcatcg tgttgtctgt actgccgttg 13260

ccacatagat catccaaatc ctaaaggatt ttgtgactta aaaggtaagt atgtacaaat 13320

acctacaact tgtgctaatg accctgtggg ttttacactt aaaaacacag tctgtaccgt 13380

ctgcggtatg tggaaaggtt atggctgtag ttgtgatcaa ctccgcgaac ccatgcttca 13440

gtcagctgat gcacaatcgt ttttaaacgg gtttgcggtg taagtgcagc ccgtcttaca 13500

ccgtgcggca caggcactag tactgatgtc gtatacaggg cttttgacat ctacaatgat 13560

aaagtagctg gttttgctaa attcctaaaa actaattgtt gtcgcttcca agaaaaggac 13620

gaagatgaca atttaattga ttcttacttt gtagttaaga gacacacttt ctctaactac 13680

caacatgaag aaacaattta taatttactt aaggattgtc cagctgttgc taaacatgac 13740

ttctttaagt ttagaataga cggtgacatg gtaccacata tatcacgtca acgtcttact 13800

aaatacacaa tggcagacct cgtctatgct ttaaggcatt ttgatgaagg taattgtgac 13860

acattaaaag aaatacttgt cacatacaat tgttgtgatg atgattattt caataaaaag 13920

gactggtatg attttgtaga aaacccagat atattacgcg tatacgccaa cttaggtgaa 13980

cgtgtacgcc aagctttgtt aaaaacagta caattctgtg atgccatgcg aaatgctggt 14040

attgttggtg tactgacatt agataatcaa gatctcaatg gtaactggta tgatttcggt 14100

gatttcatac aaaccacgcc aggtagtgga gttcctgttg tagattctta ttattcattg 14160

ttaatgccta tattaacctt gaccagggct ttaactgcag agtcacatgt tgacactgac 14220

ttaacaaagc cttacattaa gtgggatttg ttaaaatatg acttcacgga agagaggtta 14280

aaactctttg accgttattt taaatattgg gatcagacat accacccaaa ttgtgttaac 14340

tgtttggatg acagatgcat tctgcattgt gcaaacttta atgttttatt ctctacagtg 14400

ttcccaccta caagttttgg accactagtg agaaaaatat ttgttgatgg tgttccattt 14460

gtagtttcaa ctggatacca cttcagagag ctaggtgttg tacataatca ggatgtaaac 14520

ttacatagct ctagacttag ttttaaggaa ttacttgtgt atgctgctga ccctgctatg 14580

cacgctgctt ctggtaatct attactagat aaacgcacta cgtgcttttc agtagctgca 14640

cttactaaca atgttgcttt tcaaactgtc aaacccggta attttaacaa agacttctat 14700

gactttgctg tgtctaaggg tttctttaag gaaggaagtt ctgttgaatt aaaacacttc 14760

ttctttgctc aggatggtaa tgctgctatc agcgattatg actactatcg ttataatcta 14820

ccaacaatgt gtgatatcag acaactacta tttgtagttg aagttgttga taagtacttt 14880

gattgttacg atggtggctg tattaatgct aaccaagtca tcgtcaacaa cctagacaaa 14940

tcagctggtt ttccatttaa taaatggggt aaggctagac tttattatga ttcaatgagt 15000

tatgaggatc aagatgcact tttcgcatat acaaaacgta atgtcatccc tactataact 15060

caaatgaatc ttaagtatgc cattagtgca aagaatagag ctcgcaccgt agctggtgtc 15120

tctatctgta gtactatgac caatagacag tttcatcaaa aattattgaa atcaatagcc 15180

gccactagag gagctactgt agtaattgga acaagcaaat tctatggtgg ttggcacaac 15240

atgttaaaaa ctgtttatag tgatgtagaa aaccctcacc ttatgggttg ggattatcct 15300

aaatgtgata gagccatgcc taacatgctt agaattatgg cctcacttgt tcttgctcgc 15360

aaacatacaa cgtgttgtag cttgtcacac cgtttctata gattagctaa tgagtgtgct 15420

caagtattga gtgaaatggt catgtgtggc ggttcactat atgttaaacc aggtggaacc 15480

tcatcaggag atgccacaac tgcttatgct aatagtgttt ttaacatttg tcaagctgtc 15540

acggccaatg ttaatgcact tttatctact gatggtaaca aaattgccga taagtatgtc 15600

cgcaatttac aacacagact ttatgagtgt ctctatagaa atagagatgt tgacacagac 15660

tttgtgaatg agttttacgc atatttgcgt aaacatttct caatgatgat actctctgac 15720

gatgctgttg tgtgtttcaa tagcacttat gcatctcaag gtctagtggc tagcataaag 15780

aactttaagt cagttcttta ttatcaaaac aatgttttta tgtctgaagc aaaatgttgg 15840

actgagactg accttactaa aggacctcat gaattttgct ctcaacatac aatgctagtt 15900

aaacagggtg atgattatgt gtaccttcct tacccagatc catcaagaat cctaggggcc 15960

ggctgttttg tagatgatat cgtaaaaaca gatggtacac ttatgattga acggttcgtg 16020

tctttagcta tagatgctta cccacttact aaacatccta atcaggagta tgctgatgtc 16080

tttcatttgt acttacaata cataagaaag ctacatgatg agttaacagg acacatgtta 16140

gacatgtatt ctgttatgct tactaatgat aacacttcaa ggtattggga acctgagttt 16200

tatgaggcta tgtacacacc gcatacagtc ttacaggctg ttggggcttg tgttctttgc 16260

aattcacaga cttcattaag atgtggtgct tgcatacgta gaccattctt atgttgtaaa 16320

tgctgttacg accatgtcat atcaacatca cataaattag tcttgtctgt taatccgtat 16380

gtttgcaatg ctccaggttg tgatgtcaca gatgtgactc aactttactt aggaggtatg 16440

agctattatt gtaaatcaca taaaccaccc attagttttc cattgtgtgc taatggacaa 16500

gtttttggtt tatataaaaa tacatgtgtt ggtagcgata atgttactga ctttaatgca 16560

attgcaacat gtgactggac aaatgctggt gattacattt tagctaacac ctgtactgaa 16620

agactcaagc tttttgcagc agaaacgctc aaagctactg aggagacatt taaactgtct 16680

tatggtattg ctactgtacg tgaagtgctg tctgacagag aattacatct ttcatgggaa 16740

gttggtaaac ctagaccacc acttaaccga aattatgtct ttactggtta tcgtgtaact 16800

aaaaacagta aagtacaaat aggagagtac acctttgaaa aaggtgacta tggtgatgct 16860

gttgtttacc gaggtacaac aacttacaaa ttaaatgttg gtgattattt tgtgctgaca 16920

tcacatacag taatgccatt aagtgcacct acactagtgc cacaagagca ctatgttaga 16980

attactggct tatacccaac actcaatatc tcagatgagt tttctagcaa tgttgcaaat 17040

tatcaaaagg ttggtatgca aaagtattct acactccagg gaccacctgg tactggtaag 17100

agtcattttg ctattggcct agctctctac tacccttctg ctcgcatagt gtatacagct 17160

tgctctcatg ccgctgttga tgcactatgt gagaaggcat taaaatattt gcctatagat 17220

aaatgtagta gaattatacc tgcacgtgct cgtgtagagt gttttgataa attcaaagtg 17280

aattcaacat tagaacagta tgtcttttgt actgtaaatg cattgcctga gacgacagca 17340

gatatagttg tctttgatga aatttcaatg gccacaaatt atgatttgag tgttgtcaat 17400

gccagattac gtgctaagca ctatgtgtac attggcgacc ctgctcaatt acctgcacca 17460

cgcacattgc taactaaggg cacactagaa ccagaatatt tcaattcagt gtgtagactt 17520

atgaaaacta taggtccaga catgttcctc ggaacttgtc ggcgttgtcc tgctgaaatt 17580

gttgacactg tgagtgcttt ggtttatgat aataagctta aagcacataa agacaaatca 17640

gctcaatgct ttaaaatgtt ttataagggt gttatcacgc atgatgtttc atctgcaatt 17700

aacaggccac aaataggcgt ggtaagagaa ttccttacac gtaaccctgc ttggagaaaa 17760

gctgtcttta tttcacctta taattcacag aatgctgtag cctcaaagat tttgggacta 17820

ccaactcaaa ctgttgattc atcacagggc tcagaatatg actatgtcat attcactcaa 17880

accactgaaa cagctcactc ttgtaatgta aacagattta atgttgctat taccagagca 17940

aaagtaggca tactttgcat aatgtctgat agagaccttt atgacaagtt gcaatttaca 18000

agtcttgaaa ttccacgtag gaatgtggca actttacaag ctgaaaatgt aacaggactc 18060

tttaaagatt gtagtaaggt aatcactggg ttacatccta cacaggcacc tacacacctc 18120

agtgttgaca ctaaattcaa aactgaaggt ttatgtgttg acatacctgg catacctaag 18180

gacatgacct atagaagact catctctatg atgggtttta aaatgaatta tcaagttaat 18240

ggttacccta acatgtttat cacccgcgaa gaagctataa gacatgtacg tgcatggatt 18300

ggcttcgatg tcgaggggtg tcatgctact agagaagctg ttggtaccaa tttaccttta 18360

cagctaggtt tttctacagg tgttaaccta gttgctgtac ctacaggtta tgttgataca 18420

cctaataata cagatttttc cagagttagt gctaaaccac cgcctggaga tcaatttaaa 18480

cacctcatac cacttatgta caaaggactt ccttggaatg tagtgcgtat aaagattgta 18540

caaatgttaa gtgacacact taaaaatctc tctgacagag tcgtatttgt cttatgggca 18600

catggctttg agttgacatc tatgaagtat tttgtgaaaa taggacctga gcgcacctgt 18660

tgtctatgtg atagacgtgc cacatgcttt tccactgctt cagacactta tgcctgttgg 18720

catcattcta ttggatttga ttacgtctat aatccgttta tgattgatgt tcaacaatgg 18780

ggttttacag gtaacctaca aagcaaccat gatctgtatt gtcaagtcca tggtaatgca 18840

catgtagcta gttgtgatgc aatcatgact aggtgtctag ctgtccacga gtgctttgtt 18900

aagcgtgttg actggactat tgaatatcct ataattggtg atgaactgaa gattaatgcg 18960

gcttgtagaa aggttcaaca catggttgtt aaagctgcat tattagcaga caaattccca 19020

gttcttcacg acattggtaa ccctaaagct attaagtgtg tacctcaagc tgatgtagaa 19080

tggaagttct atgatgcaca gccttgtagt gacaaagctt ataaaataga agaattattc 19140

tattcttatg ccacacattc tgacaaattc acagatggtg tatgcctatt ttggaattgc 19200

aatgtcgata gatatcctgc taattccatt gtttgtagat ttgacactag agtgctatct 19260

aaccttaact tgcctggttg tgatggtggc agtttgtatg taaataaaca tgcattccac 19320

acaccagctt ttgataaaag tgcttttgtt aatttaaaac aattaccatt tttctattac 19380

tctgacagtc catgtgagtc tcatggaaaa caagtagtgt cagatataga ttatgtacca 19440

ctaaagtctg ctacgtgtat aacacgttgc aatttaggtg gtgctgtctg tagacatcat 19500

gctaatgagt acagattgta tctcgatgct tataacatga tgatctcagc tggctttagc 19560

ttgtgggttt acaaacaatt tgatacttat aacctctgga acacttttac aagacttcag 19620

agtttagaaa atgtggcttt taatgttgta aataagggac actttgatgg acaacagggt 19680

gaagtaccag tttctatcat taataacact gtttacacaa aagttgatgg tgttgatgta 19740

gaattgtttg aaaataaaac aacattacct gttaatgtag catttgagct ttgggctaag 19800

cgcaacatta aaccagtacc agaggtgaaa atactcaata atttgggtgt ggacattgct 19860

gctaatactg tgatctggga ctacaaaaga gatgctccag cacatatatc tactattggt 19920

gtttgttcta tgactgacat agccaagaaa ccaactgaaa cgatttgtgc accactcact 19980

gtcttttttg atggtagagt tgatggtcaa gtagacttat ttagaaatgc ccgtaatggt 20040

gttcttatta cagaaggtag tgttaaaggt ttacaaccat ctgtaggtcc caaacaagct 20100

agtcttaatg gagtcacatt aattggagaa gccgtaaaaa cacagttcaa ttattataag 20160

aaagttgatg gtgttgtcca acaattacct gaaacttact ttactcagag tagaaattta 20220

caagaattta aacccaggag tcaaatggaa attgatttct tagaattagc tatggatgaa 20280

ttcattgaac ggtataaatt agaaggctat gccttcgaac atatcgttta tggagatttt 20340

agtcatagtc agttaggtgg tttacatcta ctgattggac tagctaaacg ttttaaggaa 20400

tcaccttttg aattagaaga ttttattcct atggacagta cagttaaaaa ctatttcata 20460

acagatgcgc aaacaggttc atctaagtgt gtgtgttctg ttattgattt attacttgat 20520

gattttgttg aaataataaa atcccaagat ttatctgtag tttctaaggt tgtcaaagtg 20580

actattgact atacagaaat ttcatttatg ctttggtgta aagatggcca tgtagaaaca 20640

ttttacccaa aattacaatc tagtcaagcg tggcaaccgg gtgttgctat gcctaatctt 20700

tacaaaatgc aaagaatgct attagaaaag tgtgaccttc aaaattatgg tgatagtgca 20760

acattaccta aaggcataat gatgaatgtc gcaaaatata ctcaactgtg tcaatattta 20820

aacacattaa cattagctgt accctataat atgagagtta tacattttgg tgctggttct 20880

gataaaggag ttgcaccagg tacagctgtt ttaagacagt ggttgcctac gggtacgctg 20940

cttgtcgatt cagatcttaa tgactttgtc tctgatgcag attcaacttt gattggtgat 21000

tgtgcaactg tacatacagc taataaatgg gatctcatta ttagtgatat gtacgaccct 21060

aagactaaaa atgttacaaa agaaaatgac tctaaagagg gttttttcac ttacatttgt 21120

gggtttatac aacaaaagct agctcttgga ggttccgtgg ctataaagat aacagaacat 21180

tcttggaatg ctgatcttta taagctcatg ggacacttcg catggtggac agcctttgtt 21240

actaatgtga atgcgtcatc atctgaagca tttttaattg gatgtaatta tcttggcaaa 21300

ccacgcgaac aaatagatgg ttatgtcatg catgcaaatt acatattttg gaggaataca 21360

aatccaattc agttgtcttc ctattcttta tttgacatga gtaaatttcc ccttaaatta 21420

aggggtactg ctgttatgtc tttaaaagaa ggtcaaatca atgatatgat tttatctctt 21480

cttagtaaag gtagacttat aattagagaa aacaacagag ttgttatttc tagtgatgtt 21540

cttgttaaca actaaacgaa caatgtttgt ttttcttgtt ttattgccac tagtctctag 21600

tcagtgtgtt aatcttacaa ccagaactca attaccccct gcatacacta attctttcac 21660

acgtggtgtt tattaccctg acaaagtttt cagatcctca gttttacatt caactcagga 21720

cttgttctta cctttctttt ccaatgttac ttggttccat gctatacatg tctctgggac 21780

caatggtact aagaggtttg ataaccctgt cctaccattt aatgatggtg tttattttgc 21840

ttccactgag aagtctaaca taataagagg ctggattttt ggtactactt tagattcgaa 21900

gacccagtcc ctacttattg ttaataacgc tactaatgtt gttattaaag tctgtgaatt 21960

tcaattttgt aatgatccat ttttgggtgt ttattaccac aaaaacaaca aaagttggat 22020

ggaaagtgag ttcagagttt attctagtgc gaataattgc acttttgaat atgtctctca 22080

gccttttctt atggaccttg aaggaaaaca gggtaatttc aaaaatctta gggaatttgt 22140

gtttaagaat attgatggtt attttaaaat atattctaag cacacgccta ttaatttagt 22200

gcgtgatctc cctcagggtt tttcggcttt agaaccattg gtagatttgc caataggtat 22260

taacatcact aggtttcaaa ctttacttgc tttacataga agttatttga ctcctggtga 22320

ttcttcttca ggttggacag ctggtgctgc agcttattat gtgggttatc ttcaacctag 22380

gacttttcta ttaaaatata atgaaaatgg aaccattaca gatgctgtag actgtgcact 22440

tgaccctctc tcagaaacaa agtgtacgtt gaaatccttc actgtagaaa aaggaatcta 22500

tcaaacttct aactttagag tccaaccaac agaatctatt gttagatttc ctaatattac 22560

aaacttgtgc ccttttggtg aagtttttaa cgccaccaga tttgcatctg tttatgcttg 22620

gaacaggaag agaatcagca actgtgttgc tgattattct gtcctatata attccgcatc 22680

attttccact tttaagtgtt atggagtgtc tcctactaaa ttaaatgatc tctgctttac 22740

taatgtctat gcagattcat ttgtaattag aggtgatgaa gtcagacaaa tcgctccagg 22800

gcaaactgga aagattgctg attataatta taaattacca gatgatttta caggctgcgt 22860

tatagcttgg aattctaaca atcttgattc taaggttggt ggtaattata attacctgta 22920

tagattgttt aggaagtcta atctcaaacc ttttgagaga gatatttcaa ctgaaatcta 22980

tcaggccggt agcacacctt gtaatggtgt tgaaggtttt aattgttact ttcctttaca 23040

atcatatggt ttccaaccca ctaatggtgt tggttaccaa ccatacagag tagtagtact 23100

ttcttttgaa cttctacatg caccagcaac tgtttgtgga cctaaaaagt ctactaattt 23160

ggttaaaaac aaatgtgtca atttcaactt caatggttta acaggcacag gtgttcttac 23220

tgagtctaac aaaaagtttc tgcctttcca acaatttggc agagacattg ctgacactac 23280

tgatgctgtc cgtgatccac agacacttga gattcttgac attacaccat gttcttttgg 23340

tggtgtcagt gttataacac caggaacaaa tacttctaac caggttgctg ttctttatca 23400

ggatgttaac tgcacagaag tccctgttgc tattcatgca gatcaactta ctcctacttg 23460

gcgtgtttat tctacaggtt ctaatgtttt tcaaacacgt gcaggctgtt taataggggc 23520

tgaacatgtc aacaactcat atgagtgtga catacccatt ggtgcaggta tatgcgctag 23580

ttatcagact cagactaatt ctcctcggcg ggcacgtagt gtagctagtc aatccatcat 23640

tgcctacact atgtcacttg gtgcagaaaa ttcagttgct tactctaata actctattgc 23700

catacccaca aattttacta ttagtgttac cacagaaatt ctaccagtgt ctatgaccaa 23760

gacatcagta gattgtacaa tgtacatttg tggtgattca actgaatgca gcaatctttt 23820

gttgcaatat ggcagttttt gtacacaatt aaaccgtgct ttaactggaa tagctgttga 23880

acaagacaaa aacacccaag aagtttttgc acaagtcaaa caaatttaca aaacaccacc 23940

aattaaagat tttggtggtt ttaatttttc acaaatatta ccagatccat caaaaccaag 24000

caagaggtca tttattgaag atctactttt caacaaagtg acacttgcag atgctggctt 24060

catcaaacaa tatggtgatt gccttggtga tattgctgct agagacctca tttgtgcaca 24120

aaagtttaac ggccttactg ttttgccacc tttgctcaca gatgaaatga ttgctcaata 24180

cacttctgca ctgttagcgg gtacaatcac ttctggttgg acctttggtg caggtgctgc 24240

attacaaata ccatttgcta tgcaaatggc ttataggttt aatggtattg gagttacaca 24300

gaatgttctc tatgagaacc aaaaattgat tgccaaccaa tttaatagtg ctattggcaa 24360

aattcaagac tcactttctt ccacagcaag tgcacttgga aaacttcaag atgtggtcaa 24420

ccaaaatgca caagctttaa acacgcttgt taaacaactt agctccaatt ttggtgcaat 24480

ttcaagtgtt ttaaatgata tcctttcacg tcttgacaaa gttgaggctg aagtgcaaat 24540

tgataggttg atcacaggca gacttcaaag tttgcagaca tatgtgactc aacaattaat 24600

tagagctgca gaaatcagag cttctgctaa tcttgctgct actaaaatgt cagagtgtgt 24660

acttggacaa tcaaaaagag ttgatttttg tggaaagggc tatcatctta tgtccttccc 24720

tcagtcagca cctcatggtg tagtcttctt gcatgtgact tatgtccctg cacaagaaaa 24780

gaacttcaca actgctcctg ccatttgtca tgatggaaaa gcacactttc ctcgtgaagg 24840

tgtctttgtt tcaaatggca cacactggtt tgtaacacaa aggaattttt atgaaccaca 24900

aatcattact acagacaaca catttgtgtc tggtaactgt gatgttgtaa taggaattgt 24960

caacaacaca gtttatgatc ctttgcaacc tgaattagac tcattcaagg aggagttaga 25020

taaatatttt aagaatcata catcaccaga tgttgattta ggtgacatct ctggcattaa 25080

tgcttcagtt gtaaacattc aaaaagaaat tgaccgcctc aatgaggttg ccaagaattt 25140

aaatgaatct ctcatcgatc tccaagaact tggaaagtat gagcagtata taaaatggcc 25200

atggtacatt tggctaggtt ttatagctgg cttgattgcc atagtaatgg tgacaattat 25260

gctttgctgt atgaccagtt gctgtagttg tctcaagggc tgttgttctt gtggatcctg 25320

ctgcaaattt gatgaagacg actctgagcc agtgctcaaa ggagtcaaat tacattacac 25380

ataaacgaac ttatggattt gtttatgaga atcttcacaa ttggaactgt aactttgaag 25440

caaggtgaaa tcaaggatgc tactccttca gattttgttc gcgctactgc aacgataccg 25500

atacaagcct cactcccttt cggatggctt attgttggcg ttgcacttct tgctgttttt 25560

cagagcgctt ccaaaatcat aaccctcaaa aagagatggc aactagcact ctccaagggt 25620

gttcactttg tttgcaactt gctgttgttg tttgtaacag tttactcaca ccttttgctc 25680

gttgctgctg gccttgaagc cccttttctc tatctttatg ctttagtcta cttcttgcag 25740

agtataaact ttgtaagaat aataatgagg ctttggcttt gctggaaatg ccgttccaaa 25800

aacccattac tttatgatgc caactatttt ctttgctggc atactaattg ttacgactat 25860

tgtatacctt acaatagtgt aacttcttca attgtcatta cttcaggtga tggcacaaca 25920

agtcctattt ctgaacatga ctaccagatt ggtggttata ctgaaaaatg ggaatctgga 25980

gtaaaagact gtgttgtatt acacagttac ttcacttcag actattacca gctgtactca 26040

actcaattga gtacagacac tggtgttgaa catgttacct tcttcatcta caataaaatt 26100

gttgatgagc ctgaagaaca tgtccaaatt cacacaatcg acggttcatc cggagttgtt 26160

aatccagtaa tggaaccaat ttatgatgaa ccgacgacga ctactagcgt gcctttgtaa 26220

gcacaagctg atgagtacga acttatgtac tcattcgttt cggaagagac aggtacgtta 26280

atagttaata gcgtacttct ttttcttgct ttcgtggtat tcttgctagt tacactagcc 26340

atccttactg cgcttcgatt gtgtgcgtac tgctgcaata ttgttaacgt gagtcttgta 26400

aaaccttctt tttacgttta ctctcgtgtt aaaaatctga attcttctag agttcctgat 26460

cttctggtct aaacgaacta aatattatat tagtttttct gtttggaact ttaattttag 26520

ccatggcaga ttccaacggt actattaccg ttgaagagct taaaaagctc cttgaacaat 26580

ggaacctagt aataggtttc ctattcctta catggatttg tcttctacaa tttgcctatg 26640

ccaacaggaa taggtttttg tatataatta agttaatttt cctctggctg ttatggccag 26700

taactttagc ttgttttgtg cttgctgctg tttacagaat aaattggatc accggtggaa 26760

ttgctatcgc aatggcttgt cttgtaggct tgatgtggct cagctacttc attgcttctt 26820

tcagactgtt tgcgcgtacg cgttccatgt ggtcattcaa tccagaaact aacattcttc 26880

tcaacgtgcc actccatggc actattctga ccagaccgct tctagaaagt gaactcgtaa 26940

tcggagctgt gatccttcgt ggacatcttc gtattgctgg acaccatcta ggacgctgtg 27000

acatcaagga cctgcctaaa gaaatcactg ttgctacatc acgaacgctt tcttattaca 27060

aattgggagc ttcgcagcgt gtagcaggtg actcaggttt tgctgcatac agtcgctaca 27120

ggattggcaa ctataaatta aacacagacc attccagtag cagtgacaat attgctttgc 27180

ttgtacagta agtgacaaca gatgtttcat ctcgttgact ttcaggttac tatagcagag 27240

atattactaa ttattatgag gacttttaaa gtttccattt ggaatcttga ttacatcata 27300

aacctcataa ttaaaaattt atctaagtca ctaactgaga ataaatattc tcaattagat 27360

gaagagcaac caatggagat tgattaaacg aacatgaaaa ttattctttt cttggcactg 27420

ataacactcg ctacttgtga gctttatcac taccaagagt gtgttagagg tacaacagta 27480

cttttaaaag aaccttgctc ttctggaaca tacgagggca attcaccatt tcatcctcta 27540

gctgataaca aatttgcact gacttgcttt agcactcaat ttgcttttgc ttgtcctgac 27600

ggcgtaaaac acgtctatca gttacgtgcc agatcagttt cacctaaact gttcatcaga 27660

caagaggaag ttcaagaact ttactctcca atttttctta ttgttgcggc aatagtgttt 27720

ataacacttt gcttcacact caaaagaaag acagaatgat tgaactttca ttaattgact 27780

tctatttgtg ctttttagcc tttctgctat tccttgtttt aattatgctt attatctttt 27840

ggttctcact tgaactgcaa gatcataatg aaacttgtca cgcctaaacg aacatgaaat 27900

ttcttgtttt cttaggaatc atcacaactg tagctgcatt tcaccaagaa tgtagtttac 27960

agtcatgtac tcaacatcaa ccatatgtag ttgatgaccc gtgtcctatt cacttctatt 28020

ctaaatggta tattagagta ggagctagaa aatcagcacc tttaattgaa ttgtgcgtgg 28080

atgaggctgg ttctaaatca cccattcagt acatcgatat cggtaattat acagtttcct 28140

gtttaccttt tacaattaat tgccaggaac ctaaattggg tagtcttgta gtgcgttgtt 28200

cgttctatga agacttttta gagtatcatg acgttcgtgt tgttttagat ttcatctaaa 28260

cgaacaaact aaaatgtctg ataatggacc ccaaaatcag cgaaatgcac cccgcattac 28320

gtttggtgga ccctcagatt caactggcag taaccagaat ggagaacgca gtggggcgcg 28380

atcaaaacaa cgtcggcccc aaggtttacc caataatact gcgtcttggt tcaccgctct 28440

cactcaacat ggcaaggaag accttaaatt ccctcgagga caaggcgttc caattaacac 28500

caatagcagt ccagatgacc aaattggcta ctaccgaaga gctaccagac gaattcgtgg 28560

tggtgacggt aaaatgaaag atctcagtcc aagatggtat ttctactacc taggaactgg 28620

gccagaagct ggacttccct atggtgctaa caaagacggc atcatatggg ttgcaactga 28680

gggagccttg aatacaccaa aagatcacat tggcacccgc aatcctgcta acaatgctgc 28740

aatcgtgcta caacttcctc aaggaacaac attgccaaaa ggcttctacg cagaagggag 28800

cagaggcggc agtcaagcct cttctcgttc ctcatcacgt agtcgcaaca gttcaagaaa 28860

ttcaactcca ggcagcagta ggggaacttc tcctgctaga atggctggca atggcggtga 28920

tgctgctctt gctttgctgc tgcttgacag attgaaccag cttgagagca aaatgtctgg 28980

taaaggccaa caacaacaag gccaaactgt cactaagaaa tctgctgctg aggcttctaa 29040

gaagcctcgg caaaaacgta ctgccactaa agcatacaat gtaacacaag ctttcggcag 29100

acgtggtcca gaacaaaccc aaggaaattt tggggaccag gaactaatca gacaaggaac 29160

tgattacaaa cattggccgc aaattgcaca atttgccccc agcgcttcag cgttcttcgg 29220

aatgtcgcgc attggcatgg aagtcacacc ttcgggaacg tggttgacct acacaggtgc 29280

catcaaattg gatgacaaag atccaaattt caaagatcaa gtcattttgc tgaataagca 29340

tattgacgca tacaaaacat tcccaccaac agagcctaaa aaggacaaaa agaagaaggc 29400

tgatgaaact caagccttac cgcagagaca gaagaaacag caaactgtga ctcttcttcc 29460

tgctgcagat ttggatgatt tctccaaaca attgcaacaa tccatgagca gtgctgactc 29520

aactcaggcc taaactcatg cagaccacac aaggcagatg ggctatataa acgttttcgc 29580

ttttccgttt acgatatata gtctactctt gtgcagaatg aattctcgta actacatagc 29640

acaagtagat gtagttaact ttaatctcac atagcaatct ttaatcagtg tgtaacatta 29700

gggaggactt gaaagagcca ccacattttc accgaggcca cgcggagtac gatcgagtgt 29760

acagtgaaca atgctaggga gagctgccta tatggaagag ccctaatgtg taaaattaat 29820

tttagtagtg ctatccccat gtgattttaa tagcttctta ggagaatgac aaaaaaaaaa 29880

aaaaaaaaaa aaaaaaaaaa aaa 29903

<210> 14
<211> 15
<212> PRT
<213> Artificial Sequence

<220>
<223> 30-44 of SEQ ID NO1

<400> 14

Ser Ser Val Leu His Ser Thr Gln Asp Leu Phe Leu Pro Phe Phe
1               5                   10                  15

<210> 15
<211> 21
<212> PRT
<213> Artificial Sequence

<220>
<223> 48-68 of SEQ ID NO1

<400> 15

Thr Trp Phe His Ala Ile His Val Ser Gly Thr Asn Gly Thr Lys Arg
1               5                   10                  15

Phe Asp Asn Pro Val
            20

<210>  16
<211>  57
<212>  PRT
<213>  Artificial Sequence

<220>
<223>  110-166 of SEQ ID NO1

<400>  16

Asn Val Val Ile Lys Val Cys Glu Phe Gln Phe Cys Asn Asp Pro Phe
1               5                   10                  15

Leu Gly Val Tyr Tyr His Lys Asn Asn Lys Ser Trp Met Glu Ser Glu
            20                  25                  30

Phe Arg Val Tyr Ser Ser Ala Asn Asn Cys Thr Phe Glu Tyr Val Ser
        35                  40                  45

Gln Pro Phe Leu Met Asp Leu Glu Gly
    50                  55

<210>  17
<211>  15
<212>  PRT
<213>  Artificial Sequence

<220>
<223>  200-214 of SEQ ID NO1

<400>  17

Asp Leu Pro Gln Gly Phe Ser Ala Leu Glu Pro Leu Val Asp Leu
1               5                   10                  15

<210>  18
<211>  25
<212>  PRT
<213>  Artificial Sequence

<220>
<223>  226-250 of SEQ ID NO1

<400>  18

Leu Leu Ala Leu His Arg Ser Tyr Leu Thr Pro Gly Asp Ser Ser Ser
1               5                   10                  15

Gly Trp Thr Ala Gly Ala Ala Ala Tyr
            20                  25

<210>  19
<211>  25
<212>  PRT
<213>  Artificial Sequence

<220>
<223>  256-277 of SEQ ID NO1

<400>  19

Gln Pro Arg Thr Phe Leu Leu Lys Tyr Asn Glu Asn Gly Thr Ile Thr
1               5                   10                  15

Asp Ala Val Asp Cys Ala Leu Asp Pro
            20                  25

<210>  20
<211>  15
<212>  PRT
<213>  Artificial Sequence

<220>
<223>  328-342 of SEQ ID NO1

<400>  20

Asn Ala Thr Arg Phe Ala Ser Val Tyr Ala Trp Asn Arg Lys Arg
1               5                   10                  15

<210>  21
<211>  15
<212>  PRT
<213>  Artificial Sequence

<220>
<223>  399-414 of SEQ ID NO1

<400>  21

Thr Gly Lys Ile Ala Asp Tyr Asn Tyr Lys Leu Pro Asp Asp Phe
1               5                   10                  15

<210>  22
<211>  15
<212>  PRT
<213>  Artificial Sequence

<220>
<223>  434-448 of SEQ ID NO1

<400>  22

Tyr Asn Tyr Leu Tyr Arg Leu Phe Arg Lys Ser Asn Leu Lys Pro
1               5                   10                  15

<210>  23
<211>  23
<212>  PRT
<213>  Artificial Sequence

<220>
<223>  550-572 of SEQ ID NO1

<400>  23

Phe Gly Arg Asp Ile Ala Asp Thr Thr Asp Ala Val Arg Asp Pro Gln
1               5                   10                  15

Thr Leu Glu Ile Leu Asp Ile
            20

<210>  24
<211>  15
<212>  PRT
<213>  Artificial Sequence

<220>
<223>  590-604 of SEQ ID NO1

<400>  24

Ser Asn Gln Val Ala Val Leu Tyr Gln Asp Val Asn Cys Thr Glu
1               5                   10                  15

<210>  25
<211>  19
<212>  PRT
<213>  Artificial Sequence

<220>
<223>  632-650 of SEQ ID NO1

<400>  25

Ala Gly Cys Leu Ile Gly Ala Glu His Val Asn Asn Ser Tyr Glu Cys
1               5                   10                  15

Asp Ile Pro

<210>  26
<211>  15
<212>  PRT
<213>  Artificial Sequence

<220>
<223>  354-368 of SEQ ID NO1

<400>  26

Tyr Asn Ser Ala Ser Phe Ser Thr Phe Lys Cys Tyr Gly Val Ser
1               5                   10                  15

<210>  27
<211>  15
<212>  PRT
<213>  Artificial Sequence

<220>
<223>  622-636 of SEQ ID NO1

<400>  27

Ser Thr Gly Ser Asn Val Phe Gln Thr Arg Ala Gly Cys Leu Ile
1               5                   10                  15


<210>  28
<211>  15
<212>  PRT
<213>  Artificial Sequence

<220>
<223>  30-44 of SEQ ID NO1

<400>  28

Ser Ser Val Leu His Ser Thr Gln Asp Leu Phe Leu Pro Phe Phe
1               5                   10                  15


<210>  29
<211>  25
<212>  PRT
<213>  Artificial Sequence

<220>
<223>  226-250 of SEQ ID NO1

<400>  29

Leu Leu Ala Leu His Arg Ser Tyr Leu Thr Pro Gly Asp Ser Ser Ser
1               5                   10                  15


Gly Trp Thr Ala Gly Ala Ala Ala Tyr
            20                  25


<210>  30
<211>  419
<212>  PRT
<213>  SARS-CoV-2

<400>  30

Met Ser Asp Asn Gly Pro Gln Asn Gln Arg Asn Ala Pro Arg Ile Thr
1               5                   10                  15


Phe Gly Gly Pro Ser Asp Ser Thr Gly Ser Asn Gln Asn Gly Glu Arg
            20                  25                  30


Ser Gly Ala Arg Ser Lys Gln Arg Arg Pro Gln Gly Leu Pro Asn Asn
        35                  40                  45


Thr Ala Ser Trp Phe Thr Ala Leu Thr Gln His Gly Lys Glu Asp Leu
    50                  55                  60


Lys Phe Pro Arg Gly Gln Gly Val Pro Ile Asn Thr Asn Ser Ser Pro

65                  70                  75                  80

Asp Asp Gln Ile Gly Tyr Tyr Arg Arg Ala Thr Arg Arg Ile Arg Gly
                85                  90                  95

Gly Asp Gly Lys Met Lys Asp Leu Ser Pro Arg Trp Tyr Phe Tyr Tyr
            100                 105                 110

Leu Gly Thr Gly Pro Glu Ala Gly Leu Pro Tyr Gly Ala Asn Lys Asp
        115                 120                 125

Gly Ile Ile Trp Val Ala Thr Glu Gly Ala Leu Asn Thr Pro Lys Asp
    130                 135                 140

His Ile Gly Thr Arg Asn Pro Ala Asn Asn Ala Ala Ile Val Leu Gln
145                 150                 155                 160

Leu Pro Gln Gly Thr Thr Leu Pro Lys Gly Phe Tyr Ala Glu Gly Ser
                165                 170                 175

Arg Gly Gly Ser Gln Ala Ser Ser Arg Ser Ser Ser Arg Ser Arg Asn
            180                 185                 190

Ser Ser Arg Asn Ser Thr Pro Gly Ser Ser Arg Gly Thr Ser Pro Ala
        195                 200                 205

Arg Met Ala Gly Asn Gly Gly Asp Ala Ala Leu Ala Leu Leu Leu Leu
    210                 215                 220

Asp Arg Leu Asn Gln Leu Glu Ser Lys Met Ser Gly Lys Gly Gln Gln
225                 230                 235                 240

Gln Gln Gly Gln Thr Val Thr Lys Lys Ser Ala Ala Glu Ala Ser Lys
                245                 250                 255

Lys Pro Arg Gln Lys Arg Thr Ala Thr Lys Ala Tyr Asn Val Thr Gln
            260                 265                 270

Ala Phe Gly Arg Arg Gly Pro Glu Gln Thr Gln Gly Asn Phe Gly Asp
        275                 280                 285

Gln Glu Leu Ile Arg Gln Gly Thr Asp Tyr Lys His Trp Pro Gln Ile
    290                 295                 300

Ala Gln Phe Ala Pro Ser Ala Ser Ala Phe Phe Gly Met Ser Arg Ile
305                 310                 315                 320

Gly Met Glu Val Thr Pro Ser Gly Thr Trp Leu Thr Tyr Thr Gly Ala
                325                 330                 335

Ile Lys Leu Asp Asp Lys Asp Pro Asn Phe Lys Asp Gln Val Ile Leu
            340                 345                 350

Leu Asn Lys His Ile Asp Ala Tyr Lys Thr Phe Pro Pro Thr Glu Pro
        355                 360                 365

Lys Lys Asp Lys Lys Lys Lys Ala Asp Glu Thr Gln Ala Leu Pro Gln
    370                 375                 380

Arg Gln Lys Lys Gln Gln Thr Val Thr Leu Leu Pro Ala Ala Asp Leu
385                 390                 395                 400

Asp Asp Phe Ser Lys Gln Leu Gln Gln Ser Met Ser Ser Ala Asp Ser
                405                 410                 415

Thr Gln Ala

<210>  31
<211>  223
<212>  PRT
<213>  Artificial Sequence

<220>
<223>  RBD, a fragment from S1 domain from SARS-CoV-2 S protein

<400>  31

Arg Val Gln Pro Thr Glu Ser Ile Val Arg Phe Pro Asn Ile Thr Asn
1               5                   10                  15

Leu Cys Pro Phe Gly Glu Val Phe Asn Ala Thr Arg Phe Ala Ser Val
            20                  25                  30

Tyr Ala Trp Asn Arg Lys Arg Ile Ser Asn Cys Val Ala Asp Tyr Ser
        35                  40                  45

Val Leu Tyr Asn Ser Ala Ser Phe Ser Thr Phe Lys Cys Tyr Gly Val
    50                  55                  60

Ser Pro Thr Lys Leu Asn Asp Leu Cys Phe Thr Asn Val Tyr Ala Asp
65                  70                  75                  80

Ser Phe Val Ile Arg Gly Asp Glu Val Arg Gln Ile Ala Pro Gly Gln
                85                  90                  95

Thr Gly Lys Ile Ala Asp Tyr Asn Tyr Lys Leu Pro Asp Asp Phe Thr

            100                 105                 110

Gly Cys Val Ile Ala Trp Asn Ser Asn Asn Leu Asp Ser Lys Val Gly
        115                 120                 125

Gly Asn Tyr Asn Tyr Leu Tyr Arg Leu Phe Arg Lys Ser Asn Leu Lys
    130                 135                 140

Pro Phe Glu Arg Asp Ile Ser Thr Glu Ile Tyr Gln Ala Gly Ser Thr
145                 150                 155                 160

Pro Cys Asn Gly Val Glu Gly Phe Asn Cys Tyr Phe Pro Leu Gln Ser
                165                 170                 175

Tyr Gly Phe Gln Pro Thr Asn Gly Val Gly Tyr Gln Pro Tyr Arg Val
            180                 185                 190

Val Val Leu Ser Phe Glu Leu Leu His Ala Pro Ala Thr Val Cys Gly
        195                 200                 205

Pro Lys Lys Ser Thr Asn Leu Val Lys Asn Lys Cys Val Asn Phe
    210                 215                 220

<210> 32
<211> 233
<212> PRT
<213> Artificial Sequence

<220>
<223> RBD, a fragment from S1 domain from SARS-CoV-2 S protein, with
C-terminal His tag

<400> 32

Arg Val Gln Pro Thr Glu Ser Ile Val Arg Phe Pro Asn Ile Thr Asn
1               5                   10                  15

Leu Cys Pro Phe Gly Glu Val Phe Asn Ala Thr Arg Phe Ala Ser Val
            20                  25                  30

Tyr Ala Trp Asn Arg Lys Arg Ile Ser Asn Cys Val Ala Asp Tyr Ser
        35                  40                  45

Val Leu Tyr Asn Ser Ala Ser Phe Ser Thr Phe Lys Cys Tyr Gly Val
    50                  55                  60

Ser Pro Thr Lys Leu Asn Asp Leu Cys Phe Thr Asn Val Tyr Ala Asp
65                  70                  75                  80

Ser Phe Val Ile Arg Gly Asp Glu Val Arg Gln Ile Ala Pro Gly Gln

85                  90                  95

Thr Gly Lys Ile Ala Asp Tyr Asn Tyr Lys Leu Pro Asp Asp Phe Thr
            100                 105                 110

Gly Cys Val Ile Ala Trp Asn Ser Asn Asn Leu Asp Ser Lys Val Gly
        115                 120                 125

Gly Asn Tyr Asn Tyr Leu Tyr Arg Leu Phe Arg Lys Ser Asn Leu Lys
    130                 135                 140

Pro Phe Glu Arg Asp Ile Ser Thr Glu Ile Tyr Gln Ala Gly Ser Thr
145                 150                 155                 160

Pro Cys Asn Gly Val Glu Gly Phe Asn Cys Tyr Phe Pro Leu Gln Ser
                165                 170                 175

Tyr Gly Phe Gln Pro Thr Asn Gly Val Gly Tyr Gln Pro Tyr Arg Val
            180                 185                 190

Val Val Leu Ser Phe Glu Leu Leu His Ala Pro Ala Thr Val Cys Gly
        195                 200                 205

Pro Lys Lys Ser Thr Asn Leu Val Lys Asn Lys Cys Val Asn Phe Leu
    210                 215                 220

Glu His His His His His His His His
225                 230

<210>  33
<211>  588
<212>  PRT
<213>  Artificial Sequence

<220>
<223>  S2 domain from SARS-CoV-2 S protein

<400>  33

Ser Val Ala Ser Gln Ser Ile Ile Ala Tyr Thr Met Ser Leu Gly Ala
1               5                   10                  15

Glu Asn Ser Val Ala Tyr Ser Asn Asn Ser Ile Ala Ile Pro Thr Asn
            20                  25                  30

Phe Thr Ile Ser Val Thr Thr Glu Ile Leu Pro Val Ser Met Thr Lys
        35                  40                  45

Thr Ser Val Asp Cys Thr Met Tyr Ile Cys Gly Asp Ser Thr Glu Cys
    50                  55                  60

Ser Asn Leu Leu Leu Gln Tyr Gly Ser Phe Cys Thr Gln Leu Asn Arg
65                  70                  75                  80

Ala Leu Thr Gly Ile Ala Val Glu Gln Asp Lys Asn Thr Gln Glu Val
                85                  90                  95

Phe Ala Gln Val Lys Gln Ile Tyr Lys Thr Pro Pro Ile Lys Asp Phe
            100                 105                 110

Gly Gly Phe Asn Phe Ser Gln Ile Leu Pro Asp Pro Ser Lys Pro Ser
        115                 120                 125

Lys Arg Ser Phe Ile Glu Asp Leu Leu Phe Asn Lys Val Thr Leu Ala
    130                 135                 140

Asp Ala Gly Phe Ile Lys Gln Tyr Gly Asp Cys Leu Gly Asp Ile Ala
145                 150                 155                 160

Ala Arg Asp Leu Ile Cys Ala Gln Lys Phe Asn Gly Leu Thr Val Leu
                165                 170                 175

Pro Pro Leu Leu Thr Asp Glu Met Ile Ala Gln Tyr Thr Ser Ala Leu
            180                 185                 190

Leu Ala Gly Thr Ile Thr Ser Gly Trp Thr Phe Gly Ala Gly Ala Ala
        195                 200                 205

Leu Gln Ile Pro Phe Ala Met Gln Met Ala Tyr Arg Phe Asn Gly Ile
    210                 215                 220

Gly Val Thr Gln Asn Val Leu Tyr Glu Asn Gln Lys Leu Ile Ala Asn
225                 230                 235                 240

Gln Phe Asn Ser Ala Ile Gly Lys Ile Gln Asp Ser Leu Ser Ser Thr
                245                 250                 255

Ala Ser Ala Leu Gly Lys Leu Gln Asp Val Val Asn Gln Asn Ala Gln
            260                 265                 270

Ala Leu Asn Thr Leu Val Lys Gln Leu Ser Ser Asn Phe Gly Ala Ile
        275                 280                 285

Ser Ser Val Leu Asn Asp Ile Leu Ser Arg Leu Asp Lys Val Glu Ala
    290                 295                 300

Glu Val Gln Ile Asp Arg Leu Ile Thr Gly Arg Leu Gln Ser Leu Gln

305                 310                 315                 320

Thr Tyr Val Thr Gln Gln Leu Ile Arg Ala Ala Glu Ile Arg Ala Ser
                325                 330                 335

Ala Asn Leu Ala Ala Thr Lys Met Ser Glu Cys Val Leu Gly Gln Ser
            340                 345                 350

Lys Arg Val Asp Phe Cys Gly Lys Gly Tyr His Leu Met Ser Phe Pro
        355                 360                 365

Gln Ser Ala Pro His Gly Val Val Phe Leu His Val Thr Tyr Val Pro
    370                 375                 380

Ala Gln Glu Lys Asn Phe Thr Thr Ala Pro Ala Ile Cys His Asp Gly
385                 390                 395                 400

Lys Ala His Phe Pro Arg Glu Gly Val Phe Val Ser Asn Gly Thr His
                405                 410                 415

Trp Phe Val Thr Gln Arg Asn Phe Tyr Glu Pro Gln Ile Ile Thr Thr
            420                 425                 430

Asp Asn Thr Phe Val Ser Gly Asn Cys Asp Val Val Ile Gly Ile Val
        435                 440                 445

Asn Asn Thr Val Tyr Asp Pro Leu Gln Pro Glu Leu Asp Ser Phe Lys
    450                 455                 460

Glu Glu Leu Asp Lys Tyr Phe Lys Asn His Thr Ser Pro Asp Val Asp
465                 470                 475                 480

Leu Gly Asp Ile Ser Gly Ile Asn Ala Ser Val Val Asn Ile Gln Lys
                485                 490                 495

Glu Ile Asp Arg Leu Asn Glu Val Ala Lys Asn Leu Asn Glu Ser Leu
            500                 505                 510

Ile Asp Leu Gln Glu Leu Gly Lys Tyr Glu Gln Tyr Ile Lys Trp Pro
        515                 520                 525

Trp Tyr Ile Trp Leu Gly Phe Ile Ala Gly Leu Ile Ala Ile Val Met
    530                 535                 540

Val Thr Ile Met Leu Cys Cys Met Thr Ser Cys Cys Ser Cys Leu Lys
545                 550                 555                 560

Gly Cys Cys Ser Cys Gly Ser Cys Cys Lys Phe Asp Glu Asp Asp Ser
                565                 570                 575

Glu Pro Val Leu Lys Gly Val Lys Leu His Tyr Thr
            580                 585

<210>  34
<211>  255
<212>  PRT
<213>  Artificial Sequence

<220>
<223>  RBD as used in the examples

<400>  34

Met Lys His Leu Trp Phe Phe Leu Leu Leu Val Ala Ala Pro Arg Trp
1               5                   10                  15

Val Leu Ser Gly Pro Met Arg Val Gln Pro Thr Glu Ser Ile Val Arg
            20                  25                  30

Phe Pro Asn Ile Thr Asn Leu Cys Pro Phe Gly Glu Val Phe Asn Ala
        35                  40                  45

Thr Arg Phe Ala Ser Val Tyr Ala Trp Asn Arg Lys Arg Ile Ser Asn
    50                  55                  60

Cys Val Ala Asp Tyr Ser Val Leu Tyr Asn Ser Ala Ser Phe Ser Thr
65                  70                  75                  80

Phe Lys Cys Tyr Gly Val Ser Pro Thr Lys Leu Asn Asp Leu Cys Phe
                85                  90                  95

Thr Asn Val Tyr Ala Asp Ser Phe Val Ile Arg Gly Asp Glu Val Arg
            100                 105                 110

Gln Ile Ala Pro Gly Gln Thr Gly Lys Ile Ala Asp Tyr Asn Tyr Lys
        115                 120                 125

Leu Pro Asp Asp Phe Thr Gly Cys Val Ile Ala Trp Asn Ser Asn Asn
    130                 135                 140

Leu Asp Ser Lys Val Gly Gly Asn Tyr Asn Tyr Leu Tyr Arg Leu Phe
145                 150                 155                 160

Arg Lys Ser Asn Leu Lys Pro Phe Glu Arg Asp Ile Ser Thr Glu Ile
                165                 170                 175

Tyr Gln Ala Gly Ser Thr Pro Cys Asn Gly Val Glu Gly Phe Asn Cys

180                 185                 190

Tyr Phe Pro Leu Gln Ser Tyr Gly Phe Gln Pro Thr Asn Gly Val Gly
        195                 200                 205

Tyr Gln Pro Tyr Arg Val Val Val Leu Ser Phe Glu Leu Leu His Ala
    210                 215                 220

Pro Ala Thr Val Cys Gly Pro Lys Lys Ser Thr Asn Leu Val Lys Asn
225                 230                 235                 240

Lys Cys Val Asn Phe Leu Glu His His His His His His His His
                245                 250                 255

<210>  35
<211>  13
<212>  PRT
<213>  Artificial Sequence

<220>
<223>  SEQ ID NO: 1-derived peptide reactive with SARS-CoV-2 antibodies

<400>  35

Leu Thr Pro Gly Asp Ser Ser Ser Gly Trp Thr Ala Gly
1               5                   10

<210>  36
<211>  11
<212>  PRT
<213>  Artificial Sequence

<220>
<223>  SEQ ID NO1-derived peptide reactive with SARS-CoV-2 antibodies

<400>  36

Tyr Gln Ala Gly Ser Thr Pro Cys Asn Gly Val
1               5                   10

<210>  37
<211>  12
<212>  PRT
<213>  Artificial Sequence

<220>
<223>  SEQ ID NO: 1-derived peptide reactive with SARS-CoV-2 antibodies

<400>  37

Tyr Gly Phe Gln Pro Thr Asn Gly Val Gly Tyr Gln
1               5                   10

<210>  38
<211>  255

<212>  PRT
<213>  Artificial Sequence

<220>
<223>  His-tagged RBD

<400>  38

Met Lys His Leu Trp Phe Phe Leu Leu Leu Val Ala Ala Pro Arg Trp
1               5                   10                  15

Val Leu Ser Gly Pro Met Arg Val Gln Pro Thr Glu Ser Ile Val Arg
            20                  25                  30

Phe Pro Asn Ile Thr Asn Leu Cys Pro Phe Gly Glu Val Phe Asn Ala
        35                  40                  45

Thr Arg Phe Ala Ser Val Tyr Ala Trp Asn Arg Lys Arg Ile Ser Asn
    50                  55                  60

Cys Val Ala Asp Tyr Ser Val Leu Tyr Asn Ser Ala Ser Phe Ser Thr
65                  70                  75                  80

Phe Lys Cys Tyr Gly Val Ser Pro Thr Lys Leu Asn Asp Leu Cys Phe
                85                  90                  95

Thr Asn Val Tyr Ala Asp Ser Phe Val Ile Arg Gly Asp Glu Val Arg
            100                 105                 110

Gln Ile Ala Pro Gly Gln Thr Gly Lys Ile Ala Asp Tyr Asn Tyr Lys
        115                 120                 125

Leu Pro Asp Asp Phe Thr Gly Cys Val Ile Ala Trp Asn Ser Asn Asn
    130                 135                 140

Leu Asp Ser Lys Val Gly Gly Asn Tyr Asn Tyr Leu Tyr Arg Leu Phe
145                 150                 155                 160

Arg Lys Ser Asn Leu Lys Pro Phe Glu Arg Asp Ile Ser Thr Glu Ile
                165                 170                 175

Tyr Gln Ala Gly Ser Thr Pro Cys Asn Gly Val Glu Gly Phe Asn Cys
            180                 185                 190

Tyr Phe Pro Leu Gln Ser Tyr Gly Phe Gln Pro Thr Asn Gly Val Gly
        195                 200                 205

Tyr Gln Pro Tyr Arg Val Val Val Leu Ser Phe Glu Leu Leu His Ala
    210                 215                 220

Pro Ala Thr Val Cys Gly Pro Lys Lys Ser Thr Asn Leu Val Lys Asn
225                 230                 235                 240

Lys Cys Val Asn Phe Leu Glu His His His His His His His His
                245                 250                 255

<210>  39
<211>  805
<212>  PRT
<213>  Homo sapiens

<400>  39

<210>  40
<211>  11
<212>  PRT
<213>  Artificial Sequence

<220>
<223>  SEQ ID NO: 1-derived peptide reactive with SARS-CoV-2 antibodies

<400>  40

Arg Thr Trp Leu Pro Pro Ala Tyr Thr Asn Ser
1               5                   10

<210>  41
<211>  11
<212>  PRT
<213>  Artificial Sequence

<220>
<223>  SEQ ID NO: 1-derived peptide reactive with SARS-CoV-2 antibodies

<400>  41

Arg Thr Gln Leu Pro Pro Ala Tyr Thr Asn Ser
1               5                   10

<210>  42
<211>  11
<212>  PRT
<213>  Artificial Sequence

<220>
<223>  SEQ ID NO: 1-derived peptide reactive with SARS-CoV-2 antibodies

<400>  42

Ser Gly Thr Asn Gly Thr Lys Arg Phe Asp Asn
1               5                   10

<210>  43
<211>  143
<212>  PRT
<213>  Artificial Sequence

<220>
<223>  SEQ ID NO: 1-derived peptide reactive with SARS-CoV-2 antibodies

<400>  43

Met Ser His His His His His His His His Ser Pro Met Tyr Ser Ile
1               5                   10                  15

Ile Thr Pro Asn Ile Leu Arg Leu Glu Ser Glu Glu Thr Met Val Leu
            20                  25                  30

Glu Ala His Asp Ala Gln Gly Asp Val Pro Val Thr Val Thr Val His
        35                  40                  45

Asp Phe Pro Gly Lys Lys Leu Val Leu Ser Ser Glu Lys Thr Val Leu
    50                  55                  60

Thr Pro Ala Thr Asn His Met Gly Asn Val Thr Phe Thr Ile Pro Ala
65                  70                  75                  80

Asn Arg Glu Phe Lys Ser Glu Lys Gly Arg Asn Lys Phe Val Thr Val
                85                  90                  95

Gln Ala Thr Phe Gly Thr Gln Val Val Glu Lys Val Val Leu Val Ser
            100                 105                 110

Leu Gln Ser Gly Ile Glu Gly Arg Met Arg Thr Gln Leu Pro Pro Ala
        115                 120                 125

Tyr Thr Asn Ser Arg Thr Gln Leu Pro Pro Ala Tyr Thr Asn Ser
    130                 135                 140

<210>  44
<211>  264
<212>  PRT
<213>  Artificial Sequence

<220>
<223>  SEQ ID NO: 1-derived peptide reactive with SARS-CoV-2 antibodies

with GST fusion

<400> 44

Met Ser His His His His His His His His Ser Pro Met Tyr Ser Ile
1               5                   10                  15

Ile Thr Pro Asn Ile Leu Arg Leu Glu Ser Glu Glu Thr Met Val Leu
            20                  25                  30

Glu Ala His Asp Ala Gln Gly Asp Val Pro Val Thr Val Thr Val His
        35                  40                  45

Asp Phe Pro Gly Lys Lys Leu Val Leu Ser Ser Glu Lys Thr Val Leu
    50                  55                  60

Thr Pro Ala Thr Asn His Met Gly Asn Val Thr Phe Thr Ile Pro Ala
65                  70                  75                  80

Asn Arg Glu Phe Lys Ser Glu Lys Gly Arg Asn Lys Phe Val Thr Val
                85                  90                  95

Gln Ala Thr Phe Gly Thr Gln Val Val Glu Lys Val Val Leu Val Ser
            100                 105                 110

Leu Gln Ser Gly Ile Glu Gly Arg Met Met Ser His His His His His
        115                 120                 125

His His His Ser Pro Met Tyr Ser Ile Ile Thr Pro Asn Ile Leu Arg
    130                 135                 140

Leu Glu Ser Glu Glu Thr Met Val Leu Glu Ala His Asp Ala Gln Gly
145                 150                 155                 160

Asp Val Pro Val Thr Val Thr Val His Asp Phe Pro Gly Lys Lys Leu
                165                 170                 175

Val Leu Ser Ser Glu Lys Thr Val Leu Thr Pro Ala Thr Asn His Met
            180                 185                 190

Gly Asn Val Thr Phe Thr Ile Pro Ala Asn Arg Glu Phe Lys Ser Glu
        195                 200                 205

Lys Gly Arg Asn Lys Phe Val Thr Val Gln Ala Thr Phe Gly Thr Gln
    210                 215                 220

Val Val Glu Lys Val Val Leu Val Ser Leu Gln Ser Gly Ile Glu Gly
225                 230                 235                 240

Arg Met Ser Gly Thr Asn Gly Thr Lys Arg Phe Asp Asn Ser Gly Thr
                245                 250                 255

Asn Gly Thr Lys Arg Phe Asp Asn
            260

<210>  45
<211>  147
<212>  PRT
<213>  Artificial Sequence

<220>
<223>  SEQ ID NO: 1-derived peptide reactive with SARS-CoV-2 antibodies
       with GST fusion

<400>  45

Met Ser His His His His His His His His Ser Pro Met Tyr Ser Ile
1               5                   10                  15

Ile Thr Pro Asn Ile Leu Arg Leu Glu Ser Glu Glu Thr Met Val Leu
            20                  25                  30

Glu Ala His Asp Ala Gln Gly Asp Val Pro Val Thr Val Thr Val His
        35                  40                  45

Asp Phe Pro Gly Lys Lys Leu Val Leu Ser Ser Glu Lys Thr Val Leu
    50                  55                  60

Thr Pro Ala Thr Asn His Met Gly Asn Val Thr Phe Thr Ile Pro Ala
65                  70                  75                  80

Asn Arg Glu Phe Lys Ser Glu Lys Gly Arg Asn Lys Phe Val Thr Val
                85                  90                  95

Gln Ala Thr Phe Gly Thr Gln Val Val Glu Lys Val Val Leu Val Ser
            100                 105                 110

Leu Gln Ser Gly Ile Glu Gly Arg Met Leu Thr Pro Gly Asp Ser Ser
        115                 120                 125

Ser Gly Trp Thr Ala Gly Leu Thr Pro Gly Asp Ser Ser Ser Gly Trp
    130                 135                 140

Thr Ala Gly
145

<210>  46
<211>  139
<212>  PRT

<213>  Artificial Sequence

<220>
<223>  SEQ ID NO: 1-derived peptide reactive with SARS-CoV-2 antibodies
       with GST fusion

<400>  46

Met Ser His His His His His His His His Ser Pro Met Tyr Ser Ile
1               5                   10                  15

Ile Thr Pro Asn Ile Leu Arg Leu Glu Ser Glu Glu Thr Met Val Leu
            20                  25                  30

Glu Ala His Asp Ala Gln Gly Asp Val Pro Val Thr Val Thr Val His
        35                  40                  45

Asp Phe Pro Gly Lys Lys Leu Val Leu Ser Ser Glu Lys Thr Val Leu
    50                  55                  60

Thr Pro Ala Thr Asn His Met Gly Asn Val Thr Phe Thr Ile Pro Ala
65                  70                  75                  80

Asn Arg Glu Phe Lys Ser Glu Lys Gly Arg Asn Lys Phe Val Thr Val
                85                  90                  95

Gln Ala Thr Phe Gly Thr Gln Val Val Glu Lys Val Val Leu Val Ser
            100                 105                 110

Leu Gln Ser Gly Ile Glu Gly Arg Met Asn Asn Leu Asp Ser Lys Val
        115                 120                 125

Gly Gly Asn Asn Leu Asp Ser Lys Val Gly Gly
    130                 135

<210>  47
<211>  143
<212>  PRT
<213>  Artificial Sequence

<220>
<223>  SEQ ID NO: 1-derived peptide reactive with SARS-CoV-2 antibodies
       with GST fusion

<400>  47

Met Ser His His His His His His His His Ser Pro Met Tyr Ser Ile
1               5                   10                  15

Ile Thr Pro Asn Ile Leu Arg Leu Glu Ser Glu Glu Thr Met Val Leu
            20                  25                  30

Glu Ala His Asp Ala Gln Gly Asp Val Pro Val Thr Val Thr Val His
        35                  40                  45

Asp Phe Pro Gly Lys Lys Leu Val Leu Ser Ser Glu Lys Thr Val Leu
    50                  55                  60

Thr Pro Ala Thr Asn His Met Gly Asn Val Thr Phe Thr Ile Pro Ala
65                  70                  75                  80

Asn Arg Glu Phe Lys Ser Glu Lys Gly Arg Asn Lys Phe Val Thr Val
                85                  90                  95

Gln Ala Thr Phe Gly Thr Gln Val Val Glu Lys Val Val Leu Val Ser
            100                 105                 110

Leu Gln Ser Gly Ile Glu Gly Arg Met Tyr Gln Ala Gly Ser Thr Pro
        115                 120                 125

Cys Asn Gly Val Tyr Gln Ala Gly Ser Thr Pro Cys Asn Gly Val
    130                 135                 140

<210>  48
<211>  145
<212>  PRT
<213>  Artificial Sequence

<220>
<223>  SEQ ID NO: 1-derived peptide reactive with SARS-CoV-2 antibodies
       with GST fusion

<400>  48

Met Ser His His His His His His His His Ser Pro Met Tyr Ser Ile
1               5                   10                  15

Ile Thr Pro Asn Ile Leu Arg Leu Glu Ser Glu Glu Thr Met Val Leu
            20                  25                  30

Glu Ala His Asp Ala Gln Gly Asp Val Pro Val Thr Val Thr Val His
        35                  40                  45

Asp Phe Pro Gly Lys Lys Leu Val Leu Ser Ser Glu Lys Thr Val Leu
    50                  55                  60

Thr Pro Ala Thr Asn His Met Gly Asn Val Thr Phe Thr Ile Pro Ala
65                  70                  75                  80

Asn Arg Glu Phe Lys Ser Glu Lys Gly Arg Asn Lys Phe Val Thr Val
                85                  90                  95

Gln Ala Thr Phe Gly Thr Gln Val Val Glu Lys Val Val Leu Val Ser
            100                 105                 110

Leu Gln Ser Gly Ile Glu Gly Arg Met Tyr Gly Phe Gln Pro Thr Asn
        115                 120                 125

Gly Val Gly Tyr Gln Tyr Gly Phe Gln Pro Thr Asn Gly Val Gly Tyr
    130                 135                 140

Gln
145

<210>  49
<211>  188
<212>  PRT
<213>  Artificial Sequence

<220>
<223>  SEQ ID NO: 1-derived peptide reactive with SARS-CoV-2 antibodies
       with GST fusion, P1-P6

<400>  49

Met Ser His His His His His His His His Ser Pro Met Tyr Ser Ile
1               5                   10                  15

Ile Thr Pro Asn Ile Leu Arg Leu Glu Ser Glu Glu Thr Met Val Leu
            20                  25                  30

Glu Ala His Asp Ala Gln Gly Asp Val Pro Val Thr Val Thr Val His
        35                  40                  45

Asp Phe Pro Gly Lys Lys Leu Val Leu Ser Ser Glu Lys Thr Val Leu
    50                  55                  60

Thr Pro Ala Thr Asn His Met Gly Asn Val Thr Phe Thr Ile Pro Ala
65                  70                  75                  80

Asn Arg Glu Phe Lys Ser Glu Lys Gly Arg Asn Lys Phe Val Thr Val
                85                  90                  95

Gln Ala Thr Phe Gly Thr Gln Val Val Glu Lys Val Val Leu Val Ser
            100                 105                 110

Leu Gln Ser Gly Ile Glu Gly Arg Met Arg Thr Gln Leu Pro Pro Ala
        115                 120                 125

Tyr Thr Asn Ser Ser Gly Thr Asn Gly Thr Lys Arg Phe Asp Asn Leu
    130                 135                 140

Thr Pro Gly Asp Ser Ser Ser Gly Trp Thr Ala Gly Asn Asn Leu Asp
145                 150                 155                 160

Ser Lys Val Gly Gly Tyr Gln Ala Gly Ser Thr Pro Cys Asn Gly Val
                165                 170                 175

Tyr Gly Phe Gln Pro Thr Asn Gly Val Gly Tyr Gln
            180                 185

<210>  50
<211>  773
<212>  PRT
<213>  Artificial Sequence

<220>
<223>  C-terminally His-tagged extracellular domain of human ACE2

<400>  50

Met Ser Ser Ser Ser Trp Leu Leu Leu Ser Leu Val Ala Val Thr Ala
1               5                   10                  15

Ala Gln Ser Thr Ile Glu Glu Gln Ala Lys Thr Phe Leu Asp Lys Phe
            20                  25                  30

Asn His Glu Ala Glu Asp Leu Phe Tyr Gln Ser Ser Leu Ala Ser Trp
        35                  40                  45

Asn Tyr Asn Thr Asn Ile Thr Glu Glu Asn Val Gln Asn Met Asn Asn
    50                  55                  60

Ala Gly Asp Lys Trp Ser Ala Phe Leu Lys Glu Gln Ser Thr Leu Ala
65                  70                  75                  80

Gln Met Tyr Pro Leu Gln Glu Ile Gln Asn Leu Thr Val Lys Leu Gln
                85                  90                  95

Leu Gln Ala Leu Gln Gln Asn Gly Ser Ser Val Leu Ser Glu Asp Lys
            100                 105                 110

Ser Lys Arg Leu Asn Thr Ile Leu Asn Thr Met Ser Thr Ile Tyr Ser
        115                 120                 125

Thr Gly Lys Val Cys Asn Pro Asp Asn Pro Gln Glu Cys Leu Leu Leu
    130                 135                 140

Glu Pro Gly Leu Asn Glu Ile Met Ala Asn Ser Leu Asp Tyr Asn Glu
145                 150                 155                 160

Arg Leu Trp Ala Trp Glu Ser Trp Arg Ser Glu Val Gly Lys Gln Leu

                165                 170                 175

Arg Pro Leu Tyr Glu Glu Tyr Val Val Leu Lys Asn Glu Met Ala Arg
            180                 185                 190

Ala Asn His Tyr Glu Asp Tyr Gly Asp Tyr Trp Arg Gly Asp Tyr Glu
        195                 200                 205

Val Asn Gly Val Asp Gly Tyr Asp Tyr Ser Arg Gly Gln Leu Ile Glu
    210                 215                 220

Asp Val Glu His Thr Phe Glu Glu Ile Lys Pro Leu Tyr Glu His Leu
225                 230                 235                 240

His Ala Tyr Val Arg Ala Lys Leu Met Asn Ala Tyr Pro Ser Tyr Ile
                245                 250                 255

Ser Pro Ile Gly Cys Leu Pro Ala His Leu Leu Gly Asp Met Trp Gly
            260                 265                 270

Arg Phe Trp Thr Asn Leu Tyr Ser Leu Thr Val Pro Phe Gly Gln Lys
        275                 280                 285

Pro Asn Ile Asp Val Thr Asp Ala Met Val Asp Gln Ala Trp Asp Ala
    290                 295                 300

Gln Arg Ile Phe Lys Glu Ala Glu Lys Phe Phe Val Ser Val Gly Leu
305                 310                 315                 320

Pro Asn Met Thr Gln Gly Phe Trp Glu Asn Ser Met Leu Thr Asp Pro
                325                 330                 335

Gly Asn Val Gln Lys Ala Val Cys His Pro Thr Ala Trp Asp Leu Gly
            340                 345                 350

Lys Gly Asp Phe Arg Ile Leu Met Cys Thr Lys Val Thr Met Asp Asp
        355                 360                 365

Phe Leu Thr Ala His His Glu Met Gly His Ile Gln Tyr Asp Met Ala
    370                 375                 380

Tyr Ala Ala Gln Pro Phe Leu Leu Arg Asn Gly Ala Asn Glu Gly Phe
385                 390                 395                 400

His Glu Ala Val Gly Glu Ile Met Ser Leu Ser Ala Ala Thr Pro Lys
                405                 410                 415

His Leu Lys Ser Ile Gly Leu Leu Ser Pro Asp Phe Gln Glu Asp Asn
            420                 425                 430

Glu Thr Glu Ile Asn Phe Leu Leu Lys Gln Ala Leu Thr Ile Val Gly
        435                 440                 445

Thr Leu Pro Phe Thr Tyr Met Leu Glu Lys Trp Arg Trp Met Val Phe
    450                 455                 460

Lys Gly Glu Ile Pro Lys Asp Gln Trp Met Lys Lys Trp Trp Glu Met
465                 470                 475                 480

Lys Arg Glu Ile Val Gly Val Val Glu Pro Val Pro His Asp Glu Thr
                485                 490                 495

Tyr Cys Asp Pro Ala Ser Leu Phe His Val Ser Asn Asp Tyr Ser Phe
            500                 505                 510

Ile Arg Tyr Tyr Thr Arg Thr Leu Tyr Gln Phe Gln Phe Gln Glu Ala
        515                 520                 525

Leu Cys Gln Ala Ala Lys His Glu Gly Pro Leu His Lys Cys Asp Ile
    530                 535                 540

Ser Asn Ser Thr Glu Ala Gly Gln Lys Leu Phe Asn Met Leu Arg Leu
545                 550                 555                 560

Gly Lys Ser Glu Pro Trp Thr Leu Ala Leu Glu Asn Val Val Gly Ala
                565                 570                 575

Lys Asn Met Asn Val Arg Pro Leu Leu Asn Tyr Phe Glu Pro Leu Phe
            580                 585                 590

Thr Trp Leu Lys Asp Gln Asn Lys Asn Ser Phe Val Gly Trp Ser Thr
        595                 600                 605

Asp Trp Ser Pro Tyr Ala Asp Gln Ser Ile Lys Val Arg Ile Ser Leu
    610                 615                 620

Lys Ser Ala Leu Gly Asp Lys Ala Tyr Glu Trp Asn Asp Asn Glu Met
625                 630                 635                 640

Tyr Leu Phe Arg Ser Ser Val Ala Tyr Ala Met Arg Gln Tyr Phe Leu
                645                 650                 655

Lys Val Lys Asn Gln Met Ile Leu Phe Gly Glu Glu Asp Val Arg Val
            660                 665                 670

Ala Asn Leu Lys Pro Arg Ile Ser Phe Asn Phe Phe Val Thr Ala Pro
        675                 680                 685

Lys Asn Val Ser Asp Ile Ile Pro Arg Thr Glu Val Glu Lys Ala Ile
    690                 695                 700

Arg Met Ser Arg Ser Arg Ile Asn Asp Ala Phe Arg Leu Asn Asp Asn
705                 710                 715                 720

Ser Leu Glu Phe Leu Gly Ile Gln Pro Thr Leu Gly Pro Pro Asn Gln
                725                 730                 735

Pro Pro Val Ser Leu Glu Gly Ser Gly Ser Gly Ser His His His His
            740                 745                 750

His His His His Gly Ser Gly Leu Asn Asp Ile Phe Glu Ala Gln Lys
        755                 760                 765

Ile Glu Trp His Glu
    770

<210>  51
<211>  648
<212>  PRT
<213>  Artificial Sequence

<220>
<223>  SEQ ID NO1 with mutations of SARS-CoV-2 U.K. variant B.1.1.7

<400>  51

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile Ser Gly Thr Asn Gly Thr Lys Arg Phe Asp Asn
    50                  55                  60

Pro Val Leu Pro Phe Asn Asp Gly Val Tyr Phe Ala Ser Thr Glu Lys
65                  70                  75                  80

Ser Asn Ile Ile Arg Gly Trp Ile Phe Gly Thr Thr Leu Asp Ser Lys
                85                  90                  95

Thr Gln Ser Leu Leu Ile Val Asn Asn Ala Thr Asn Val Val Ile Lys
        100                 105                 110

Val Cys Glu Phe Gln Phe Cys Asn Asp Pro Phe Leu Gly Val Tyr Tyr
        115                 120                 125

His Lys Asn Asn Lys Ser Trp Met Glu Ser Glu Phe Arg Val Tyr Ser
    130                 135                 140

Ser Ala Asn Asn Cys Thr Phe Glu Tyr Val Ser Gln Pro Phe Leu Met
145                 150                 155                 160

Asp Leu Glu Gly Lys Gln Gly Asn Phe Lys Asn Leu Arg Glu Phe Val
                165                 170                 175

Phe Lys Asn Ile Asp Gly Tyr Phe Lys Ile Tyr Ser Lys His Thr Pro
            180                 185                 190

Ile Asn Leu Val Arg Asp Leu Pro Gln Gly Phe Ser Ala Leu Glu Pro
        195                 200                 205

Leu Val Asp Leu Pro Ile Gly Ile Asn Ile Thr Arg Phe Gln Thr Leu
    210                 215                 220

Leu Ala Leu His Arg Ser Tyr Leu Thr Pro Gly Asp Ser Ser Ser Gly
225                 230                 235                 240

Trp Thr Ala Gly Ala Ala Ala Tyr Tyr Val Gly Tyr Leu Gln Pro Arg
                245                 250                 255

Thr Phe Leu Leu Lys Tyr Asn Glu Asn Gly Thr Ile Thr Asp Ala Val
            260                 265                 270

Asp Cys Ala Leu Asp Pro Leu Ser Glu Thr Lys Cys Thr Leu Lys Ser
        275                 280                 285

Phe Thr Val Glu Lys Gly Ile Tyr Gln Thr Ser Asn Phe Arg Val Gln
    290                 295                 300

Pro Thr Glu Ser Ile Val Arg Phe Pro Asn Ile Thr Asn Leu Cys Pro
305                 310                 315                 320

Phe Gly Glu Val Phe Asn Ala Thr Arg Phe Ala Ser Val Tyr Ala Trp
                325                 330                 335

Asn Arg Lys Arg Ile Ser Asn Cys Val Ala Asp Tyr Ser Val Leu Tyr
            340                 345                 350

Asn Ser Ala Ser Phe Ser Thr Phe Lys Cys Tyr Gly Val Ser Pro Thr
        355                 360                 365

Lys Leu Asn Asp Leu Cys Phe Thr Asn Val Tyr Ala Asp Ser Phe Val
    370                 375                 380

Ile Arg Gly Asp Glu Val Arg Gln Ile Ala Pro Gly Gln Thr Gly Lys
385                 390                 395                 400

Ile Ala Asp Tyr Asn Tyr Lys Leu Pro Asp Asp Phe Thr Gly Cys Val
                405                 410                 415

Ile Ala Trp Asn Ser Asn Asn Leu Asp Ser Lys Val Gly Gly Asn Tyr
            420                 425                 430

Asn Tyr Leu Tyr Arg Leu Phe Arg Lys Ser Asn Leu Lys Pro Phe Glu
        435                 440                 445

Arg Asp Ile Ser Thr Glu Ile Tyr Gln Ala Gly Ser Thr Pro Cys Asn
    450                 455                 460

Gly Val Glu Gly Phe Asn Cys Tyr Phe Pro Leu Gln Ser Tyr Gly Phe
465                 470                 475                 480

Gln Pro Thr Tyr Gly Val Gly Tyr Gln Pro Tyr Arg Val Val Val Leu
                485                 490                 495

Ser Phe Glu Leu Leu His Ala Pro Ala Thr Val Cys Gly Pro Lys Lys
            500                 505                 510

Ser Thr Asn Leu Val Lys Asn Lys Cys Val Asn Phe Asn Phe Asn Gly
        515                 520                 525

Leu Thr Gly Thr Gly Val Leu Thr Glu Ser Asn Lys Lys Phe Leu Pro
    530                 535                 540

Phe Gln Gln Phe Gly Arg Asp Ile Asp Asp Thr Thr Asp Ala Val Arg
545                 550                 555                 560

Asp Pro Gln Thr Leu Glu Ile Leu Asp Ile Thr Pro Cys Ser Phe Gly
                565                 570                 575

Gly Val Ser Val Ile Thr Pro Gly Thr Asn Thr Ser Asn Gln Val Ala
            580                 585                 590

Val Leu Tyr Gln Gly Val Asn Cys Thr Glu Val Pro Val Ala Ile His
        595                 600                 605

Ala Asp Gln Leu Thr Pro Thr Trp Arg Val Tyr Ser Thr Gly Ser Asn
    610                 615                 620

Val Phe Gln Thr Arg Ala Gly Cys Leu Ile Gly Ala Glu His Val Asn
625                 630                 635                 640

Asn Ser Tyr Glu Cys Asp Ile His
                645

<210>  52
<211>  650
<212>  PRT
<213>  Artificial Sequence

<220>
<223>  SEQ ID NO1 with mutations of SARS-CoV-2 South African variant
       B.1.351

<400>  52

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu
                165                 170                 175

Phe Val Phe Lys Asn Ile Asp Gly Tyr Phe Lys Ile Tyr Ser Lys His
            180                 185                 190

Thr Pro Ile Asn Leu Val Arg Asp Leu Pro Gln Gly Phe Ser Ala Leu
        195                 200                 205

Glu Pro Leu Val Asp Leu Pro Ile Gly Ile Asn Ile Thr Arg Phe Gln
    210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Asn Ile Ala Asp Tyr Asn Tyr Lys Leu Pro Asp Asp Phe Thr Gly

                405                 410                 415

Cys Val Ile Ala Trp Asn Ser Asn Asn Leu Asp Ser Lys Val Gly Gly
            420                 425                 430

Asn Tyr Asn Tyr Leu Tyr Arg Leu Phe Arg Lys Ser Asn Leu Lys Pro
        435                 440                 445

Phe Glu Arg Asp Ile Ser Thr Glu Ile Tyr Gln Ala Gly Ser Thr Pro
    450                 455                 460

Cys Asn Gly Val Lys Gly Phe Asn Cys Tyr Phe Pro Leu Gln Ser Tyr
465                 470                 475                 480

Gly Phe Gln Pro Thr Tyr Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Gly Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro
                645                 650

<210>  53
<211>  650
<212>  PRT
<213>  Artificial Sequence

<220>
<223>  SEQ ID NO1 with mutations of SARS-CoV-2 Brazilian variant P.1

<400>  53

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu
                165                 170                 175

Phe Val Phe Lys Asn Ile Asp Gly Tyr Phe Lys Ile Tyr Ser Lys His
            180                 185                 190

Thr Pro Ile Asn Leu Val Arg Asp Leu Pro Gln Gly Phe Ser Ala Leu
        195                 200                 205

Glu Pro Leu Val Asp Leu Pro Ile Gly Ile Asn Ile Thr Arg Phe Gln

210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Lys Ile Ala Asp Tyr Asn Tyr Lys Leu Pro Asp Asp Phe Thr Gly
                405                 410                 415

Cys Val Ile Ala Trp Asn Ser Asn Asn Leu Asp Ser Lys Val Gly Gly
            420                 425                 430

Asn Tyr Asn Tyr Leu Tyr Arg Leu Phe Arg Lys Ser Asn Leu Lys Pro
        435                 440                 445

Phe Glu Arg Asp Ile Ser Thr Glu Ile Tyr Gln Ala Gly Ser Thr Pro
    450                 455                 460

Cys Asn Gly Val Lys Gly Phe Asn Cys Tyr Phe Pro Leu Gln Ser Tyr
465                 470                 475                 480

Gly Phe Gln Pro Thr Tyr Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Asp Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro
                645                 650

<210>  54
<211>  648
<212>  PRT
<213>  Artificial Sequence

<220>
<223>  SEQ ID NO1 with mutations of SARS-CoV-2 Mink Variant from Denmark

<400>  54

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val

20                  25                  30

Leu His Ser Thr Gln Asp Leu Phe Leu Pro Phe Phe Ser Asn Val Thr
        35                  40                  45

Trp Phe His Ala Ile Ser Gly Thr Asn Gly Thr Lys Arg Phe Asp Asn
    50                  55                  60

Pro Val Leu Pro Phe Asn Asp Gly Val Tyr Phe Ala Ser Thr Glu Lys
65                  70                  75                  80

Ser Asn Ile Ile Arg Gly Trp Ile Phe Gly Thr Thr Leu Asp Ser Lys
                85                  90                  95

Thr Gln Ser Leu Leu Ile Val Asn Asn Ala Thr Asn Val Val Ile Lys
            100                 105                 110

Val Cys Glu Phe Gln Phe Cys Asn Asp Pro Phe Leu Gly Val Tyr Tyr
        115                 120                 125

His Lys Asn Asn Lys Ser Trp Met Glu Ser Glu Phe Arg Val Tyr Ser
    130                 135                 140

Ser Ala Asn Asn Cys Thr Phe Glu Tyr Val Ser Gln Pro Phe Leu Met
145                 150                 155                 160

Asp Leu Glu Gly Lys Gln Gly Asn Phe Lys Asn Leu Arg Glu Phe Val
                165                 170                 175

Phe Lys Asn Ile Asp Gly Tyr Phe Lys Ile Tyr Ser Lys His Thr Pro
            180                 185                 190

Ile Asn Leu Val Arg Asp Leu Pro Gln Gly Phe Ser Ala Leu Glu Pro
        195                 200                 205

Leu Val Asp Leu Pro Ile Gly Ile Asn Ile Thr Arg Phe Gln Thr Leu
    210                 215                 220

Leu Ala Leu His Arg Ser Tyr Leu Thr Pro Gly Asp Ser Ser Ser Gly
225                 230                 235                 240

Trp Thr Ala Gly Ala Ala Ala Tyr Tyr Val Gly Tyr Leu Gln Pro Arg
                245                 250                 255

Thr Phe Leu Leu Lys Tyr Asn Glu Asn Gly Thr Ile Thr Asp Ala Val
            260                 265                 270

Asp Cys Ala Leu Asp Pro Leu Ser Glu Thr Lys Cys Thr Leu Lys Ser
        275                 280                 285

Phe Thr Val Glu Lys Gly Ile Tyr Gln Thr Ser Asn Phe Arg Val Gln
    290                 295                 300

Pro Thr Glu Ser Ile Val Arg Phe Pro Asn Ile Thr Asn Leu Cys Pro
305                 310                 315                 320

Phe Gly Glu Val Phe Asn Ala Thr Arg Phe Ala Ser Val Tyr Ala Trp
                325                 330                 335

Asn Arg Lys Arg Ile Ser Asn Cys Val Ala Asp Tyr Ser Val Leu Tyr
            340                 345                 350

Asn Ser Ala Ser Phe Ser Thr Phe Lys Cys Tyr Gly Val Ser Pro Thr
        355                 360                 365

Lys Leu Asn Asp Leu Cys Phe Thr Asn Val Tyr Ala Asp Ser Phe Val
    370                 375                 380

Ile Arg Gly Asp Glu Val Arg Gln Ile Ala Pro Gly Gln Thr Gly Lys
385                 390                 395                 400

Ile Ala Asp Tyr Asn Tyr Lys Leu Pro Asp Asp Phe Thr Gly Cys Val
                405                 410                 415

Ile Ala Trp Asn Ser Asn Asn Leu Asp Ser Lys Val Gly Gly Asn Tyr
            420                 425                 430

Asn Tyr Leu Phe Arg Leu Phe Arg Lys Ser Asn Leu Lys Pro Phe Glu
        435                 440                 445

Arg Asp Ile Ser Thr Glu Ile Tyr Gln Ala Gly Ser Thr Pro Cys Asn
    450                 455                 460

Gly Val Glu Gly Phe Asn Cys Tyr Phe Pro Leu Gln Ser Tyr Gly Phe
465                 470                 475                 480

Gln Pro Thr Asn Gly Val Gly Tyr Gln Pro Tyr Arg Val Val Val Leu
                485                 490                 495

Ser Phe Glu Leu Leu His Ala Pro Ala Thr Val Cys Gly Pro Lys Lys
            500                 505                 510

Ser Thr Asn Leu Val Lys Asn Lys Cys Val Asn Phe Asn Phe Asn Gly
        515                 520                 525

Leu Thr Gly Thr Gly Val Leu Thr Glu Ser Asn Lys Lys Phe Leu Pro
    530                 535                 540

Phe Gln Gln Phe Gly Arg Asp Ile Ala Asp Thr Thr Asp Ala Val Arg
545                 550                 555                 560

Asp Pro Gln Thr Leu Glu Ile Leu Asp Ile Thr Pro Cys Ser Phe Gly
                565                 570                 575

Gly Val Ser Val Ile Thr Pro Gly Thr Asn Thr Ser Asn Gln Val Ala
            580                 585                 590

Val Leu Tyr Gln Gly Val Asn Cys Thr Glu Val Pro Val Ala Ile His
        595                 600                 605

Ala Asp Gln Leu Thr Pro Thr Trp Arg Val Tyr Ser Thr Gly Ser Asn
    610                 615                 620

Val Phe Gln Thr Arg Ala Gly Cys Leu Ile Gly Ala Glu His Val Asn
625                 630                 635                 640

Asn Ser Tyr Glu Cys Asp Ile Pro
                645

<210>  55
<211>  9
<212>  PRT
<213>  Artificial Sequence

<220>
<223>  SEQ ID NO: 1-derived peptide reactive with SARS-CoV-2 antibodies

<400>  55

Asn Asn Leu Asp Ser Lys Val Gly Gly
1               5

<210>  56
<211>  67
<212>  PRT
<213>  Artificial Sequence

<220>
<223>  SEQ ID NO: 1-derived peptide reactive with SARS-CoV-2 antibodies
       with GST fusion, P1-P6

<400>  56

Arg Thr Gln Leu Pro Pro Ala Tyr Thr Asn Ser Ser Gly Thr Asn Gly
1               5                   10                  15

Thr Lys Arg Phe Asp Asn Leu Thr Pro Gly Asp Ser Ser Ser Gly Trp

20                       25                       30

Thr Ala Gly Asn Asn Leu Asp Ser Lys Val Gly Gly Tyr Gln Ala Gly
        35                       40                       45

Ser Thr Pro Cys Asn Gly Val Tyr Gly Phe Gln Pro Thr Asn Gly Val
    50                       55                       60

Gly Tyr Gln
65

## Description

The present invention relates to a method for diagnosing a SARS-CoV-2 infection comprising the step of detecting the presence or absence of an antibody to SEQ ID NO: 1, preferably IgA class antibody, in a sample from a subject, a method for the differential diagnosis of a coronavirus infection, a use of an antibody to SEQ ID NO:1, preferably IgA class antibody for diagnosing a SARS-CoV-2 infection or for the differential diagnosis of a coronavirus infection, preferably for distinguishing between a SARS-CoV-2, MERS, NL63, 229E, OC43 and HKU1 infection, and a kit comprising a polypeptide comprising SEQ ID NO:1 or a variant thereof, preferably coated to a diagnostically useful carrier and one or more, preferably all reagents from the group comprising an antibody to SEQ ID NO:1, a washing buffer, a means for detecting the presence of an antibody, preferably IgA class antibody, preferably a secondary antibody binding specifically to IgA class antibodies, preferably comprising a detectable label, and a dilution buffer.

At the end of 2019, a rising number of pneumonia patients with unknown pathogen emerged from Wuhan, the capital of Hubei province, China, to nearly the entirety of China. A novel coronavirus was isolated and based on its phylogeny, taxonomy and established practice, the Coronavirus Study Group (CSG) recognized it as a sister to severe acute respiratory syndrome coronavirus (SARS-CoV-1) and labeled it as severe acute respiratory syndrome coronavirus 2 (SARS-CoV-2). Although SARS-CoV-2 is generally less pathogenic than SARS-CoV-1 and Middle East respiratory syndrome coronavirus (MERS-CoV), it has a relatively high transmissibility. Since symptoms may be mild and may be confused with a cold, there is the danger that patients may be unaware that they have been infected and may help the virus spread further.

SARS-CoV-2 is a coronavirus which has four major structural proteins, specifically the spike (S), envelope (E), membrane (M) and nucleocapsid (N) proteins. The N protein holds the RNA genome, while the other three structural proteins are components of the viral envelope. The S protein is responsible for allowing the virus to attach and fuse to the membrane of a host cell. It comprises an S1 domain which mediates the attachment and an S2 domain which mediates the fusion of the viral cellular membrane with the host cell. The S1 domain comprises the receptor binding domain (RBD), the binding site to the receptor angiotensin converting enzyme 2 (ACE2) on human host cells. Therefore, the RBD is a binding site of neutralizing antibodies which block the interaction between the virus and its host cells, thus conferring immunity. By contrast to SARS-CoV-1 and SARS-CoV-2, which are associated with a high mortality and severe illness, other coronaviruses exist which are associated with a mild and passing illness, such as coronaviruses 229E, NL63, OC43 and HKU1. These coronaviruses are frequently associated with common cold, in particular among children.

Owing to the high transmissibility and health impact, reliable methods for the diagnosis of SARS-CoV-2 infection are paramount. In the past, a combination of serological and real-time reverse transcriptionpolymerase chain reaction (RT-PCR) methods were used to detect and confirm infections with coronaviruses.

RT-PCR methods are based on the detection of one or more nucleic acids of the coronavirus of interest, more specifically of the viral ribonucleic acid (RNA). RT-PCR-based methods are rapid and, based on samples from throat or nasal pharyngeal swabs, can be used during the first week of illness for the detection of SARS-CoV-2. However, their usefulness very much depends on how long the nucleic acid is detectable in samples, which varies from virus to virus. In particular, a negative result from a diagnostic test performed on a sample obtained at an early stage of the disease may be meaningless. Moreover, generally a sample from the upper respiratory tract of the patient is required. Improper or insufficient recovery of such a sample, prolonged transportation time and associated degradation of the viral RNA, or instrument malfunction may also lead to false-negative results. Therefore, several samples should be examined, one from week 1 of the infection and a follow-up sample obtained 3 to 4 weeks later.

Corman *et al.* published a real-time RT-PCR based assay for the detection of SARS-CoV-2 (Corman VM, Landt O, Kaiser M, et al. Detection of 2019 novel coronavirus (2019-nCoV) by real-time RT-PCR. Euro Surveill. 2020;25(3):2000045. doi:10.2807/1560-7917.ES.2020.25.3.2000045), first published in January 2020 by the WHO on their webpage.

Serological tests are also frequently used. These tests are based on the detection of antibodies directed against certain virus-specific antigens. However, serological assays also suffer from severe impediments:
One frequently observed issue is their limited sensitivity. in order for serological assays to yield a positive result, the presence of virus-antigen specific antibodies in the patient's blood is required. Generally, the time point from which an antibody is first produced and detectable after an infection with a new virus cannot be predicted, because it depends on a multitude of factors, including the virus itself, the viral load i.e., the quantity of virus in a given amount of patient's blood, patient-related factors such as age, gender, health condition, immune status, etc., the immunoglobulin class of the antibody of interest, and the antigen target chosen to set up a diagnostic test. Particularly at very early stages of the disease, i.e. before the patient's body has been able to mount a specific antibody response in detectable quantities, serological assays may give a negative result, despite an ongoing infection.

Further serological testing, taking into account whether an antibody to be detected belongs to a certain immunoglobulin (Ig) class, may help monitor the course of the disease and distinguish an acute from a past infection or a vaccination. Most serological assays currently available for detection of viral infections including SARS-CoV-1 infection hence focus on the detection of IgG, or IgG and IgM class antibodies. However, besides their risk of yielding false-negative results specifically in very early stages of infection due to the intrinsic delay of antibody response, serological assays for diagnosing SARS-CoV-1 infection are also prone to false-positive results, presumably due to a high seroprevalence in the population of antibodies against common seasonal coronoviruses (CoV) and the associated presence of cross-reactive antibodies against conserved parts of immunogenic virus proteins. This cross-reactivity against such antigenically closely related seasonal coronaviruses also disallowed a differential diagnosis, *for example* a differentiation of the life-threatening variant SARS-CoV-1 from other coronaviruses. Challenges and pitfalls of serological assays for diagnosing and differentiating coronoviruses, specifically for diagnosing SARS-CoV-1, have been reviewed in Meyer, Drosten & Müller, Virus Research 194 (2014); 175-186. These multiple challenges associated with the serological diagnosis of earlier coronoviruses, e.g. poor sensitivity in early stages of infection; and poor specificity caused by the presence of cross-reactive antibodies, also apply to the development of diagnostic assays for detection of the recently emerged severe acute respiratory syndrome coronavirus 2 (SARS-CoV-2).

In view of the current severe threat posed by the ongoing global SARS-CoV-2 pandemic and the above mentioned issues of available assays for diagnosing a corresponding infection, there is an urgent need in the art for improved, in particular more sensitive, more specific, and hence more reliable means and methods for diagnosing a SARS-CoV-2 infection, particularly for the early diagnosis of a SARS-CoV-2 infection, and for differentiation thereof from infections with other coronaviruses, such as the common seasonal coronoviruses, in order to enable a more targeted virus-specific and hence more effective treatment.

The present invention addresses this need by providing diagnostic means and methods overcoming these previous impediments.

Specifically, the present invention addresses, without intended to be limiting, the following problems:
One problem addressed by the present invention is to provide an assay and reagents for the early serological detection of SARS-CoV-2. This is particularly important, as patients may still suffer from active disease and be contagious, even if a negative PCR result was obtained. N.B. that the Center for Disease Control and Prevention (CDC) recommends that patients remain isolated for at least 10 days since symptom onset and up to 20 days in cases of severe illness.

Another problem addressed by the present invention is to provide an assay that may be used to distinguish known coronavirus infections, in particular a SARS-CoV-2 infection from other coronavirus infections, preferably coronaviruses associated with mild cold-like symptoms or other pathogens or causes of such symptoms. Preferably, the infections can be distinguished at an early stage.

Another problem addressed by the present invention is to provide an assay with high optimized reliability, in particular with regard to sensitivity and/or specificity, preferably sensitivity.

Another problem addressed by the present invention is to provide an assay with high sensitivity, for patients lacking antibodies to the SARS-CoV-2 N protein or lacking IgM class antibodies.

Another problem addressed by the present invention is to provide an assay with long-lasting high sensitivity.

Previous assays for diagnosing coronavirus infections are described, for example, in the following documents: WO2014/045254 discloses assays for diagnosing a Middle East respiratory syndromerelated coronavirus (MERS-CoV) infection, including serlogical assays wherein antibodies against viral proteinaceaous antigens are detected in a sample. However, no practical evidence of such an assay is shown. US2005/0112559 discloses SARS-CoV-1-related methods and agents. The nucleocapsid (N) protein is considered the major diagnostic antigen. WO2005/118813 discloses a variant of SARS-CoV-1-related S protein and the detection of its presence, or the presence of antibodies binding to it, in samples. US2006/0188519 discloses peptides for the diagnosis of a SARS-CoV-1 infection. Hsueh et al. reported that IgG could be detected as early as four days after the onset of a SARS-CoV-1 infection, simultaneously as or one day *earlier* than IgM and IgA (Hsue, P. R., Huang, L. M., Chen, P. J., Kao, C. L., and Yang P. C. (2004) Chronological evolution of IgM, IgA, IgG and neutralization antibodies after infection with SARS-associated coronavirus, Clinical Microbiology and Infection, 10(12), 1062-1066). Reusken et al. disclose the specific, but not necessarily sensitive detection of antibodies to a SARS-CoV-2 spike protein fragment (Specific serology for emerging human coronaviruses by protein microarray. Euro Surveill. 2013;18(14). Yu *et al.* (2020) Measures for diagnosing and treating infections by a novel coronavirus responsible for a pneumonia outbreak originating in Wuhn, China, Microbes and Infection 22 (2020), 74-79 disclose, without pointing to specific antigens, that immunological methods may be used for detecting a SARS-CoV-2 infection, but are associated with poor sensitivity and specificity.

The above-discussed problems are solved by the present invention, as described in the present specification and in the claims.

In a first aspect, the invention provides a method for diagnosing a SARS-CoV-2 infection, comprising the step of detecting the presence or absence of an antibody to SEQ ID NO:1, preferably IgM, IgG and/or IgA class antibody, more preferably IgA class antibody, in a sample from a subject. Whenever reference to a SEQ ID NO is made throughout this specification and the SEQ ID NO denotes an amino acid sequence, it is intended to denote a polypeptide comprising said corresponding amino acid string even if in that connection the term "polypeptide" is not specifically mentioned. The term "comprising" has two meanings in connection with this invention, namely "containing" and "consisting of."

In a second aspect, the invention provides a method for the differential diagnosis of a coronavirus infection, preferably for distinguishing between a SARS-CoV; preferably SARS-CoV-2; MERS, NL63, 229E, OC43 and HKU1 infection, comprising the step of detecting the presence or absence of an antibody to SEQ ID NO: 1 in a sample from a subject, preferably an IgA and/or IgG class antibody, more preferably an IgA class antibody.

In a preferred embodiment, the presence of an IgG and/or IgM class antibody to SEQ ID NO: 1 is detected in addition to an IgA class antibody to SEQ ID NO: 1.

In accordance with the present invention, the "subject", or interchangeably referred to herein as "patient", refers to a mammal, preferably a human, but may alternatively also refer to a different mammal, such as a non-human primate or other mammalian animal or even a non-mammalian animal capable of producing an antibody to SEQ ID NO: 1 or variant thereof.

In accordance with the present invention, the term "sample" from a subject may be a sample of any bodily fluid or tissue of said subject that may comprise an antibody. Exemplary bodily fluids include for example blood, saliva, nasal mucus or lymph fluid. In a preferred embodiment, the sample is a blood sample, preferably selected from the group comprising whole blood, serum, plasma, capillary blood, arterial blood, venous blood or any mixture thereof. The capillary blood is preferably in the form of a dried blot spot, which may be prepared by the patient, sent to the lab, followed by extraction of the blood. The skilled person is aware of various means and methods that may be applied to obtain a sample from a subject suitable for the purposes of the herein disclosed methods, products and uses and in the required quantities. In certain embodiments, the sample may be obtained from the subject by a physician, whereas in other cases, the sample may be obtained by the subject itself, for example, by using minimal invasive means, such as finger pricking to draw blood ("finger-stick blood").

It is also understood that, for the purposes of the herein disclosed invention, the sample may originate from a single subject, i.e., a single individuum, but may alternatively also comprise samples from more than one subject, wherein said samples from more than one subject are pooled into a single sample. For example, in certain cases, it might be more efficient in terms of ressources and experimental time, to first analyze a pooled sample comprising samples from a group of subjects (e.g. members of a common household, school class, sports team, or same company department), and only in case of the detection of the presence of an antibody to SEQ ID NO: 1 or a variant thereof, to conduct a second analysis wherein samples from individual subjects are assayed separately.

Various methods or uses according to the invention can be conducted with a sample from a subject as described herein. These methods or uses can also be characterized as *"in vitro"* methods or *"in vitro"* uses.

In accordance with the present invention, the term "secondary antibody" in its broadest sense is to be understood to refer to any kind of "binding moiety", preferably binding protein, capable of specific binding to an IgA, IgG and/or IgM class antibody or a fragment thereof such as a constant domain of a particular Ig class of a selected species, preferably human species. Non-limiting examples of binding moieties include antibodies, for example antibodies immunologically or genetically derived from any species, for example human, chicken, camel, llama, lamprey, shark, goat, rodent, cow, dog, rabbit, etc., antibody fragments, domains or parts thereof, for example Fab, Fab', F(ab')₂, scFab, Fv, scFv, VH, VHH, VL, VLRs, and the like, diabodies, monoclonal antibodies (mAbs), polyclonal antibodies (pAbs), mAbdAbs, phage display-derived binders, affibodies, heteroconjugate antibodies, bispecific antibodies, evibodies, lipocalins, anticalins, affibodies, avimers, maxibodies, heat shock proteins such as GroEL and GroES, trans-bodies, DARPins, aptamers, C-type lectin domains such as tetranectins; human γ-crystallin and human ubiquitin-derived binders such as affilins, PDZ domain-derived binders; scorpion toxin and/or Kunitz-type domain binders, fibronectin-derived binders such as adnectins, receptors, ligands, lectins, streptavidin, biotin, including derivatives and/or combinations thereof such as bi-/multi-specific formats formed from two or more of these binding molecules. Various antibody-derived and alternative (*i.e.* non-antibody) binding protein scaffolds including methods of generation thereof are known in the art (e.g. reviewed in Chiu ML et al., Antibodies (Basel), (2019);8(4):55; Simeon R. & Chen Z., Protein Cell. (2018);9(1):3-14; and Chapter 7 - Non-Antibody Scaffolds from Handbook of Therapeutic Antibodies (2007) edited by Stefan Dübel.

All these types of molecules are well known in the art. Some examples are described in more detail herein below.

"Evibodies" are engineered binding proteins derived from the variable(V)-set Ig-like scaffold of the T-cell surface receptor Cytotoxic T Lymphocyte-associated Antigen 4 (CTLA-4). Loops corresponding to CDRs of antibodies can be substituted with heterologous sequences to confer different binding properties. Methods of making Evibodies are known in the art and are described, for example, U.S. Patent No. 7,166,697.

"Lipocalins" are a family of extracellular proteins which transport small hydrophobic molecules such as steroids, bilins, retinoids and lipids. They have a rigid beta-sheet secondary structure with a number of loops at the open end of the conical structure which can be engineered to bind to different target antigens. "Anticalins", also termed "Affilins", in accordance with the present invention, are between 160-180 amino acids in size, and are derived from lipocalins (Rothe C & Skerra A., BioDrugs. (2018);32(3):233-243; Gebauer M & Skerra A, Curr Opin Biotechnol. (2019); 60:230-241).

"Affibodies" are a family of antibody mimetics that is derived from the Z-domain of staphylococcal protein A. Affibodies are structurally based on a three-helix bundle domain. An affibody has a molecular mass of around 6 kDa and is stable at high temperatures and under acidic or alkaline conditions. Target specificity is obtained by randomization of amino acids located in two alpha-helices involved in the binding activity of the parent protein domain (Feldwisch, J & Tolmachev, V. (2012) Methods Mol. Biol. 899:103-126). Methods of making affibodies are known in the art and are described in Wikman M etal., Protein Eng Des Sel. (2004);17(5):455-62.

"Avimers", short for avidity multimers), are a class of artificial multi-domain proteins which specifically bind certain antigens via multiple binding sites. This protein is also known as "maxibody" or low-density lipoprotein receptor (LDLR) domain A. It consists of two or more (poly)peptide sequences, which are based on A domains. The A domains are 30 to 35 amino acids scaffolds (~4 kDa) derived from extracellular cysteine-rich cell surface receptor proteins and stabilized by disulfide bond formation and complexation of calcium ions. The scaffold structure is maintained by 12 conserved amino acids, leaving all the remaining non-conserved residues amenable to randomization and ligand binding. Avimers are highly thermostable. Due to their small size, avimers often consist of multiple A-domains with each binding to a different site on the target, thereby achieving increased affinity through avidity (Silverman J et al. (2005), Nat Biotechnol 23:1556-1561).

"DARPins" are designed ankyrin repeat domains and based on tightly packed ankyrin repeats, each forming a β-turn and two antiparallel α-helices. DARPins usually carry three repeats corresponding to an artificial consensus sequence, whereby a single repeat typically consists of 33 amino acids, six of which form the binding surface. During recombinant library design, these sites are used to introduce the codons of random amino acids. DARPins are typically formed by two or three of the binding motifs contained between the N- and C-terminal motifs shielding the hydrophobic regions. DARPins are small proteins (~14-18 kDa) that are extremely thermostable and resistant to proteases and denaturing agents (Plückthun A., Annu Rev Pharmacol Toxicol. (2015);55:489-511).

"Kunitz-type domain binders" are ~60-amino-acid polypeptides (~7 kDa) derived from the active motif of Kunitz-type protease inhibitors such as aprotinin (bovine pancreatic trypsin inhibitor), Alzheimer's amyloid precursor protein, and tissue factor pathway inhibitor. The hydrophobic core of the Kunitz domain is composed of a twisted two-stranded antiparallel β-sheet and two α-helices stabilized by three pairs of disulfide bonds. Residues in the three loops can be substituted without destabilizing the structural framework (Hosse RJ et al. (2006). Protein Sci 15:14-27; Simeon R. & Chen Z. Protein Cell. (2018);9(1):3-14).

"Adnectins" is a class of binding proteins having a scaffold which consists of a backbone of the natural amino acid sequence of the 10th domain of the 15 repeating units of human fibronectin type III (FN3). The molecule adopts a β-sandwich fold with seven strands connected by six loops similar like an immunoglobulin domain, but without any disulfide bonds. Three loops at one end of the β-sandwich can be engineered to enable an adnectin to specifically recognize a target of interest. Non-loop residues have also been found to expand the available binding footprint. Ligand-binding adnectin variants with binding affinities in the nanomolar to picomolar range have been selected via mRNA, phage, and yeast display (Hackel BJ, et al. (2008) J Mol Biol 381:1238-1252).

Means and methods for developing, screening and identification of suitable binding molecules of various scaffolds, including, without being limiting, those described herein above, toward desired target structures, such as the IgA and/or IgG class antibodies, are well known and routinely employed in the art. Exemplary nowadays routinely-performed methods include, without intended to being limiting, high-throughput (HT) combinatorial library-based display and selection methods, such as phage display, ribosome display, mRNA display, and cell surface display (e.g. yeast display).

In a preferred embodiment, the secondary antibody is an immunoglobulin (Ig), preferably IgG raised in a non-human species, wherein said secondary antibody specifically binds immunoglobulins of one or more specific Ig classes or fragments thereof (e.g. a constant domain of a particular Ig class) of another selected species, preferably human species. An example is a polyclonal antibody raised in goat that specifically recognizes human IgA (i.e., a polyclonal goat anti-human IgA antibody). In another preferred embodiment, the secondary antibody is a monoclonal antibody that specifically binds immunoglobulins of one or more specific Ig classes or fragments thereof (e.g. a constant domain of a particular Ig class) of another selected species, preferably human species. Means and methods for producing (mono- or polyclonal) antibodies capable of specific binding of one or more selected target antigens are well known in the art.

In certain embodiments, the secondary antibody may be chosen to specifically bind to only one, only two, or all three classes of Ig antibodies, i.e. IgA, IgG and/or IgM. In certain embodiments, instead of using only one single kind of secondary antibody, a mixture of several different secondary antibodies may be used, wherein the different secondary antibodies either bind to the same one or different Ig classes (e.g., a mixture of different antibodies (e.g., polyclonal antibodies) all binding to IgA), or to, e.g. IgA and IgG or IgM or wherein the different secondary antibodies bind to different individual target structures (e.g. one kind of secondary antibody specifically binding to IgA, and another specifically binding to IgG).

In a preferred embodiment, the antibody to SEQ ID NO: 1 is detected using a labeled secondary antibody, preferably a labeled secondary antibody binding to IgA, IgG and/or IgM class antibodies.

As used herein, the term "labeled", with regard to the secondary antibody, is intended to embrace such embodiments wherein the secondary antibody is labelled by coupling, preferably physically linking, a detectable substance, such as a radioactive agent or a other molecule providing a detectable signal, such as, without intended to being limiting, a fluorophore, such as a small organic chemical fluorphore or fluorescent protein, or an enzymatically active label, i.e. an enzyme, such as alkaline phosphatase, whose presence can be assessed and optionally be quantified based on its reactivity with, and/or conversion of, a substrate substance. Various suitable detectable labels are known in the art and some of which are also described herein below.

In a preferred embodiment, the antibody, preferably IgA, IgG or IgM class antibody, is detected using a method selected from the group comprising colorimetry, immunofluorescence, detection of enzymatic activity, chemiluminscence and radioactivity.

In a preferred embodiment, the infection is detected at an early stage. In a preferred embodiment, the term "early stage", as referred to herein in the context of the course of a SARS-CoV-2 infection, refers to, in such cases where the time-point of infection is known, e.g. infection of a subject in a laboratory experiment, any time point within less than 14 days, preferably less than six days after the time-point of infection, i.e. the time-point of initial contact between the virus and the subject's body. In another preferred embodiment, the term "early stage", as referred to herein in the context of the course of a SARS-CoV-2 infection, refers to any time-point within less than 14 days, preferably less than six days after the onset of illness (also termed in the field of virology as "days post onset of illness" (dpoi)"), i.e. after the first occurrence of one or more typical SAR-CoV-2 infection-associated clinical symptoms, such as defined herein. The person skilled in the art is aware that upon infection, components of a corona virus can be detected directly, for example using PCR for detecting nucleic acid and immunoassays for detecting virus antigens in samples. After the peak of the infection with the maximum virus load, possibly only few days after the first contact of the patient with the virus, the concentration of virus decreases which makes the direct detection increasingly difficult and finally impossible, at the latest when the virus is absent in samples. Meanwhile, as soon as virus components are present in the blood of the patient, the production of antibodies against virus components is triggered. In another preferred embodiment, the term "early stage" refers to the time window between the first contact between virus and patient or the onset of illness, preferably the onset of illness, and the production of detectable IgG antibodies to the virus, more preferably before IgG antibodies are the dominant immunglobulin class, i.e. are present at a higher concentration than IgA and IgM, most preferably before the peak of IgG concentration is reached. Thus, the detection of IgA and IgM antibodies can be a contribution to the diagnosis of an infection that is still acute, with detectable symptoms or not, but before the full IgG immune response is developed.

In a 3^{rd} aspect, the invention provides a use of an antibody to SEQ ID NO: 1, preferably IgA class antibody, for diagnosing a SARS-CoV-2 infection or for the differential diagnosis of a coronavirus infection, preferably for distinguishing between a SARS-CoV, preferably SARS-CoV-2, MERS, NL63, 229E, OC43 and HKU1 infection.

In a preferred embodiment, the use is for the early diagnosis or early stage diagnosis of a SARS-CoV-2 infection.

In a 4^{th} aspect, the invention provides a kit comprising a polypeptide comprising SEQ ID NO: 1 or a variant thereof, preferably coated to a diagnostically useful carrier and one or more, preferably all reagents from the group comprising an antibody to SEQ ID NO: 1, a washing buffer, a means for detecting the presence or absence of an antibody to SEQ ID NO: 1, preferably an IgA, IgG and/or IgM class antibody, more preferably an IgA class antibody, preferably a secondary antibody binding specifically to IgA IgG and/or IgM, more preferably IgA class antibodies, preferably comprising a detectable label, and a dilution buffer.

In a preferred embodiment, the diagnostically useful carrier is selected from the group comprising a bead, preferably a magnetic or paramagnetic bead, a test strip, a microtiter plate, a membrane, preferably from the group comprising a nitrocellulose membrane, western blot, line blot and dot blot, a lateral flow device, a glass surface, a slide, a microarray, a chromatography column and a biochip and is preferably a microtiter plate.

In a preferred embodiment, the kit comprises two or more, preferably three or more calibrators.

In a preferred embodiment, each calibrator is a recombinant antibody binding to SEQ ID NO: 1 which is preferably recognized by a secondary antibody binding to IgA class antibodies. In certain preferred embodiments, the calibrator is a recombinant antibody binding to SEQ ID NO: 1 or variant thereof, and said recombinant antibody is preferably an immunoglobulin (Ig) of the same Ig class as the Ig class to which the secondary antibody binds.

In a 5^{th} aspect, the invention provides a use of a polypeptide comprising SEQ ID NO: 1 or a variant thereof and/or an antibody to SEQ ID NO: 1, preferably IgA class antibody, for the manufacture of a diagnostic kln accordance with this aspect, the invention particularly relates to a use of a polypeptide comprising SEQ ID NO: 1 or a variant thereof for the manufacture of a diagnostic kit. Moreover, the invention also provides a use of an antibody to SEQ ID NO: 1, preferably IgA, IgM and/or IgG, more preferably IgA class antibody, for the manufacture of a diagnostic kit.

The diagnostic kit is preferably for the diagnosis of a SARS-CoV-2 infection, or for the differential diagnosis of a coronavirus infection, preferably for distinguishing between a SARS-CoV, preferably SARS-CoV-2, MERS, NL63, 229E, OC43 and HKU1 infection. Accordingly, the invention relates to a use of a polypeptide comprising SEQ ID NO: 1 or a variant thereof for the manufacture of a diagnostic kit for the diagnosis of a SARS-CoV-2 infection. Said diagnosis of a SARS-CoV-2 infection may, for example, comprise a step of detecting the presence or absence of an antibody to SEQ ID NO: 1, preferably an IgA IgM and/or IgG, more preferably IgA class antibody to SEQ ID NO: 1, in a sample from a subject. Said diagnosis of a SARS-CoV-2 infection may also comprise a step of obtaining a sample from a subject, and a step of detecting the presence or absence of an antibody to SEQ ID NO: 1, preferably an IgA, IgM and/or IgG, more preferably IgA class antibody to SEQ ID NO: 1, in the sample from the subject. The invention further relates to a use of a polypeptide comprising SEQ ID NO: 1 or a variant thereof for the manufacture of a diagnostic kit for the differential diagnosis of a coronavirus infection, preferably for distinguishing between a SARS-CoV, preferably SARS-CoV-2, MERS, NL63, 229E, OC43 and HKU1 infection. Said differential diagnosis may, for example, comprise a step of detecting the presence or absence of an antibody to SEQ ID NO: 1 in a sample from a subject, preferably an IgA, IgM and/or IgG, more preferably IgA and/or IgG class antibody to SEQ ID NO: 1, most preferably an IgA class antibody to SEQ ID NO: 1. Said differential diagnosis may also comprise a step of obtaining a sample from a subject, and a step of detecting the presence or absence of an antibody to SEQ ID NO: 1 in the sample from the subject, preferably an IgA, IgM and/or IgG, more preferably IgA and/or IgG class antibody to SEQ ID NO: 1, most preferably an IgA class antibody class antibody to SEQ ID NO: 1.

In accordance with this aspect, the invention likewise provides a polypeptide comprising SEQ ID NO: 1 or a variant thereof for use in diagnosis, for example for use in a diagnostic method practised on the human or animal body. In particular, the invention provides a polypeptide comprising SEQ ID NO: 1 or a variant thereof for use in the diagnosis of a SARS-CoV-2 infection. Said diagnosis of a SARS-CoV-2 infection may, for example, comprise a step of detecting the presence or absence of an antibody to SEQ ID NO: 1, preferably an IgA, IgM and/or IgG, more preferably IgA and/or IgG class antibody to SEQ ID NO: 1, most preferably an IgA class antibody to SEQ ID NO: 1, in a sample from a subject. Said diagnosis of a SARS-CoV-2 infection may also comprise a step of obtaining a sample from a subject, and a step of detecting the presence or absence of an antibody to SEQ ID NO: 1, preferably an IgA, IgM and/or IgG, more preferably IgA and/or IgG class antibody to SEQ ID NO: 1, most preferably an IgA class antibody to SEQ ID NO: 1, in the sample from the subject. The invention further provides a polypeptide comprising SEQ ID NO: 1 or a variant thereof for use in the differential diagnosis of a coronavirus infection, preferably for distinguishing between a SARS-CoV (preferably SARS-CoV-2), MERS, NL63, 229E, OC43 and HKU1 infection. Said differential diagnosis may, for example, comprise a step of detecting the presence or absence of an antibody to SEQ ID NO: 1 in a sample from a subject, preferably an IgA, IgM and/or IgG, more preferably IgA and/or IgG class antibody to SEQ ID NO: 1, most preferably an IgA class antibody to SEQ ID NO: 1. Said differential diagnosis may also comprise a step of obtaining a sample from a subject, and a step of detecting the presence or absence of an antibody to SEQ ID NO: 1 in the sample from the subject, preferably an IgA, IgM and/or IgG, more preferably IgA and/or IgG class antibody to SEQ ID NO: 1, most preferably an IgA class antibody to SEQ ID NO: 1.

The invention further provides an antibody to SEQ ID NO: 1, preferably an IgA, IgM and/or IgG, more preferably IgA and/or IgG class antibody to SEQ ID NO: 1, most preferably an IgA class antibody, for use in diagnosis, for example for use in a diagnostic method practised on the human or animal body. In particular, the invention relates to an antibody to SEQ ID NO: 1, preferably an IgA, IgM and/or IgG, more preferably IgA and/or IgG class antibody to SEQ ID NO: 1, most preferably an IgA class antibody, for use in the diagnosis of a SARS-CoV-2 infection. The invention further relates to an antibody to SEQ ID NO: 1, preferably an IgA, IgM and/or IgG, more preferably IgA and/or IgG class antibody to SEQ ID NO: 1, most preferably an IgA class antibody, for use in the differential diagnosis of a coronavirus infection, preferably for distinguishing between a SARS-CoV, preferably SARS-CoV-2, MERS, NL63, 229E, OC43 and HKU1 infection.

In a 6^{th} aspect, the invention provides a use of a recombinant antibody binding to SEQ ID NO: 1 which is preferably recognized by a secondary antibody binding to IgA class antibodies as a calibrator for the early diagnosis of a SARS-CoV-2 infection.

In accordance with this aspect, the invention also provides a use of a recombinant antibody, for example a recombinant IgA class antibody, binding to SEQ ID NO: 1 as a calibrator, preferably as a calibrator for the detection of an antibody to SEQ ID NO: 1, preferably an IgA class antibody to SEQ ID NO: 1, in a sample from a subject. Such a calibrator can be used, in particular, in a method of diagnosing a SARS-CoV-2 infection for in a method for the differential diagnosis of a coronavirus infection, preferably for distinguishing between a SARS-CoV, preferably SARS-CoV-2, MERS, NL63, 229E, OC43 and HKU1 infection. The invention thus likewise provides a use of a recombinant antibody, for example a recombinant IgA class antibody, binding to SEQ ID NO: 1 as a calibrator, preferably for the diagnosis, particularly for the early diagnosis, of a SARS-CoV-2 infection.

In a preferred embodiment, the human subject is a vaccinated human subject.

In a preferred embodiment, the method further comprises evaluating the result for the diagnosis. In a preferred embodiment, the method further comprises transferring the result of the diagnosis or the evaluation to a different location.

Various antigens and antibodies have been used as the basis for the immunological detection of coronaviruses, among them the whole virus or any of the structural proteins or fragments thereof as well as antibodies binding to these antigens.

The present invention is based on the inventors' surprising finding that antibodies against SEQ ID NO: 1 can be detected at an early stage of the infection. They may be detected for the purpose of determining the presence of an infection at an early stage of the infection.

In other words, the results disclosed herein demonstrate a superior sensitivity, preferably *as measured by the number of correctly positive determined samples relative to the total number* of *samples examined,* for diagnosing a SARS-CoV-2 infection already at early stage of infection. An important contribution to the observed sensitivity increase arises from the surprising finding that IgA class antibodies to SEQ IDNO:1 become detectable earlier than antibodies of other Ig classes, such as IgG antibodies in many patients, including a subpopulation of patients who lack detectable IgM antibodies.

Moreover, the invention is based on the surprising finding that antibodies to SEQ ID NO: 1, preferably IgG and/or IgA, persist longer and are detectable for a longer period of time than antibodies to SARS-CoV-2 N protein.

Also, there is surprisingly low cross reactivity with a range of antibodies in samples from patients infected with other coronaviruses, preferably other than SARS-CoV-1 and SARS-CoV-2, which contributes to the superior specificity, preferably *as measured by the high number of correctly identified negative samples, i.e. low number of false-postive identified samples relative to the total number of samples examined,* of detection of antibodies to SEQ ID NO: 1. In particular, the inventors have found that the specificity of SEQ ID NO1 as an antigen is superior compared to SEQ ID NO: 33 (S2 domain) (Okba et al., Severe Acute Respiratory Syndrome Coronavirus 2-Specific Antibody Responses in Coronavirus Disease Patients. Emerg Infect Dis. 2020 Jul;26(7):1478-1488, prepublished on medRxiv 2020.03.18.20038059).

In a preferred embodiment, the term "diagnosis", as used herein, is to be understood in its broadest possible sense and may refer to any kind of procedure aiming to obtain information instrumental in the assessment whether a patient suffered, suffers or is likely or more likely than the average or a comparative subject, the latter preferably having similar symptoms, to suffer from a certain disease or disorder in the past, at the time of the diagnosis or in the future, to find out how the disease is progressing or is likely to progress in the future or to evaluate the responsiveness of a patient or patients in general with regard to a treatment, preferably a vaccine, or to find out whether a sample is from such a patient. Such information may be used for a clinical diagnosis but may also be obtained by an experimental and/or research laboratory for the purpose of general research, for example to determine the proportion of subjects suffering from the disease in a patient cohort or in a population. In other words, the term "diagnosis" comprises not only diagnosing, but also prognosticating and/or monitoring the course of a disease or disorder, including monitoring the response of one or more patients to the administration of a drug or candidate drug, for example to determine its efficacy. Again, the early emergence and/or the long persistence of IgA and/or IgG antibodies to SEQ ID NO:1 may be exploited. While the result may be assigned to a specific patient for clinical diagnostic applications and may be communicated to a medical doctor or institution treating said patient, for example by telephone, fax, letter or in an electronic format such as e-mail or using a data base, this is not necessarily the case for other applications, for example in diagnostics for research purposes, where it may be sufficient to assign the results to a sample from an anonymized patient or a patient cohort. In a preferred embodiment, the person to be diagnosed, i.e., the "subject" or "patient", is an anonymous blood donor whose blood may be donated or used to obtain therapeutically useful antibodies. Preferably, the disease is a SARS infection, including SARS-CoV-1 and SARS-CoV-2, more preferably a SARS-CoV-2 infection.

In a preferred embodiment, the methods, products and uses according to the present invention may be used for interaction studies, including determining whether a drug candidate or other compound, including a candidate vaccine, or any bodily compound such as a blocking antibody is present and may interfere with the binding of an antibody to SARS-CoV-2 or may affect any downstream process. In a preferred embodiment, they may be used for monitoring the immune response, more preferably the emergence and/or titer of antibodies to a polypeptide comprising, preferably consisting of SEQ ID NO: 1, following the administration of an immunogenic composition comprising a polypeptide comprising SEQ ID NO: 1 or an immunogenic variant thereof, for example to a mammal, which may be a mammal other than a human, such as a laboratory animal. The detection of IgA and/or IgG antibodies, preferably in the late phase of the immunization of a subject, more preferably for the purpose of increasing the sensitivity of the detection or optimizing the sensitivity of the detection, is particularly preferred. In a preferred embodiment, the immunization is the result of a SARS-CoV-2 infection or the result of an administration of a vaccine comprising SEQ ID NO: 1 or a variant thereof. The increase or optimization may be in comparison to other antibodies against SARS-CoV-2 or components thereof, for example SEQ ID NO: 30 (N protein), preferably IgG class antibodies to SEQ ID NO30. In a preferred embodiment, the term "late phase", as used herein, refers to the period beginning with day 20, 30, 40, 50, 60, 61, 70, 80 or 90 after the onset of the disease or the first administration of a vaccine, preferably day 60. Preferably it lasts for 28 days, 1, 2, 3, 4, 5, 6, 9, 12, 15, 18, 24, 36, 48, 60 or more months, preferably 3 or more months. In a preferred embodiment, a method or use may be for the long-term monitoring of an immunization. The immunization may be the result of a SARS-CoV-2 infection or of a vaccination, preferably with SEQ ID NO1 or a variant thereof. At least one sample obtained during the late phase is analyzed, preferably two or more. A sample may be obtained and analyzed at least once a week or once a months during the late phase.

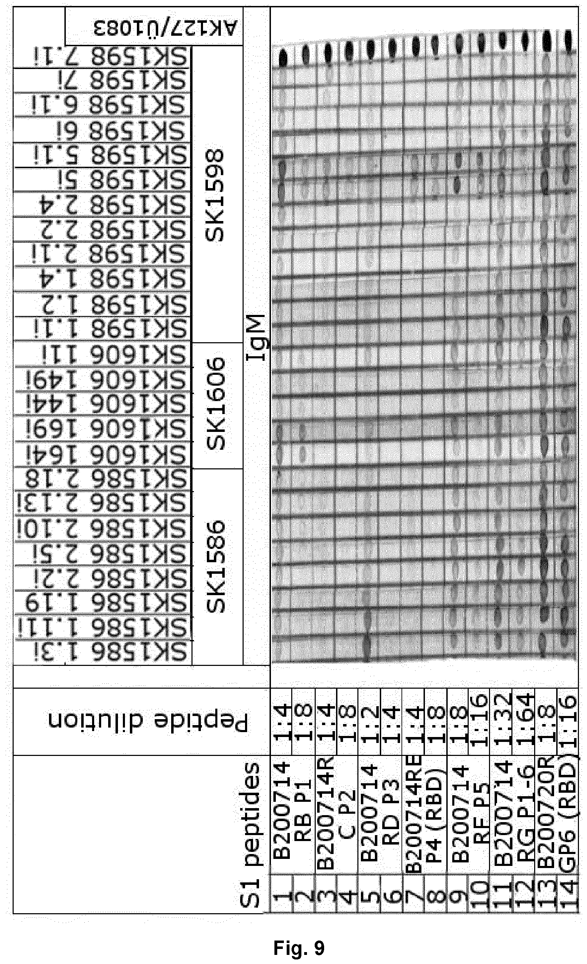

The subject is likely or more likely to suffer from a SARS-CoV-2 infection if an IgA and/or IgG and/or IgM antibody to SEQ ID NO: 1 is detected in a sample from them. The person skilled in the art is familiar with general principles in virology regarding the interpretation of results reflecting the presence or absence of antibodies (for example, Doerr, H. W., and Gerlich, W., Medizinische Virologie: Grundlagen, Diagnostik, Prävention und Therapie, Thieme 2010, for example Fig. 9.7 therein). Briefly, the first detection of specific antibodies to virus-specific antigens may be used for the initial diagnosis of an infection. IgA antibodies are usually not diagnostically relevant except for specific cases, such as in cases of infections by entero viruses (Gressner/Arndt, Lexikon der Medizinischen Labordiagnostik, 2. Auflage, Springer, 2013, page 1387), but if they appear, their titer will typically be lower than IgM and IgG class antibodies, and they may appear later. A low concentration of IgG class antibodies, preferably in the absence of IgM and IgA class antibodies, indicates an immunization in the past. An increasing IgG class antibody titer or high concentration may be indicative of an acute infection or reinfection. In a preferred embodiment, the presence of an antibody to SEQ ID NO: 1 indicates an immunization, either as a result of a previous or ongoing SARS-CoV-2 infection or a vaccination. In a preferred embodiment, a successful vaccination with a vaccine comprising a polypeptide comprising SEQ ID NO: 1 or a variant thereof (or with a nucleic acid encoding a polypeptide comprising SEQ ID NO: 1 or variant thereof, e.g. a RNA-based vaccine), may be distinguished from a SARS-CoV-2 infection by confirming the absence of antibodies to SARS-CoV-2 antigens other than SEQ ID NO: 1, preferably by confirming the absence of antibodies to SEQ ID NO: 30 (SARS-CoV-2 N protein).

The sample is preferably a mammalian sample, i.e., a sample from a mammal, more preferably a human sample, i.e., a sample from a human.

The term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. According to the invention, it can be distinguished if a patient, preferably one already suspected to have a coronavirus infection, suffers from a SARS, preferably SARS-CoV-1 and/or SARS-CoV-2 infection, or another coronavirus infection, preferably from the group comprising MERS, NL63, 229E, OC43 and HKU1. NL63, 229E, OC43 and HKU1 are associated with a significant number of cases of common cold, hence the differential diagnosis may involve distinguishing between SARS-CoV-1 and/or SARS-CoV-2 and a cold. For example, a patient may initially be suspected of suffering from a coronavirus infection owing to a possible exposure to a risk environment and/or based on common symptoms such as fever, cough and shortness of breath. A PCR (for example Corman VM, Landt O, Kaiser M, et al. Detection of 2019 novel coronavirus (2019-nCoV) by real-time RT-PCR. Euro Surveill. 2020;25(3):2000045. doi:10.2807/1560-7917.ES.2020.25.3.2000045) and/or an immunoassay may then be carried out. If the PCR is negative, for example because the sample was taken a few days after the infection, the inventive method may be used to detect the presence or absence of an antibody to SEQ ID NO: 1. In addition, the presence or absence of an antibody to an antigen from another coronavirus may be detected, preferably from the group comprising SARS-CoV-1, MERS, NL63, 229E, OC43 and HKU1, more preferably MERS, NL63, 229E, OC43 and HKU1. Antibodies to homologues and variants of SEQ ID NO: 1 from SARS-CoV-2 may be detected. Based on specific clinical symptoms such as headache and body pains, which are more characteristic of SARS-CoV-1, or loss of taste and smell and sore throat, which are more characteristic for SARS-CoV-2, the diagnosis may be finalized. It may be considered whether the patient has been exposed to infected patients. For example, SARS-CoV-1 cases are extremely rare, so many patients suffering from common SARS-CoV-1 and SARS-CoV-2 symptoms in a SARS-CoV-2 pandemic can be assumed to suffer from an infection with the latter coronavirus. Distinction between SARS-CoV-1 and SARS-CoV-2 is possible based on the different time-resolved immunoglobulin (Ig) class signature, in particular the later emergence of IgA class antibodies in SARS-CoV-1 (Hsue, P. R., Huang, L. M., Chen, P. J., Kao, C. L., and Yang P. C. (2004) Chronological evolution of IgM, IgA, IgG and neutralization antibodies after infection with SARS-associated coronavirus, Clinical Microbiology and Infection, 10(12), 1062-1066). Antibody levels may be monitored, for example over several weeks, for example to detect the disappearance or emergence of an antibody of interest, which may help distinguish a primary and a secondary infection or immunization, for example as a result of vaccination, or recognize an infection with more than one coronavirus.

In a preferred embodiment, the term "SARS-CoV-2", as used herein, refers to a virus characterized by the genome deposited on GenBank under accession code MN908947 or SEQ ID NO: 13, preferably as shown in SEQ ID NO: 13, and derivatives thereof having at least 80, preferably 85, preferably 88, preferably 90, preferably 91, preferably 92, preferably 93, preferably 94, preferably 95, preferably 96, preferably 97, preferably 98, preferably 99, preferably 99.5, preferably 99.8, preferably 99.9 or 99.99 percent sequence identity over the entire genome nucleotide sequence. All data base entries or product codes used herein correspond to the version online at the earliest priority or filing date of the application. For example, for the SARS-CoV-2 genome sequence deposited under accession code MN908947, the version MN908947.3 (published on January 17, 2020) was online at the earliest priority or filing date of the present application. The nucleotide sequence disclose in MN908947.3 is identical with SEQ ID NO13. More preferably, mutants such as those from the group comprising the U.K. variant B.1.1.7, the South African variant B.1.351, the Brazilian variant P.1 and the Mink Variant from Denmark are included.

In a preferred embodiment, the SARS-CoV-2 infection to be diagnosed is or may be associated with the U.K. variant B.1.1.7 characterized by a spike protein having, with reference to SEQ ID NO1, one or more mutations, preferably all mutations from the group comprising deletion(s) in His 54 and/or Val55, Gln486Tyr, Ala555Asp, Asp599Gly and Pro666His. Preferably, a variant of SEQ ID NO1 having one or more mutations, preferably all from the group comprising deletion(s) in His 54 and/or Val55, Gln486Tyr, Ala555Asp, Asp599Gly and Pro666His, is used for reagents, methods and uses according to the present invention. A variant of SEQ ID NO1 comprising all these mutations is represented by SEQ ID NO: 51.

In a preferred embodiment, the SARS-CoV-2 infection to be diagnosed is or may be associated with the South African variant B.1.351 characterized by a spike protein having, with reference to SEQ ID NO1, one or more mutations, preferably all mutations from the group comprising Lys402Gln, Glu469Lys, Gln486Tyr and Asp599Gly. Preferably, a variant of SEQ ID NO1 having one or more mutations, preferably all from the group comprising Lys402Gln, Glu469Lys, Gln486Tyr and Asp599Gly, is used for reagents, methods and uses according to the present invention. A variant of SEQ ID NO1 comprising all these mutations is represented by SEQ ID NO: 52.

In a preferred embodiment, the SARS-CoV-2 infection to be diagnosed is or may be associated with the Brazilian variant P.1 characterized by a spike protein having, with reference to SEQ ID NO1, one or more mutations, preferably all mutations from the group comprising Glu469Lys and Gln486Tyr. Preferably, a variant of SEQ ID NO1 having one or more mutations, preferably all from the group comprising Glu469Lys and Gln486Tyr, is used for reagents, methods and uses according to the present invention. A variant of SEQ ID NO1 comprising all these mutations is represented by SEQ ID NO: 53.

In a preferred embodiment, the SARS-CoV-2 infection to be diagnosed is or may be associated with the Mink Variant from Denmark characterized by a spike protein having, with reference to SEQ ID NO1, one or more mutations, preferably all mutations from the group comprising deletion(s) in His 54 and/or Val55,Asp599Gly and Tyr438Phe. Preferably, a variant of SEQ ID NO1 having one or more mutations, preferably all from the group comprising deletion(s) in His 54 and/or Val55, Asp599Gly and Tyr438Phe, is used for reagents, methods and uses according to the present invention. A variant of SEQ ID NO1 comprising all these mutations is represented by SEQ ID NO: 54.

In a preferred embodiment, the term "diagnosis" means that the method or product or use may be used for aiding in the diagnosis of a disease or identifying a subject with a risk of suffering from a disease. The term "diagnosis" may also refer to a method or agent used to choose the most promising treatment regime for a patient. In other words, the method or agent may relate to selecting a treatment regimen for a subject.

In a preferred embodiment, the method according to the present invention comprises the step of providing the diagnostically useful carrier and a sample from a patient suspected of being infected, preferably a mammalian, more preferably a human patient. The carrier is coated with the polypeptide comprising SEQ ID NO: 1 or variant thereof. The carrier may then be contacted with the sample under conditions allowing for binding of any antibodies to the polypeptide comprising SEQ ID NO: 1 or variant thereof. The sample may then be removed and the carrier may be washed to remove any remaining sample. A secondary antibody or similar reagent or means binding to the antibody to be detected and carrying a detectable label may then be contacted with the carrier under conditions allowing formation of a complex between any bound antibody and the secondary antibody. The carrier may be washed then to remove non-bound secondary antibody. Finally, the presence of the antibody is detected by checking whether the secondary antibody may be detected.

In a preferred embodiment, the term "SARS-CoV-2 infection" or similar terms, as used herein, refers to an infection of a subject, preferably a human subject, with SARS-CoV-2, i.e., the presence of the virus, at least temporarily, in the body of said subject, more preferably together with detectable levels of the virus itself and/or one or more biomarker from the group comprising a SARS-CoV-2 polypeptide from the group comprising the structural proteins, in particular S and N, more preferably the S1 domain, and antibodies binding to them, and/or a nucleic acid from SARS-CoV-2, the latter detectable by PCR. The subject may have clinical symptoms, preferably one or more, more preferably all from the group comprising fever, tiredness, dry cough, nasal congestion, runny nose, and sore throat. More severe symptoms include breathing difficulties, chest pain or pressure and sudden confusion. The disease may lead to complications such as pneumonia, acute respiratory distress syndrome, sepsis, septic shock and kidney failure. However, many infected subjects have mild symptoms only and may not even be aware of their infection.

In a preferred embodiment, the method is used more than once to examine samples from the same patient, preferably on different days. For example, the presence or absence of antibodies may be detected on a daily basis over one or two weeks. In a preferred embodiment, at least 2, 3, 4, 5, 6, 7, 8, 9 or 10 samples are examined on different days. In a preferred embodiment, samples are taken at least over a period of at least 2, 3, 4, 5, 6, 7, 8, 9, 10, 11, 12, 13, 14, 15 or 16 weeks.

The methods, reagents and uses according to the present invention may also be used for screening the potency and the usefulness of an antiviral drug or a vaccine or vaccine candidate. In this regard, it is of note that the method of the invention has been validated in a vaccinated human, i.e. an antibody to the polypeptide comprising the amino acid sequence of SEQ ID NO: 1 could be detected in a human vaccinated with a composition comprising a polypeptide omprising SEQ ID NO1. They may be used as part of vaccination trials and studies aiming to confirm the quality of vaccines in human and other subjects including laboratory animals or to confirm that a subject's immune system responds to the administration of a vaccine. Preferably the vaccine is based on SEQ ID NO: 1 or a fragment thereof. The methods, reagents and uses may be used to initially diagnose a patient before the initial administration of a vaccine to check whether said patient is already immunized, for example as a result of a previous infection with SARS-CoV-2 or a previous vaccination. For example, the patient may be selected for inclusion in or exclusion from a study or trial and monitored over time. In particular already infected subjects may be excluded from testing a vaccine candidate. It can be confirmed whether a previous vaccination is still effective, preferably based on the detection of IgA and/or IgG class antibodies to SEQ ID NO:1.

The methods and reagents according to the present invention may also be used for screening whether donated blood is contaminated with coronavirus or whether a blood donor produces antibodies to SARS-CoV-2, preferably SEQ ID NO: 1, which may be extracted from his donated blood, for example for therapeutic uses. Following the detection of the presence or absence of IgG, IgM and/or IgA class antibodies, preferably IgG and IgA, more preferably IgA, to SEQ ID NO: 1, an antibody to SARS-CoV-2, preferably to SEQ ID NO: 1 may be purified from the blood donor's blood, for example by affinity chromatography or by contacting the carrier according to the present invention with the donated blood under conditions that are compatible with the formation of a complex comprising the antibody to SEQ ID NO: 1 and the carrier, removal of any remaining donated blood and separation of the antibody from the carrier. In a preferred embodiment, donated blood is selected from the group comprising whole blood, plasma, and serum, and may contain an anti-clotting reagent. Also a compound selected from the group comprising citrate, phosphate, dextrose and adenine, preferably all of them, may be present.

The antibody to be detected binds preferably specifically to SEQ ID NO: 1. Specific binding preferably means that the binding reaction is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

The teachings of the present invention may not only be carried out using polypeptides having the exact sequences referred to in this application explicitly, such as SEQ ID NO: 1, for example by function, name, sequence or accession number, or 6

In accordance with the invention, the term "variant", as used herein, also refers to at least one fragment of the full length sequence referred to, or a polypeptide comprising said fragment, more specifically to one or more amino acid or nucleic acid sequences which are, relative to the full length sequence, truncated at one or both termini by one or more amino acids. Such a fragment comprises or encodes a (poly-)peptide having at least 10, 15, 25, 50, 75, 100, 150, 200, 250, 300, 400, 500 or 600 successive amino acids of the original sequence or a variant thereof. For example, fragments of SEQ ID NO: 1 include SEQ ID NO: 12 and SEQ ID NO: 31. Two or more copies of a fragment may be fused for increased sensitivity.

It is also understood that the term "variant", as used herein, also embraces such polypeptides or fragments thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or maintain the fold or structure of the polypeptide may be deleted or substituted and/or one or more such essential amino acids may be replaced in a conservative manner and/or amino acids are added or deleted such that the biological activity of the polypeptide is at least partially preserved. For example, fragments of SEQ ID NO:1 in comprise SEQ ID NO:5, SEQ ID NO:12, SEQ ID NOs:14-29, SEQ ID NOs:35-37 and SEQ IDNOs:40-43. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

SARS-CoV-2-related publications on specific amino acid sequences such as Beal *et al.* may aid the skilled one in designing variants (Beal, J., Mitechell, T., Wyschogrod, W., Manthey, J, and Clore, A. (2020) Highly Distinguished Amino Acid Sequences of 2019-nCoV (Wuhan Coronavirus) doi: https://doi.org/10.1101/2020.01.31.929497), as well as publications relating to SARS-CoV, for example Hua *et al.* (Hua, R., Zhou, Y., Wang, Y., Hua, Y and Tong, T. (2004) Identification of two antigenic epitopes on SARS-CoV spike protein, BBR 319, 929-935), wherein homologous epitopes may be found and SARS-CoV-2 epitopes be identified on account of their homology. For example, possible epitopes may be derived from SEQ ID NO: 5. Dahlke *et al.* present an epitope mapping based on a microarray comprising overlapping 15mer peptides derived from the S1 polypeptide (Dahlke, C., Heidepriem, J., Kobbe, R., Santer R., Koch, T., Fathi, A., Ly, M. L, Schmiedel, S., Seeberger, P. H., ID-UKE COVID-19 study group, Addo, M. M., and Loeffler, F. F. (2020) https://doi.org/10.1101/2020.04.14.20059733doi). More specifically, peptides comprising amino acid sequences SSVLHSTQDLFLPFF (SEQ ID NO: 14, which is 30-44 of SEQ ID NO: 1), TWFHAIHVSGTNGTKRFDNPV (SEQ ID NO: 15, which is 48-68), NVVIKVCEFQFCNDPFLGVYYHKNNKSWMESEFRVYSSANNCTFEYVSQPFLMDLEG (SEQ ID NO: 16, which is 110-166), DLPQGFSALEPLVDL (SEQ ID NO: 17, which is 200-214), LLALHRSYLTPGDSSSGWTAGAAAY (SEQ ID NO: 18, which is 226-250), QPRTFLLKYNENGTITDAVDCALDP (SEQ ID NO: 19, which is 256-277), NATRFASVYAWNRKR (SEQ ID NO: 20, which is 328-342), TGKIADYNYKLPDDF (SEQ ID NO: 21, which is 399-414), YNYLYRLFRKSNLKP (SEQ ID NO: 22, which is 434-448), FGRDIADTTDAVRDPQTLEILDI (SEQ ID NO: 23, which is 550-572), SNQVAVLYQDVNCTE (SEQ ID NO: 24, which is 590-604), AGCLIGAEHVNNSYECDIP (SEQ ID NO: 25, which is 632-650) of S1 protein were identified as IgAreactive epitopes. Peptides comprising amino acid sequences YNSASFSTFKCYGVS (SEQ ID NO: 26, which is 354-368), STGSNVFQTRAGCLI (SEQ ID NO: 27, which is 622-636) of S1 protein were identified as IgG-reactive epitopes. Peptides comprising amino acid sequences SSVLHSTQDLFLPFF (SEQ ID NO: 28, which is 30-44) and LLALHRSYLTPGDSSSGWTAGAAAY (SEQ ID NO: 29, which is 226-250) of S1 protein were identified as IgM-reactive epitopes. Moreover, the inventors have shown that peptides having sequences RTQLPPAYTNS (SEQ ID NO: 41), LTPGDSSSGWTAG (SEQ ID NO: 35), YQAGSTPCNGV (SEQ ID NO: 36) and YGFQPTNGVGYQ (SEQ ID NO: 37) are reactive with antibodies from SARS-CoV-2 patients. Many other publications can be used by the person skilled in the art as guidance when designing variants (Zhang et al. (2020) Mining of epitopes on spike protein of SARS-CoV-2 from COVID-19 patients, Cell Res 30, 702-704 (2020). https://doi.org/10.1038/s41422-020-0366-x; Poh, C.M., Carissimo, G., Wang, B. et al. Two linear epitopes on the SARS-CoV-2 spike protein that elicit neutralising antibodies in COVID-19 patients. Nat Commun 11, 2806 (2020). https://doi.org/10.1038/s41467-020-16638-2; Wang et al. (2020) SARS-CoV-2 Proteome Microarray for Mapping COVID-19 Antibody Interactions at Amino Acid Resolution, ACS Cent. Sci. 2020, 6, 12, 2238-2249). In a preferred embodiment, a polypeptide is used which comprises SEQ ID NO: 1 or a variant thereof and is folded and, more preferably comprises at least 150, 200, 250, 300, 400, 500 or 600 successive amino acids of SEQ ID NO: 1.

In a preferred embodiment, variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, affinity tags, other antigens and the like. For example, SEQ ID NO: 3, SEQ ID NO: 32, SEQ ID NO 34 and SEQ ID NO: 38 are fusion proteins according to the present invention.

According to the present invention, a medical or diagnostic device such as the diagnostically useful carrier may be prepared by expressing a recombinant variant of SEQ ID NO: 1 comprising an affinity tag, optionally with an artificial linker which may include a protease cleavage site, in a cell such as a eukaryotic (such as a CHO or HEK293 cell) or prokaryotic (such as an Ecoli cell) cell, contacting the expressed variant with a ligand binding specifically to the affinity tag, which ligand is immobilized on a solid phase, washing the solid phase such that non-specifically bound material from the cell is removed and eluting the expressed variant from the solid phase, preferably by adding an excess of nonimmobilized ligand. The variant may then be immobilized on the device. Optionally, the affinity tag may be removed by contacting the variant with a protease, preferably a protease recognizing the protease cleavage site, preferably before the immobilization. The affinity tag may be selected from the group of tags comprising His, 18A, ACP, aldehyde, Avi, BCCP, calmodulin-binding peptide (CBP), chitin binding domain (CBD), E-Tag, ELK16, FLAG, flash, poly-glutamate, poly-aspartate, GST, Green fluorescent protein, HA, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag. Useful proteases include, but are not limited to TEV, Thrombin, Faktor Xa or Enteropeptidase. Suitable linkers comprising a protease cleavage site (e.g. between SEQ ID NO: : 1 or variant thereof and the affinity tag) may be comprised in the variant, and be part of the coding sequence of commercially available expression vectors, for example pET vector series (Novagen) that may be used for expression of said variant.

The variant of the polypeptide has biological activity. In a preferred embodiment, such biological activity is the ability to bind to the respective antibody. In a preferred embodiment it comprises an epitope having the ability or it has itself the ability to bind to an antibody to SEQ ID NO: 1, preferably an IgA class antibody to SEQ ID NO: 1, preferably from a sample from a patient suffering from SARS-CoV-2, wherein more preferably the epitope comprises a sequence comprising at least 5, 6, 7 or 8 amino acid residues. More preferably, it does not bind specifically to homologues of SEQ ID NO: 1 from other coronaviruses, preferably from the group comprising MERS (SEQ ID NO: 6), NL63 (SEQ ID NO: 10), 229E (SEQ ID NO: 7), OC43 (SEQ ID NO: 8) and HKU1 (SEQ ID NO: 9), more preferably from the group comprising SARS-CoV-1 (SEQ ID NO: 11), MERS, NL63, 229E, OC43 and HKU1, wherein specific binding is preferably defined and determined as outlined above, using Biacore equipment.

The person skilled in the art is familiar how to make diagnostically useful reagents based on suitable amino acid sequences and is able to provide both linear peptides, for example by chemical synthesis, and polypeptides, for example by recombinant expression. In particular, the person skilled in the art is aware that both conformational and sequential epitopes exist, and that a suitable strategy for expression and purification must be chosen to obtain a diagnostically useful polypeptide which may be used to detect an antibody such as the antibody to SEQ ID NO: 1 (for example Reischl, U., Molecular Diagnosis of Infectious diseases, Humana Press, 1998, for example chapters 12, 15, 16, 18 and 19). The person skilled in the art is also familiar with ways to evaluate the usefulness of recombinant proteins for test systems (for example Reischl, U., Molecular Diagnosis of Infectious diseases, Humana Press, 1998, for example chapters 21-26).

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. In a preferred embodiment, the complex is detected using a method selected from the group comprising immunodiffusion techniques, immunoelectrophoretic techniques, light scattering immunoassays, agglutination techniques, labeled immunoassays from the group comprising radiolabeled immunoassays, chemiluminscence immunoassays, immunofluorescence techniques and immunoprecipitation techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. Preferably the test format is an ELISA and a microtiter plate comprising wells is used as a diagnostically useful carrier.

Preferably a polypeptide comprising SEQ ID NO: 1 or a variant thereof is immobilized on a solid phase of the diagnostically useful carrier. It may be directly immobilized on the solid phase when contacted with the sample, but a competitive assay, a capture bridge assay, an immunometric assay, a classspecific second antibody on the solid phase, a class capture assay, direct or indirect, may also be used. The principle of each of these formats is detailed in The Immunoassay Handbook, 3rd edition, edited by David Wild, Elsevier, 2005. More preferably, the solid phase is a test strip or a well of a microtiter plate for ELISA, most preferably a well of a microtiter plate for ELISA.

In a preferred embodiment, a competitive assay format is used, wherein the antibody to be detected competes with another antibody to SEQ ID NO: 1 or another ligand binding specifically to SEQ ID NO: 1. The ligand binding specifically to SEQ ID NO: 1 may be selected from the group comprising an aptamer or antibody binding to SEQ ID NO: 1 and the human ACE2 receptor (SEQ ID NO: 39) or a variant thereof, the natural binding partner of the SARS-CoV-2 spike protein. Such a method may comprise providing a polypeptide comprising SEQ ID NO: 1 or variant thereof and the ligand binding specifically to SEQ ID NO: 1, preferably from the group comprising an antibody, an aptamer and the ACE receptor or a variant thereof. If the antibody to be detected is present, it will interfere with the formation of the complex or partially or fully displace the ligand binding specifically to SEQ ID NO: 1, thus reducing the number of the complexes. Any complex comprising the antibody to be detected may then be detected by precipitating the complex, for example using an affinity ligand attached to the polypeptide comprising SEQ ID NO: 1 or to a molecule binding to the antibody to be detected such as a secondary antibody. Examples of affinity ligands include glutathione and biotin. A binding partner of the affinity ligand may be coated on a solid phase which binds to the affinity ligand such as GST or streptavidin, respectively. Any complex precipitated may then be detected, preferably by detecting a detectable label attached to the ligand binding specifically to the polypeptide comprising SEQ ID NO: 1 or attached to the polypeptide comprising SEQ ID NO: 1, respectively. A specific competitive test has been published by Tan et al. (2020) A SARS-CoV-2 surrogate virus neutralization test based on antibody-mediated blockage of ACE2-spike protein protein interation, Nature Biotechnology 38 (1073-1078).

Alternatively, the complex may be formed on the surface of a carrier if the ligand binding specifically to SEQ ID NO: 1 or the polypeptide comprising SEQ ID NO: 1 or a variant is coated on said surface. Normally, the presence or absence of IgA, IgM and IgG class antibodies interfering with the complex formation will be detected using such a format. Specific IgA class antibodies may be detected by isolating the antibodies of the Ig class of interest before, preferably from the group comprising IgA, IgM and IgG class antibodies preferably Ig, for example using Protein A or Protein G or a secondary antibody. The person skilled in the art is familiar with the synthesis, selection and use of aptamers (Thiviyanathan V, Gorenstein DG. Aptamers and the next generation of diagnostic reagents. Proteomics Clin Appl. 2012;6(11-12):563-573. doi:10.1002/prca.201200042) and antibodies (Hust M, Frenzel A, Schirrmann T, Dübel S. Selection of recombinant antibodies from antibody gene libraries. Methods Mol Biol. 2014;1101:305-20; Hanack K, Messerschmidt K, Listek M. Antibodies and Selection of Monoclonal Antibodies. Adv Exp Med Biol. 2016;917:11-22; Harold F. Stills, in The Laboratory Rabbit, Guinea Pig, Hamster, and Other Rodents, 2012) and with generating and selecting antibodies binding to specific targets (Lottspeich/Engels, Bioanalytic, Chapter 6 and references therein, Springer 2012).

In another preferred embodiment, a complex comprising a first and a second polypeptide comprising SEQ ID NO: 1 or a variant thereof, such as RBD, and the antibody to be detected may be formed in a liquid phase if the antibody is present in the sample, since the antibody has two or more binding sites, each binding to a polypeptide comprising SEQ ID NO: 1. Any complex comprising the antibody to be detected may then be detected by precipitating the complex, for example using an affinity ligand attached to the first polypeptide such as glutathione or biotin and a binding partner coated on a solid phase which binds to the affinity ligand such as GST or streptavidin, respectively, which solid phase may for example be a bead. Any complex precipitated may then be detected, preferably by detecting a detectable label attached to the second polypeptide. Alternatively, the complex may be formed on the surface of a carrier if the first polypeptide comprising SEQ ID NO: 1 or a variant thereof is coated on said surface. Alternatively, the first and the second polypeptide may each be labeled with two different labels which are detectable only if they are in close proximity, for example when bridged by the antibody to be detected. Again, the presence or absence of IgA, IgM and IgG class antibodies will be detected unless the specific Ig class antibodies have been isolated before, preferably from the group comprising IgA, IgM and IgG class antibodies, preferably IgA, for example using Protein A or Protein G or a secondary antibody. Preferably, in an early phase of the infection, when IgG antibodies have not yet been produced, IgA antibodies will predominantly or only be detected.

Any polypeptide, for example a polypeptide comprising SEQ ID NO: 1 or a secondary antibody, may be provided in any form and at any degree of purification, from tissues, fluids or cells comprising said polypeptide in an endogenous form, more preferably cells overexpressing the polypeptide, crude or enriched lysates of such cells, to purified and/or isolated polypeptide which may be essentially pure. In a preferred embodiment, the polypeptide is a native polypeptide, wherein the term "native polypeptide", as used herein, refers to a folded polypeptide, more preferably to a folded polypeptide purified from cells, more preferably from prokaryotic or eukaryotic, preferably mammalian cells. A glycosylated form of the polypeptide may be used. Secondary antibodies are preferably pure, for example following purification based on Protein A or G as an affinity ligand. In a preferred embodiment, any polypeptide used or provided according to the present invention is used or provided in a folded state, in the absence of significant concentrations of denaturing reagents such as thiol-containing compounds such as DTT.

In a preferred embodiment, the presence or absence of an IgA, IgM and/or IgG class antibody to SEQ ID NO: 1 is detected. In another preferred embodiment, the presence or absence of IgA is detected only. In another preferred embodiment, the presence or absence of IgM is detected only. In another preferred embodiment, the presence or absence of IgG is detected only.

In a preferred embodiment, the presence or absence of IgA and the presence or absence of IgG is detected. More preferably, a secondary antibody to IgA class antibodies and a secondary antibody to IgG class antibodies are used or provided for this detection. In another preferred embodiment, the presence or absence of IgM and the presence or absence of IgG is detected. More preferably, a secondary antibody to IgM class antibodies and a secondary antibody to IgG class antibodies are used or provided for this detection. In another preferred embodiment, the presence or absence of IgM and the presence or absence of IgA is detected. More preferably, a secondary antibody to IgM class antibodies and a secondary antibody to IgA class antibodies are used or provided for this detection.

In a preferred embodiment, the presence or absence of an antibody, for example IgA, IgM and/or IgG to SEQ ID NO: 1, may be detected using a polypeptide comprising SEQ ID NO: 1 or a variant thereof, but the polypeptide may comprise additional sequences, preferably artificial sequences for example linkers or binding epitopes. However, such additional sequences are chosen such that the ability of SEQ ID NO: 1 or a variant thereof to bind specifically to the antibody to be detected or the diagnostic reliability, in particular sensitivity and/or specificity, is not significantly altered, let alone abolished. For example, a domain binding to SEQ ID NO: 1 or a fragment thereof, thus masking an epitope, should not be fused to the polypeptide or be present. According to the present invention, a secondary antibody detecting IgA, IgM and/or IgG class immunoglobulins to a SARS-CoV-2 antigen, preferably from the group comprising an antigen comprising SEQ ID NO: 1 or a variant thereof, an antigen comprising SEQ ID NO: 30 or a variant thereof, an antigen comprising SEQ ID NO: 31 or a variant thereof and an antigen comprising SEQ ID NO: 33 or a variant thereof, preferably all, may be detected or used for the diagnosis, preferably early diagnosis of a SARS-CoV-2 infection. An antigen selected from the group comprising an antigen comprising SEQ ID NO: 30 or a variant thereof, an antigen comprising SEQ ID NO: 31 or a variant thereof and an antigen comprising SEQ ID NO: 33 or a variant thereof, preferably all, may be coated on a diagnostically useful carrier, preferably spatially separated or in a mixture, and contacted with a sample for detecting an antibody binding specifically to the respective antigen or antigens. In a preferred embodiment, the detection of an antibody to SEQ ID NO: 1 in addition to the detection of an antibody to such a SARS-CoV-2 antigen other than SEQ ID NO: 1 may increase the overall sensitivity of the assay, in particular diagnosing a SARS-CoV-2 infection at an early stage or for the differential diagnosis at an early stage.

In a preferred embodiment, the products, methods and uses of the present invention are configured such that presence or absence of an antibody to SEQ ID NO: 1 can be distinguished from the presence of an antibody to another SARS-CoV-2 antigen or one or more other antigen, preferably from SARS-CoV-2 N protein, more preferably from the group comprising SARS-CoV-2 N protein, SARS-CoV-2 M protein and SARS-CoV-2 E protein, most preferably from the group comprising SARS-CoV-2 N protein, SARS-CoV-2 M protein SARS-CoV-2 E protein and SARS-CoV-2 S protein epitopes other than those present on SEQ ID NO: 1. In a more preferred embodiment, a polypeptide comprising SEQ ID NO: 1 or a variant thereof is spatially separated from such other SARS-CoV-2 antigens or other coronavirus antigens when used to detect the presence or absence of an antibody to SEQ ID NO: 1. For example, the polypeptide comprising SEQ ID NO: 1 or a variant may be pure and/or isolated on a carrier. For example, it could be on a blot or microtiter well or bead, spatially separate from other antigens.

In a preferred embodiment, the products, methods and uses of the present invention are configured such that presence or absence of an antibody to SEQ ID NO: 1 can be detected, without determining whether the antibody detected belongs to a certain immunoglobulin class. This is often referred to as detecting the "total antibodies" to one or more antigens such as SEQ ID NO: 1. Detecting total antibodies, including IgA and IgG, increases the sensitvitity of the assay, since IgA class antibodies present at an early stage and IgA and IgG class antibodies to SEQ ID NO: 1 at a late phase are detected as part of the total antibodies to SEQ ID NO: 1. Preferably, an antibody to SEQ ID NO: 1 can be distinguished from an antibody to another SARS-CoV-2 antigen such as N protein (SEQ ID NO: 30) or S2 domain (SEQ ID NO: 32), more preferably because other SARS-CoV-2 antigens are absent or spatially separated or an antibody to such another SARS-CoV-2 produces a signal which can be distinguished from a signal produced by an antibody to SEQ ID NO: 1.

In a preferred embodiment, the products, methods and uses of the present invention are configured such that presence or absence of an antibody to SEQ ID NO: 1 can be detected, without determining whether the antibody detected binds to SEQ ID NO: 1 or to another another SARS-CoV-2 antigen such as N protein or S2 protein. This may be accomplished by using a mixture of antigens comprising SEQ ID NO: 1 or a variant thereof in combination with N protein (SEQ ID NO: 30) or a variant thereof or S2 domain (SEQ ID NO: 32) or a variant thereof. In a more preferred embodiment, the whole spike protein comprising S1 and S2 domain (SEQ ID NO: 32) may be used, optionally in combination with N protein (SEQ ID NO: 30). Again, the sensitivity of such an assay is increased since antibodies to SEQ ID NO: 1 including IgA and IgG, increases the sensitvitity of the assay, since IgA class antibodies present at an early stage and IgA and IgG class antibodies to SEQ ID NO: 1 at a late phase are detected as part of the total antibodies to SEQ ID NO: 1.

In a preferred embodiment, the products, methods and uses of the present invention are configured such that presence or absence of an antibody to SEQ ID NO: 1 can be detected, without determining whether the antibody detected belongs to a certain immunoglobulin class. This procedure is often referred to as detecting the "total antibodies" to one or more antigens such as SEQ ID NO: 1. Detecting total antibodies, including IgA and IgG, increases the sensitvitity of the assay, since IgA class antibodies present at an early stage are detected as part of the antibodies to SEQ ID NO: 1.

In a preferred embodiment, the absence of an antibody to SEQ ID NO: 1 may be detected, using an isolated, pure and/or recombinant polypeptide comprising SEQ ID NO: 1 or a variant thereof.

In a preferred embodiment, the presence or absence of an antibody to SARS-CoV-2 N protein, defined by SEQ ID NO: 30, is determined in addition, preferably an IgA, IgG and/or IgM antibody, more preferably IgG or IgM, most preferably IgG. The carrier according to the invention may be coated with a polypeptide comprising the SARS-CoV-2 N protein defined by SEQ ID NO: 30 or a variant thereof for this purpose.

In a preferred embodiment, the presence or absence of an antibody to the receptor binding domain (RBD) of the SARS-CoV-2 S1 domain defined by SEQ ID NO: 31 is detected. The carrier according to the invention may be coated with a polypeptide comprising SEQ ID NO: 31 or a variant thereof for this purpose.

In a preferred embodiment, the presence or absence of an antibody to the SARS-CoV-2 S2 domain defined by SEQ ID NO: 32 of the SARS-CoV-2 spike protein is detected. The carrier according to the invention may comprise a polypeptide comprising SEQ ID NO: 32 or a variant thereof for this purpose.

In a preferred embodiment, a secondary antibody is an antibody binding to all antibodies from an antibody or immunglobulin class, preferably a human antibody class, preferably IgA and/or IgG and/or IgM antibodies, preferably IgA. Secondary antibodies typically recognize the constant domain of said class or are polyvalent, with binding sites to various epitopes across the sequence or 3D structure shared by antibodies of said Ig class. Secondary antibodies are typically from a mammal other than a human or from a bird, preferably from chicken, rabbit, mouse, rat, horse, pig, donkey, goat, cow, camel, llama, or non-human primate. A wide range of them is commercially available.

According to the present invention, the SARS-CoV-2 infection may be detected at increased sensitivity at an early stage, preferably 5 or fewer days after the onset of disease symptoms.

In a further preferred embodiment, the method the invention further comprises evaluating the result for a diagnosis. The evaluation in accordance with the present invention is carried out to decide whether a treatment for the individual tested is required. The evaluation of the result of the diagnosis may include involving a physician in order to select an appropriate treatment if the diagnosis was positive. Importantly, the method of the invention excluding the evaluation may be carried out at one location such as a country and the evaluation of the results of the diagnosis may be carried out at a differenct location

In a different preferred embodiment, the method of the invention further comprises transferring the result of the diagnosis or the evaluation to a different location. This preferred embodiment relates to cases where the method of the invention, optionally including the evaluation step, are carried out in one location such as one country and the results of the diagnosis and evaluation, respectively, are transferred to a different location such as a different country. The transfer may be effected by any means available to the skilled person. This includes electronic transfer of the data as well as factual transfer of read material. According to this preferred embodiment, in particular the physician or clinic treating the patient in case of a positive result may take the appropriate steps for successful treatment. The diagnostically useful carrier is preferably selected from the group comprising a glass slide, preferably for microscopy, a biochip, a microarray, a microtiter plate, a lateral flow device, a test strip, a membrane, e.g. a nitrocellulose membrane, preferably a line blot, a chromatography column and a bead, preferably a microtiter plate.

In a preferred embodiment, the diagnostically useful carrier is a line blot (Raoult, D., and Dasch, G. A. (1989), The line blot: an immunoassay for monoclonal and other antibodies. Its application to the serotyping of gram-negative bacteria. J. Immunol. Methods, 125 (1-2), 57-65; WO2013041540). In a preferred embodiment, the term "line blot", as used herein, refers to a test strip, more preferably membrane-based, that has been coated with one or more means for specifically capturing an antibody, preferably each of these means is a polypeptide. If two or more means are used, they are preferably spatially separated on the carrier. Preferably, the width of the bands is at least 30, more preferably 40, 50, 60, 70 or 80 % the width of the test strip. The line blot may comprise one or more control bands for confirming that it has been contacted with sample sufficiently long and under adequate conditions, in particular in the presence of human serum, or with a secondary antibody, respectively. A line blot is preferably made from a nitrocellulose membrane.

In another preferred embodiment, the diagnostically useful carrier is a bead. Various beads for numerous applications are commercially available, mainly based on carbohydrate, for example sepharose or agarose, or plastic. They may contain active or activatable chemical groups such as a carboxyl or tosyl or ester group, which can be utilized for the immobilization of a means for specifically capturing an antibody. Preferably, the beads are beads having an average diameter of from 0.1 µm to 10 µm, from 0.5 µm to 8 µm, from 0.75 µm to 7 µm or from 1 µm to 6 µm. The beads can be coated with the means for specifically capturing an antibody directly or *via* affinity ligands, for example biotin or glutathione and streptavidin or GST, respectively. For example, the bead may be coated with biotin or glutathione and the antigen may be fused with streptavidin or glutathione-S-transferase or a variant thereof, respectively. Preferably, the bead is provided in the form of an aqueous suspension having a bead content of from 10 to 90%, preferably from 20 to 80%, preferably from 30 to 70%, more preferably from 40 to 60% (w/w). The person skilled in the art is familiar with such beads (Diamindis, E. P., Chriopoulus, T. K., Immunoassays, 1996, Academic Press), which are commercially available, for example Bio-Plex COOH beads MC10026-01 or 171-506011 from Bio-Rad.

In a particularly preferred embodiment, the beads are paramagnetic beads, which can be easily concentrated on a surface with the aid of a magnet. For this purpose, commercial paramagnetic beads usually contain a paramagnetic mineral, for example iron oxide. A multiplicity of suitable paramagnetic beads is commercially available. A bead may be labeled with a detectable label.

In a preferred embodiment, a paramagnetic bead is used and washed or incubated in buffer by applying a magnetic field to concentrate and immobilize the beads, following removal of the buffer present and addition of new buffer. The magnetic field may then be discontinued to make the suspension of the beads in the new buffer more efficient. A buffer may be any buffered solution used according to the present invention including a diluted patient sample, an incubation buffer or a buffer comprising a secondary antibody.

In a preferred embodiment, the antibody is detected using a chemiluminescent label. In a preferred embodiment, this is a chemiluminescent enzyme, preferably selected from the group comprising luciferase, peroxidase, alkaline phosphatase and □-galactosidase or a variant thereof, which may turn over a chemiluminescent substrate without being consumed itself (Kricka, L. J. (2003). Clinical applications of chemiluminescence. Analytica chimica acta, 500(1): 279-286). In another preferred embodiment, the chemiluminescent label is a small organic compound having no enzymatic activity catalyzing a chemiluminescence reaction, which emits a chemiluminescence signal upon being degraded when contacted with a chemiluminescence substrate solution which comprises inorganic and/or non-enzymatic organic compounds that are required for emitting the signal. Preferably, the small organic compound having no enzymatic activity is selected from the group comprising acridinium esters (Weeks, I., Beheshti, I., McCapra, F., Campbell, A. K., Woodhead, J. S. (1983) Acridinium esters as high specific activity labels in immunoassay. Clin Chem 29: 1474-1479) and luminol or a chemiluminescent derivative thereof such as isoluminol. Such small organic compounds may be coupled to the secondary antibody. In the case of luminol, the substrate solution comprises H₂O₂ at a high pH. In the case of an acridinium ester, a mixture of H₂O₂ and sodium hydroxide is frequently used. The small organic compound is consumed upon emission of the chemiluminescence signal. In a preferred embodiment the chemiluminescence of the chemiluminescent label is detected for 1 to 60 seconds, preferably for 2 to 20 seconds, more preferably 3 to 15 seconds following initiation of the chemiluminescent detection reaction. In another preferred embodiment the chemiluminescence of the chemiluminescent label is detected for at least 0.5, 1, 1.5, 2, 2.5 or 3 seconds.In another preferred embodiment, the carrier is a microtiter plate comprising at least 8 wells that may be used for ELISA. At least one of the wells is coated with the means for specifically capturing an antibody, either directly or indirectly, preferably a polypeptide comprising SEQ ID NO: 1 or a variant thereof. At least 3, preferably 4, more preferably 5 calibrators, at defined concentrations may be used to set up a calibration curve for semi-quantitative analysis. When the inventive method is carried out, the calibrators, which typically cover a range of concentrations covering the calibratring curve, may be processed and developed in parallel to the samples. A secondary antibody comprising a detectable label such as an enzymatically active label may be provided, for example a label having horse radish peroxidase activity or alkaline phosphatase activity or an enzyme capable of chemiluminescence.

In another preferred embodiment, the carrier is a microarray. In a preferred embodiment, the term "microarray", as used herein, refers to a chip spotted with a variety of spatially separate antigens, preferably at least 20, preferably 30, 40, 50, 80 or 100. Preferably each antigen is a peptide comprising or consisting of 5 to 25, preferably 7 to 15 successive amino acids spanning a fragment of SEQ ID NO: 1, more preferably spanning the RBD (SEQ ID NO: 31). A secondary antibody comprising a label, preferably a fluorescent label, may be used for the detection. Preferably other antigens are spotted, more preferably from the group of polypeptides comprising SEQ ID NO: 30 (SARS-CoV-2 N protein) and SEQ ID NO: 33 (SARS-CoV-2 S2 protein).

In another preferred embodiment, a glass slide is used, which is on or part of a carrier for microscopic immunofluorescence analysis. A cell, preferably a eukaryotic cell such as a HEK293 cell is on the slide. It may be covered with a mounting buffer. Various compositions and methods are described in the state of the art, for example in "Mountants and Antifades", published by Wright Cell Imaging Facility, Toronto Western Research Institute University Health Network, (https://de.scribd.com/document/47879592/Mountants-Antifades), Krenek et al. (1989) Comparison of antifading agents used in immunofluorescence, J. Immunol. Meth 117, 91-97 and Nairn et al. (1969) Microphotometry in Immunofluorescence, Clin. Exp. Immunol. 4, 697-705. The cell expresses, preferably overexpresses a polypeptide comprising SEQ ID NO: 1 or a variant thereof. The carrier may comprise a mock-transfected cell, which has been transfected with the same vector as the cell overexpressing a polypeptide comprising SEQ ID NO: 1 or a variant thereof, but without the nucleic acid encoding for the latter. Such mock-transfected cell may serve as a negative control. Another cell may comprise an additional coronavirus, preferably SARS, more preferably SARS-CoV-2 antigen, for example N protein, S2 protein or RBD to detect an antibody.

According to the present invention, immunofluorescence may be used to detect an antibody. The person skilled in the art is familiar with the method (Storch, W. B., Immunofluorescence in Clinical Immunology: A Primer and Atlas, Birkhäuser, 2000; Wesseling JG, Godeke GJ, Schijns VE, Prevec L, Graham FL, Horzinek MC, Rottier PJ. Mouse hepatitis virus spike and nucleocapsid proteins expressed by adenovirus vectors protect mice against a lethal infection. J Gen Virol. 1993 Oct;74 (Pt 10):2061-9. doi: 10.1099/0022-1317-74-10-2061. PMID: 8409930.). Briefly, an antigen, preferably a polypeptide comprising SEQ ID NO: 1 or a variant thereof, is immobilized on a carrier which may be a cell expressing said antibody and contacted with a sample, followed by detection of the antibody to be detected by fluorescence, preferably using a means for detecting the antibody labeled with a fluorescent label. In a preferred embodiment, the cell is a eukaryotic cell overexpressing the polypeptide, such as a cell selected from the group comprising HEK, Hela, CHO and Jurkat cells and derivatives thereof. In a preferred embodiment, the cell is a recombinant cell overexpressing the polypeptide, which is preferably under the control of a heterologous strong promoter.

According to the present invention, a lateral flow device may be used to detect an antibody. The person skilled in the art is familiar with lateral flow devices for this purpose (Lateral Flow Immunoassay, edited by Raphael Wong, Harley Tse, 2009, Springer; Paper-based diagnostics: Current Status and Future applications, Kevin J. Land, Springer 2019). Briefly, a lateral flow assay may be based on a membrane such as a nitrocellulose membrane which comprises a polypeptide comprising SEQ ID NO: 1 or a variant thereof comprising a detectable label. If the membrane is contacted with a sample, an antibody to be detected will bind to the antigen. The resulting complex will move driven by capillary forces on the membrane and will be immobilized on a test line on the membrane comprising a means for detecting the antibody, typically a secondary antibody binding to the immunglobulin class or classes of the antibody or the antibodies to be detected such as IgG and/or IgA and/or IgM. Preferably nanoparticles or beads are used as labels, for example gold nanoparticles or latex beads.

According to the present invention, a polypeptide, preferably the polypeptide comprising SEQ ID NO: 1 or a variant thereof, may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Green M. R. and Sambrook, J. (2012), Molecular Cloning - A Laboratory Manual, Fourth Edition, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, i.e. at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

In a preferred embodiment, a detectable label is used to detect an antibody according to the present invention, which is a label that may be used to distinguish a population of molecules from others using biophysical detection methods. It is preferably selected from the group comprising a fluorescent, a radioactive, a chemiluminescent label, a heavy metal such as gold label, a nanoparticle, a bead or an enzymatically active label, preferably one catalyzing a colorimetric reaction. In a preferred embodiment, a fluorescent label is selected from the group comprising Alexa dyes, FITC, TRITC and green fluorescent protein (GFP). Iodine-125 may be used as radioactive label. In a preferred embodiment, an enzymatically active label is selected from the group comprising horseradish peroxidase, glucose oxidase, beta galactosidase, alkaline phosphatase and luciferase. The person skilled in the art is able to choose suitable labels and to attach them to proteins, nucleic acids and other molecules (Hassanzadeh L, Chen S, Veedu RN. Radiolabeling of Nucleic Acid Aptamers for Highly Sensitive Disease-Specific Molecular Imaging. Pharmaceuticals (Basel). 2018;11(4):106. Published 2018 Oct 15. doi:10.3390/ph11040106 Hassanzadeh L, Chen S, Veedu RN. Radiolabeling of Nucleic Acid Aptamers for Highly Sensitive Disease-Specific Molecular Imaging. Pharmaceuticals (Basel). 2018;11(4):106. Published 2018 Oct 15. doi:10.3390/ph11040106, Bioconjugate Techniques, 3rd Edition (2013) by Greg T. Hermanson, Obermaier C, Griebel A, Westermeier R. Principles of protein labeling techniques. Methods Mol Biol. 2015;1295:153-65), and a wide range of labeled molecules are commercially available.

According to the present invention, a means for detecting the presence of antibodies to SEQ ID NO: 1 is provided. In a preferred embodiment, a secondary antibody comprising a detectable label may be used to detect IgA and/or IgG and/or IgM, preferably IgA class antibodies to SEQ ID NO: 1, more preferably SEQ ID NO: 1 and another coronavirus antigen. A protein having peroxidase activity may be used as an enzymatically active label. Preferably the secondary antibody recognizes mammalian, more preferably human antibodies. If antibodies from one Ig class are to be detected, two secondary antibodies may be used, preferably one binding to IgA class antibodies and one binding to IgG class antibodies. The two secondary antibodies may be in a mixture or separate, preferably separate to allow separate detection of antibodies from different Ig classes such as IgA, IgM and IgG antibodies, preferably IgG and IgA for example to obtain information regarding the course of the disease, based on the fact that IgA class antibodies emerge earlier in many patients than IgG class antibodies. Alternatively, one secondary antibody binding to antibodies from more than one Ig class may be used, such as from the group comprising IgG, IgA and IgM, preferably one binding to IgG and IgA class antibodies. A secondary antibody may be a polyclonal or monoclonal antibody. In a preferred embodiment, the secondary antibody is from a mammal other than a human but binds to human antibodies of a certain Ig class, preferably IgA, IgG and/or IgM. The person skilled in the art is familiar with the production and use of secondary antibodies (Kalyuzhny, A., Immunohistochemistry, Essential Elements and Beyound, Springer, 2017, in particular chapter 4; Howard, G. C., and Bethell, D. R., Basic Methods in Antibody Production and Characterization, 2000, CRC press). A variety of secondary antibodies, optionally with labels such as a fluorescent label, is commercially available, for example FITC-labeled secondary antibodies Cat # H15101, Cat # 62-8411 Cat # A24459, horseradish peroxidase labeled secondary antibodies Cat # 31420, Cat # SA1-35467, Cat # SA1-35467, Cat # SA1-35467 and others from Thermo Fisher. Labeled fragments of secondary antibodies or aptamers may also be used. The person skilled in the art is familiar with the synthesis, selection and use of aptamers, which may also be used as means for detecting the presence of antibody to SEQ ID NO: 1, for example when binding specifically to the antibody or antibodies to be detected, preferably IgG and/or IgA and/or IgM antibodies (Thiviyanathan V, Gorenstein DG. Aptamers and the next generation of diagnostic reagents. Proteomics Clin Appl. 2012;6(11-12):563-573. doi:10.1002/prca.201200042) and the generation of specific antibodies (Hust M, Frenzel A, Schirrmann T, Dübel S. Selection of recombinant antibodies from antibody gene libraries. Methods Mol Biol. 2014; 1101:305-20; Hanack K, Messerschmidt K, Listek M. Antibodies and Selection of Monoclonal Antibodies. Adv Exp Med Biol. 2016;917:11-22; Harold F. Stills, in The Laboratory Rabbit, Guinea Pig, Hamster, and Other Rodents, 2012). Such aptamers may bind specifically to the constant region or epitopes in other parts of the antibody or antibodies to be detected.

In another preferred embodiment, a polypeptide comprising SEQ ID NO: 1 or a variant thereof, preferably the receptor binding domain (SEQ ID NO: 31), may be used as a means for detecting the presence of an antibody to SEQ ID NO: 1. More specifically, said polypeptide may be coated to a diagnostically useful carrier and may be used to capture any antibody to be detected. Since human antibodies have more than one binding site, only one antigen binding site of a captured antibody may be occupied. Subsequent addition of another polypeptide comprising SEQ ID NO: 1 or a variant thereof, which is not coated on the carrier, may lead to the occupation of another binding site by this newly added polypeptide. The complex comprising a coated polypeptide, the antibody to be detected and the other polypeptide may then be detected. More preferably, the other polypeptide carries a detectable label, and this is used to detect the complex. In this embodiment, all antibodies, more specifically IgA, IgM and IgG class antibodies may be detected.

In another preferred embodiment, a polypeptide comprising SEQ ID NO: 39 (ACE2) or a variant thereof binding to the receptor binding domain in SEQ ID NO: 1, optionally in combination with a polypeptide comprising SEQ ID NO: 1 or a variant thereof binding to the ACE2 receptor, may be used as a means for detecting the presence of an antibody to SEQ ID NO: 1. The competitive assay format is then used to detect its presence.

In another preferred embodiment, specific proteins binding to distinct immunoglobulin classes may be labeled with detectable labels and used as a means for detecting the presence of an antibody to SEQ ID NO: 1. For example, protein G, A and L are bacterial proteins which bind to IgG class antibodes, (L. Bjorck, G. Kronvall: Purification and some properties of streptococcal protein G, a novel IgG-binding reagent. In: Journal of Immunology. 133(2)/1984.), whereas jacalin (Abcam, ThermoFisher) may be used for binding of IgA class antibodies (see e.g. Choe et al., Materials (Basel). 2016 Dec; 9(12): 994; Wilkinson & Neville Vet Immunol Immunopathol. 1988 Mar;18(2):195-8)..

In a preferred embodiment, a kit according to the present invention comprises a polypeptide comprising SEQ ID NO: 1 or a variant thereof, preferably coated to a diagnostically useful carrier, more preferably a microtiter plate, and one or more, preferably all reagents from the group comprising a calibrator, a positive control, a negative control, a washing buffer, a means for detecting the presence of an antibody to SEQ ID NO: 1, preferably an IgA, IgM and/or IgG class antibody, preferably a secondary antibody binding specifically antibodies, more preferably IgA, IgM and/or IgG class antibodies, wherein the secondary antibody may comprise a detectable label, a sample buffer, a detection solution, preferably a chromogen/substrate solution, a stop solution and a protective foil.

In a preferred embodiment, a calibrator is a reagent that binds to a polypeptide comprising SEQ ID NO: 1 or a variant thereof and is preferably recognized by secondary antibodies recognizing IgA, IgM and/or IgG class antibodies. The calibrator may be an IgA antibody to SEQ ID NO: 1. Alternatively, the calibrator may be a chimeric antibody, preferably comprising the contant region or other regions or epitopes shared by distinct immunoglobulin classes, preferably IgA and/or IgG and/or IgM, and a variable region, in particular a binding site wich is derived from an artificial antibody. The person skilled in the art is familiar with the design, production and use of such calibrators (Lütkecosmann S, Faupel T, Porstmann S, Porstmann T, Micheel B, Hanack K. A cross-reactive monoclonal antibody as universal detection antibody in autoantibody diagnostic assays. Clin Chim Acta. 2019 Dec;499:87-92. doi: 10.1016/j.cca.2019.09.003. Epub 2019 Sep 4. PMID: 31493374, Hackett J Jr, Hoff-Velk J, Golden A, Brashear J, Robinson J, Rapp M, Klass M, Ostrow DH, Mandecki W. Recombinant mouse-human chimeric antibodies as calibrators in immunoassays that measure antibodies to Toxoplasma gondii. J Clin Microbiol. 1998 May;36(5):1277-84. doi:, WO2009081165A1). In a preferred embodiment, a positive control is a solution comprising a compound such as antibody to SEQ ID NO: 1, preferably from the group comprising IgA, IgG and IgM class antibodies, more preferably IgA, from the sample of a patient suffering from SARS-CoV-2 at an amount that a positive result is obtained using the method according to the present invention. A negative control is a reagent that lacks such a compound and could comprise serum from a healthy person. A washing buffer may be used to wash the the carrier such as a microtiter plate after the incubation to remove unspecific antibodies and could be PBS. The means for detecting the presence of an antibody could be a secondary antibody binding to the antibody class to be detected, preferably human IgA, IgM and/or IgG class antibodies, and is labeled with a detectable label, preferably with an enzyme, more preferably with an enzyme having peroxidase activity. The sample buffer may be used to dilute patient sample and may be PBS. The detection solution may yield a signal in the presence of the labeled secondary antibody and is preferably a colordeveloping solution and more preferably 3,3',5,5' tetramethylbenzidine/H₂O₂. The stop solution may be added to a reaction to stop the reaction of the detection solution and may comprise a strong acid, preferably 0.5 M sulphuric acid. The protective foil may be placed on top of the carrier such as a microtiter plate to avoid evaporation.

Any reagent used according to the invention may comprise a preservative, for example azide.

According to the present invention, a use of a polypeptide comprising SEQ ID NO: 1 or a variant thereof or an antibody to SEQ ID NO: 1, preferably an IgA class antibody, for the manufacture of a diagnostic kit is provided. In a preferred embodiment, the polypeptide or antibody is packaged as one of the components of such a kit. The polypeptide or antibody may be used to confirm the quality upon production of the kit. For example, the antibody may be used as a positive control to confirm the reactivity of a polypeptide comprising SEQ ID NO: 1 which is a component of the kit, either separate or coated on a diagnostically useful carrier. The polypeptide may be used to confirm the reactivity of an antibody binding specifically to SEQ ID NO: 1 which is part of the kit. The polypeptide may be used to coat a diagnostically useful carrier as part of the manufacture. Both the polypeptide and antibody may be used to determine the concentration of the antibody or polypeptide, respectively, in buffered solutions used to make the carrier and the kit or to check whether or not a cell is expressing a polypeptide comprising SEQ ID NO: 1.

According to the present invention, an antibody to SEQ ID NO: 1, preferably IgA class antibody, is used for diagnosing a SARS-CoV-2 infection or for the differential diagnosis according to the present invention. The use may relate to detecting the antibody itself or in combination with other antibodies to SEQ ID NO: 1 or antibodies to other SARS-CoV-2 antigens, thus increasing the overall sensitivity of the diagnostic assay. In a more preferred embodiment, an IgA class antibody is used to increase the sensitivity of a diagnostic assay, in particular at an early stage of the infection. This may be accomplished by detecting not only IgG, but also IgA class antibodies, optionally also IgM class antibodies to SEQ ID NO: 1 or IgA only. In a more preferred embodiment, an IgG class antibody is used to increase the sensitivity of a diagnostic assay, in particular over a period of time which sees a decline in the concentration of antibodies to other antigens, for example IgG class antibodies to the N protein of SARS-CoV-2 such as during the late phase of a SARS-CoV-2 infection. This may be accomplished by detecting not only IgG, but also IgA class antibodies, optionally also IgM class antibodies to SEQ ID NO: 1 or IgG only. In another preferred embodiment, the antibody may be used for the diagnosis by calibrating a device or assay using a calibrator comprising said antibody, preferably an IgA, IgM and/or IgG class antibody, preferably all. In a preferred embodiment, IgA and/or IgG antibody to SEQ ID NO: 1 is used to increase the sensitivity at a late phase of the immunization. The antibody may be used for the validation of an assay, for calibration, for confirming the quality of reagents or assay materials such as a diagnostically useful carrier or as a positive control.

According to the present invention, a polypepide comprising SEQ ID NO:1 or a variant thereof or an antibody to SEQ ID NO1, preferably an IgA class antibody is used for the manufacture of a diagnostic kit, preferably for the diagnosis of SARS-CoV-2, wherein an IgA class antibody binding specifically to SEQ ID NO: 1 is detected.

A use of an antibody to SEQ ID NO: 1, preferably IgA, IgM and/or IgG, more preferably IgA class antibody, for increasing the sensitivity of the detection of a SARS-CoV-2 infection, preferably at an early stage of the infection, more preferably 5 or fewer days after the onset of disease symptoms. In another preferred embodiment, the use may be at a late phase of the infection. In a preferred embodiment, the presence of or absence IgA, IgM and IgG to SEQ DI NO1 is detected.

The present invention comprises a range of novel nucleic acid and polypeptide sequences, including in particular the sequences described in the following and/or in the sequence listing forming part of the present specification. It will be understood that in case of conflict between any sequence shown herein below and the corresponding sequence described in the sequence listing, the present invention specifically and invididually relates to each one of the respective sequences, i.e., to the sequence described herein below and also to the sequence described in the sequence listing.
SEQ ID NO: 1 (SARS-CoV-2 S1 domain from S Protein) 
VNLTTRTQLPPAYTNSFTRGVYYPDKVFRSSVLHSTQDLFLPFFSNVTWFHAIHVSGTNGTKRFDNPV
LPFNDGVYFASTEKSNIIRGWIFGTTLDSKTQSLLIVNNATNVVIKVCEFQFCNDPFLGVYYHKNNKSW
MESEFRVYSSANNCTFEYVSQPFLMDLEGKQGNFKNLREFVFKNIDGYFKIYSKHTPINLVRDLPQGFS
ALEPLVDLPIGINITRFQTLLALHRSYLTPGDSSSGWTAGAAAYYVGYLQPRTFLLKYNENGTITDAVDC
ALDPLSETKCTLKSFTVEKGIYQTSNFRVQPTESIVRFPNITNLCPFGEVFNATRFASVYAWNRKRISN
CVADYSVLYNSASFSTFKCYGVSPTKLNDLCFTNVYADSFVIRGDEVRQIAPGQTGKIADYNYKLPDDF
TGCVIAWNSNNLDSKVGGNYNYLYRLFRKSNLKPFERDISTEIYQAGSTPCNGVEGFNCYFPLQSYGF
QPTNGVGYQPYRVVVLSFELLHAPATVCGPKKSTNLVKNKCVNFNFNGLTGTGVLTESNKKFLPFQQ
FGRDIADTTDAVRDPQTLEILDITPCSFGGVSVITPGTNTSNQVAVLYQDVNCTEVPVAIHADQLTPTW
RVYSTGSNVFQTRAGCLIGAEHVNNSYECDIPIGAGICASYQTQTNSPRRAR
SEQ ID NO: 2 (SARS-CoV-2 S1 domain from S Protein, C-terminally his-tagged, as expressed in cells for the example) 
MFVFLVLLPLVSSQCVNLTTRTQLPPAYTNSFTRGVYYPDKVFRSSVLHSTQDLFLPFFSNVTWFHAIH
VSGTNGTKRFDNPVLPFNDGVYFASTEKSNIIRGWIFGTTLDSKTQSLLIVNNATNVVIKVCEFQFCND
PFLGVYYHKNNKSWMESEFRVYSSANNCTFEYVSQPFLMDLEGKQGNFKNLREFVFKNIDGYFKIYSK
HTPINLVRDLPQGFSALEPLVDLPIGINITRFQTLLALHRSYLTPGDSSSGWTAGAAAYYVGYLQPRTFL
LKYNENGTITDAVDCALDPLSETKCTLKSFTVEKGIYQTSNFRVQPTESIVRFPNITNLCPFGEVFNATR
FASVYAWNRKRISNCVADYSVLYNSASFSTFKCYGVSPTKLNDLCFTNVYADSFVIRGDEVRQIAPGQ
TGKIADYNYKLPDDFTGCVIAWNSNNLDSKVGGNYNYLYRLFRKSNLKPFERDISTEIYQAGSTPCNGV
EGFNCYFPLQSYGFQPTNGVGYQPYRVVVLSFELLHAPATVCGPKKSTNLVKNKCVNFNFNGLTGTG
VLTESNKKFLPFQQFGRDIADTTDAVRDPQTLEILDITPCSFGGVSVITPGTNTSNQVAVLYQDVNCTE
VPVAIHADQLTPTWRVYSTGSNVFQTRAGCLIGAEHVNNSYECDIPIGAGICASYQTQTNSPRRARLE
HHHHHHHH
SEQ ID NO: 3 (SARS-CoV-2 S1 domain from S Protein, C-terminally his-tagged, as after cleavage of signal peptide, used in the examples) 
VNLTTRTQLPPAYTNSFTRGVYYPDKVFRSSVLHSTQDLFLPFFSNVTWFHAIHVSGTNGTKRFDNPV
LPFNDGVYFASTEKSNIIRGWIFGTTLDSKTQSLLIVNNATNVVIKVCEFQFCNDPFLGVYYHKNNKSW
MESEFRVYSSANNCTFEYVSQPFLMDLEGKQGNFKNLREFVFKNIDGYFKIYSKHTPINLVRDLPQGFS
ALEPLVDLPIGINITRFQTLLALHRSYLTPGDSSSGWTAGAAAYYVGYLQPRTFLLKYNENGTITDAVDC
ALDPLSETKCTLKSFTVEKGIYQTSNFRVQPTESIVRFPNITNLCPFGEVFNATRFASVYAWNRKRISN
CVADYSVLYNSASFSTFKCYGVSPTKLNDLCFTNVYADSFVIRGDEVRQIAPGQTGKIADYNYKLPDDF
TGCVIAWNSNNLDSKVGGNYNYLYRLFRKSNLKPFERDISTEIYQAGSTPCNGVEGFNCYFPLQSYGF
QPTNGVGYQPYRVVVLSFELLHAPATVCGPKKSTNLVKNKCVNFNFNGLTGTGVLTESNKKFLPFQQ
FGRDIADTTDAVRDPQTLEILDITPCSFGGVSVITPGTNTSNQVAVLYQDVNCTEVPVAIHADQLTPTW
RVYSTGSNVFQTRAGCLIGAEHVNNSYECDIPIGAGICASYQTQTNSPRRARLEHHHHHHHH
**SEQ ID NO: 4 (SARS-CoV-2 S1 domain from S Protein, nucleotide sequence encoding SEQ ID NO: 2)** - only in sequence listing
**SEQ ID NO: 5 (possible SARS-CoV-2 S1 epitope)** - only in sequence listing
**SEQ ID NO: 6 (S1[MERS_CoV])** - only in sequence listing
**SEQ ID NO: 7 (S1[HCoV-229E])** - only in sequence listing
**SEQ ID NO: 8 (S1[HCoV-OC43])** - only in sequence listing
**SEQ ID NO: 9 (S1[HCoV-HKU1])** - only in sequence listing
**SEQ ID NO: 10 (S1[HCoV-NL63])** - only in sequence listing
**SEQ ID NO: 11 (S1[SARS_CoV])** - only in sequence listing
**SEQ ID NO: 12 (fragment of SEQ ID NO:** 1) - only in sequence listing
**SEQ ID NO: 13 (genome of SARS-CoV-2 isolate Wuhan-Hu-1 genome, identitical to Genbank MN908947): [ONLY IN SEQUENCE LISTING]** 
ATTAAAGGTTTATACCTTCCCAGGTAACAAACCAACCAACTTTCGATCTCTTGTAGATCTGTTCTC
TAAACGAACTTTAAAATCTGTGTGGCTGTCACTCGGCTGCATGCTTAGTGCACTCACGCAGTATA
ATTAATAACTAATTACTGTCGTTGACAGGACACGAGTAACTCGTCTATCTTCTGCAGGCTGCTTAC
GGTTTCGTCCGTGTTGCAGCCGATCATCAGCACATCTAGGTTTCGTCCGGGTGTGACCGAAAGG
TAAGATGGAGAGCCTTGTCCCTGGTTTCAACGAGAAAACACACGTCCAACTCAGTTTGCCTGTTT
TACAGGTTCGCGACGTGCTCGTACGTGGCTTTGGAGACTCCGTGGAGGAGGTCTTATCAGAGGC
ACGTCAACATCTTAAAGATGGCACTTGTGGCTTAGTAGAAGTTGAAAAAGGCGTTTTGCCTCAAC
TTGAACAGCCCTATGTGTTCATCAAACGTTCGGATGCTCGAACTGCACCTCATGGTCATGTTATG
GTTGAGCTGGTAGCAGAACTCGAAGGCATTCAGTACGGTCGTAGTGGTGAGACACTTGGTGTCC
TTGTCCCTCATGTGGGCGAAATACCAGTGGCTTACCGCAAGGTTCTTCTTCGTAAGAACGGTAAT
AAAGGAGCTGGTGGCCATAGTTACGGCGCCGATCTAAAGTCATTTGACTTAGGCGACGAGCTTG
GCACTGATCCTTATGAAGATTTTCAAGAAAACTGGAACACTAAACATAGCAGTGGTGTTACCCGT
GAACTCATGCGTGAGCTTAACGGAGGGGCATACACTCGCTATGTCGATAACAACTTCTGTGGCC
CTGATGGCTACCCTCTTGAGTGCATTAAAGACCTTCTAGCACGTGCTGGTAAAGCTTCATGCACT
TTGTCCGAACAACTGGACTTTATTGACACTAAGAGGGGTGTATACTGCTGCCGTGAACATGAGCA
TGAAATTGCTTGGTACACGGAACGTTCTGAAAAGAGCTATGAATTGCAGACACCTTTTGAAATTAA
ATTGGCAAAGAAATTTGACACCTTCAATGGGGAATGTCCAAATTTTGTATTTCCCTTAAATTCCATA
ATCAAGACTATTCAACCAAGGGTTGAAAAGAAAAAGCTTGATGGCTTTATGGGTAGAATTCGATCT
GTCTATCCAGTTGCGTCACCAAATGAATGCAACCAAATGTGCCTTTCAACTCTCATGAAGTGTGAT
CATTGTGGTGAAACTTCATGGCAGACGGGCGATTTTGTTAAAGCCACTTGCGAATTTTGTGGCAC
TGAGAATTTGACTAAAGAAGGTGCCACTACTTGTGGTTACTTACCCCAAAATGCTGTTGTTAAAAT
TTATTGTCCAGCATGTCACAATTCAGAAGTAGGACCTGAGCATAGTCTTGCCGAATACCATAATGA
ATCTGGCTTGAAAACCATTCTTCGTAAGGGTGGTCGCACTATTGCCTTTGGAGGCTGTGTGTTCT
CTTATGTTGGTTGCCATAACAAGTGTGCCTATTGGGTTCCACGTGCTAGCGCTAACATAGGTTGT
AACCATACAGGTGTTGTTGGAGAAGGTTCCGAAGGTCTTAATGACAACCTTCTTGAAATACTCCA
AAAAGAGAAAGTCAACATCAATATTGTTGGTGACTTTAAACTTAATGAAGAGATCGCCATTATTTT
GGCATCTTTTTCTGCTTCCACAAGTGCTTTTGTGGAAACTGTGAAAGGTTTGGATTATAAAGCATT
 
CAAACAAATTGTTGAATCCTGTGGTAATTTTAAAGTTACAAAAGGAAAAGCTAAAAAAGGTGCCTG
GAATATTGGTGAACAGAAATCAATACTGAGTCCTCTTTATGCATTTGCATCAGAGGCTGCTCGTGT
TGTACGATCAATTTTCTCCCGCACTCTTGAAACTGCTCAAAATTCTGTGCGTGTTTTACAGAAGGC
CGCTATAACAATACTAGATGGAATTTCACAGTATTCACTGAGACTCATTGATGCTATGATGTTCAC
ATCTGATTTGGCTACTAACAATCTAGTTGTAATGGCCTACATTACAGGTGGTGTTGTTCAGTTGAC
TTCGCAGTGGCTAACTAACATCTTTGGCACTGTTTATGAAAAACTCAAACCCGTCCTTGATTGGCT
TGAAGAGAAGTTTAAGGAAGGTGTAGAGTTTCTTAGAGACGGTTGGGAAATTGTTAAATTTATCTC
AACCTGTGCTTGTGAAATTGTCGGTGGACAAATTGTCACCTGTGCAAAGGAAATTAAGGAGAGTG
TTCAGACATTCTTTAAGCTTGTAAATAAATTTTTGGCTTTGTGTGCTGACTCTATCATTATTGGTGG
AGCTAAACTTAAAGCCTTGAATTTAGGTGAAACATTTGTCACGCACTCAAAGGGATTGTACAGAAA
GTGTGTTAAATCCAGAGAAGAAACTGGCCTACTCATGCCTCTAAAAGCCCCAAAAGAAATTATCTT
CTTAGAGGGAGAAACACTTCCCACAGAAGTGTTAACAGAGGAAGTTGTCTTGAAAACTGGTGATT
TACAACCATTAGAACAACCTACTAGTGAAGCTGTTGAAGCTCCATTGGTTGGTACACCAGTTTGTA
TTAACGGGCTTATGTTGCTCGAAATCAAAGACACAGAAAAGTACTGTGCCCTTGCACCTAATATGA
TGGTAACAAACAATACCTTCACACTCAAAGGCGGTGCACCAACAAAGGTTACTTTTGGTGATGAC
ACTGTGATAGAAGTGCAAGGTTACAAGAGTGTGAATATCACTTTTGAACTTGATGAAAGGATTGAT
AAAGTACTTAATGAGAAGTGCTCTGCCTATACAGTTGAACTCGGTACAGAAGTAAATGAGTTCGC
CTGTGTTGTGGCAGATGCTGTCATAAAAACTTTGCAACCAGTATCTGAATTACTTACACCACTGGG
CATTGATTTAGATGAGTGGAGTATGGCTACATACTACTTATTTGATGAGTCTGGTGAGTTTAAATT
GGCTTCACATATGTATTGTTCTTTCTACCCTCCAGATGAGGATGAAGAAGAAGGTGATTGTGAAG
AAGAAGAGTTTGAGCCATCAACTCAATATGAGTATGGTACTGAAGATGATTACCAAGGTAAACCTT
TGGAATTTGGTGCCACTTCTGCTGCTCTTCAACCTGAAGAAGAGCAAGAAGAAGATTGGTTAGAT
GATGATAGTCAACAAACTGTTGGTCAACAAGACGGCAGTGAGGACAATCAGACAACTACTATTCA
AACAATTGTTGAGGTTCAACCTCAATTAGAGATGGAACTTACACCAGTTGTTCAGACTATTGAAGT
GAATAGTTTTAGTGGTTATTTAAAACTTACTGACAATGTATACATTAAAAATGCAGACATTGTGGAA
GAAGCTAAAAAGGTAAAACCAACAGTGGTTGTTAATGCAGCCAATGTTTACCTTAAACATGGAGG
AGGTGTTGCAGGAGCCTTAAATAAGGCTACTAACAATGCCATGCAAGTTGAATCTGATGATTACA
TAGCTACTAATGGACCACTTAAAGTGGGTGGTAGTTGTGTTTTAAGCGGACACAATCTTGCTAAA
CACTGTCTTCATGTTGTCGGCCCAAATGTTAACAAAGGTGAAGACATTCAACTTCTTAAGAGTGCT
TATGAAAATTTTAATCAGCACGAAGTTCTACTTGCACCATTATTATCAGCTGGTATTTTTGGTGCT
GACCCTATACATTCTTTAAGAGTTTGTGTAGATACTGTTCGCACAAATGTCTACTTAGCTGTCTTT
GATAAAAATCTCTATGACAAACTTGTTTCAAGCTTTTTGGAAATGAAGAGTGAAAAGCAAGTTGAA
CAAAAGATCGCTGAGATTCCTAAAGAGGAAGTTAAGCCATTTATAACTGAAAGTAAACCTTCAGTT
GAACAGAGAAAACAAGATGATAAGAAAATCAAAGCTTGTGTTGAAGAAGTTACAACAACTCTGGAA
GAAACTAAGTTCCTCACAGAAAACTTGTTACTTTATATTGACATTAATGGCAATCTTCATCCAGATT
CTGCCACTCTTGTTAGTGACATTGACATCACTTTCTTAAAGAAAGATGCTCCATATATAGTGGGTG
ATGTTGTTCAAGAGGGTGTTTTAACTGCTGTGGTTATACCTACTAAAAAGGCTGGTGGCACTACT
GAAATGCTAGCGAAAGCTTTGAGAAAAGTGCCAACAGACAATTATATAACCACTTACCCGGGTCA
GGGTTTAAATGGTTACACTGTAGAGGAGGCAAAGACAGTGCTTAAAAAGTGTAAAAGTGCCTTTT
ACATTCTACCATCTATTATCTCTAATGAGAAGCAAGAAATTCTTGGAACTGTTTCTTGGAATTTGC
GAGAAATGCTTGCACATGCAGAAGAAACACGCAAATTAATGCCTGTCTGTGTGGAAACTAAAGCC
ATAGTTTCAACTATACAGCGTAAATATAAGGGTATTAAAATACAAGAGGGTGTGGTTGATTATGGT
 
GCTAGATTTTACTTTTACACCAGTAAAACAACTGTAGCGTCACTTATCAACACACTTAACGATCTAA
ATGAAACTCTTGTTACAATGCCACTTGGCTATGTAACACATGGCTTAAATTTGGAAGAAGCTGCTC
GGTATATGAGATCTCTCAAAGTGCCAGCTACAGTTTCTGTTTCTTCACCTGATGCTGTTACAGCGT
ATAATGGTTATCTTACTTCTTCTTCTAAAACACCTGAAGAACATTTTATTGAAACCATCTCACTTGC
TGGTTCCTATAAAGATTGGTCCTATTCTGGACAATCTACACAACTAGGTATAGAATTTCTTAAGAG
AGGTGATAAAAGTGTATATTACACTAGTAATCCTACCACATTCCACCTAGATGGTGAAGTTATCAC
CTTTGACAATCTTAAGACACTTCTTTCTTTGAGAGAAGTGAGGACTATTAAGGTGTTTACAACAGT
AGACAACATTAACCTCCACACGCAAGTTGTGGACATGTCAATGACATATGGACAACAGTTTGGTC
CAACTTATTTGGATGGAGCTGATGTTACTAAAATAAAACCTCATAATTCACATGAAGGTAAAACATT
TTATGTTTTACCTAATGATGACACTCTACGTGTTGAGGCTTTTGAGTACTACCACACAACTGATCC
TAGTTTTCTGGGTAGGTACATGTCAGCATTAAATCACACTAAAAAGTGGAAATACCCACAAGTTAA
TGGTTTAACTTCTATTAAATGGGCAGATAACAACTGTTATCTTGCCACTGCATTGTTAACACTCCA
ACAAATAGAGTTGAAGTTTAATCCACCTGCTCTACAAGATGCTTATTACAGAGCAAGGGCTGGTG
AAGCTGCTAACTTTTGTGCACTTATCTTAGCCTACTGTAATAAGACAGTAGGTGAGTTAGGTGATG
TTAGAGAAACAATGAGTTACTTGTTTCAACATGCCAATTTAGATTCTTGCAAAAGAGTCTTGAACG
TGGTGTGTAAAACTTGTGGACAACAGCAGACAACCCTTAAGGGTGTAGAAGCTGTTATGTACATG
GGCACACTTTCTTATGAACAATTTAAGAAAGGTGTTCAGATACCTTGTACGTGTGGTAAACAAGCT
ACAAAATATCTAGTACAACAGGAGTCACCTTTTGTTATGATGTCAGCACCACCTGCTCAGTATGAA
CTTAAGCATGGTACATTTACTTGTGCTAGTGAGTACACTGGTAATTACCAGTGTGGTCACTATAAA
CATATAACTTCTAAAGAAACTTTGTATTGCATAGACGGTGCTTTACTTACAAAGTCCTCAGAATACA
AAGGTCCTATTACGGATGTTTTCTACAAAGAAAACAGTTACACAACAACCATAAAACCAGTTACTT
ATAAATTGGATGGTGTTGTTTGTACAGAAATTGACCCTAAGTTGGACAATTATTATAAGAAAGACA
ATTCTTATTTCACAGAGCAACCAATTGATCTTGTACCAAACCAACCATATCCAAACGCAAGCTTCG
ATAATTTTAAGTTTGTATGTGATAATATCAAATTTGCTGATGATTTAAACCAGTTAACTGGTTATAA
GAAACCTGCTTCAAGAGAGCTTAAAGTTACATTTTTCCCTGACTTAAATGGTGATGTGGTGGCTAT
TGATTATAAACACTACACACCCTCTTTTAAGAAAGGAGCTAAATTGTTACATAAACCTATTGTTTGG
CATGTTAACAATGCAACTAATAAAGCCACGTATAAACCAAATACCTGGTGTATACGTTGTCTTTGG
AGCACAAAACCAGTTGAAACATCAAATTCGTTTGATGTACTGAAGTCAGAGGACGCGCAGGGAAT
GGATAATCTTGCCTGCGAAGATCTAAAACCAGTCTCTGAAGAAGTAGTGGAAAATCCTACCATAC
AGAAAGACGTTCTTGAGTGTAATGTGAAAACTACCGAAGTTGTAGGAGACATTATACTTAAACCAG
CAAATAATAGTTTAAAAATTACAGAAGAGGTTGGCCACACAGATCTAATGGCTGCTTATGTAGACA
ATTCTAGTCTTACTATTAAGAAACCTAATGAATTATCTAGAGTATTAGGTTTGAAAACCCTTGCTAC
TCATGGTTTAGCTGCTGTTAATAGTGTCCCTTGGGATACTATAGCTAATTATGCTAAGCCTTTTCT
TAACAAAGTTGTTAGTACAACTACTAACATAGTTACACGGTGTTTAAACCGTGTTTGTACTAATTAT
ATGCCTTATTTCTTTACTTTATTGCTACAATTGTGTACTTTTACTAGAAGTACAAATTCTAGAATTAA
AGCATCTATGCCGACTACTATAGCAAAGAATACTGTTAAGAGTGTCGGTAAATTTTGTCTAGAGGC
TTCATTTAATTATTTGAAGTCACCTAATTTTTCTAAACTGATAAATATTATAATTTGGTTTTTACTATT
AAGTGTTTGCCTAGGTTCTTTAATCTACTCAACCGCTGCTTTAGGTGTTTTAATGTCTAATTTAGG
CATGCCTTCTTACTGTACTGGTTACAGAGAAGGCTATTTGAACTCTACTAATGTCACTATTGCAAC
CTACTGTACTGGTTCTATACCTTGTAGTGTTTGTCTTAGTGGTTTAGATTCTTTAGACACCTATCCT
TCTTTAGAAACTATACAAATTACCATTTCATCTTTTAAATGGGATTTAACTGCTTTTGGCTTAGTTG
CAGAGTGGTTTTTGGCATATATTCTTTTCACTAGGTTTTTCTATGTACTTGGATTGGCTGCAATCA
 
TGCAATTGTTTTCAGCTATTTTGCAGTACATTTTATTAGTAATTCTTGGCTTATGTGGTTAATAAT
TAATCTTGTACAAATGGCCCCGATTTCAGCTATGGTTAGAATGTACATCTTCTTTGCATCATTTTAT
TATGTATGGAAAAGTTATGTGCATGTTGTAGACGGTTGTAATTCATCAACTTGTATGATGTGTTAC
AAACGTAATAGAGCAACAAGAGTCGAATGTACAACTATTGTTAATGGTGTTAGAAGGTCCTTTTAT
GTCTATGCTAATGGAGGTAAAGGCTTTTGCAAACTACACAATTGGAATTGTGTTAATTGTGATACA
TTCTGTGCTGGTAGTACATTTATTAGTGATGAAGTTGCGAGAGACTTGTCACTACAGTTTAAAAGA
CCAATAAATCCTACTGACCAGTCTTCTTACATCGTTGATAGTGTTACAGTGAAGAATGGTTCCATC
CATCTTTACTTTGATAAAGCTGGTCAAAAGACTTATGAAAGACATTCTCTCTCTCATTTTGTTAACT
TAGACAACCTGAGAGCTAATAACACTAAAGGTTCATTGCCTATTAATGTTATAGTTTTTGATGGTA
AATCAAAATGTGAAGAATCATCTGCAAAATCAGCGTCTGTTTACTACAGTCAGCTTATGTGTCAAC
CTATACTGTTACTAGATCAGGCATTAGTGTCTGATGTTGGTGATAGTGCGGAAGTTGCAGTTAAA
ATGTTTGATGCTTACGTTAATACGTTTTCATCAACTTTTAACGTACCAATGGAAAAACTCAAAACAC
TAGTTGCAACTGCAGAAGCTGAACTTGCAAAGAATGTGTCCTTAGACAATGTCTTATCTACTTTTA
TTTCAGCAGCTCGGCAAGGGTTTGTTGATTCAGATGTAGAAACTAAAGATGTTGTTGAATGTCTTA
AATTGTCACATCAATCTGACATAGAAGTTACTGGCGATAGTTGTAATAACTATATGCTCACCTATA
ACAAAGTTGAAAACATGACACCCCGTGACCTTGGTGCTTGTATTGACTGTAGTGCGCGTCATATT
AATGCGCAGGTAGCAAAAAGTCACAACATTGCTTTGATATGGAACGTTAAAGATTTCATGTCATTG
TCTGAACAACTACGAAAACAAATACGTAGTGCTGCTAAAAAGAATAACTTACCTTTTAAGTTGACA
TGTGCAACTACTAGACAAGTTGTTAATGTTGTAACAACAAAGATAGCACTTAAGGGTGGTAAAATT
GTTAATAATTGGTTGAAGCAGTTAATTAAAGTTACACTTGTGTTCCTTTTTGTTGCTGCTATTTTCT
ATTTAATAACACCTGTTCATGTCATGTCTAAACATACTGACTTTTCAAGTGAAATCATAGGATACAA
GGCTATTGATGGTGGTGTCACTCGTGACATAGCATCTACAGATACTTGTTTTGCTAACAAACATG
CTGATTTTGACACATGGTTTAGCCAGCGTGGTGGTAGTTATACTAATGACAAAGCTTGCCCATTG
ATTGCTGCAGTCATAACAAGAGAAGTGGGTTTTGTCGTGCCTGGTTTGCCTGGCACGATATTACG
CACAACTAATGGTGACTTTTTGCATTTCTTACCTAGAGTTTTTAGTGCAGTTGGTAACATCTGTTA
CACACCATCAAAACTTATAGAGTACACTGACTTTGCAACATCAGCTTGTGTTTTGGCTGCTGAATG
TACAATTTTTAAAGATGCTTCTGGTAAGCCAGTACCATATTGTTATGATACCAATGTACTAGAAGG
TTCTGTTGCTTATGAAAGTTTACGCCCTGACACACGTTATGTGCTCATGGATGGCTCTATTATTCA
ATTTCCTAACACCTACCTTGAAGGTTCTGTTAGAGTGGTAACAACTTTTGATTCTGAGTACTGTAG
GCACGGCACTTGTGAAAGATCAGAAGCTGGTGTTTGTGTATCTACTAGTGGTAGATGGGTACTTA
ACAATGATTATTACAGATCTTTACCAGGAGTTTTCTGTGGTGTAGATGCTGTAAATTTACTTACTAA
TATGTTTACACCACTAATTCAACCTATTGGTGCTTTGGACATATCAGCATCTATAGTAGCTGGTGG
TATTGTAGCTATCGTAGTAACATGCCTTGCCTACTATTTTATGAGGTTTAGAAGAGCTTTTGGTGA
ATACAGTCATGTAGTTGCCTTTAATACTTTACTATTCCTTATGTCATTCACTGTACTCTGTTTAACA
CCAGTTTACTCATTCTTACCTGGTGTTTATTCTGTTATTTACTTGTACTTGACATTTTATCTTACTAA
TGATGTTTCTTTTTTAGCACATATTCAGTGGATGGTTATGTTCACACCTTTAGTACCTTTCTGGATA
ACAATTGCTTATATCATTTGTATTTCCACAAAGCATTTCTATTGGTTCTTTAGTAATTACCTAAAGA
GACGTGTAGTCTTTAATGGTGTTTCCTTTAGTACTTTTGAAGAAGCTGCGCTGTGCACCTTTTTGT
TAAATAAAGAAATGTATCTAAAGTTGCGTAGTGATGTGCTATTACCTCTTACGCAATATAATAGATA
CTTAGCTCTTTATAATAAGTACAAGTATTTTAGTGGAGCAATGGATACAACTAGCTACAGAGAAGC
TGCTTGTTGTCATCTCGCAAAGGCTCTCAATGACTTCAGTAACTCAGGTTCTGATGTTCTTTACCA
ACCACCACAAACCTCTATCACCTCAGCTGTTTTGCAGAGTGGTTTTAGAAAAATGGCATTCCCATC
 
TGGTAAAGTTGAGGGTTGTATGGTACAAGTAACTTGTGGTACAACTACACTTAACGGTCTTTGGC
TTGATGACGTAGTTTACTGTCCAAGACATGTGATCTGCACCTCTGAAGACATGCTTAACCCTAATT
ATGAAGATTTACTCATTCGTAAGTCTAATCATAATTTCTTGGTACAGGCTGGTAATGTTCAACTCA
GGGTTATTGGACATTCTATGCAAAATTGTGTACTTAAGCTTAAGGTTGATACAGCCAATCCTAAGA
CACCTAAGTATAAGTTTGTTCGCATTCAACCAGGACAGACTTTTTCAGTGTTAGCTTGTTACAATG
GTTCACCATCTGGTGTTTACCAATGTGCTATGAGGCCCAATTTCACTATTAAGGGTTCATTCCTTA
ATGGTTCATGTGGTAGTGTTGGTTTTAACATAGATTATGACTGTGTCTCTTTTTGTTACATGCACC
ATATGGAATTACCAACTGGAGTTCATGCTGGCACAGACTTAGAAGGTAACTTTTATGGACCTTTTG
TTGACAGGCAAACAGCACAAGCAGCTGGTACGGACACAACTATTACAGTTAATGTTTTAGCTTGG
TTGTACGCTGCTGTTATAAATGGAGACAGGTGGTTTCTCAATCGATTTACCACAACTCTTAATGAC
TTTAACCTTGTGGCTATGAAGTACAATTATGAACCTCTAACACAAGACCATGTTGACATACTAGGA
CCTCTTTCTGCTCAAACTGGAATTGCCGTTTTAGATATGTGTGCTTCATTAAAAGAATTACTGCAA
AATGGTATGAATGGACGTACCATATTGGGTAGTGCTTTATTAGAAGATGAATTTACACCTTTTGAT
GTTGTTAGACAATGCTCAGGTGTTACTTTCCAAAGTGCAGTGAAAAGAACAATCAAGGGTACACA
CCACTGGTTGTTACTCACAATTTTGACTTCACTTTTAGTTTTAGTCCAGAGTACTCAATGGTCTTT
GTTCTTTTTTTTGTATGAAAATGCCTTTTTACCTTTTGCTATGGGTATTATTGCTATGTCTGCTTTT
GCAATGATGTTTGTCAAACATAAGCATGCATTTCTCTGTTTGTTTTTGTTACCTTCTCTTGCCACTG
TAGCTTATTTTAATATGGTCTATATGCCTGCTAGTTGGGTGATGCGTATTATGACATGGTTGGATA
TGGTTGATACTAGTTTGTCTGGTTTTAAGCTAAAAGACTGTGTTATGTATGCATCAGCTGTAGTGT
TACTAATCCTTATGACAGCAAGAACTGTGTATGATGATGGTGCTAGGAGAGTGTGGACACTTATG
AATGTCTTGACACTCGTTTATAAAGTTTATTATGGTAATGCTTTAGATCAAGCCATTTCCATGTGG
GCTCTTATAATCTCTGTTACTTCTAACTACTCAGGTGTAGTTACAACTGTCATGTTTTTGGCCAGA
GGTATTGTTTTTATGTGTGTTGAGTATTGCCCTATTTTCTTCATAACTGGTAATACACTTCAGTGTA
TAATGCTAGTTTATTGTTTCTTAGGCTATTTTTGTACTTGTTACTTTGGCCTCTTTTGTTTACTCAA
CCGCTACTTTAGACTGACTCTTGGTGTTTATGATTACTTAGTTTCTACACAGGAGTTTAGATATAT
GAATTCACAGGGACTACTCCCACCCAAGAATAGCATAGATGCCTTCAAACTCAACATTAAATTGTT
GGGTGTTGGTGGCAAACCTTGTATCAAAGTAGCCACTGTACAGTCTAAAATGTCAGATGTAAAGT
GCACATCAGTAGTCTTACTCTCAGTTTTGCAACAACTCAGAGTAGAATCATCATCTAAATTGTGGG
CTCAATGTGTCCAGTTACACAATGACATTCTCTTAGCTAAAGATACTACTGAAGCCTTTGAAAAAA
TGGTTTCACTACTTTCTGTTTTGCTTTCCATGCAGGGTGCTGTAGACATAAACAAGCTTTGTGAAG
AAATGCTGGACAACAGGGCAACCTTACAAGCTATAGCCTCAGAGTTTAGTTCCCTTCCATCATAT
GCAGCTTTTGCTACTGCTCAAGAAGCTTATGAGCAGGCTGTTGCTAATGGTGATTCTGAAGTTGT
TCTTAAAAAGTTGAAGAAGTCTTTGAATGTGGCTAAATCTGAATTTGACCGTGATGCAGCCATGCA
ACGTAAGTTGGAAAAGATGGCTGATCAAGCTATGACCCAAATGTATAAACAGGCTAGATCTGAGG
ACAAGAGGGCAAAAGTTACTAGTGCTATGCAGACAATGCTTTTCACTATGCTTAGAAAGTTGGATA
ATGATGCACTCAACAACATTATCAACAATGCAAGAGATGGTTGTGTTCCCTTGAACATAATACCTC
TTACAACAGCAGCCAAACTAATGGTTGTCATACCAGACTATAACACATATAAAAATACGTGTGATG
GTACAACATTTACTTATGCATCAGCATTGTGGGAAATCCAACAGGTTGTAGATGCAGATAGTAAAA
TTGTTCAACTTAGTGAAATTAGTATGGACAATTCACCTAATTTAGCATGGCCTCTTATTGTAACAG
CTTTAAGGGCCAATTCTGCTGTCAAATTACAGAATAATGAGCTTAGTCCTGTTGCACTACGACAGA
TGTCTTGTGCTGCCGGTACTACACAAACTGCTTGCACTGATGACAATGCGTTAGCTTACTACAAC
ACAACAAAGGGAGGTAGGTTTGTACTTGCACTGTTATCCGATTTACAGGATTTGAAATGGGCTAG
 
ATTCCCTAAGAGTGATGGAACTGGTACTATCTATACAGAACTGGAACCACCTTGTAGGTTTGTTAC
AGACACACCTAAAGGTCCTAAAGTGAAGTATTTATACTTTATTAAAGGATTAAACAACCTAAATAGA
GGTATGGTACTTGGTAGTTTAGCTGCCACAGTACGTCTACAAGCTGGTAATGCAACAGAAGTGCC
TGCCAATTCAACTGTATTATCTTTCTGTGCTTTTGCTGTAGATGCTGCTAAAGCTTACAAAGATTAT
CTAGCTAGTGGGGGACAACCAATCACTAATTGTGTTAAGATGTTGTGTACACACACTGGTACTGG
TCAGGCAATAACAGTTACACCGGAAGCCAATATGGATCAAGAATCCTTTGGTGGTGCATCGTGTT
GTCTGTACTGCCGTTGCCACATAGATCATCCAAATCCTAAAGGATTTTGTGACTTAAAAGGTAAGT
ATGTACAAATACCTACAACTTGTGCTAATGACCCTGTGGGTTTTACACTTAAAAACACAGTCTGTA
CCGTCTGCGGTATGTGGAAAGGTTATGGCTGTAGTTGTGATCAACTCCGCGAACCCATGCTTCA
GTCAGCTGATGCACAATCGTTTTTAAACGGGTTTGCGGTGTAAGTGCAGCCCGTCTTACACCGTG
CGGCACAGGCACTAGTACTGATGTCGTATACAGGGCTTTTGACATCTACAATGATAAAGTAGCTG
GTTTTGCTAAATTCCTAAAAACTAATTGTTGTCGCTTCCAAGAAAAGGACGAAGATGACAATTTAA
TTGATTCTTACTTTGTAGTTAAGAGACACACTTTCTCTAACTACCAACATGAAGAAACAATTTATAA
TTTACTTAAGGATTGTCCAGCTGTTGCTAAACATGACTTCTTTAAGTTTAGAATAGACGGTGACAT
GGTACCACATATATCACGTCAACGTCTTACTAAATACACAATGGCAGACCTCGTCTATGCTTTAAG
GCATTTTGATGAAGGTAATTGTGACACATTAAAAGAAATACTTGTCACATACAATTGTTGTGATGA
TGATTATTTCAATAAAAAGGACTGGTATGATTTTGTAGAAAACCCAGATATATTACGCGTATACGC
CAACTTAGGTGAACGTGTACGCCAAGCTTTGTTAAAAACAGTACAATTCTGTGATGCCATGCGAA
ATGCTGGTATTGTTGGTGTACTGACATTAGATAATCAAGATCTCAATGGTAACTGGTATGATTTCG
GTGATTTCATACAAACCACGCCAGGTAGTGGAGTTCCTGTTGTAGATTCTTATTATTCATTGTTAA
TGCCTATATTAACCTTGACCAGGGCTTTAACTGCAGAGTCACATGTTGACACTGACTTAACAAAGC
CTTACATTAAGTGGGATTTGTTAAAATATGACTTCACGGAAGAGAGGTTAAAACTCTTTGACCGTT
ATTTTAAATATTGGGATCAGACATACCACCCAAATTGTGTTAACTGTTTGGATGACAGATGCATTC
TGCATTGTGCAAACTTTAATGTTTTATTCTCTACAGTGTTCCCACCTACAAGTTTTGGACCACTAG
TGAGAAAAATATTTGTTGATGGTGTTCCATTTGTAGTTTCAACTGGATACCACTTCAGAGAGCTAG
GTGTTGTACATAATCAGGATGTAAACTTACATAGCTCTAGACTTAGTTTTAAGGAATTACTTGTGTA
TGCTGCTGACCCTGCTATGCACGCTGCTTCTGGTAATCTATTACTAGATAAACGCACTACGTGCT
TTTCAGTAGCTGCACTTACTAACAATGTTGCTTTTCAAACTGTCAAACCCGGTAATTTTAACAAAGA
CTTCTATGACTTTGCTGTGTCTAAGGGTTTCTTTAAGGAAGGAAGTTCTGTTGAATTAAAACACTT
CTTCTTTGCTCAGGATGGTAATGCTGCTATCAGCGATTATGACTACTATCGTTATAATCTACCAAC
AATGTGTGATATCAGACAACTACTATTTGTAGTTGAAGTTGTTGATAAGTACTTTGATTGTTACGAT
GGTGGCTGTATTAATGCTAACCAAGTCATCGTCAACAACCTAGACAAATCAGCTGGTTTTCCATTT
AATAAATGGGGTAAGGCTAGACTTTATTATGATTCAATGAGTTATGAGGATCAAGATGCACTTTTC
GCATATACAAAACGTAATGTCATCCCTACTATAACTCAAATGAATCTTAAGTATGCCATTAGTGCAA
AGAATAGAGCTCGCACCGTAGCTGGTGTCTCTATCTGTAGTACTATGACCAATAGACAGTTTCAT
CAAAAATTATTGAAATCAATAGCCGCCACTAGAGGAGCTACTGTAGTAATTGGAACAAGCAAATTC
TATGGTGGTTGGCACAACATGTTAAAAACTGTTTATAGTGATGTAGAAAACCCTCACCTTATGGGT
TGGGATTATCCTAAATGTGATAGAGCCATGCCTAACATGCTTAGAATTATGGCCTCACTTGTTCTT
GCTCGCAAACATACAACGTGTTGTAGCTTGTCACACCGTTTCTATAGATTAGCTAATGAGTGTGCT
CAAGTATTGAGTGAAATGGTCATGTGTGGCGGTTCACTATATGTTAAACCAGGTGGAACCTCATC
AGGAGATGCCACAACTGCTTATGCTAATAGTGTTTTTAACATTTGTCAAGCTGTCACGGCCAATGT
TAATGCACTTTTATCTACTGATGGTAACAAAATTGCCGATAAGTATGTCCGCAATTTACAACACAG
 
ACTTTATGAGTGTCTCTATAGAAATAGAGATGTTGACACAGACTTTGTGAATGAGTTTTACGCATA
TTTGCGTAAACATTTCTCAATGATGATACTCTCTGACGATGCTGTTGTGTGTTTCAATAGCACTTA
TGCATCTCAAGGTCTAGTGGCTAGCATAAAGAACTTTAAGTCAGTTCTTTATTATCAAAACAATGT
TTTTATGTCTGAAGCAAAATGTTGGACTGAGACTGACCTTACTAAAGGACCTCATGAATTTTGCTC
TCAACATACAATGCTAGTTAAACAGGGTGATGATTATGTGTACCTTCCTTACCCAGATCCATCAAG
AATCCTAGGGGCCGGCTGTTTTGTAGATGATATCGTAAAAACAGATGGTACACTTATGATTGAAC
GGTTCGTGTCTTTAGCTATAGATGCTTACCCACTTACTAAACATCCTAATCAGGAGTATGCTGATG
TCTTTCATTTGTACTTACAATACATAAGAAAGCTACATGATGAGTTAACAGGACACATGTTAGACAT
GTATTCTGTTATGCTTACTAATGATAACACTTCAAGGTATTGGGAACCTGAGTTTTATGAGGCTAT
GTACACACCGCATACAGTCTTACAGGCTGTTGGGGCTTGTGTTCTTTGCAATTCACAGACTTCAT
TAAGATGTGGTGCTTGCATACGTAGACCATTCTTATGTTGTAAATGCTGTTACGACCATGTCATAT
CAACATCACATAAATTAGTCTTGTCTGTTAATCCGTATGTTTGCAATGCTCCAGGTTGTGATGTCA
CAGATGTGACTCAACTTTACTTAGGAGGTATGAGCTATTATTGTAAATCACATAAACCACCCATTA
GTTTTCCATTGTGTGCTAATGGACAAGTTTTTGGTTTATATAAAAATACATGTGTTGGTAGCGATA
ATGTTACTGACTTTAATGCAATTGCAACATGTGACTGGACAAATGCTGGTGATTACATTTTAGCTA
ACACCTGTACTGAAAGACTCAAGCTTTTTGCAGCAGAAACGCTCAAAGCTACTGAGGAGACATTT
AAACTGTCTTATGGTATTGCTACTGTACGTGAAGTGCTGTCTGACAGAGAATTACATCTTTCATGG
GAAGTTGGTAAACCTAGACCACCACTTAACCGAAATTATGTCTTTACTGGTTATCGTGTAACTAAA
AACAGTAAAGTACAAATAGGAGAGTACACCTTTGAAAAAGGTGACTATGGTGATGCTGTTGTTTAC
CGAGGTACAACAACTTACAAATTAAATGTTGGTGATTATTTTGTGCTGACATCACATACAGTAATG
CCATTAAGTGCACCTACACTAGTGCCACAAGAGCACTATGTTAGAATTACTGGCTTATACCCAACA
CTCAATATCTCAGATGAGTTTTCTAGCAATGTTGCAAATTATCAAAAGGTTGGTATGCAAAAGTAT
TCTACACTCCAGGGACCACCTGGTACTGGTAAGAGTCATTTTGCTATTGGCCTAGCTCTCTACTA
CCCTTCTGCTCGCATAGTGTATACAGCTTGCTCTCATGCCGCTGTTGATGCACTATGTGAGAAGG
CATTAAAATATTTGCCTATAGATAAATGTAGTAGAATTATACCTGCACGTGCTCGTGTAGAGTGTT
TTGATAAATTCAAAGTGAATTCAACATTAGAACAGTATGTCTTTTGTACTGTAAATGCATTGCCTGA
GACGACAGCAGATATAGTTGTCTTTGATGAAATTTCAATGGCCACAAATTATGATTTGAGTGTTGT
CAATGCCAGATTACGTGCTAAGCACTATGTGTACATTGGCGACCCTGCTCAATTACCTGCACCAC
GCACATTGCTAACTAAGGGCACACTAGAACCAGAATATTTCAATTCAGTGTGTAGACTTATGAAAA
CTATAGGTCCAGACATGTTCCTCGGAACTTGTCGGCGTTGTCCTGCTGAAATTGTTGACACTGTG
AGTGCTTTGGTTTATGATAATAAGCTTAAAGCACATAAAGACAAATCAGCTCAATGCTTTAAAATGT
TTTATAAGGGTGTTATCACGCATGATGTTTCATCTGCAATTAACAGGCCACAAATAGGCGTGGTAA
GAGAATTCCTTACACGTAACCCTGCTTGGAGAAAAGCTGTCTTTATTTCACCTTATAATTCACAGA
ATGCTGTAGCCTCAAAGATTTTGGGACTACCAACTCAAACTGTTGATTCATCACAGGGCTCAGAA
TATGACTATGTCATATTCACTCAAACCACTGAAACAGCTCACTCTTGTAATGTAAACAGATTTAATG
TTGCTATTACCAGAGCAAAAGTAGGCATACTTTGCATAATGTCTGATAGAGACCTTTATGACAAGT
TGCAATTTACAAGTCTTGAAATTCCACGTAGGAATGTGGCAACTTTACAAGCTGAAAATGTAACAG
GACTCTTTAAAGATTGTAGTAAGGTAATCACTGGGTTACATCCTACACAGGCACCTACACACCTCA
GTGTTGACACTAAATTCAAAACTGAAGGTTTATGTGTTGACATACCTGGCATACCTAAGGACATGA
CCTATAGAAGACTCATCTCTATGATGGGTTTTAAAATGAATTATCAAGTTAATGGTTACCCTAACAT
GTTTATCACCCGCGAAGAAGCTATAAGACATGTACGTGCATGGATTGGCTTCGATGTCGAGGGG
TGTCATGCTACTAGAGAAGCTGTTGGTACCAATTTACCTTTACAGCTAGGTTTTTCTACAGGTGTT
 
AACCTAGTTGCTGTACCTACAGGTTATGTTGATACACCTAATAATACAGATTTTTCCAGAGTTAGT
GCTAAACCACCGCCTGGAGATCAATTTAAACACCTCATACCACTTATGTACAAAGGACTTCCTTGG
AATGTAGTGCGTATAAAGATTGTACAAATGTTAAGTGACACACTTAAAAATCTCTCTGACAGAGTC
GTATTTGTCTTATGGGCACATGGCTTTGAGTTGACATCTATGAAGTATTTTGTGAAAATAGGACCT
GAGCGCACCTGTTGTCTATGTGATAGACGTGCCACATGCTTTTCCACTGCTTCAGACACTTATGC
CTGTTGGCATCATTCTATTGGATTTGATTACGTCTATAATCCGTTTATGATTGATGTTCAACAATG
GGGTTTTACAGGTAACCTACAAAGCAACCATGATCTGTATTGTCAAGTCCATGGTAATGCACATGT
AGCTAGTTGTGATGCAATCATGACTAGGTGTCTAGCTGTCCACGAGTGCTTTGTTAAGCGTGTTG
ACTGGACTATTGAATATCCTATAATTGGTGATGAACTGAAGATTAATGCGGCTTGTAGAAAGGTTC
AACACATGGTTGTTAAAGCTGCATTATTAGCAGACAAATTCCCAGTTCTTCACGACATTGGTAACC
CTAAAGCTATTAAGTGTGTACCTCAAGCTGATGTAGAATGGAAGTTCTATGATGCACAGCCTTGTA
GTGACAAAGCTTATAAAATAGAAGAATTATTCTATTCTTATGCCACACATTCTGACAAATTCACAGA
TGGTGTATGCCTATTTTGGAATTGCAATGTCGATAGATATCCTGCTAATTCCATTGTTTGTAGATT
TGACACTAGAGTGCTATCTAACCTTAACTTGCCTGGTTGTGATGGTGGCAGTTTGTATGTAAATAA
ACATGCATTCCACACACCAGCTTTTGATAAAAGTGCTTTTGTTAATTTAAAACAATTACCATTTTTC
TATTACTCTGACAGTCCATGTGAGTCTCATGGAAAACAAGTAGTGTCAGATATAGATTATGTACCA
CTAAAGTCTGCTACGTGTATAACACGTTGCAATTTAGGTGGTGCTGTCTGTAGACATCATGCTAAT
GAGTACAGATTGTATCTCGATGCTTATAACATGATGATCTCAGCTGGCTTTAGCTTGTGGGTTTAC
AAACAATTTGATACTTATAACCTCTGGAACACTTTTACAAGACTTCAGAGTTTAGAAAATGTGGCTT
TTAATGTTGTAAATAAGGGACACTTTGATGGACAACAGGGTGAAGTACCAGTTTCTATCATTAATA
ACACTGTTTACACAAAAGTTGATGGTGTTGATGTAGAATTGTTTGAAAATAAAACAACATTACCTGT
TAATGTAGCATTTGAGCTTTGGGCTAAGCGCAACATTAAACCAGTACCAGAGGTGAAAATACTCA
ATAATTTGGGTGTGGACATTGCTGCTAATACTGTGATCTGGGACTACAAAAGAGATGCTCCAGCA
CATATATCTACTATTGGTGTTTGTTCTATGACTGACATAGCCAAGAAACCAACTGAAACGATTTGT
GCACCACTCACTGTCTTTTTTGATGGTAGAGTTGATGGTCAAGTAGACTTATTTAGAAATGCCCGT
AATGGTGTTCTTATTACAGAAGGTAGTGTTAAAGGTTTACAACCATCTGTAGGTCCCAAACAAGCT
AGTCTTAATGGAGTCACATTAATTGGAGAAGCCGTAAAAACACAGTTCAATTATTATAAGAAAGTT
GATGGTGTTGTCCAACAATTACCTGAAACTTACTTTACTCAGAGTAGAAATTTACAAGAATTTAAAC
CCAGGAGTCAAATGGAAATTGATTTCTTAGAATTAGCTATGGATGAATTCATTGAACGGTATAAAT
TAGAAGGCTATGCCTTCGAACATATCGTTTATGGAGATTTTAGTCATAGTCAGTTAGGTGGTTTAC
ATCTACTGATTGGACTAGCTAAACGTTTTAAGGAATCACCTTTTGAATTAGAAGATTTTATTCCTAT
GGACAGTACAGTTAAAAACTATTTCATAACAGATGCGCAAACAGGTTCATCTAAGTGTGTGTGTTC
TGTTATTGATTTATTACTTGATGATTTTGTTGAAATAATAAAATCCCAAGATTTATCTGTAGTTTCTA
AGGTTGTCAAAGTGACTATTGACTATACAGAAATTTCATTTATGCTTTGGTGTAAAGATGGCCATG
TAGAAACATTTTACCCAAAATTACAATCTAGTCAAGCGTGGCAACCGGGTGTTGCTATGCCTAATC
TTTACAAAATGCAAAGAATGCTATTAGAAAAGTGTGACCTTCAAAATTATGGTGATAGTGCAACAT
TACCTAAAGGCATAATGATGAATGTCGCAAAATATACTCAACTGTGTCAATATTTAAACACATTAAC
ATTAGCTGTACCCTATAATATGAGAGTTATACATTTTGGTGCTGGTTCTGATAAAGGAGTTGCACC
AGGTACAGCTGTTTTAAGACAGTGGTTGCCTACGGGTACGCTGCTTGTCGATTCAGATCTTAATG
ACTTTGTCTCTGATGCAGATTCAACTTTGATTGGTGATTGTGCAACTGTACATACAGCTAATAAAT
GGGATCTCATTATTAGTGATATGTACGACCCTAAGACTAAAAATGTTACAAAAGAAAATGACTCTA
AAGAGGGTTTTTTCACTTACATTTGTGGGTTTATACAACAAAAGCTAGCTCTTGGAGGTTCCGTG
 
GCTATAAAGATAACAGAACATTCTTGGAATGCTGATCTTTATAAGCTCATGGACACTTCGCATGG
TGGACAGCCTTTGTTACTAATGTGAATGCGTCATCATCTGAAGCATTTTTAATTGGATGTAATTAT
CTTGGCAAACCACGCGAACAAATAGATGGTTATGTCATGCATGCAAATTACATATTTTGGAGGAAT
ACAAATCCAATTCAGTTGTCTTCCTATTCTTTATTTGACATGAGTAAATTTCCCCTTAAATTAAGGG
GTACTGCTGTTATGTCTTTAAAAGAAGGTCAAATCAATGATATGATTTTATCTCTTCTTAGTAAAGG
TAGACTTATAATTAGAGAAAACAACAGAGTTGTTATTTCTAGTGATGTTCTTGTTAACAACTAAACG
AACAATGTTTGTTTTTCTTGTTTTATTGCCACTAGTCTCTAGTCAGTGTGTTAATCTTACAACCAGA
ACTCAATTACCCCCTGCATACACTAATTCTTTCACACGTGGTGTTTATTACCCTGACAAAGTTTTC
AGATCCTCAGTTTTACATTCAACTCAGGACTTGTTCTTACCTTTCTTTTCCAATGTTACTTGGTTCC
ATGCTATACATGTCTCTGGGACCAATGGTACTAAGAGGTTTGATAACCCTGTCCTACCATTTAATG
ATGGTGTTTATTTTGCTTCCACTGAGAAGTCTAACATAATAAGAGGCTGGATTTTTGGTACTACTT
TAGATTCGAAGACCCAGTCCCTACTTATTGTTAATAACGCTACTAATGTTGTTATTAAAGTCTGTG
AATTTCAATTTTGTAATGATCCATTTTTGGGTGTTTATTACCACAAAAACAACAAAAGTTGGATGGA
AAGTGAGTTCAGAGTTTATTCTAGTGCGAATAATTGCACTTTTGAATATGTCTCTCAGCCTTTTCTT
ATGGACCTTGAAGGAAAACAGGGTAATTTCAAAAATCTTAGGGAATTTGTGTTTAAGAATATTGAT
GGTTATTTTAAAATATATTCTAAGCACACGCCTATTAATTTAGTGCGTGATCTCCCTCAGGGTTTTT
CGGCTTTAGAACCATTGGTAGATTTGCCAATAGGTATTAACATCACTAGGTTTCAAACTTTACTTG
CTTTACATAGAAGTTATTTGACTCCTGGTGATTCTTCTTCAGGTTGGACAGCTGGTGCTGCAGCTT
ATTATGTGGGTTATCTTCAACCTAGGACTTTTCTATTAAAATATAATGAAAATGGAACCATTACAGA
TGCTGTAGACTGTGCACTTGACCCTCTCTCAGAAACAAAGTGTACGTTGAAATCCTTCACTGTAG
AAAAAGGAATCTATCAAACTTCTAACTTTAGAGTCCAACCAACAGAATCTATTGTTAGATTTCCTAA
TATTACAAACTTGTGCCCTTTTGGTGAAGTTTTTAACGCCACCAGATTTGCATCTGTTTATGCTTG
GAACAGGAAGAGAATCAGCAACTGTGTTGCTGATTATTCTGTCCTATATAATTCCGCATCATTTTC
CACTTTTAAGTGTTATGGAGTGTCTCCTACTAAATTAAATGATCTCTGCTTTACTAATGTCTATGCA
GATTCATTTGTAATTAGAGGTGATGAAGTCAGACAAATCGCTCCAGGGCAAACTGGAAAGATTGC
TGATTATAATTATAAATTACCAGATGATTTTACAGGCTGCGTTATAGCTTGGAATTCTAACAATCTT
GATTCTAAGGTTGGTGGTAATTATAATTACCTGTATAGATTGTTTAGGAAGTCTAATCTCAAACCTT
TTGAGAGAGATATTTCAACTGAAATCTATCAGGCCGGTAGCACACCTTGTAATGGTGTTGAAGGT
TTTAATTGTTACTTTCCTTTACAATCATATGGTTTCCAACCCACTAATGGTGTTGGTTACCAACCAT
ACAGAGTAGTAGTACTTTCTTTTGAACTTCTACATGCACCAGCAACTGTTTGTGGACCTAAAAAGT
CTACTAATTTGGTTAAAAACAAATGTGTCAATTTCAACTTCAATGGTTTAACAGGCACAGGTGTTC
TTACTGAGTCTAACAAAAAGTTTCTGCCTTTCCAACAATTTGGCAGAGACATTGCTGACACTACTG
ATGCTGTCCGTGATCCACAGACACTTGAGATTCTTGACATTACACCATGTTCTTTTGGTGGTGTCA
GTGTTATAACACCAGGAACAAATACTTCTAACCAGGTTGCTGTTCTTTATCAGGATGTTAACTGCA
CAGAAGTCCCTGTTGCTATTCATGCAGATCAACTTACTCCTACTTGGCGTGTTTATTCTACAGGTT
CTAATGTTTTTCAAACACGTGCAGGCTGTTTAATAGGGGCTGAACATGTCAACAACTCATATGAGT
GTGACATACCCATTGGTGCAGGTATATGCGCTAGTTATCAGACTCAGACTAATTCTCCTCGGCGG
GCACGTAGTGTAGCTAGTCAATCCATCATTGCCTACACTATGTCACTTGGTGCAGAAAATTCAGTT
GCTTACTCTAATAACTCTATTGCCATACCCACAAATTTTACTATTAGTGTTACCACAGAAATTCTAC
CAGTGTCTATGACCAAGACATCAGTAGATTGTACAATGTACATTTGTGGTGATTCAACTGAATGCA
GCAATCTTTTGTTGCAATATGGCAGTTTTTGTACACAATTAAACCGTGCTTTAACTGGAATAGCTG
TTGAACAAGACAAAAACACCCAAGAAGTTTTTGCACAAGTCAAACAAATTTACAAAACACCACCAA
 
TTAAAGATTTTGGTGGTTTTAATTTTTCACAAATATTACCAGATCCATCAAAACCAAGCAAGAGGTC
ATTTATTGAAGATCTACTTTTCAACAAAGTGACACTTGCAGATGCTGGCTTCATCAAACAATATGG
TGATTGCCTTGGTGATATTGCTGCTAGAGACCTCATTTGTGCACAAAAGTTTAACGGCCTTACTGT
TTTGCCACCTTTGCTCACAGATGAAATGATTGCTCAATACACTTCTGCACTGTTAGCGGGTACAAT
CACTTCTGGTTGGACCTTTGGTGCAGGTGCTGCATTACAAATACCATTTGCTATGCAAATGGCTT
ATAGGTTTAATGGTATTGGAGTTACACAGAATGTTCTCTATGAGAACCAAAAATTGATTGCCAACC
AATTTAATAGTGCTATTGGCAAAATTCAAGACTCACTTTCTTCCACAGCAAGTGCACTTGGAAAAC
TTCAAGATGTGGTCAACCAAAATGCACAAGCTTTAAACACGCTTGTTAAACAACTTAGCTCCAATT
TTGGTGCAATTTCAAGTGTTTTAAATGATATCCTTTCACGTCTTGACAAAGTTGAGGCTGAAGTGC
AAATTGATAGGTTGATCACAGGCAGACTTCAAAGTTTGCAGACATATGTGACTCAACAATTAATTA
GAGCTGCAGAAATCAGAGCTTCTGCTAATCTTGCTGCTACTAAAATGTCAGAGTGTGTACTTGGA
CAATCAAAAAGAGTTGATTTTTGTGGAAAGGGCTATCATCTTATGTCCTTCCCTCAGTCAGCACCT
CATGGTGTAGTCTTCTTGCATGTGACTTATGTCCCTGCACAAGAAAAGAACTTCACAACTGCTCCT
GCCATTTGTCATGATGGAAAAGCACACTTTCCTCGTGAAGGTGTCTTTGTTTCAAATGGCACACA
CTGGTTTGTAACACAAAGGAATTTTTATGAACCACAAATCATTACTACAGACAACACATTTGTGTCT
GGTAACTGTGATGTTGTAATAGGAATTGTCAACAACACAGTTTATGATCCTTTGCAACCTGAATTA
GACTCATTCAAGGAGGAGTTAGATAAATATTTTAAGAATCATACATCACCAGATGTTGATTTAGGT
GACATCTCTGGCATTAATGCTTCAGTTGTAAACATTCAAAAAGAAATTGACCGCCTCAATGAGGTT
GCCAAGAATTTAAATGAATCTCTCATCGATCTCCAAGAACTTGGAAAGTATGAGCAGTATATAAAA
TGGCCATGGTACATTTGGCTAGGTTTTATAGCTGGCTTGATTGCCATAGTAATGGTGACAATTAT
GCTTTGCTGTATGACCAGTTGCTGTAGTTGTCTCAAGGGCTGTTGTTCTTGTGGATCCTGCTGCA
AATTTGATGAAGACGACTCTGAGCCAGTGCTCAAAGGAGTCAAATTACATTACACATAAACGAACT
TATGGATTTGTTTATGAGAATCTTCACAATTGGAACTGTAACTTTGAAGCAAGGTGAAATCAAGGA
TGCTACTCCTTCAGATTTTGTTCGCGCTACTGCAACGATACCGATACAAGCCTCACTCCCTTTCG
GATGGCTTATTGTTGGCGTTGCACTTCTTGCTGTTTTTCAGAGCGCTTCCAAAATCATAACCCTCA
AAAAGAGATGGCAACTAGCACTCTCCAAGGGTGTTCACTTTGTTTGCAACTTGCTGTTGTTGTTT
GTAACAGTTTACTCACACCTTTTGCTCGTTGCTGCTGGCCTTGAAGCCCCTTTTCTCTATCTTTAT
GCTTTAGTCTACTTCTTGCAGAGTATAAACTTTGTAAGAATAATAATGAGGCTTTGGCTTTGCTGG
AAATGCCGTTCCAAAAACCCATTACTTTATGATGCCAACTATTTTCTTTGCTGGCATACTAATTGTT
ACGACTATTGTATACCTTACAATAGTGTAACTTCTTCAATTGTCATTACTTCAGGTGATGGCACAA
CAAGTCCTATTTCTGAACATGACTACCAGATTGGTGGTTATACTGAAAAATGGGAATCTGGAGTAA
AAGACTGTGTTGTATTACACAGTTACTTCACTTCAGACTATTACCAGCTGTACTCAACTCAATTGA
GTACAGACACTGGTGTTGAACATGTTACCTTCTTCATCTACAATAAAATTGTTGATGAGCCTGAAG
AACATGTCCAAATTCACACAATCGACGGTTCATCCGGAGTTGTTAATCCAGTAATGGAACCAATTT
ATGATGAACCGACGACGACTACTAGCGTGCCTTTGTAAGCACAAGCTGATGAGTACGAACTTATG
TACTCATTCGTTTCGGAAGAGACAGGTACGTTAATAGTTAATAGCGTACTTCTTTTTCTTGCTTTC
GTGGTATTCTTGCTAGTTACACTAGCCATCCTTACTGCGCTTCGATTGTGTGCGTACTGCTGCAA
TATTGTTAACGTGAGTCTTGTAAAACCTTCTTTTTACGTTTACTCTCGTGTTAAAAATCTGAATTCT
TCTAGAGTTCCTGATCTTCTGGTCTAAACGAACTAAATATTATATTAGTTTTTCTGTTTGGAACTTT
AATTTTAGCCATGGCAGATTCCAACGGTACTATTACCGTTGAAGAGCTTAAAAAGCTCCTTGAACA
ATGGAACCTAGTAATAGGTTTCCTATTCCTTACATGGATTTGTCTTCTACAATTTGCCTATGCCAA
CAGGAATAGGTTTTTGTATATAATTAAGTTAATTTTCCTCTGGCTGTTATGGCCAGTAACTTTAGCT
 
TGTTTTGTGCTTGCTGCTGTTTACAGAATAAATTGGATCACCGGTGGAATTGCTATCGCAATGGC
TTGTCTTGTAGGCTTGATGTGGCTCAGCTACTTCATTGCTTCTTTCAGACTGTTTGCGCGTACGC
GTTCCATGTGGTCATTCAATCCAGAAACTAACATTCTTCTCAACGTGCCACTCCATGGCACTATTC
TGACCAGACCGCTTCTAGAAAGTGAACTCGTAATCGGAGCTGTGATCCTTCGTGGACATCTTCGT
ATTGCTGGACACCATCTAGGACGCTGTGACATCAAGGACCTGCCTAAAGAAATCACTGTTGCTAC
ATCACGAACGCTTTCTTATTACAAATTGGGAGCTTCGCAGCGTGTAGCAGGTGACTCAGGTTTTG
CTGCATACAGTCGCTACAGGATTGGCAACTATAAATTAAACACAGACCATTCCAGTAGCAGTGAC
AATATTGCTTTGCTTGTACAGTAAGTGACAACAGATGTTTCATCTCGTTGACTTTCAGGTTACTAT
AGCAGAGATATTACTAATTATTATGAGGACTTTTAAAGTTTCCATTTGGAATCTTGATTACATCATA
AACCTCATAATTAAAAATTTATCTAAGTCACTAACTGAGAATAAATATTCTCAATTAGATGAAGAGC
AACCAATGGAGATTGATTAAACGAACATGAAAATTATTCTTTTCTTGGCACTGATAACACTCGCTA
CTTGTGAGCTTTATCACTACCAAGAGTGTGTTAGAGGTACAACAGTACTTTTAAAAGAACCTTGCT
CTTCTGGAACATACGAGGGCAATTCACCATTTCATCCTCTAGCTGATAACAAATTTGCACTGACTT
GCTTTAGCACTCAATTTGCTTTTGCTTGTCCTGACGGCGTAAAACACGTCTATCAGTTACGTGCCA
GATCAGTTTCACCTAAACTGTTCATCAGACAAGAGGAAGTTCAAGAACTTTACTCTCCAATTTTTC
TTATTGTTGCGGCAATAGTGTTTATAACACTTTGCTTCACACTCAAAAGAAAGACAGAATGATTGA
ACTTTCATTAATTGACTTCTATTTGTGCTTTTTAGCCTTTCTGCTATTCCTTGTTTTAATTATGCTTA
TTATCTTTTGGTTCTCACTTGAACTGCAAGATCATAATGAAACTTGTCACGCCTAAACGAACATGA
AATTTCTTGTTTTCTTAGGAATCATCACAACTGTAGCTGCATTTCACCAAGAATGTAGTTTACAGTC
ATGTACTCAACATCAACCATATGTAGTTGATGACCCGTGTCCTATTCACTTCTATTCTAAATGGTAT
ATTAGAGTAGGAGCTAGAAAATCAGCACCTTTAATTGAATTGTGCGTGGATGAGGCTGGTTCTAA
ATCACCCATTCAGTACATCGATATCGGTAATTATACAGTTTCCTGTTTACCTTTTACAATTAATTGC
CAGGAACCTAAATTGGGTAGTCTTGTAGTGCGTTGTTCGTTCTATGAAGACTTTTTAGAGTATCAT
GACGTTCGTGTTGTTTTAGATTTCATCTAAACGAACAAACTAAAATGTCTGATAATGGACCCCAAA
ATCAGCGAAATGCACCCCGCATTACGTTTGGTGGACCCTCAGATTCAACTGGCAGTAACCAGAAT
GGAGAACGCAGTGGGGCGCGATCAAAACAACGTCGGCCCCAAGGTTTACCCAATAATACTGCGT
CTTGGTTCACCGCTCTCACTCAACATGGCAAGGAAGACCTTAAATTCCCTCGAGGACAAGGCGTT
CCAATTAACACCAATAGCAGTCCAGATGACCAAATTGGCTACTACCGAAGAGCTACCAGACGAAT
TCGTGGTGGTGACGGTAAAATGAAAGATCTCAGTCCAAGATGGTATTTCTACTACCTAGGAACTG
GGCCAGAAGCTGGACTTCCCTATGGTGCTAACAAAGACGGCATCATATGGGTTGCAACTGAGGG
AGCCTTGAATACACCAAAAGATCACATTGGCACCCGCAATCCTGCTAACAATGCTGCAATCGTGC
TACAACTTCCTCAAGGAACAACATTGCCAAAAGGCTTCTACGCAGAAGGGAGCAGAGGCGGCAG
TCAAGCCTCTTCTCGTTCCTCATCACGTAGTCGCAACAGTTCAAGAAATTCAACTCCAGGCAGCA
GTAGGGGAACTTCTCCTGCTAGAATGGCTGGCAATGGCGGTGATGCTGCTCTTGCTTTGCTGCT
GCTTGACAGATTGAACCAGCTTGAGAGCAAAATGTCTGGTAAAGGCCAACAACAACAAGGCCAAA
CTGTCACTAAGAAATCTGCTGCTGAGGCTTCTAAGAAGCCTCGGCAAAAACGTACTGCCACTAAA
GCATACAATGTAACACAAGCTTTCGGCAGACGTGGTCCAGAACAAACCCAAGGAAATTTTGGGGA
CCAGGAACTAATCAGACAAGGAACTGATTACAAACATTGGCCGCAAATTGCACAATTTGCCCCCA
GCGCTTCAGCGTTCTTCGGAATGTCGCGCATTGGCATGGAAGTCACACCTTCGGGAACGTGGTT
GACCTACACAGGTGCCATCAAATTGGATGACAAAGATCCAAATTTCAAAGATCAAGTCATTTTGCT
GAATAAGCATATTGACGCATACAAAACATTCCCACCAACAGAGCCTAAAAAGGACAAAAAGAAGAA
GGCTGATGAAACTCAAGCCTTACCGCAGAGACAGAAGAAACAGCAAACTGTGACTCTTCTTCCTG
 
CTGCAGATTTGGATGATTTCTCCAAACAATTGCAACAATCCATGAGCAGTGCTGACTCAACTCAG
GCCTAAACTCATGCAGACCACACAAGGCAGATGGGCTATATAAACGTTTTCGCTTTTCCGTTTAC
GATATATAGTCTACTCTTGTGCAGAATGAATTCTCGTAACTACATAGCACAAGTAGATGTAGTTAA
CTTTAATCTCACATAGCAATCTTTAATCAGTGTGTAACATTAGGGAGGACTTGAAAGAGCCACCAC
ATTTTCACCGAGGCCACGCGGAGTACGATCGAGTGTACAGTGAACAATGCTAGGGAGAGCTGCC
TATATGGAAGAGCCCTAATGTGTAAAATTAATTTTAGTAGTGCTATCCCCATGTGATTTTAATAGCT
TCTTAGGAGAATGACAAAAAAAAAAAAAAAAAAAAAAAAAAAAAAAA
**SEQ ID NO: 14, which is 30-44 of SEQ ID NO: 1** - only in sequence listing
**SEQ ID NO: 15, which is 48-68 of SEQ ID NO: 1-** only in sequence listing
**SEQ ID NO: 16, which is 110-166 of SEQ ID NO: 1** - only in sequence listing
**SEQ ID NO: 17, which is 200-214 of SEQ ID NO: 1** - only in sequence listing
**SEQ ID NO: 18, which is 226-250 of SEQ ID NO: 1** - only in sequence listing
**SEQ ID NO: 19, which is 256-277 of SEQ ID NO: 1** - only in sequence listing
**SEQ ID NO: 20, which is 328-342 of SEQ ID NO: 1** - only in sequence listing
**SEQ ID NO: 21, which is 399-414 of SEQ ID NO: 1** - only in sequence listing
**SEQ ID NO**: **22, which is 434-448 of SEQ ID NO: 1** - only in sequence listing
**SEQ ID NO: 23, which is 550-572 of SEQ ID NO: 1** - only in sequence listing
**SEQ ID NO: 24, which is 590-604 of SEQ ID NO: 1** - only in sequence listing
**SEQ ID NO: 25, which is 632-650 of SEQ ID NO: 1** - only in sequence listing
**SEQ ID NO: 26, which is 354-368 of SEQ ID NO: 1** - only in sequence listing
**SEQ ID NO: 27, which is 622-636 of SEQ ID NO: 1** - only in sequence listing
**SEQ ID NO: 28, which is 30-44 of SEQ ID NO: 1** - only in sequence listing
**SEQ ID NO: 29, which is 226-250 of SEQ ID NO: 1** - only in sequence listing
**SEQ ID NO: 30: SARS-CoV-2 N protein** 
MSDNGPQNQRNAPRITFGGPSDSTGSNQNGERSGARSKQRRPQGLPNNTASWFTALTQHGKEDLK
FPRGQGVPINTNSSPDDQIGYYRRATRRIRGGDGKMKDLSPRWYFYYLGTGPEAGLPYGANKDGIIW
VATEGALNTPKDHIGTRNPANNAAIVLQLPQGTTLPKGFYAEGSRGGSQASSRSSSRSRNSSRNSTP
GSSRGTSPARMAGNGGDAALALLLLDRLNQLESKMSGKGQQQQGQTVTKKSAAEASKKPRQKRTAT
KAYNVTQAFGRRGPEQTQGNFGDQELIRQGTDYKHWPQIAQFAPSASAFFGMSRIGMEVTPSGTWL
TYTGAIKLDDKDPNFKDQVILLNKHIDAYKTFPPTEPKKDKKKKADETQALPQRQKKQQTVTLLPAADL
DDFSKQLQQSMSSADSTQA
**SEQ ID NO: 31: RBD, a fragment from S1 domain from SARS-CoV-2 S protein** 
RVQPTESIVRFPNITNLCPFGEVFNATRFASVYAWNRKRISNCVADYSVLYNSASFSTFKCYGVSPTKL
NDLCFTNVYADSFVIRGDEVRQIAPGQTGKIADYNYKLPDDFTGCVIAWNSNNLDSKVGGNYNYLYRL
FRKSNLKPFERDISTEIYQAGSTPCNGVEGFNCYFPLQSYGFQPTNGVGYQPYRVVVLSFELLHAPAT
VCGPKKSTNLVKNKCVNF
SEQ ID NO: 32: RBD, a fragment from S1 domain from SARS-CoV-2 S protein, with C-terminal His tag 
RVQPTESIVRFPNITNLCPFGEVFNATRFASVYAWNRKRISNCVADYSVLYNSASFSTFKCYGVSPTKL
NDLCFTNVYADSFVIRGDEVRQIAPGQTGKIADYNYKLPDDFTGCVIAWNSNNLDSKVGGNYNYLYRL
FRKSNLKPFERDISTEIYQAGSTPCNGVEGFNCYFPLQSYGFQPTNGVGYQPYRVVVLSFELLHAPAT
VCGPKKSTNLVKNKCVNFLEHHHHHHHH
SEQ ID NO: 33: S2 domain from SARS-CoV-2 S protein 
SVASQSIIAYTMSLGAENSVAYSNNSIAIPTNFTISVTTEILPVSMTKTSVDCTMYICGDSTECSNLLLQY
GSFCTQLNRALTGIAVEQDKNTQEVFAQVKQIYKTPPIKDFGGFNFSQILPDPSKPSKRSFIEDLLFNKV
TLADAGFIKQYGDCLGDIAARDLICAQKFNGLTVLPPLLTDEMIAQYTSALLAGTITSGWTFGAGAALQI
PFAMQMAYRFNGIGVTQNVLYENQKLIANQFNSAIGKIQDSLSSTASALGKLQDVVNQNAQALNTLVK
QLSSNFGAISSVLNDILSRLDKVEAEVQIDRLITGRLQSLQTYVTQQLIRAAEIRASANLAATKMSECVLG
QSKRVDFCGKGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHTSPDVDLGDIS
GINASVVNIQKEIDRLNEVAKNLNESLIDLQELGKYEQYIKWPWYIWLGFIAGLIAIVMVTIMLCCMTSCC
SCLKGCCSCGSCCKFDEDDSEPVLKGVKLHYT
SEQ ID NO: 34: RBD as used in the examples 
MKHLWFFLLLVAAPRWVLSGPMRVQPTESIVRFPNITNLCPFGEVFNATRFASVYAWNRKRISNCVAD
YSVLYNSASFSTFKCYGVSPTKLNDLCFTNVYADSFVIRGDEVRQIAPGQTGKIADYNYKLPDDFTGCV
IAWNSNNLDSKVGGNYNYLYRLFRKSNLKPFERDISTEIYQAGSTPCNGVEGFNCYFPLQSYGFQPTN
GVGYQPYRVVVLSFELLHAPATVCGPKKSTNLVKNKCVNFLEHHHHHHHH
**SEQ ID NO: 35 (SEQ ID NO: 1-derived peptide reactive with SARS-CoV-2 antibodies)** LTPGDSSSGWTAG
**SEQ ID NO: 36 (SEQ ID NO: 1-derived peptide reactive with SARS-CoV-2 antibodies)** YQAGSTPCNGV
**SEQ ID NO: 37 (SEQ ID NO: 1-derived peptide reactive with SARS-CoV-2 antibodies)** YGFQPTNGVGYQ
**SEQ ID NO: 38: His-tagged RBD** 
MKHLWFFLLLVAAPRWVLSGPMRVQPTESIVRFPNITNLCPFGEVFNATRFASVYAWNRKRISNCVAD
YSVLYNSASFSTFKCYGVSPTKLNDLCFTNVYADSFVIRGDEVRQIAPGQTGKIADYNYKLPDDFTGCV
IAWNSNNLDSKVGGNYNYLYRLFRKSNLKPFERDISTEIYQAGSTPCNGVEGFNCYFPLQSYGFQPTN
GVGYQPYRVVVLSFELLHAPATVCGPKKSTNLVKNKCVNFLEHHHHHHHH
SEQ ID NO: 39: human Angiotensin-converting enzyme 2 (ACE2)
SEQ ID NO: 40 (SEQ ID NO: 1-derived peptide reactive with SARS-CoV-2 antibodies) RTWLPPAYTNS
SEQ ID NO: 41 (SEQ ID NO: 1-derived peptide reactive with SARS-CoV-2 antibodies) RTQLPPAYTNS
SEQ ID NO: 42 (SEQ ID NO: 1-derived peptide reactive with SARS-CoV-2 antibodies) SGTNGTKRFDN
SEQ ID NO: 43 (SEQ ID NO: 1-derived peptide reactive with SARS-CoV-2 antibodies) 
MSHHHHHHHHSPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGIEGRMRTQLPPAYTNSRTQLP
PAYTNS
SEQ ID NO: 44 (SEQ ID NO: 1-derived peptide reactive with SARS-CoV-2 antibodies with GST fusion) 
MSHHHHHHHHSPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGIEGRMMSHHHHHHHHSPMY
SIITPNILRLESEETMVLEAHDAQGDVPVTVTVHDFPGKKLVLSSEKTVLTPATNHMGNVTFTIPANREF
KSEKGRNKFVTVQATFGTQVVEKVVLVSLQSGIEGRMSGTNGTKRFDNSGTNGTKRFDN
SEQ ID NO: 45 (SEQ ID NO: 1-derived peptide reactive with SARS-CoV-2 antibodies with GST fusion) 
MSHHHHHHHHSPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGIEGRMLTPGDSSSGWTAGLT
PGDSSSGWTAG
SEQ ID NO: 46 (SEQ ID NO: 1-derived peptide reactive with SARS-CoV-2 antibodies with GST fusion) 
MSHHHHHHHHSPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGIEGRMNNLDSKVGGNNLDSK
VGG
SEQ ID NO: 47 (SEQ ID NO: 1-derived peptide reactive with SARS-CoV-2 antibodies with GST fusion) 
MSHHHHHHHHSPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGIEGRMYQAGSTPCNGVYQAG
STPCNGV
SEQ ID NO: 48 (SEQ ID NO: 1-derived peptide reactive with SARS-CoV-2 antibodies with GST fusion) 
MSHHHHHHHHSPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGIEGRMYGFQPTNGVGYQYGF
QPTNGVGYQ
SEQ ID NO: 49 (SEQ ID NO: 1-derived peptide reactive with SARS-CoV-2 antibodies with GST fusion, P1-P6) 
MSHHHHHHHHSPMYSIITPNILRLESEETMVLEAHDAQGDVPVTVTVHDFPGKKLVLSSEKTVLTPATN
HMGNVTFTIPANREFKSEKGRNKFVTVQATFGTQVVEKVVLVSLQSGIEGRMRTQLPPAYTNSSGTN
GTKRFDNLTPGDSSSGWTAGNNLDSKVGGYQAGSTPCNGVYGFQPTNGVGYQ
SEQ ID NO: 50 (C-terminally His-tagged extracellular domain of human ACE2) 
MSSSSWLLLSLVAVTAAQSTIEEQAKTFLDKFNHEAEDLFYQSSLASWNYNTNITEENVQNMNNAGDK
WSAFLKEQSTLAQMYPLQEIQNLTVKLQLQALQQNGSSVLSEDKSKRLNTILNTMSTIYSTGKVCNPD
NPQECLLLEPGLNEIMANSLDYNERLWAWESWRSEVGKQLRPLYEEYVVLKNEMARANHYEDYGDY
WRGDYEVNGVDGYDYSRGQLIEDVEHTFEEIKPLYEHLHAYVRAKLMNAYPSYISPIGCLPAHLLGDM
WGRFWTNLYSLTVPFGQKPNIDVTDAMVDQAWDAQRIFKEAEKFFVSVGLPNMTQGFWENSMLTDP
GNVQKAVCHPTAWDLGKGDFRILMCTKVTMDDFLTAHHEMGHIQYDMAYAAQPFLLRNGANEGFHE
AVGEIMSLSAATPKHLKSIGLLSPDFQEDNETEINFLLKQALTIVGTLPFTYMLEKWRWMVFKGEIPKDQ
WMKKWWEMKREIVGVVEPVPHDETYCDPASLFHVSNDYSFIRYYTRTLYQFQFQEALCQAAKHEGP
LHKCDISNSTEAGQKLFNMLRLGKSEPWTLALENVVGAKNMNVRPLLNYFEPLFTWLKDQNKNSFVG
WSTDWSPYADQSIKVRISLKSALGDKAYEWNDNEMYLFRSSVAYAMRQYFLKVKNQMILFGEEDVRV
ANLKPRISFNFFVTAPKNVSDIIPRTEVEKAIRMSRSRINDAFRLNDNSLEFLGIQPTLGPPNQPPVSLEG
SGSGSHHHHHHHHGSGLNDIFEAQKIEWHE
**SEQ ID NO: 51 (SEQ ID NO1 with mutations of SARS-CoV-2 U.K. variant B.1.1.7-** only in sequence listing
**SEQ ID NO: 52 (SEQ ID NO1 with mutations of SARS-CoV-2 South African variant B.1.351): - only in sequence listing**
**SEQ ID NO: 53 (SEQ ID NO1 with mutations of SARS-CoV-2 Brazilian variant P.1):** - only in **sequence listing**
**SEQ ID NO: 54 (SEQ ID NO1 with mutations of SARS-CoV-2 Mink Variant from Denmark):** - only in sequence listing
**SEQ ID NO55: (SEQ ID NO: 1-derived peptide reactive with SARS-CoV-2 antibodies)** NNLDSKVGG
**SEQ ID NO: 56 (SEQ ID NO: 1-derived peptide reactive with SARS-CoV-2 antibodies with GST** fusion, P1-P6)

RTQLPPAYTNSSGTNGTKRFDNLTPGDSSSGWTAGNNLDSKVGGYQAGSTPCNGVYGFQPTNGVG
YQ

The present invention is further illustrated by the following examples, sequences and figures from which further features, embodiments, aspects and advantages of the present invention may be taken. All methods and materials similar or equivalent to those described herein can be used in the practice or testing of the present invention, with suitable methods and materials being described herein. All publications, patent applications, patents, and other references mentioned herein are incorporated by reference in their entirety.
Fig. 1 shows a time course of antibody levels to SEQ ID NO: 1 (IgA, IgG) and N protein (IgG, IgM) monitored in two patients (Fig. 1), as described in Example 3.
Fig. 2 shows monitoring of another time course with a patient infected with SARS-CoV-2. IgA class antibodies to SEQ ID NO: 1 (squares), IgG class antibodies to SEQ ID NO: 1 (triangles) and IgG class antibodies to N protein (circles) were determined.

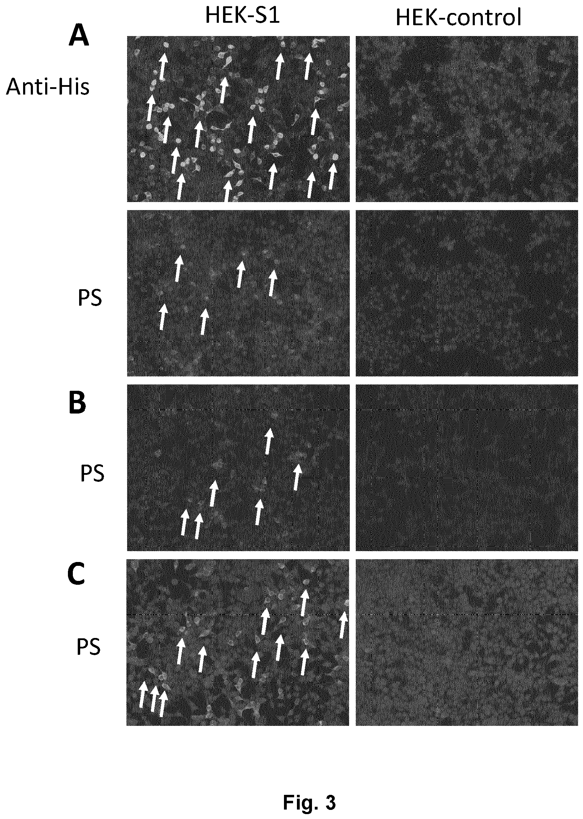

**Fig. 3** shows IFA using acetone-fixed S1-expressing or control plasmid-transfected HEK293 cells **(A)** incubated directly with anti-His tag (1:200) or patient serum (PS, 1:100) in the first step and antimouse-IgG-FITC or anti-human-IgG-FITC in the second step, **(B)** incubated 30 min with PBS prior to the two step incubation with PS3 as described in A, or **(C)** incubated 30 min with PBS, followed by an incubation with PS3 (1:100) in the first step, anti-human-IgG-Biotin (1:200) in the second step and ExtrAvidin-FITC (1:2000) in the third step. S1-expressing cells had been transfected with a pTriEx vector expressing SEQ ID NO: 2 using standard methods. Representative cells identified as reactive with patient antibody were marked using arrows.
Fig. 4 shows the detection of IgG, IgA and IgM antibodies from the sample of a patient based on dot blot analysis using a fusion protein comprising RBD.
Fig. 5 shows the detection of IgG, IgA and IgM antibodies from the sample of a patient based on dot blot analysis using a polypeptide comprising SEQ ID NO: 1.
Fig. 6 shows the detection of IgM antibodies from the sample of a patient based on dot blot analysis using fragments of SEQ ID NO: 1 and fusion proteins thereof.
Fig. 7 shows the detection of IgA antibodies from the sample of a patient based on dot blot analysis using fragments of SEQ ID NO: 1 and fusion proteins thereof.
Fig. 8 shows the detection of IgG antibodies from the sample of a patient based on dot blot analysis using fragments of SEQ ID NO: 1 and fusion proteins thereof.
Fig. 9 shows the time-dependent detection of IgM antibodies from samples from patients based on dot blot analysis using fragments of SEQ ID NO: 1 and fusion proteins thereof.
Fig. 10 shows the time-dependent detection of IgG antibodies from samples from patients based on dot blot analysis using fragments of SEQ ID NO: 1 and fusion proteins thereof.
**Fig. 11** shows the time-dependent detection of IgA antibodies from samples from patients based on dot blot analysis using fragments of SEQ ID NO: 1 and fusion proteins thereof.
**Fig. 12** shows the detection of IgA antibodies from the sample of a patient based on Western analysis using a polypeptide comprising RBD.
**Fig. 13** shows the detection of IgM antibodies from the sample of a patient based on Western blot analysis using a polypeptide comprising RBD.
**Fig.** 14 shows the detection of IgG antibodies from the sample of a patient based on Western blot analysis using a polypeptide comprising RBD.
**Fig. 15** shows the time-dependent detection of IgA antibodies from samples of patients based on Western blot analysis using a polypeptide comprising SEQ ID NO: 1.
**Fig. 16** shows the time-dependent detection of IgM antibodies from samples of patients based on Western blot analysis using polypeptide comprising SEQ ID NO: 1.
**Fig. 17** shows the time-dependent detection of IgG antibodies from samples of patients based on Western blot analysis using a polypeptide comprising SEQ ID NO: 1.

### Example 1: Detection of antibodies to SEQ ID NO: 1 using an ELISA immunoassay

### Samples

Eight samples from patients tested SARS-CoV-2 positive by PCR as described by Corman *et al.* (Corman et al. (2020) Diagnostic detection of 2019-nCoV by real-time RT-PCR, https://www.who.int/docs/default-source/coronaviruse/protocol-v2-1.pdf?sfvrsn=a9ef618c 2) were obtained 6 to 14 days after the infection and 14 samples from such patients obtained at an earlier time point after the infection were available.

In addition, a range of samples containing various coronaviruses was available, including 18 samples from patients infected with MERS, three samples from patients infected with SARS-CoV-1, four patients with NL63, three patients with 229E, six patients with OC43 and three patients with HKU1.

**Preparation of microtiter plates coated with antigen:** SEQ ID NO: 2 was expressed in HEK293T cells using standard cloning of SEQ ID NO: 4 into the pTriEx-1 plasmid with an artificial signal sequence and a C-terminal His tag, resulting in the expression of SEQ ID NO: 2 and, after removal of the signal peptide, SEQ ID NO: 3. Transfected cells were cultured at 37°C and 8.5% CO₂ in Dulbecco's modified eagle's medium with 10% fetal calf serum, 100 U/ml penicillin and 0.1 mg/ml streptomycin for three to five days. Cells were harvested, resuspended in 20 mM Tris-HCl pH 7.4, 10% (w/v) sucrose, 5 mM EDTA, 1 mM PMSF and stored at -80°C until further use.

To prepare SEQ ID NO: 3, cell culture supernatant was adjusted to 5 mmol/l tris chloride pH 8.0, 164 mmol/l sodium chloride, 50 mmol/l magnesium chloride, 20 mmol/l imidazole, 0,1% Triton X-100, cleared by centrifugation for 30 minutes at 17,600xg, 4°C, applied to Nickel Rapid Run (Agarose Bead Technologies, Miami, FL, USA) equilibrated with 5 mmol/l tris chloride pH 8.0, 300 mmol/l sodium chloride, 20 mmol/l imidazole and eluted by increasing the imidazole concentration to 150 mmol/l. All fractions containing SEQ ID NO: 3 were pooled and concentrated by ultrafiltration (VivaSpin, Sartorius, Göttingen, Germany). The final preparation was stored at -80°C until further use.

The final protein preparation of SEQ ID NO: 3 was treated with or without 16 mmol/l dithiotreitol and incubated at 70°C or at room temperature for 10 minutes, followed by SDS gel electrophoresis and Coomassie staining. Protein identity was verified by mass spectrometry.

For use in microtiter ELISA the purified protein was diluted in PBS to final concentrations of approximately 1.5 µg/ml and used to coat ELISA microtiter plates (Nunc, Roskilde, Denmark) overnight.

**Experimental procedure:** Samples were diluted 1:101 in IgG sample buffer, applied to microtiter plates and incubated as described for commercial EUROIMMUN ELISA Test-Kits, using reagents commercially available (e.g. EI 2260-9601 G/A, which is a buffer having essentially physiological conditions regarding salt concentration and pH). The manual of EI 2260-9601 G/A was followed. In brief: 60 min at 37 °C; 3 washing steps using washing buffer; addition of 100 µl of peroxidase-labelled anti-human IgG conjugate (rabbit) or anti-human IgA conjugate (rabbit) per well; incubation for 30 min at 37 °C; 3 washing steps using EUROIMMUN washing buffer; addition of 100 µl of chromogen/substrate solution (TMB/H₂O₂) per well; incubation for 30 min at room temperature; addition of 100 µl stop-solution (0.5 M sulfuric acid); measurement of optical density at 450 nm against 630 nm as a reference.

Calibration was carried out using commercially available calibrators (product number EI 2606-9601 A, EUROIMMUN Medizinische Labordiagnostika AG). A ratio was calculated by dividing extinction of the control or patient sample by the extinction of the calibrator. Results below 0.8 were considered negative, results between 0.8 and 1.1 borderline, and results of more than 1.1 positive.

**Results: The primary data are shown in Table 1:**

| | | **raw data** | | **Cut-Off OD** | **raw data** | | **Cut-Off OD** |
|---|---|---|---|---|---|---|---|
| | | | | **0,100** | | | **0,200** |
| | | **IgG** (OD) | **dil.** 1: | **IgG** (Ratio) | **IgA** (OD) | **dil. 1:** | **IgA** (Ratio) |
| | | | | **pos**: **≥ 1,1** | | | **pos: ≥ 1,1** |
| | | | | bl: 0,8-1,0 | | | bl: 0,8-1,0 |
| **1** | Calibrator | 1,911 | 300 | **19,1** | 3,055 | 600 | **15,3** |
| **2** | Calibrator | 1,593 | 600 | **15,9** | 2,204 | 1200 | **11,0** |
| **3** | Calibrator | 1,077 | 1200 | **10,8** | 1,068 | 2400 | **5,3** |
| **4** | Calibrator | 0,697 | 2400 | **7,0** | 0,529 | 4800 | **2,6** |
| **5** | Calibrator | 0,441 | 4800 | **4,4** | 0,314 | 9600 | **1,6** |
| **6** | Calibrator | 0,248 | 9600 | **2,5** | 0,155 | 19200 | 0,8 |
| **7** | Calibrator | 0,132 | 19200 | **1,3** | 0,078 | 38400 | 0,4 |
| **8** | Calibrator | 0,066 | 38400 | 0,7 | 0,051 | 76800 | 0,3 |
| **9** | SARS-CoV-2* | 0,157 | | **1,6** | 1,402 | | **7,0** |
| **10** | SARS-CoV-2* | 0,075 | | 0,8 | 0,377 | | 1,9 |
| **11** | SARS-CoV-2* | 0,276 | | **2,8** | 9,999 | | **50,0** |
| **12** | SARS-CoV-2* | 0,027 | | 0,3 | 0,027 | | 0,1 |
| 13 | SARS-CoV-2* | 0,023 | | 0,2 | 0,064 | | 0,3 |
| **14** | SARS-CoV-2* | 0,119 | | **1,2** | 1,142 | | **5,7** |
| **15** | SARS-CoV-2* | 0,031 | | 0,3 | 0,203 | | **1,0** |
| **16** | SARS-CoV-2* | 0,079 | | 0,8 | 0,385 | | **1,9** |
| **17** | SARS-COV-2** | 0,021 | | 0,2 | 0,055 | | 0,3 |
| **18** | SARS-COV-2** | 0,019 | | 0,2 | 0,084 | | **0,4** |
| **19** | SARS-COV-2** | 0,021 | | 0,2 | 0,225 | | **1,1** |
| **20** | SARS-COV-2** | 0,018 | | 0,2 | 0,192 | | **1,0** |
| **21** | SARS-COV-2** | 0,033 | | 0,3 | 0,599 | | **3,0** |
| **22** | SARS-COV-2** | 0,029 | | 0,3 | 0,331 | | **1,7** |
| **23** | SARS-COV-2** | 0,013 | | 0,1 | 0,030 | | 0,2 |
| **24** | SARS-COV-2** | 0,017 | | 0,2 | 0,097 | | 0,5 |
| **25** | SARS-COV-2** | 0,027 | | 0,3 | 0,214 | | **1,1** |
| **26** | SARS-COV-2** | 0,016 | | 0,2 | 0,021 | | 0,1 |
| **27** | SARS-COV-2** | 0,014 | | 0,1 | 0,073 | | **0,4** |
| **28** | SARS-COV-2** | 0,014 | | 0,1 | 0,042 | | 0,2 |
| **29** | SARS-COV-2** | 0,015 | | 0,2 | 0,030 | | 0,2 |
| **30** | SARS-COV-2** | 0,015 | | 0,2 | 0,079 | | 0,4 |
| **31** | MERS 1 | 0,013 | | 0,1 | 0,042 | | 0,2 |
| **32** | MERS 2 | 0,008 | | 0,1 | 0,017 | | 0,1 |
| **33** | MERS 3 | 0,011 | | 0,1 | 0,038 | | 0,2 |
| **34** | MERS 4 | 0,008 | | 0,1 | 0,011 | | 0,1 |
| **35** | MERS 5 | 0,008 | | 0,1 | 0,027 | | 0,1 |
| **36** | MERS 6 | 0,008 | | 0,1 | 0,035 | | 0,2 |
| **37** | MERS 7 | 0,009 | | 0,1 | 0,013 | | 0,1 |
| **38** | MERS 8 | 0,017 | | 0,2 | 0,036 | | 0,2 |
| **39** | MERS 9 | 0,010 | | 0,1 | 0,024 | | 0,1 |
| **40** | MERS 10 | 0,007 | | 0,1 | 0,012 | | 0,1 |
| **41** | MERS 11 | 0,020 | | 0,2 | 0,026 | | 0,1 |
| **42** | MERS 12 | 0,008 | | 0,1 | 0,026 | | 0,1 |
| **43** | MERS 13 | 0,015 | | 0,2 | 0,021 | | 0,1 |
| **44** | MERS 14 | 0,012 | | 0,1 | 0,036 | | 0,2 |
| **45** | MERS 15 | 0,024 | | 0,2 | 0,066 | | 0,3 |
| **46** | MERS 16 | 0,021 | | 0,2 | 0,080 | | 0,4 |
| **47** | MERS 17 | 0,008 | | 0,1 | 0,039 | | 0,2 |
| **48** | MERS 18 | 0,029 | | 0,3 | 0,104 | | 0,5 |
| **49** | SARS-1 | 0,214 | | **2,1** | 0,285 | | **1,4** |
| **50** | SARS-1 | 0,596 | | **6,0** | 0,227 | | **1,1** |
| **51** | SARS-1 | 0,128 | | **1,3** | 0,260 | | **1,3** |
| **52** | OC43 | 0,035 | | 0,4 | 0,126 | | 0,6 |
| **53** | OC43 | 0,029 | | 0,3 | 0,098 | | 0,5 |
| **54** | OC43 | 0,016 | | 0,2 | 0,041 | | 0,2 |
| **55** | OC43 | 0,011 | | 0,1 | 0,048 | | 0,2 |
| **68** | NL63 | 0,013 | | 0,1 | 0,023 | | 0,1 |
| **69** | NL63 | 0,027 | | 0,3 | 0,039 | | 0,2 |
| **70** | NL63 | 0,036 | | 0,4 | 0,057 | | 0,3 |
| **71** | NL63 | 0,019 | | 0,2 | 0,030 | | 0,2 |
| **72** | 229E | 0,041 | | 0,4 | 0,045 | | 0,2 |
| **73** | 229E | 0,022 | | 0,2 | 0,024 | | 0,1 |
| **74** | 229E | 0,030 | | 0,3 | 0,034 | | 0,2 |
| **75** | OC43 | 0,050 | | 0,5 | 0,100 | | 0,5 |
| **76** | OC43 | 0,079 | | 0,8 | 0,201 | | 1,0 |
| **77** | HKU1 | 0,023 | | 0,2 | 0,054 | | 0,3 |
| **78** | HKU1 | 0,019 | | 0,2 | 0,055 | | 0,3 |
| **79** | HKU1 | 0,010 | | 0,1 | 0,029 | | 0,1 |

| | | | | | | | |
|---|---|---|---|---|---|---|---|
| (*late stage; ** < day 6) | | | | | | | |

### Conclusions

The results show that antibodies to SEQ ID NO: 1 may be used to diagnose a SARS-CoV-2 infection in samples from human patients.

Comparison of the data obtained with secondary antibodies recognizing IgG and IgA class antibodies shows that the detection of IgA antibodies is more sensitive, at least at an early stage of the disease: 4/14 patient samples taken at an earlier stage of the infection, before six days post onset of illness, could be correctly identified as positive when IgA class antibodies were detected, while the detection of IgG in the same samples gave negative results.

Both assays showed cross-reactivity with samples from SARS-CoV-1 patients, but virtually none of the samples from patients infected with MERS, NL63, 229E, OC43 and HKU1. Distinction between SARS-CoV-1 and SARS-CoV-2 is possible based on the different time-resolved Ig class signature, in particular the later emergence of IgA class antibodies in SARS-CoV-1 (Hsue, P. R., Huang, L. M., Chen, P. J., Kao, C. L., and Yang P. C. (2004) Chronological evolution of IgM, IgA, IgG and neutralization antibodies after infection with SARS-associated coronavirus, Clinical Microbiology and Infection, 10(12), 1062-1066). Not in the least, hardly any cases of SARS-CoV have been reported since the outbreak of SARS-CoV-2.

Various post published publications by independent researchers confirmed the inventors' findings:
Jääskelainen *et al.* concluded from a comparative study with six commercially available serological assays for detection of SARS-CoV-2 IgG, IgA and/or IgM antibodies that, among the six assays tested, the EUROIMMUN assay based on the detection of IgA to S1 provided the highest sensitivity (Jääskelainen, A. J., Kuivanen, S., Kekäläinen, E., Ahava, M.J., Loginov, R., Kallio-Kokko, H., Vapalahti, O., Jarva, H., Kurkela, and Lappalainen, M. (2020), J. Clin. Virology 129, 104512). Okba et al. obtained similar results. (Okba et al., Severe Acute Respiratory Syndrome Coronavirus 2-Specific Antibody Responses in Coronavirus Disease Patients. Emerg Infect Dis. 2020 Jul;26(7):1478-1488, prepublished on medRxiv 2020.03.18.20038059).Beavis *et al.* confirmed that the sensitivity of the IgA-based assay according to the present invention is superior at an early stage of the disease, while the IgG assay is superior at a later stage (Beavis, K. G., Mathushek, S. M., Abeleda, A. P. F., Bethel, C., Hunt, C., Gillen, S., Moran, A., and Tesic, V. (2020) Evalutaion of the EUROIMMUN Anti-SARS-CoV-2 ELISA Assay for detection of IgA and IgG antibodies, J. Clin. Virology 129, 104468).

In summary, the assay according to the present invention may be used for the early detection of diagnostically relevant antibodies to SEQ ID NO: 1. In some patients, true positive results can be found before six days have passed since the onset of symptoms. Therefore, the assay helps close or at least narrow down the diagnostic gap between the period when PCR-based assays and immunoassays may be used.

### Example 2: An extended ELISA study aiming to further characterize the diagnostic reliability and relevance of tests for the detection of IgA antibodies to SEQ ID NO: 1

For the purpose of determining the sensitivity of the instant assay, the presence or absence of IgA antibodies was detected in 166 samples from 152 European patients using EUROIMMUN product no. EI 2606-9601 A, based on an assay similar as described in Example 1. In each of the subject samples, a SARS-CoV-2 infection had been confirmed using RT-PCR according to Corman VM, et al. Detection of 2019 novel coronavirus (2019-nCoV) by real-time RT-PCR. Euro Surveill 25(3): pii=2000045 (2020-01-23) based on a sample from an early stage of the infection.

Briefly, this assay is based on an ELISA using a microtiter plate coated with an antigen comprising purified SEQ ID NO: 1. Following incubation with samples and extensive washing steps using physiological buffers, a secondary antibody to IgA labeled with an enzymatically active label is used to specifically decorate IgA antibodies to SEQ ID NO: 1, followed by incubation of a chromogenic substrate. Further details can be found in the manual supplied with the product (EUROIMMUN product no. EI 2606-9601 A), which product manual is herewith incorporated by reference.

The serological characterization was carried out based on samples obtained in the following course of the infection.

A sensitivity of 60.2% regarding IgA to SEQ ID NO: 1 was determined with samples obtained until day 10 post onset of symptoms (or obtained until day 10 after direct detection of infection by positive RT-PCR). The sensitivity was 98.6% for samples obtained after day 10.

The results and immunoresponses are highly individual though. For example, a minority of patients may not show IgA, IgG or IgM responses at all.

Taken together, this further study also consistently demonstrates that the specific detection of IgA class antibodies directed against SEQ ID NO: 1 provides particularly high sensitivity for detecting SARS-CoV-2 infection already at an early stage of disease.

### Example 3: Persistence of various diagnostically relevant antibodies over time in SARS-CoV-2 patients as shown by ELISA

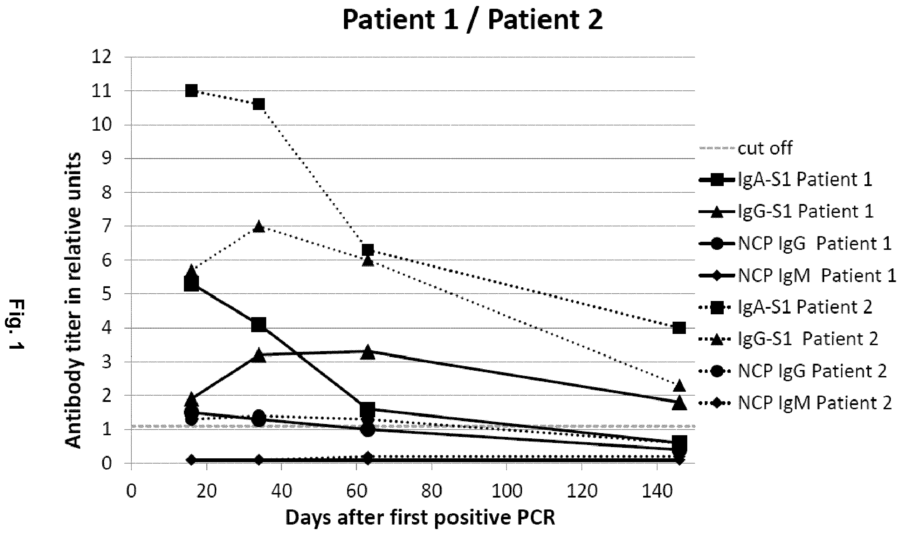

The time course of antibody levels to SEQ ID NO: 1 (IgA, IgG) and N protein (IgG, IgM) was monitored in two patients (Fig. 1). Both presented with a mild course of the disease, but the infection had been confirmed by RT-PCR. Typical specific symptoms such as temporary loss of smell were observed. EUROIMMUN products EI 2606-9601 A and EI 2606-9601 G (S1 protein as antigen, IgA or IgG detection, respectively) and EI 2606-9601-2 G and EI 2606-9601-2 M (N protein as antigen, IgG or IgM detection, respectively) were used according to the manufacturer's instructions, which are incorporated here by reference. The principle and major components of the tests are outlined in Example 2; however, different antigens (N protein as antigen instead of a polypeptide comprising SEQ ID NO: 1) as well as different secondary antibodies (binding to IgA, IgG or IgM) were used depending on which antibody was to be detected. Additional information is available in the manufacturer's instructions of EI 2606-9601 A, EI 2606-9601 G, EI 2606-9601-2 G and EI 2606-9601-2 M (EUROIMMUN).

The course of the disease was monitored over more than four months. IgA and IgG to SEQ ID NO: 1 and IgG, but no IgM to N protein were detectable initially. In both patients, at least one of IgA or IgG to SEQ ID NO: 1 was detectable after four months, whereas IgG to N protein was absent or only weak as early as two months after the first positive PCR.

These results show that antibodies to SEQ ID NO: 1 persist at least in some patients longer than those to N protein do. The level of IgA antibodies decreases more rapidly than the IgG antibody level, but IgA antibodies may still be predominant as they disappear, owing to their initially higher signal.

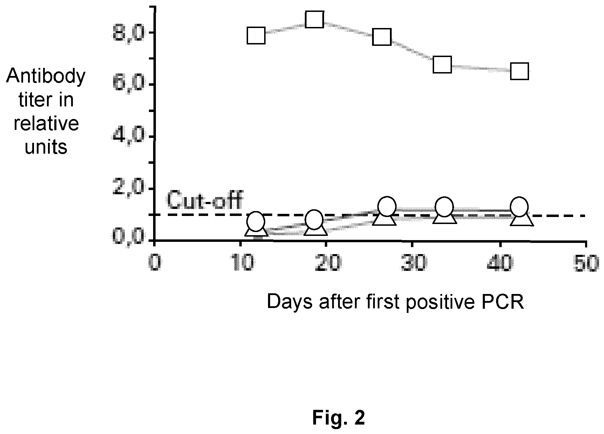

The monitoring of another time course with a third patient is shown in Fig. 2. Essentially, IgA class antibodies to SEQ ID NO: 1 (squares), IgG class antibodies to SEQ ID NO: 1 (triangles) and IgG class antibodies to N protein (circles) were determined using the same methods. As evident from Fig. 2, IgA class antibodies to S1 [SEQ ID NO: 1] were detectable over the entire time period monitored, i.e. even after 40 days after RT-PCR-confirmed SAR-CoV-2 infection, whereas levels of IgG class antibodies to either SEQ ID NO: 1 or to SARS-CoV-2 N protein remained under (or close to) the cut-off. Thus, these data additionally confirm the superior sensitivity of the instant assay which is based on the specific detection of the IgA class antibodies bound to the SEQ ID NO: 1 antigen.

### Example 4: Chemiluminescence-based assay for detection of antibodies to SEQ ID NO: 1 used to monitor various antibodies over time

A EUROIMMUN Random Access RA 10 Analyzer (YG 0710-0101) was used according to the manufacturer's instructions and default settings, including a reagent cartridge (LS 1254-10010 G).

Tosylactivated paramagnetic beads (M-280, Invitrogen) were coated using the manufacturer's instructions. Briefly, beads were washed in coating buffer (0.1 M sodium phosphate pH 7.4) for 10 minutes at 37 °C on an IKA Roller 10 (supplied by VWR), followed by magnetic concentration of beads and removal of supernatant. Recombinant polypeptide (SEQ ID NO: 3) purified according to Example 1 was added in the same buffer, followed by addition of 3 M ammonium sulphate and incubation under the same conditions for 19 h, followed by two washing steps using washing buffer (PBS pH 7.4 0.1% BSA 0.2% Tween-20), blocking in the same buffer for 4 h at 37 °C (PBS pH 7.4 0.1% BSA 0.2% Tween-20) and two additional wash steps. Beads were stored in the same buffer for at least 16 h at 4 °C.

The assay was carried out by mixing paramagnetic beads with sample buffer (BSA/Tween-20 in Tris-HCI EDTA pH 7. 20 µl suspension of beads (1 mg/ml) was contacted with 5 µl of sample (i.e. a blood sample of a patient) in a total of 200 µl in sample buffer. After incubation for ten minutes, beads were washed thrice in sample buffer, followed by addition of 160 µl IgG / IgA conjugate (EUROIMMUN Medizinische Labordiagnostik AG, LK 0711-10010, essentially an IgG or IgA-specific secondary antibody labeled with acridinium ester) and incubation for 10 minutes at 37 °C. After three washing steps, alkaline hydrogen peroxide was rapidly added and mixed to trigger the emission of light, followed by immediate luminescence detection for 10 seconds. Results are shown in Table 2:

| | | ELISA | ELISA |
|---|---|---|---|
| | | **IgA** | **IgG** |
| | | Ratio **pos: ≥ 1,1** bl: 0,8-<1,1 | Ratio **pos: ≥** 1,1 bl: 0,8-<1,1 |
| Nr. | | | |
| **1** | IgG / IgA | **8,2** | **2,2** |
| **2** | positive | **12,7** | **6,3** |
| **3** | samples | **13,1** | **5,4** |
| **4** | Negative | 0,3 | 0,3 |
| **5** | samples | 0,3 | 0,1 |
| **6** | | 0,3 | 0,0 |
| **7** | | 0,4 | 0,1 |
| **8** | | 0,5 | 0,3 |
| **15** | IgM positive samples | **2,3** | 2, 1 |
| **16** | | **1,1** | **5,6** |
| **17** | | 0,9 | **6,5** |
| **20** | **blank** | | |

| | Chemiluminescence | | Chemiluminescence | | Chemiluminescence | |
|---|---|---|---|---|---|---|
| | **IgG** | | **IgA** | | **IgM** | |
| | MW (RLU) Cutoff 40.000 | Ratio **pos:** ≥ **1,1** bl: 0,8-1,1 | MW (RLU) Cutoff 50.000 | Ratio **pos:** ≥ **1,1** bl: 0,8-1,1 | MW (RLU) Cutoff 50.000 | Ratio **pos: ≥ 1,1** bl: 0,8-1,1 |
| **1** | 36.051 | 0,9 | 79.014 | **1,6** | 9.535 | 0,2 |
| **2** | 128.217 | **3,2** | 244.029 | **4,9** | 24.354 | 0,5 |
| **3** | 223.597 | **5,6** | 307.108 | **6,1** | 21.478 | 0,4 |
| **4** | 3.222 | 0,1 | 6.861 | 0,1 | 4.495 | 0,1 |
| **5** | 4.152 | 0,1 | 4.707 | 0,1 | 2.095 | 0,0 |
| **6** | 2.005 | 0,1 | 3.943 | 0,1 | 2.133 | 0,0 |
| **7** | 15.280 | 0,4 | 9.498 | 0,2 | 4.929 | 0,1 |
| **8** | 11.401 | 0,3 | 10.970 | 0,2 | 2.745 | 0,1 |
| **15** | | | | | 9.292 | 1,0 |
| **16** | | | | | 50.715 | **1,3** |
| **17** | | | | | 62.684 | 0,0 |
| **20** | | 576 | | | 1.826 | 0,0 |

These results show that antibodies to SEQ ID NO:1 can be detected using chemiluminescence. There is a good correlation with results obtained by ELISA. Therefore, chemiluminescence can be used to practice the present invention.

### Example 5: Indirect immunofluorescence assay (IFA) with HEK-S1 cells expressing SEQ ID NO: 2

IFA was conducted using slides with a biochip array of recombinant HEK293 cells expressing SARS-CoV-2 S1 protein or HEK293 control cells to demonstrate that this method may be used for the detection of antibodies to SEQ ID NO: 1.

Each biochip mosaic was incubated with 35 µL of 1:100 PBS-diluted serum samples at room temperature for 30 min, washed with PBS-Tween and immersed in PBS-Tween for 5 min. In the second step, fluorescein isothiocyanate (FITC)-labelled goat anti-human IgG (EUROIMMUN Medizinische Labordiagnostika AG, Lübeck) was applied and incubated at room temperature for 30 min. Slides were washed again with a flush of PBS-Tween and then immersed in PBS-Tween for 5 min. Slides were embedded in PBS-buffered, DABCO containing glycerol (approximately 10 µL per field) and examined by fluorescence microscopy. Alternatively, slides were incubated 30 min with PBS prior to serum incubation and bound human IgGs were detected with by 30 min incubation with anti-human-IgG-Biotin (1:200, 109-065-098, Dianova), followed by a washing step as described above and 30 min incubation with ExtrAvidin-FITC (1:2000, E2761, Sigma-Aldrich), followed by another washing step. Samples were classified as positive or negative based on the fluorescence intensity of the transfected cells in direct comparison with control-transfected cells and control samples. Results were evaluated by two independent observers using a EUROStar II microscope (EUROIMMUN Medizinische Labordiagnostika AG, Lübeck, Germany). Reagents were obtained from Merck, Darmstadt, Germany or Sigma-Aldrich, Heidelberg, Germany if not specified otherwise.

The sera of a S1 IgG ELISA-positive patient serum (PS) showed a positive reaction with S1 (SEQ ID NO: 2) but not control transfected HEK cells (**Fig. 3**), whereas none of the 49 S1 IgG ELISA-negative control sera reacted. Signal intensity of the patient serum was improved by incubating the HEK-S1 cells with PBS prior to serum incubation and detection of human IgG antibodies with anti-human-IgG-Biotin/ExtrAvidin-FITC. Altogether 15/24 S1 IgG ELISA-positive patient sera showed a positive reaction in HEK-S1 IFA with anti-human-IgG-Biotin/ExtrAvidin-FITC.

Therefore, IFT is another method which can be used to practice the invention.

### Example 6: Vaccination studies

A healthy subject received on day 1 an injection into the musculus quadriceps of 12.86 µg recombinant S1 protein in physiological PBS (SEQ ID NO: 3). Alum adjuvans (Twinrix for adults, EMRA-MED Arzneimittel GmbH) was applied according to the manufacturer's instructions. On days 9, 21 and 28, the subject received an injection of another 12.86 µg S1 protein in physiological PBS buffer and 10 µl Imject alaun adjuvans (Thermo Scientific Imject Alum Adjuvant Alaun) in 500 µl sodium chloride solution. Blood samples were obtained on days 10, 23 and 29.

Presence of IgG and IgA antibodies to SEQ ID NO1 (S1) and N protein was determined in serum samples from the healthy subject using serological kits (EUROIMMUN Medizinische Labordiagnostika AG, EI 2606-9601 A, EI 2606-9601 G, EI 2606-9601-2 G and EI 2606-9601-2 M, as described in Examples 2 and 3) according to the manufacturer's instructions. Determined antibody titers are shown in Table 3.

**Table 3: Antibodies to SARS-CoV-2 antigens in a subject vaccinated using S1 protein**

| Day | IgA (S1) (Cut off: <0.8) | IgG (S1) (Cut off: <0.8) | IgG (NCP) (Cut off: <0.8) | IgM (NCP) (Cut off: <0.8) |
|---|---|---|---|---|
| 10 | 0.5 | 0.5 | - | - |
| 23 | 1.0 | 0.2 | - | - |
| 29 | 3.5 | 6.2 | 0.6 | 0.7 |

As positive and negative controls, samples from patients suffering from SARS-CoV-2 infection (i.e., expected presence of antibodies to SARs-CoV-2) and samples from healthy blood donors (i.e., expected absence of antibodies to SARS-CoV-2) were used; see Tables 4 and 5, respectively.

In two patients suffering from SARS-CoV-2 infection (positive controls), samples were obtained 17 and 19 days after the onset of symptoms (usually 5-6 days after the infection). Determined antibody titers are shown in Table 4.

**Table 4: Antibodies to SARS-CoV-2 antigens in two patients suffering from SARS-CoV-2 infection**

| Day | IgA (S1) (Cut off: <0.8) | IgG (S1) (Cut off: <0.8) | IgG (N) (Cut off: <0.8) | IgM (N) (Cut off: <0.8) |
|---|---|---|---|---|
| Patient 1 | 2,6 | 1,8 | 1,7 | 1,3 |
| Patient 2 | 1.3 | 6.3 | 3.5 | 11.4 |

In four healthy subjects who did not receive any vaccination (negative controls), samples were obtained. Antibody titers are shown in Table 5.

**Table 5: Antibodies to SARS-CoV-2 antigens in healthy subjects**

| Day | IgA (S1) (Cut off: <0.8) | IgG (S1) (Cut off: <0.8) | IgG (NCP) (Cut off: <0.8) | IgM (NCP) (Cut off: <0.8) |
|---|---|---|---|---|
| Patient 1 | 0,0 | 0,1 | 0,0 | 0,5 |
| Patient 2 | 0,2 | 0,3 | 0,1 | 0,4 |
| Patient 3 | 0,1 | 0,1 | 0,1 | 0,2 |
| Patient 4 | 0,1 | 0,1 | 0,1 | 0,1 |

These results show that subjects vaccinated with SEQ ID NO: 1 or a variant thereof can be distinguished from infected subjects or subjects treated with a vaccine which does not comprise SEQ ID NO: 1 or a variant thereof by using an assay based on the detection of an antibody to SEQ ID NO: 1. While antibodies to S1 comprised in the vaccine can be detected in both, antibodies to the N protein are only detected in infected patients, but not in the vaccinated subjects.

### Example 7: Detection of antibodies to RBD and S1 using a dot blot

**Reagents:** RBD (SEQ ID NO: 34) and S1 (SEQ ID NO: 3) antigens were obtained as described in Example 1. Dilutions of the samples from patients suffering from SARS-CoV-2 as shown by a positive PCR test (1075, 1076, 1078, 1079, 1080, 1084, 1085, 1098, 1099, 1100) in dilution buffer (3% Bovine Serum Albumin in 1x Universal buffer (10x concentrate, product number 20125896) were prepared. Samples from healthy blood donors (BS01, BS10, BS25, BS32, BS43) were used as additional negative controls.

Monoclonal antibodies AK78, AK76 and AK80, used as positive controls, were each monoclonal anti-His tag antibodies (Lindner P, Bauer K, Krebber A, Nieba L, Kremmer E, Krebber C, Honegger A, Klinger B, Mocikat R, Plückthun A. Specific detection of his-tagged proteins with recombinant anti-His tag scFv-phosphatase or scFv-phage fusions. Biotechniques. 1997 Jan;22(1):140-9).

Blot strips were made by transferring 1 µl of the antigen solution (2.69 mg ml⁻¹) on the strip. The membrane was a 0.22 µm cellulose nitrate membrane (Sartorius).

Secondary antibodies to IgA, IgG and IgM ("Anti-human-IgA-AP", "Anti-human-IgG-AP" and "Anti-human-IgM-AP", respectively) were from EUROIMMUN (Alkaline phosphatase-labelled anti-human IgA/G/M (goat), product no. ZD 1129 A / G / M) as was NBT/BCIP, product no. 10 123964)

**Method:** Blot strips were blocked by incubation for 15 minutes in washing buffer (3% Bovine Serum Albumin in 1x Universal buffer (10x concentrate product number 20125896)), followed by incubation of the strip in appropriately diluted sample for 3 hours at room temperature, followed by three washing steps using washing buffer, followed by incubation of the strip in appropriately diluted sample for another 30 minutes, followed again by the three washing steps, followed by staining by incubation in NBT/BCIP solution for ten minutes, which was stopped by washing the strips thrice thoroughly in demineralized water.

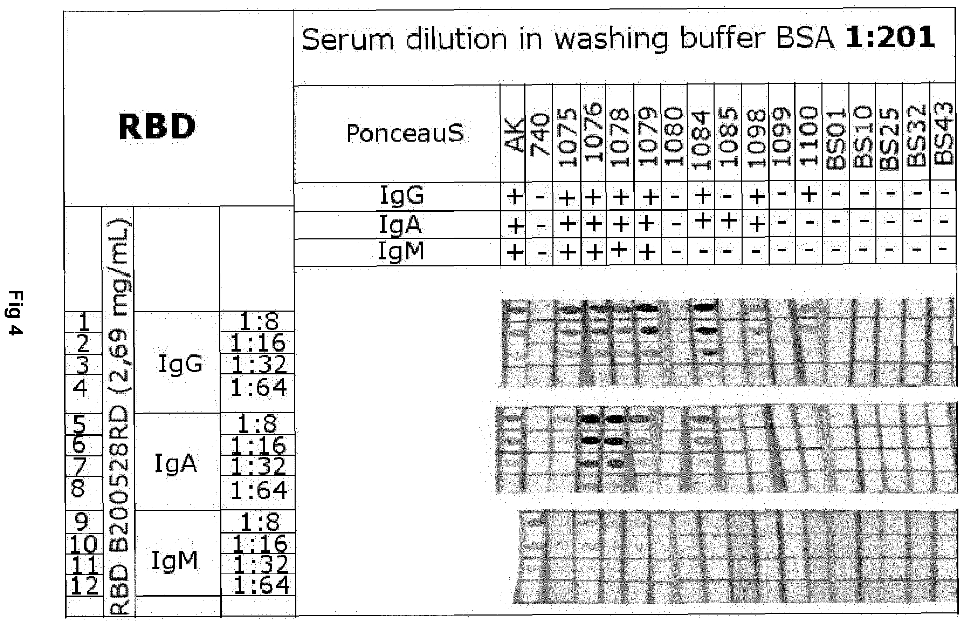

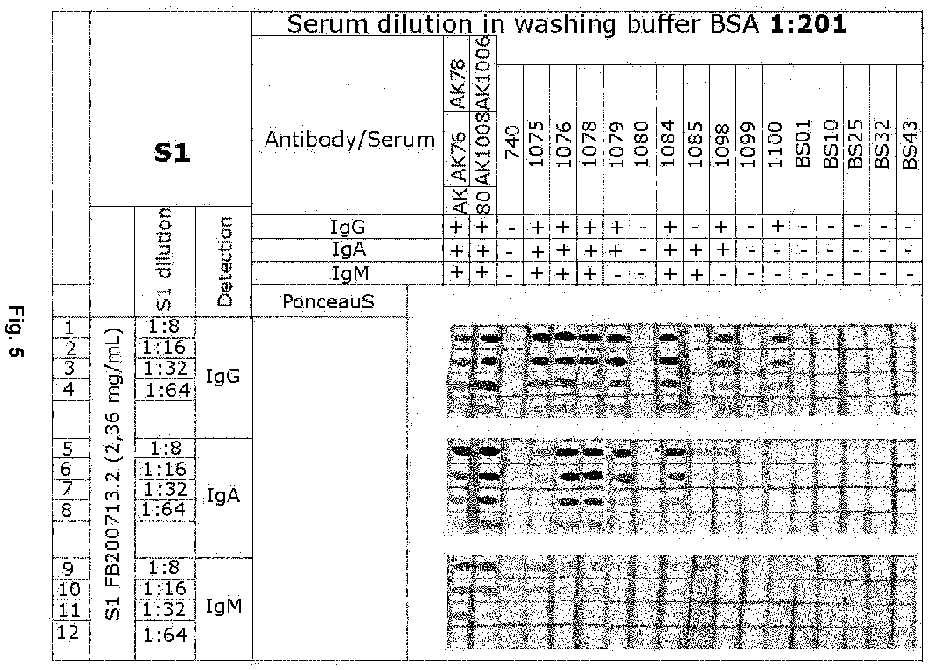

**Results:** **Figs. 4** and **5** show the results of dot blot analysis of the samples and controls. The assay could be established successfully as judged by positive results using the monoclonal antibodies instead of samples and no signals if negative controls were used.

While both S1 and RBD can be used to detect IgM, IgA and IgG antibodies, reactions are generally slightly stronger if S1 is used, suggesting that both the RBD and sequences flanking it contain epitopes or parts thereof. Moreover, part of the antigen-antibody interaction may be affected by the fold of the antigen.

Interestingly, IgA and IgM, but no IgG antibodies could be detected in patient 1085, confirming the ELISA-based results that detection of IgA antibodies enhances the sensitivity, particularly in addition to IgG. The patient may have been examined at an early stage of the disease when no IgG antibodies were detectable yet. Vice versa, only IgG antibodies were detectable in patient 1100, whose sample may have been obtained at a later stage of the disease, following disappearance of IgA antibodies. IgA and IgG antibodies gave generally stronger signals than IgM antibodies.

### Example 8: Detection of antibodies to peptides derived from RBD and S1, using a dot blot

Peptides P1 (RTQLPPAYTNS, SEQ ID NO: 41), P2 (SGTNGTKRFDN, SEQ ID NO: 42), P3 (LTPGDSSSGWTAG, SEQ ID NO: 35), P4 (NNLDSKVGG, SEQ ID NO: 55), P5 (YQAGSTPCNGV, SEQ ID NO: 36), P6 (YGFQPTNGVGYQ, SEQ ID NO: 37) and a fusion comprising P1-P6 (SEQ ID NO: 56) were expressed as N-terminal His tag fusions comprising a protease cleavage site (sequence of fusion comprising P1: SEQ ID NO: 43; P2: SEQ ID NO: 44; P3: SEQ ID NO: 45; P4: SEQ ID NO: 46; P5; SEQ ID NO: 47; P6: SEQ ID NO: 48; fusion comprising P1-P6: SEQ ID NO: 49 by *E*. *coli* Rosetta(DE3)pLacl cells using standard methods based on the pET24d plasmid, at 37°C for 3 h in LB Medium, containing Kanamycin and Chloramphenicol, using IPTG induction. Cells were harvested, resuspended in Phosphate-Buffered Saline and stored at -20°C until further use.

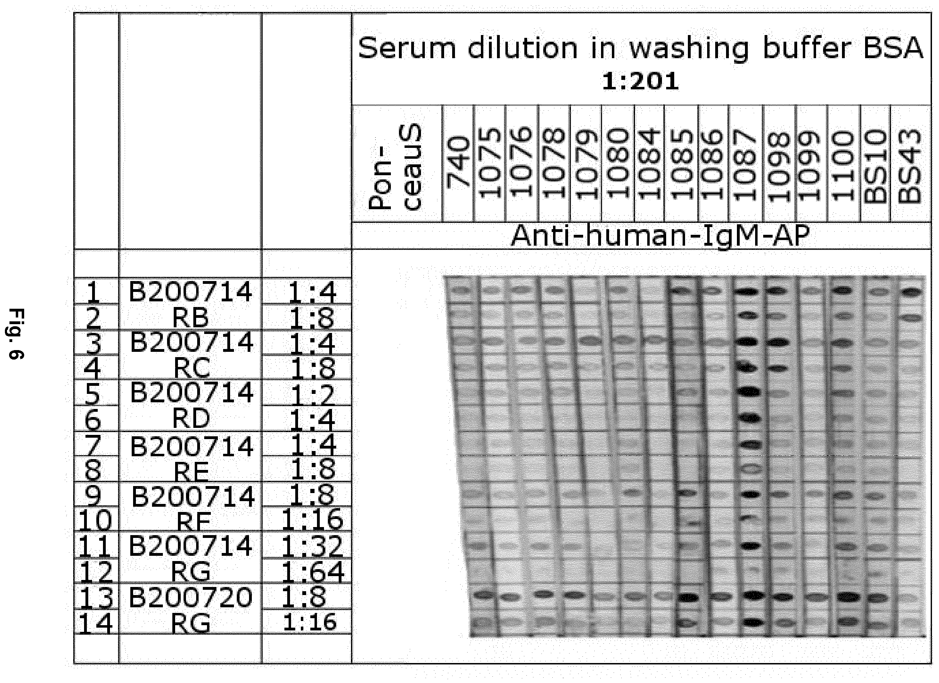

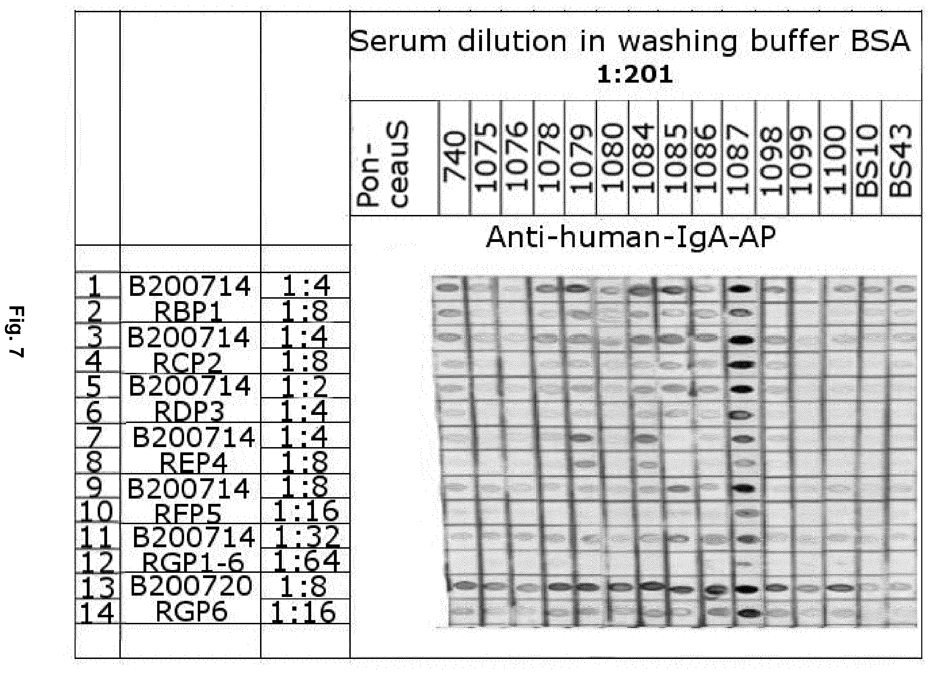

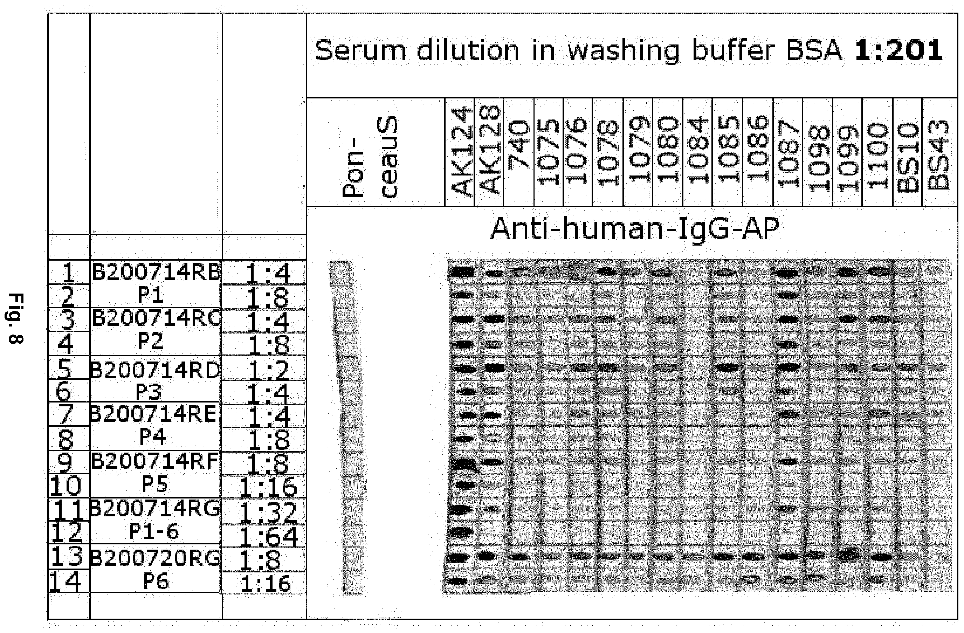

**Figs. 6****,** **7** and **8** show the results of the dot blot assays using the P1 to P6 constructs. P6, which is part of the RBD, showed the strongest reaction no matter whether IgA, IgM or IgG were detected, but some reactivity could also be demonstrated with P1, P2 (except for IgM), P3 and P4 (except for IgM) and P5. Reactions with IgG and IgA were generally stronger than IgM.

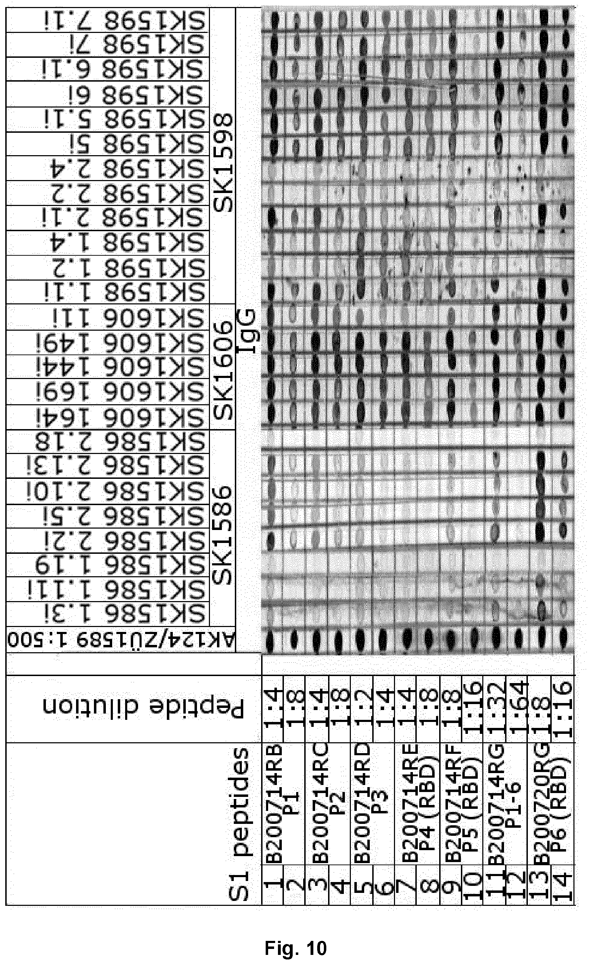

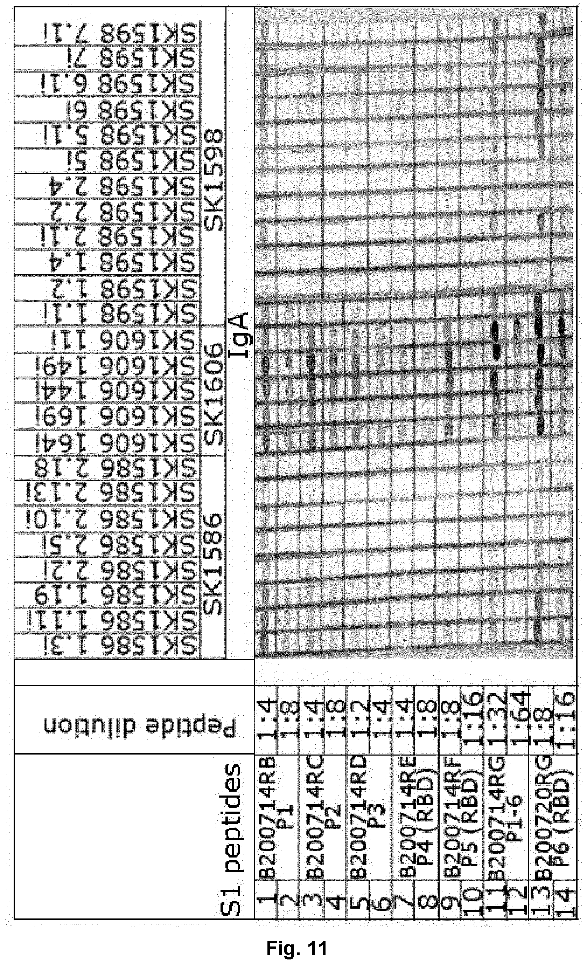

Samples representing time courses with more than one samples from several patients demonstrated that the dot blot may be used to monitor patients. For example, patient SK1586 (three time points) has the strongest IgM reaction with the first sample (1.3i), while the signal is weaker if the second (1.11.i) and the third sample (1.19) are used, in particular with regard to P3 (Fig. 9).The same samples showed a stronger reaction if peptides P1 and P6 were used to detect IgA (Fig. 10). The reaction was generally stronger if IgG antibodies were detected, but a time-dependent reaction can be monitored using the same samples, especially peptides P6, P1 and the fusion protein comprising P1 to P6 (**Fig. 11**). Examples 7 and 8 show that antibodies to SEQ ID NO:1 can be detected using dot blot and various fragmens of SEQ ID NO: 1.

### Example 9: Detection of antibodies to RBD and S1 using a Western Blot

A non-reducing SDS PAGE was run using 2 µg of RBD (SEQ ID NO: 34) or S1 (SEQ ID NO: 3). A sample comprising 10 µl of protein was mixed with 4 µl NuPAGE-PP (NuPage LDS Sample Buffer (4x), Firma Fisher Scientific GmbH) and 1 µl of EU-PBS (Phosphate-Buffered Saline), incubated for ten minutes at 70 °C. 12 µl of the resulting solution were applied per lane to a non-reducing 2D gel (NuPAGE 4-12% Bis-Tris Gel 1.0 mm x 2 D, Fisher Scientific GmbH) and subjected to electrophoresis in running buffer (NuPage MOPS SDS Running Buffer, Fisher Scientific GmbH). Separated Protein bands were transferred to a nitrocellulose membrane for 60 minutes at 400 mA (PowerPac HC Power Supply, Firma Bio-Rad). Membrane strips could be stained using PonceauS, followed by washing in 50 mM Tris, but were in any event subjected to blocking for 15 minutes in washing buffer (3% Bovine Serum Albumin in 1x Universal buffer (10x concentrate order no.: 20125896), followed by incubation of the strip in appropriately diluted sample for 3 hours at room temperature, followed by three washing steps using washing buffer, followed by incubation of the strip in appropriately diluted sample for another 30 minutes, followed again by the three washing steps, followed by staining by incubation in NBT/BCIP solution for ten minutes, which was stopped by washing the strips thrice thoroughly in demineralized water.

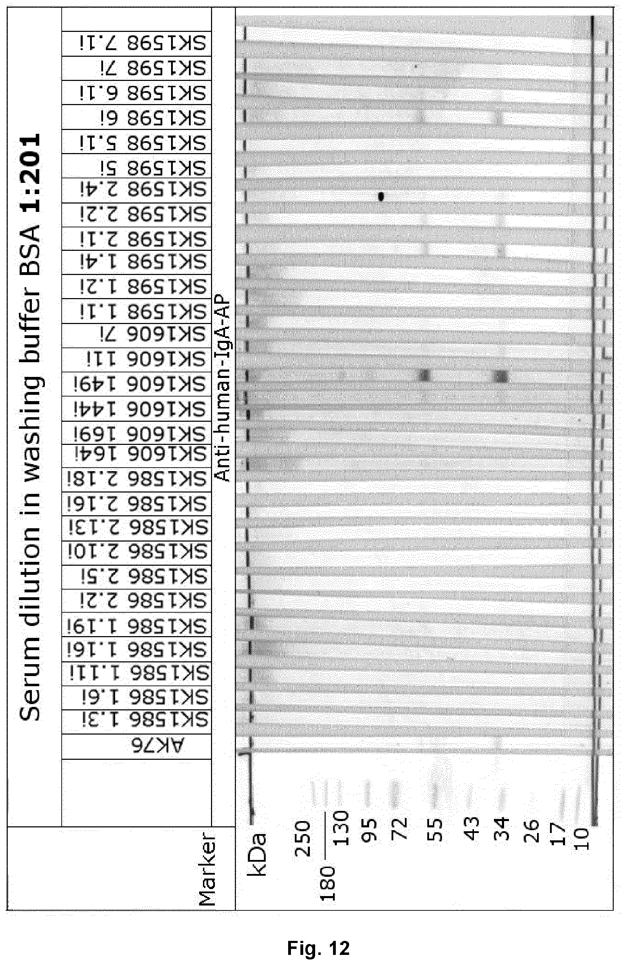

**Results:** Positive control (anti-His antibodies as before) and positive samples from SARS-CoV-2 infected patients show that dimers, trimers and tetramers were detected in addition to RBD monomers. A weak reaction was detected using secondary antibodies detecting IgA class antibodies with six sera, a weak reaction with another three sera and a strong reaction with one serum. If two or more samples from different time points were available and reactive, a decrease of the concentration of IgA antibodies was observed, for example with samples SK159822.1i to 2.4i, as well as with samples 6i and 61i and 7i and 7.1i (**Fig. 12**).

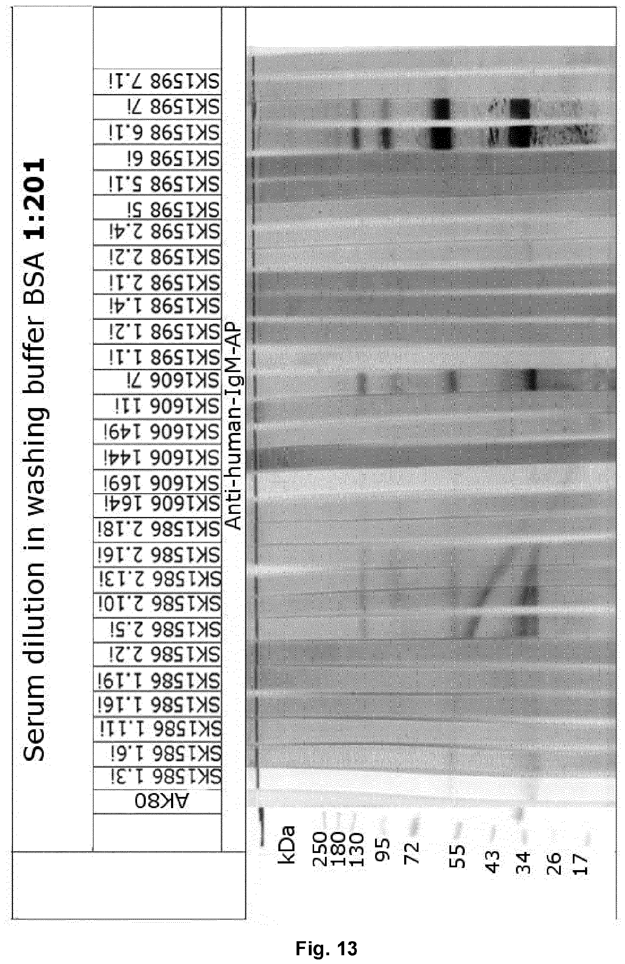

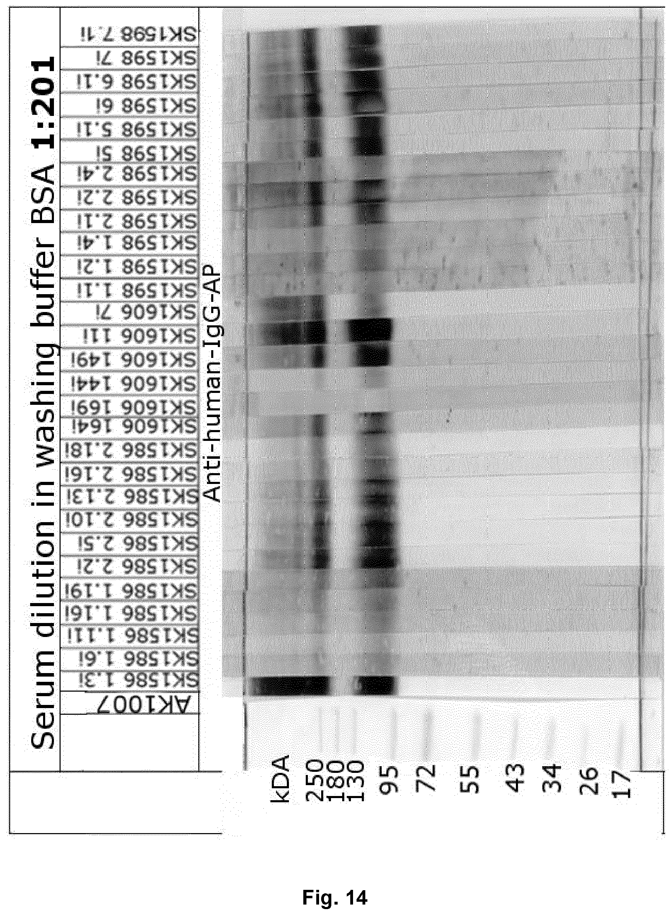

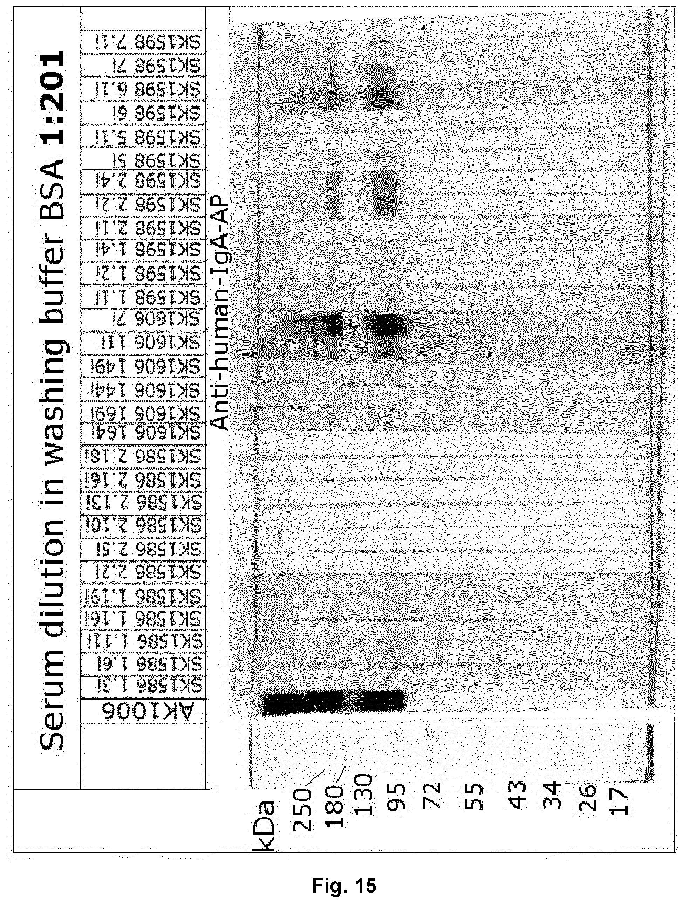

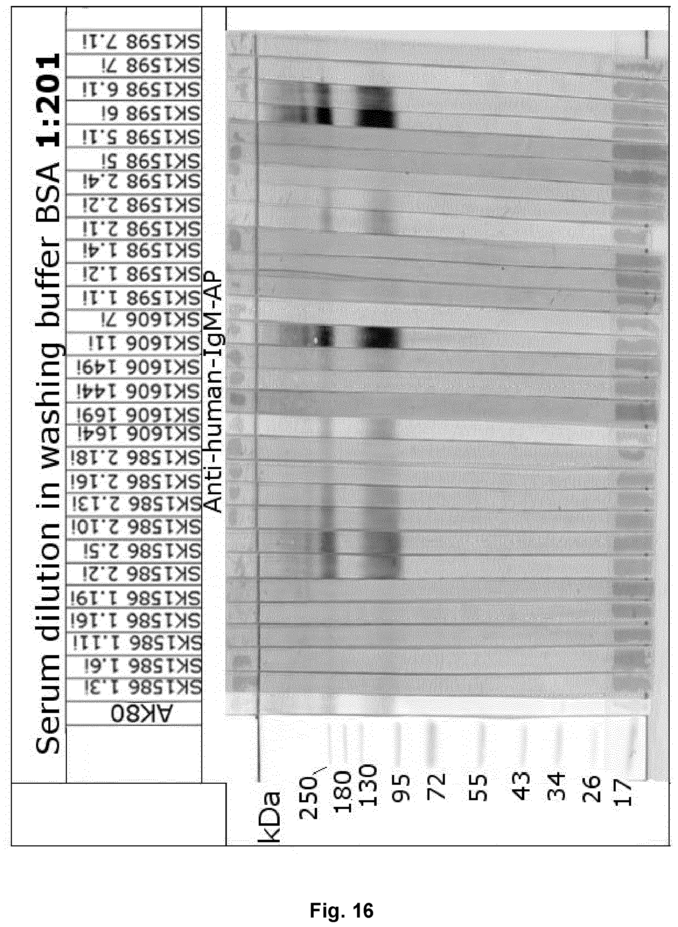

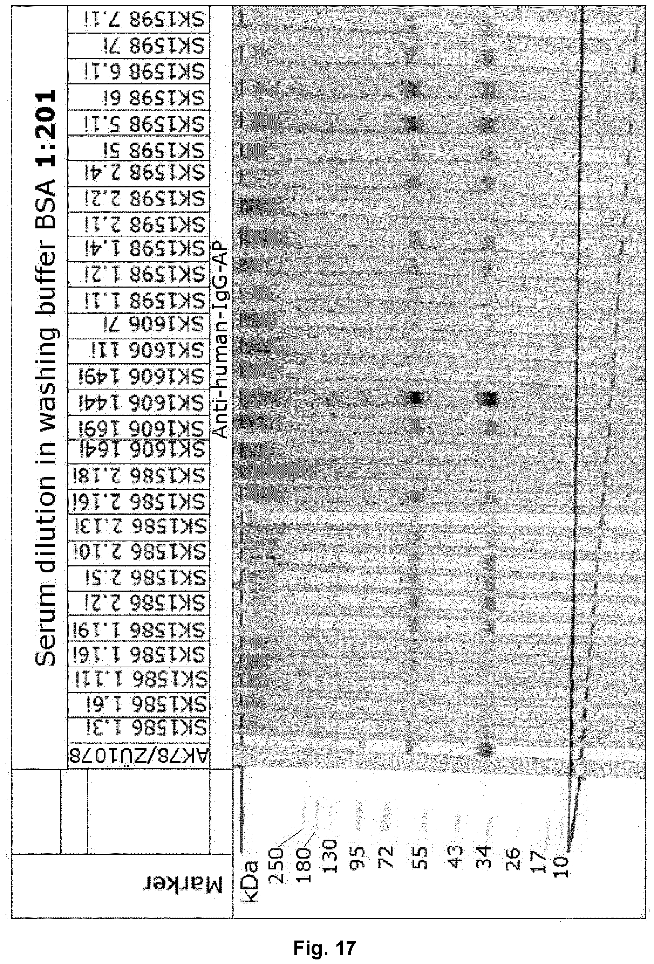

As for the detection of IgG class antibodies, all samples which were positive, as judged by ELISA, showed a reaction (**Fig. 13**). Sample SK1606 169i was negative based on the results of both methods. As for the detection of IgM class antibodies, a decreasing concentration of antibodies was detected in some cases, notably SK15862.2 to SK15862.16 as well as SK159986i to SK1599861i, if two or more samples from different time points were available (**Fig. 14**). Results were highly comparable if S1 was used rather than RBD as antigen **(****Figs. 15****,** **16** and **17****).**

It is concluded that Western blotting is another method that may be used to practice the present invention.

### Example 10: Detection of IgA, IgM and IgG antibodies to RBD using a competitive test format

A EUROIMMUN SARS-CoV-2 NeutraLISA kit (EI 2606-9601-4) was used for the following experiment according to manufacturer's instructions unless specified to the contrary.

Briefly, a microtiter plate coated with the S1 domain of the spike protein of SARS-CoV-2 expressed recombinantly in the human cell line HEK293 (SEQ ID NO: 3) was used. In the first step of the analysis, the controls and samples (blood samples of a subject) were diluted with a sample buffer containing soluble biotinylated human ACE2 (SEQ ID NO: 50) and incubated in the reagent wells of a microtiter plate. Neutralising antibodies present in the sample compete with the ACE2 for the binding sites of the coated SARS-CoV-2 S1 spike protein. Unbound ACE2 and unbound sample was removed in a subsequent washing step. To detect bound ACE2, a second incubation was performed using peroxidase-labelled streptavidin, which binds to the biotinylated ACE2 immobilized on the antigen on the microtiter plate and catalyses a colour reaction in the third step. The intensity of the produced colour is inversely proportional to the concentration of neutralising antibodies in the sample as shown in **Table 6:**

| | ACE2-Concen-tration 6,0 µg/ml | | | | |
|---|---|---|---|---|---|
| Probe | | Positive sample | Positive sample | | Negative sample |
| 1:2,5 | | 0,248 | 0,166 | | 1,177 |
| 1:5 | | 0,342 | 0,294 | | 1,208 |
| 1:10 | | 0,521 | 0,446 | | 1,213 |
| 1:20 | | 0,697 | 0,605 | | 1,190 |
| 1:40 | | 0,870 | 0,738 | | 1,231 |
| 1:80 | | 0,910 | 0,829 | | 1,172 |
| 1:160 | | 0,932 | 0,877 | | 1,187 |
| Blank | | 0,878 | 0,911 | | 1,107 |

The results show that an increasing dilution of a positive sample, i.e. a decreasing concentration of neutralizing antibodies, leads to an increase in the produced colour. By contrast, the signal is high and does not correlate with the dilution of the sample if a negative sample is used.

This confirms that the assay can be used to detect and to quantify the neutralizing antibodies against the RBD in a sample.

### Example 11: Detection of antibodies to SARS-CoV-2 antigens S1 and N proteins by ELISA

A panel comprising several samples taken at different time points after the infection from each of 43 German COVID-19 patients was used to detect the presence or absence of antibodies over time. All sera were tested for antibodies against the S1 domain (SEQ ID NO: 3) of the SARS-CoV-2 spike domain (IgA and IgG ELISA kits, EUROIMMUN, products as in Examples 2 and 3) and antibodies against the N protein (IgG and IgM ELISA kits, EUROIMMUN, as in Examples 2 and 3).

Between >10 to <21 days after the onset of illness IgG and IgA antibodies against S1 of SARS-CoV-2 were detected in 70.4% and 88.9% of the samples, while IgG and IgM against N protein were detected in 86.2% and 50%, respectively. In six patients, IgA antibody to S1 was the first antibody that could be detected, while only in two cases an antibody *other than* IgA to S1 was the first antibody to be detected. In 30 patients no IgM antibody to N protein could be detected at any time.

More than 60 days after the onset of illness) IgG and IgA antibodies against S1 of SARS-CoV-2 were detected in 85.1% and 80.5% of the samples, while IgG and IgM against N protein were detected in 81.4% and 0%, respectively. In four patients, IgG to S1 was detectable, but not IgG to N protein. By contrast, only in one patient IgG to N protein was detectable, but not IgG to S1.

These results confirm that the detection of IgA to S1 is the most sensitive assay for the early detection of an antibody response against SARS-CoV-2, more specifically its S1 protein. By contrast, the detection of IgG to S1 is particularly sensitive at a later stage of the infection. Both assays may be combined by detection of IgA to S1 and IgG to S1 in one reaction or in separate reactions for increased sensitivity, optionally in combination with additional assays for increased sensitivity over an extended period of time.

### Example 12: Detection of antibodies to SARS-CoV-2 antigens S1 and N proteins by ELISA during the late phase of a SARS-CoV-2 infection

Using the same methodology as in Example 11, samples from another cohort of 15 patients suffering from a SARS-CoV-2 infection taken 21 days or later after the onset of illness were obtained and tested for the presence of IgG and IgM antibodies to N protein and IgA and IgG antibodies to the S1 protein. The results are shown in **Table 7:**

| Assay | Positive | Borderline | negative | Sensitivity |
|---|---|---|---|---|
| NCP ELISA IgG | 13 | 0 | 2 | 86,7% |
| NCP ELISA IgM | 5 | 2 | 8 | 46,7% |
| S1 ELISA IgG | 14 | 0 | 1 | 93,3% |
| S1 ELISA IgA | 12 | 3 | 0 | 100% |

The study on this additional cohort confirms that the sensitivity of diagnosis is highest at the late phase of a SARS-CoV-2 infection if antibodies to the S1 protein are detected.

### Example 13: The specificity of the detection of antibodies to SARS-CoV-2 antigens S1 and N proteins

The specificity of the Anti-SARS-CoV-2 ELISA (IgA, EI 2606-9601 A, carried out according to manufacturer's instructions, more details in Examples 2 and 3) was determined by analyzing 210 patient samples that were positive, for instance, for antibodies against other human pathogenic coronaviruses, other pathogens or for rheumatoid factors. Additionally, 1052 samples from blood donors, children and pregnant women obtained before the occurrence of SARS-CoV-2 (before January 2020) were analysed Results in the borderline range (n=9) were not included in the calculation of the specificity. This resulted in a specificity of 98.3% as shown in **Table 8.**

The specificity of the Anti-SARS-CoV-2 ELISA (IgG, EI 2606-9601 G, carried out according to manufacturer's instructions, more details in Examples 2 and 3) was determined in the same manner, based on 222 positive patient samples and 1052 samples from blood donors, children and pregnant. This resulted in a specificity of 98.3% as shown in **Table 8:**

| Panel | n | Specificity IgA ELISA in % | Panel | n | Specificity IgG ELISA in % |
|---|---|---|---|---|---|
| Blood donors | 849 | 98.2 | Blood donors | 849 | 99.5 |
| Pregnant women | 99 | 97 | Pregnant women | 199 | 99.5 |
| Children | 104 | 100 | Children | 74 | 100 |
| Elderly people | 97 | 99 | Elderly people | 97 | 100 |
| Infections with other human pathogenic coronaviruses | 11 | 100 | Infections with other human pathogenic coronaviruses | 23 | 100 |
| Influenza (freshly vaccinated) | 40 | 100 | Influenza (freshly vaccinated) | 40 | 100 |
| Acute EBV infections & heterophile antibodies | 22 | 90.5 | Acute EBV infections & heterophile antibodies | 22 | 100 |
| Rheumatoid factors | 40 | 100 | Rheumatoid factors | 40 | |
| Total | 1262 | 98.3 | Total | 1344 | 99.6 |

## Claims

1. A method comprising the step of detecting the presence or absence of an IgM and/or IgA class antibody to SEQ ID NO1 in a blood sample from a subject, comprising
coating a carrier with an isolated polypeptide comprising SEQ ID NO1 or a variant thereof and
contacting the carrier with the sample under conditions allowing for binding of any antibodies to the polypeptide,
wherein the polypeptide
a) consists of the full length-sequence of SEQ ID NO1, optionally fused to one or more artificial linkers or affinity tags, or
b) the variant is a truncated SEQ ID NO1, *i*. *e*. less than the full-length sequence of SEQ ID NO1, wherein the truncation is at the N-terminus, C-terminus or both, optionally fused to one or more artificial linkers or affinity tags, and
(i) comprises a fragment of SEQ ID NO1 comprising at least 25, 50, 75, 100, 150, 200, 250, 300, 400, 500 or 600 successive amino acids of SEQ ID NO1 and/or
(ii) comprises a fragment of SEQ ID NO1 comprising at least 200 successive amino acids with an identity to the reference fragment of SEQ ID NO1 of at least 80, 85, 90, 92, 94,95, 96, 97, 98, 99 %,
and wherein the variant has the ability to bind to an antibody to SEQ ID NO1 from a sample from a patient suffering from SARS-CoV-2.

2. A kit comprising a carrier coated with an isolated polypeptide comprising SEQ ID NO1 or a variant thereof, a blood sample and a means for detecting the presence of an IgM and/or IgA class antibody to SEQ ID NO1,
wherein the polypeptide
a) consists of the full length-sequence of SEQ ID NO1, optionally fused to one or more artificial linkers or affinity tags, or
b) the variant is a truncated SEQ ID NO1, *i*. *e*. less than the full-length sequence of SEQ ID NO1, wherein the truncation is at the N-terminus, C-terminus or both, optionally fused to one or more artificial linkers or affinity tags, and
(i) comprises a fragment of SEQ ID NO1 comprising at least 25, 50, 75, 100, 150, 200, 250, 300, 400, 500 or 600 successive amino acids of SEQ ID NO1 and/or
(ii) comprises a fragment of SEQ ID NO1 comprising at least 200 successive amino acids with an identity to the reference fragment of SEQ ID NO1 of at least 80, 85, 90, 92, 94,95, 96, 97, 98, 99 %,
and wherein the variant has the ability to bind to an antibody to SEQ ID NO1 from a sample from a patient suffering from SARS-CoV-2.

3. The kit according to claim 2, wherein the kit comprises in addition one or more, preferably all reagents from the group comprising a calibrator, a positive control, a negative control, a wash buffer, a sample buffer, a detection solution, a stop solution, a protective foil and an antibody to SEQ ID NO1.

4. The kit according to any of claims 2 to 3, wherein the antibody to SEQ ID NO1 is selected from the group comprising an IgA, IgG and IgM class antibody.

5. The kit according to any of claims 2 to 4, wherein the means for detecting an antibody to SEQ ID NO1 detects IgG and/or IgM class antibodies in addition to IgA class antibodies to SEQ ID NO1.

6. A use of an isolated polypeptide comprising SEQ ID NO1 or a variant thereof for the manufacture of the diagnostic kit according to any of claims 2 to 5,
wherein the isolated polypeptide
a) consists of the full length-sequence of SEQ ID NO1, optionally fused to one or more artificial linkers or affinity tags, or
b) the variant is a truncated SEQ ID NO1, *i*. *e*. less than the full-length sequence of SEQ ID NO1, wherein the truncation is at the N-terminus, C-terminus or both, optionally fused to one or more artificial linkers or affinity tags, and
(i) comprises a fragment of SEQ ID NO1 comprising at least 25, 50, 75, 100, 150, 200, 250, 300, 400, 500 or 600 successive amino acids of SEQ ID NO1 and/or
(ii) comprises a fragment of SEQ ID NO1 comprising at least 200 successive amino acids with an identity to the reference fragment of SEQ ID NO1 of at least 80, 85, 90, 92, 94,95, 96, 97, 98, 99 %, and wherein the variant has the ability to bind to an antibody to SEQ ID NO1 from a sample suffering from SARS-CoV-2.

7. The use according to claim 6, comprising the step coating a carrier with the isolated polypeptide comprising a variant of SEQ ID NO1.

8. A use of an isolated polypeptide comprising SEQ ID NO1 or a variant thereof or of a kit for detecting an IgM and/or IgA class antibody to SEQ ID NO1 in a blood sample,
wherein the kit comprises a carrier coated with an isolated polypeptide comprising SEQ ID NO1 or a variant thereof and a means for detecting the presence of an IgM and/or IgA class antibody to SEQ ID NO1,
wherein the polypeptide
a) consists of the full length-sequence of SEQ ID NO1, optionally fused to one or more artificial linkers or affinity tags, or
b) the variant is a truncated SEQ ID NO1, *i*. *e*. less than the full-length sequence of SEQ ID NO1, wherein the truncation is at the N-terminus, C-terminus or both, optionally fused to one or more artificial linkers or affinity tags, and
(i) comprises a fragment of SEQ ID NO1 comprising at least 25, 50, 75, 100, 150, 200, 250, 300, 400, 500 or 600 successive amino acids of SEQ ID NO1 and/or
(ii) comprises a fragment of SEQ ID NO1 comprising at least 200 successive amino acids with an identity to the reference fragment of SEQ ID NO1 of at least 80, 85, 90, 92, 94,95, 96, 97, 98, 99 %,
and wherein the variant has the ability to bind to an antibody to SEQ ID NO1 from a sample from a patient suffering from SARS-CoV-2.

9. The method or use according to any of claims 1 and 6 to 8, wherein the blood sample is from a mammalian, preferably human subject.

10. The method, kit or use according to any of claims 1 to 9, wherein the blood sample is selected from the group comprising whole blood, serum and plasma.

11. The method, kit or use according to any of claims 1 to 10, wherein SARS-CoV-2 is a virus **characterized by** the genome deposited on GenBank under accession code MN908947 and derivatives thereof having at least 80, preferably 85, preferably 88, preferably 90, preferably 91, preferably 92, preferably 93, preferably 94, preferably 95, preferably 96, preferably 97, preferably 98, preferably 99, preferably 99.5, preferably 99.8, preferably 99.9 or 99.99 percent sequence identity over the entire genome nucleotide sequence.

12. The method or use according to any of claims 1 or 8 to 11, wherein the method or use is for aiding in the diagnosis of a SARS-CoV-2 infection or identifying a subject at risk of suffering from a SARS-CoV-2 infection.

13. The method according to any of method or use according to any of claims 1 or 8 to 12, wherein it is detected whether an IgG, IgM or IgA class antibody is present or absent.

14. The method or use according to any of claims 1 or 8 to 13, wherein the antibody is detected using a method selected from the group comprising colorimetry, immunofluorescence, detection of enzymatic activity, chemiluminscence and radioactivity.

15. The method, kit or use according to any of claims 1 to 14, wherein the carrier is selected from the group comprising a bead, preferably a paramagnetic bead, a test strip, a microtiter plate, a membrane, preferably from the group comprising western blot, line blot and dot blot, a lateral flow device, a glass surface, a slide, a microarray and a biochip and is preferably a microtiter plate.

16. The method or use according any of claims 1 or 8 to 15, comprising the step contacting the carrier with a means binding to the antibody which carries a detectable label and detecting the presence of the antibody.

17. The method or use according any of claims 1 or 8 to 16, wherein the subject is likely or more likely than the average subject to suffer from a SARS-CoV-2 infection if an IgA or IgG or IgM antibody to SEQ ID NO1 is detected in the sample.

18. The method, kit or use according to any of claims 1 to 17, wherein the polypeptide
a) consists of the full length-sequence of SEQ ID NO12, optionally fused to one or more artificial linkers or affinity tags, or
b) the variant is a truncated SEQ ID NO12, *i*. *e*. less than the full-length sequence of SEQ ID NO1, wherein the truncation is at the N-terminus, C-terminus or both, optionally fused to one or more artificial linkers or affinity tags, and
(i) comprises a fragment of SEQ ID NO12 comprising at least 25, 50, 75, 100, 150, 200, 250, 300, 400, 500 or 600 successive amino acids of SEQ ID NO1 and/or
(ii) comprises a fragment of SEQ ID NO12 comprising at least 25, preferably 50, more preferably 200 successive amino acids with an identity to the reference fragment of SEQ ID NO1 of at least 80, 85, 90, 92, 94,95, 96, 97, 98, 99 %,
and wherein the variant has the ability to bind to an antibody to SEQ ID NO12 from a sample from a patient suffering from SARS-CoV-2.
